# EUROPÄISCHE PATENTSCHRIFT

(11) **EP 0 873 305 B1**
(45) Veröffentlichungstag und Bekanntmachung des Hinweises auf die Patenterteilung: **28.03.2001**
(21) Anmeldenummer: 96938034.4
(22) Anmeldetag: 30.10.1996
(51) Int. Cl.: C07C 251/88, A01N 37/50

(54) **AZINOOXIMETHER, VERFAHREN UND ZWISCHENPRODUKTE ZU IHRER HERSTELLUNG UND IHRE VERWENDUNG ZUR BEKÄMPFUNG VON SCHADPILZEN UND TIERISCHEN SCHÄDLINGEN**
AZINOOXIMETHERS, PROCESSES AND INTERMEDIATE PRODUCTS FOR MANUFACTURING SAME, AND THE USES THEREOF IN COMBATING HARMFUL FUNGI AND PESTS
AZINOOXIMETHERS, PROCEDE DE PRODUCTION ET PRODUITS INTERMEDIAIRES UTILISES, UTILISATION DE CES ETHERS DANS LA LUTTE CONTRE LES CHAMPIGNONS ET ANIMAUX NUISIBLES

(30) Priorität: 15.11.1995 DE 19542629
(43) Veröffentlichungstag der Anmeldung: 28.10.1998
(73) Patentinhaber: BASF AKTIENGESELLSCHAFT, 67056 Ludwigshafen (DE)
(72) Erfinder: BAYER, Herbert, D-68159 Mannheim (DE); SAUTER, Hubert, D-68167 Mannheim (DE); OBERDORF, Klaus, D-69117 Heidelberg (DE); GRAMMENOS, Wassilios, D-67063 Ludwigshafen (DE); GROTE, Thomas, D-67105 Schifferstadt (DE); KIRSTGEN, Reinhard, D-67434 Neustadt (DE); MÜLLER, Bernd, D-67227 Frankenthal (DE); MÜLLER, Ruth, D-67159 Friedelsheim (DE); RÖHL, Franz, D-67105 Schifferstadt (DE); AMMERMANN, Eberhard, D-64646 Heppenheim (DE); HARRIES, Volker, D-67227 Frankenthal (DE); LORENZ, Gisela, D-67434 Hambach (DE); STRATHMANN, Siegfried, D-67117 Limburgerhof (DE)
(86) Internationale Anmeldenummer: EP9604712
(87) Internationale Veröffentlichungsnummer: WO9718187

(56) Entgegenhaltungen:
- WO-A-95/18789
- MENDELEEV COMMUN. (1995), (6), 229-31 CODEN: MENCEX;ISSN: 0959-9436, 1995, XP000617737 AZEV, YURII A. ET AL: "Transformations of 6-phenyl-1,2,4-triazine 4-oxides in reactions with nucleophiles"
- LIEBIGS ANN. CHEM. (1989), (11), 1071-4 CODEN: LACHDL;ISSN: 0170-2041, 1989, XP000644510 GNICHTEL, HORST ET AL: "Configuration of 1,2-diketone hydrazone oximes"
- TETRAHEDRON LETT. (1973), (16), 1429-32 CODEN: TELEAY, 1973, XP000644515 NEUNHOEFFER, HANS ET AL: "Synthesis of 1,2,4-triazines. VI. Synthesis of 6-substituted triazines"
- LIEBIGS ANN. CHEM. (1989), (11), 1071-4 CODEN: LACHDL;ISSN: 0170-2041, 1989, XP000644507 GNICHTEL, HORST ET AL: "Configuration of 1,2-diketone hydrazone oximes"

## Beschreibung

Die vorliegende Erfindung betrifft Azinooximether der Formel I 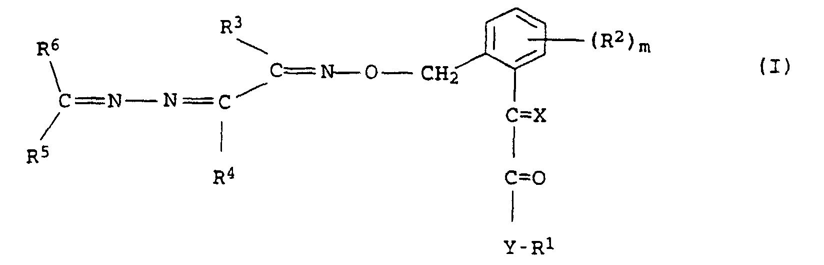 in der die Variablen die folgenden Bedeutungen haben:
- X: NOCH₃, CHOCH₃ oder CHCH₃;
- Y: O oder NZ, wobei Z für Wasserstoff oder C₁-C₄-Alkyl steht;
- R¹: Wasserstoff oder C₁-C₄-Alkyl;
- R²: Cyano, Nitro, Halogen, C₁-C₄-Alkyl, Trifluormethyl oder C₁-C₄-Alkoxy;
- m: 0, 1 oder 2, wobei die Reste R² verschieden sein können, wenn m für 2 steht;
- R³: Wasserstoff, Cyano, Alkyl, Halogenalkyl, Alkoxy oder Cycloalkyl;
- R⁴_{,} R⁵, R⁶: unabhängig voneinander: Wasserstoff oder gegebenenfalls substituiertes: Alkyl, Alkenyl, Alkinyl, Cycloalkyl, Cycloalkenyl, Heterocyclyl, Aryl oder Heteroaryl,
sowie deren Salze.

Außerdem betrifft die Erfindung Verfahren zur Herstellung dieser Verbindungen sowie sie enthaltende Mittel zur Bekämpfung tierischer Schädlinge und Schadpilze.

Phenylessigsäure-Derivate des Typs I mit fungizider und insektizider Wirkung sind aus folgender Druckschrift bekannt:
WO-A 95/18789. Hinsichtlich ihrer Wirkung vermögen die dort offenbarten Wirkstoffe jedoch noch nicht zu befriedigen.

Der vorliegenden Erfindung lagen daher neue Verbindungen dieses Typs mit verbesserter Wirkung gegen Schadpilze und Schädlinge als Aufgabe zugrunde.

Demgemäß wurden die eingangs definierten Azinooximether I gefunden. Außerdem wurden Verfahren und Zwischenprodukte zu ihrer Herstellung sowie sie enthaltende Mittel zur Bekämpfung von tierischen Schädlingen und Schadpilzen und ihre Verwendung hierzu gefunden.

Die Verbindungen I sind auf verschiedenen Wegen nach an sich bekannten Verfahren erhältlich.

Grundsätzlich ist es bei der Synthese der Verbindungen I unerheblich, ob zunächst die Gruppierung -C(X)-CO-YR¹ oder die Gruppierung -CH₂ON=C (R³)-C (R⁴)=N-N=C (R⁵) R⁶ aufgebaut wird.

Der Aufbau der Gruppierung -C(X)-CO-YR¹ ist beispielsweise aus folgenden Druckschriften bekannt: EP-A 422 597, EP-A 463 488, EP-A 370 629, EP-A 460 575, EP-A 472 300, WO-A 90/07493, WO-A 92/13830, WO-A 92/18487, DE-Anm. P 44 20 416.7.
1.1 Man geht beim Aufbau der Gruppierung-CH₂ON=C(R³) -C(R⁴)=N-N=C(R⁵)R⁶ im allgemeinen so vor, daß man ein Benzylderivat der Formel II mit einem Hydroxyimin der Formel III umsetzt. 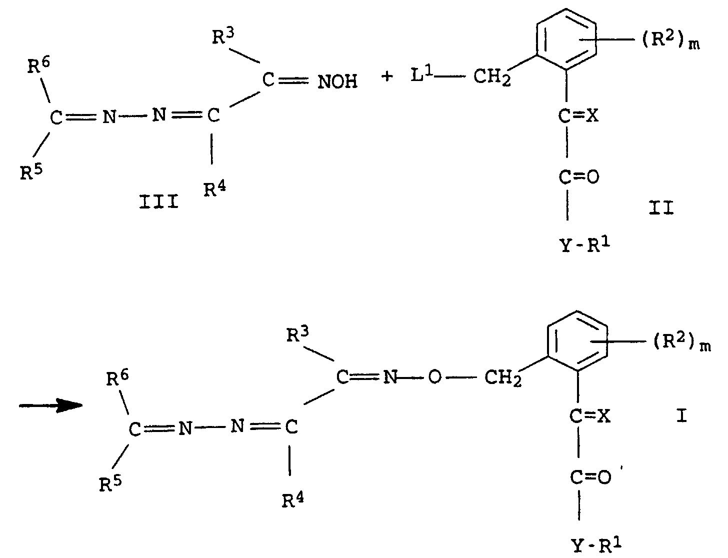 L¹ in der Formel II steht für eine nukleophil austauschbare Abgangsgruppe, z.B. Halogen oder eine Sulfonatgruppe, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Natriumhydrid, Kaliumhydroxid, Kaliumcarbonat oder Triethylamin, gemäß den in Houben-Weyl, 4. Auflage, Bd. E 14b, S. 370 ff. und Bd. 10/1, S. 1189 ff. beschriebenen Methoden.
1.2 Die Hydroxyimine III erhält man beispielsweise durch Umsetzung eines Hydrazons V mit einer Carbonylverbindung der Formel VI. 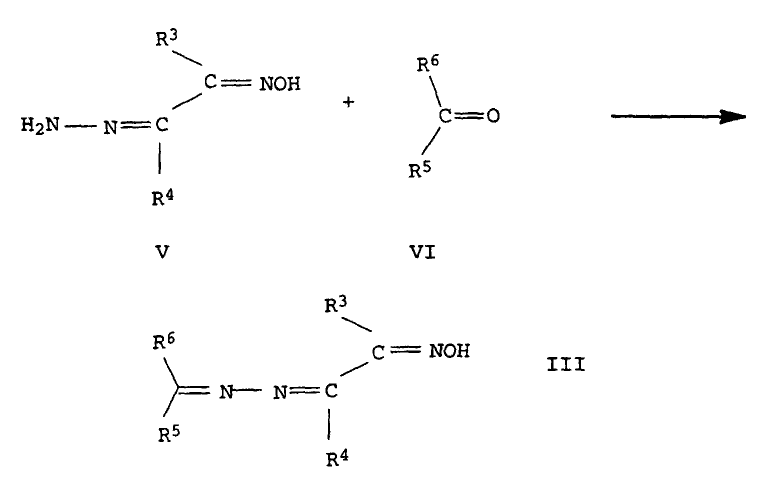
   Die Umsetzung erfolgt, gegebenenfalls unter Säurekatalyse, in an sich bekannter Weise in einem inerten organischen Lösungsmittel (vgl. Bull. Soc. Chim. Fr. 713 (1968); Houben-Weyl, 4. Auflage, Bd. X/2, Seite 104 ff.).
   Die Hydrazone V sind bekannt oder können nach an sich bekannten Methoden hergestellt werden (vgl. J. Med. Chem. 21, 623 (1978); J. Chem. Soc. 101, 2238 (1912); J. Org. Chem. 25, 313 (1960)).
1.3 Eine weitere Möglichkeit zur Herstellung der Hydroxyimine III besteht darin, daß man ein Carbonylhydroxyiminoderivat IV mit einem Hydrazon der Formel VII, wie unter 1.2 beschrieben, umsetzt. 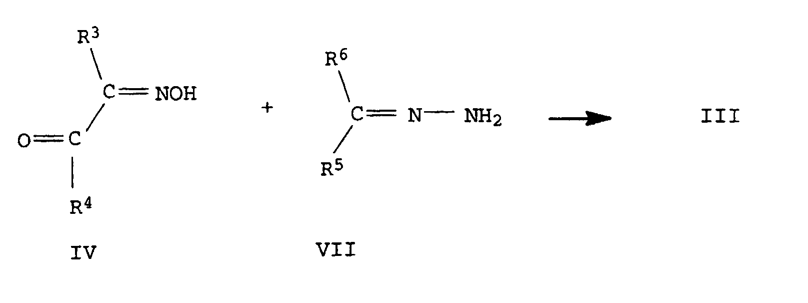
2. Alternativ können die Verbindungen I auch dadurch erhalten werden, daß man das Benzylderivat II zunächst mit dem Carbonylhydroxyiminoderivat IV in ein entsprechendes Benzyloxim der Formel VIII, und VIII anschließend mit einer Verbindung VII (s.o.) zu I umsetzt. 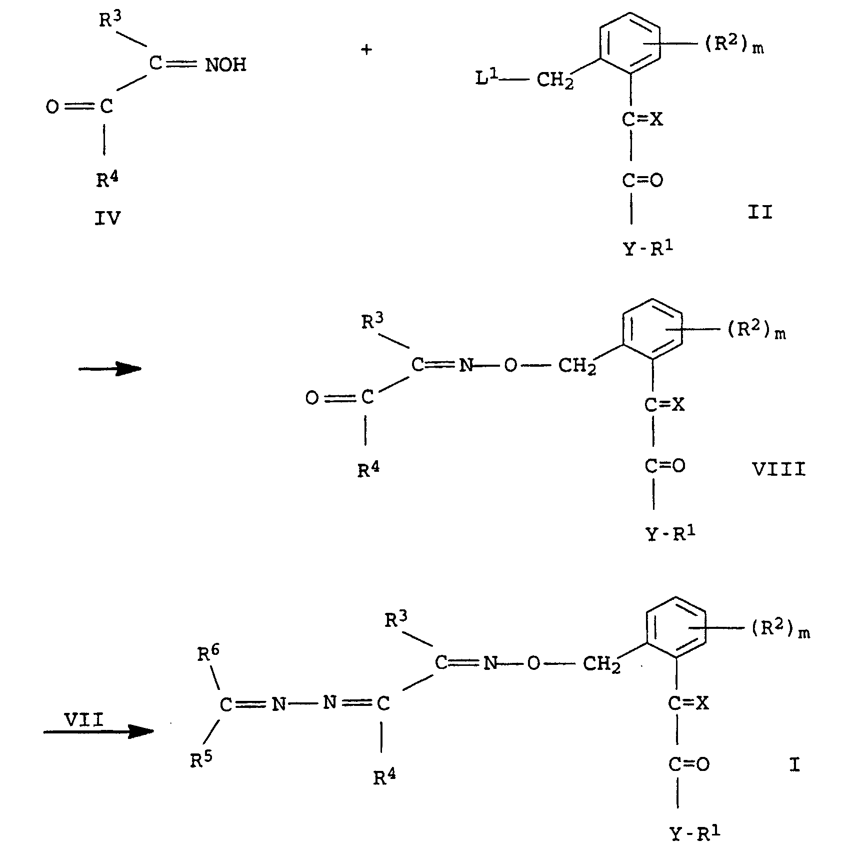
   Die Umsetzung von IV mit II erfolgt analog zur Synthese, wie sie unter 1.1 beschrieben ist. Die Umsetzung von VIII mit VII erfolgt analog zur Synthese, wie sie unter 1.2 beschrieben ist.
3.1 Die Synthese der Verbindungen I ist weiterhin möglich, indem man eine Verbindung der Formel VIII zunächst mit Hydrazinhydrat zum Hydrazon der Formel IX und dieses anschließend mit einer Carbonylverbindung der Formel VI umsetzt. 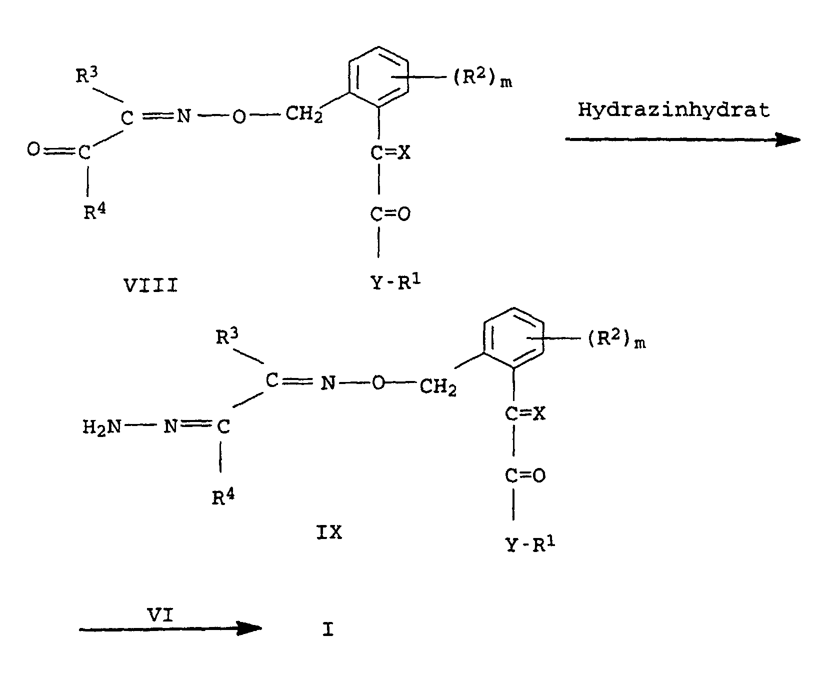
   Die Umsetzungen erfolgen analog zur Synthese, wie sie unter 1.2 beschrieben ist.
3.2 Die Synthese der Derivate IX ist auch dadurch möglich, daß man ein Benzylderivat der Formel II mit einem Hydroxyimin der Formel V umsetzt. 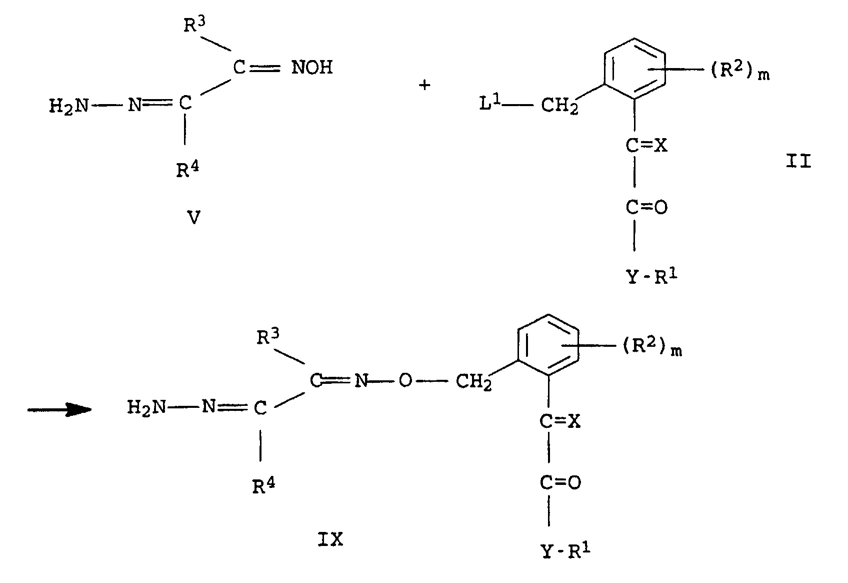
   L¹ in der Formel II steht für eine nucleophil austauschbare Abgangsgruppe, z.B. Halogen oder eine Sulfonatgruppe, vorzugsweise Chlor, Brom, Iod, Mesylat, Tosylat oder Triflat.
   Die Umsetzung erfolgt analog zur Synthese, wie sie unter 1.1 beschrieben ist.
4. Des weiteren erhält man die Verbindungen I dadurch, daß man eine Verbindung III gemäß der EP-A 493 711 mit einem Lacton X zunächst in die entsprechende Benzoesäure XI überführt und XI über die entsprechenden Halogenide XII in die Cyanocarbonsäuren XIII überführt, welche im Wege der Pinner-Reaktion (Angew. Chem. 94, 1 (1982)) in die α-Ketoester XIV überführt und ggf. weiter zu den α-Ketoamiden XV umgesetzt werden (vgl. EP-A 348 766, DE-A 37 05 389, EP-A 178 826, DE-A 36 23 921, Houben-Weyl, 4. Auflage, Bd. E5, S. 941 ff.). 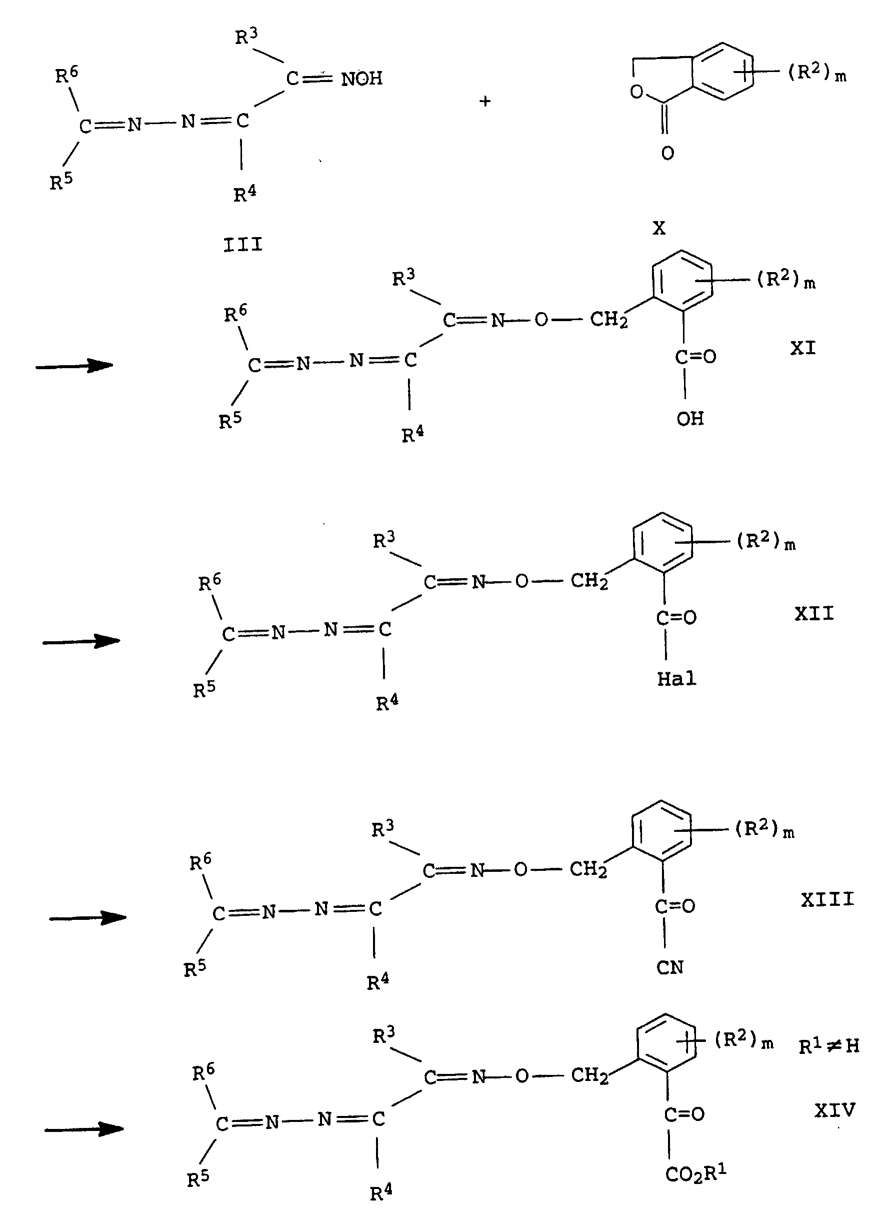 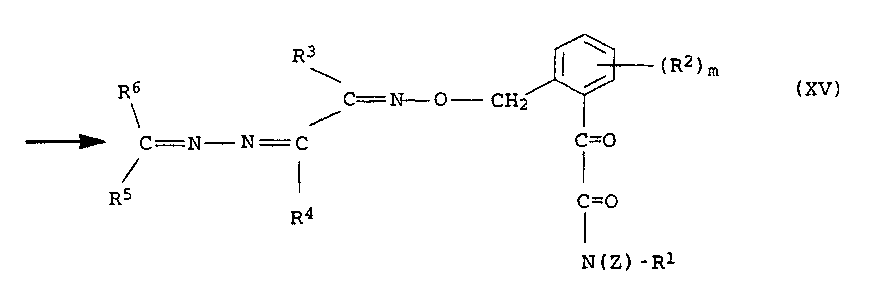 (Z =Wasserstoff oder C₁-C₄-Alkyl)
Die α-Ketoester XIV und die α-Ketoamide XV können gemäß üblichen Verfahren in die Verbindungen I überführt werden (vgl. EP-A 178 826, EP-A 348 766, DE-A 36 23 921, DE-A 37 05 389 sowie die eingangs zitierte Literatur).

Verbindungen I, in denen R¹ Wasserstoff bedeutet, erhält man nach diesem Verfahren durch Verseifung der Ester XIV und anschließende Umsetzung zu I.

Die Verbindungen I, in denen Y für NZ steht, können auch aus den Estern (Y=O) durch Umsetzung mit den entsprechenden Aminen HN(Z)R¹ erhalten werden.

Die Verbindungen II sind bekannt aus EP-A 513 580, EP-A 477 631, EP-A 463 488, EP-A 251 082, EP-A 400 417 und/oder EP-A 585 751 oder können nach den dort beschriebenen Methoden hergestellt werden.

Die Verbindungen I können bei der Herstellung aufgrund ihrer C=C und C=N Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung der Isomeren nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen, in der behandelten Pflanze oder im zu bekämpfenden Schadpilz oder tierischen Schädling erfolgen.

In Bezug auf die C=X Doppelbindung werden hinsichtlich ihrer Wirksamkeit die E-Isomere der Verbindungen I bevorzugt (Konfiguration bezogen auf die -OCH₃ bzw. die -CH₃ Gruppe im Verhältnis zur -COYR¹ Gruppe).

In Bezug auf die -C(R³)=NOCH₂- Doppelbindung werden hinsichtlich ihrer Wirksamkeit die cis-Isomere der Verbindungen I bevorzugt (Konfiguration bezogen auf den Rest R³ im Verhältnis zur -OCH₂-Gruppe).

Teil der Erfindung sind auch die Salze der säurebeständigen Verbindungen I, welche basische Zentren, vor allem basische Stickstoffatome enthalten, insbesondere mit Mineralsäuren wie Schwefelsäure und Phosphorsäure oder Lewis-Säuren wie Zinkchlorid. Üblicherweise kommt es hierbei auf die Art des Salzes nicht an. Im Sinne der Erfindung sind solche Salze bevorzugt, die die von Schadpilzen oder tierischen Schädlingen freizuhaltenden Pflanzen, Flächen, Materialien oder Räume nicht schädigen und die Wirkung der Verbindungen I nicht beeinträchtigen. Besonders bedeutsam sind derartige, für landwirtschaftliche Zwecke geeignete Salze.

Die Salze der Verbindungen I sind in an sich bekannter Weise zugänglich, vor allem durch Umsetzen der entsprechenden Biphenylamide I mit den genannten Säuren in Wasser oder einem inerten organischen Lösungsmittel bei Temperaturen von -80 bis 120°C, vorzugsweise 0 bis 60°C.

Bei den eingangs angegebenen Definitionen der Verbindungen I wurden Sammelbegriffe verwendet, die allgemein repräsentativ für die folgenden Gruppen stehen:
**Halogen:** Fluor, Chlor, Brom und Jod;
**Alkyl:** geradkettige oder verzweigte Alkylgruppen mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl;
**Alkylamino:** eine Aminogruppe, welche eine geradkettige oder verzweigte Alkylgruppe mit 1 bis 6 Kohlenstoffatomen wie vorstehend genannt trägt;
**Dialkylamino:** eine Aminogruppe, welche zwei voneinander unabhängige, geradkettige oder verzweigte Alkylgruppen mit jeweils 1 bis 6 Kohlenstoffatomen wie vorstehend genannt, trägt;
**Alkylcarbonyl:** geradkettige oder verzweigte Alkylgruppen mit 1 bis 10 Kohlenstoffatomen, welche über eine Carbonylgruppe (-CO-) an das Gerüst gebunden sind;
**Alkylsulfonyl:** geradkettige oder verzweigte Alkylgruppen mit 1 bis 6 oder 10 Kohlenstoffatomen, welche über eine Sulfonylgruppe (-SO₂-) an das Gerüst gebunden sind;
**Alkylsulfoxyl:** geradkettige oder verzweigte Alkylgruppen mit 1 bis 6 Kohlenstoffatomen, welche über eine Sulfoxylgruppe (-S(=O)-) an das Gerüst gebunden sind;
**Alkylaminocarbonyl:** Alkylaminogruppen mit 1 bis 6 Kohlenstoffatomen wie vorstehend genannt, welche über eine Carbonylgruppe (-CO-) an das Gerüst gebunden sind;
**Dialkylaminocarbonyl:** Dialkylaminogruppen mit jeweils 1 bis 6 Kohlenstoffatomen pro Alkylrest wie vorstehend genannt, welche über eine Carbonylgruppe (-CO-) an das Gerüst gebunden sind;
**Alkylaminothiocarbonyl:** Alkylaminogruppen mit 1 bis 6 Kohlenstoffatomen wie vorstehend genannt, welche über eine Thiocarbonylgruppe (-CS-) an das Gerüst gebunden sind;
**Dialkylaminothiocarbonyl:** Dialkylaminogruppen mit jeweils 1 bis 6 Kohlenstoffatomen pro Alkylrest wie vorstehend genannt, welche über eine Thiocarbonylgruppe (-CS-) an das Gerüst gebunden sind;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 4 oder 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
**Alkoxycarbonyl:** geradkettige oder verzweigte Alkylgruppen mit 1 bis 6 Kohlenstoffatomen, welche über eine Oxycarbonylgruppe (-OC(=O)-) an das Gerüst gebunden sind;
**Alkylendioxy:** z.B. C₁-C₄-Alkylendioxy: geradkettige oder verzweigte Alkylengruppen mit 1 bis 4 Kohlenstoffatomen, welche an zwei Stellen über je ein Sauerstoffatom (-O-) in das Gerüst eingebunden oder an das Gerüst gebunden sind wie Methylendioxy (-O-CH₂-O-) oder 2,2-Propylendioxy (-O-C(CH₃)₂-O-);
**Halogenalkoxy:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, und wobei diese Gruppen über ein Sauerstoffatom an das Gerüst gebunden sind;
**Alkylthio:** geradkettige oder verzweigte Alkylgruppen mit 1 bis 4 oder 6 Kohlenstoffatomen wie vorstehend genannt, welche über ein Schwefelatom (-S-) an das Gerüst gebunden sind, z.B. C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
**Cycloalkyl:** monocyclische Alkylgruppen mit 3 bis 6 Kohlenstoffringgliedern, z.B. Cyclopropyl, Cyclobutyl, Cyclopentyl und Cyclohexyl;
**Alkenyl:** geradkettige oder verzweigte Alkenylgruppen mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-1-methyl-2-propenyl, 1-Ethyl-2-methyl-1-propenyl und 1-Ethyl-2-methyl-2-propenyl;
**Alkenyloxy:** geradkettige oder verzweigte Alkenylgruppen mit 2 bis 6 Kohlenstoffatomen und einer Doppelbindung in einer beliebigen Position, welche über ein Sauerstoffatom (-O-) an das Gerüst gebunden sind;
**Alkenylthio bzw. Alkenylamino:** geradkettige oder verzweigte Alkenylgruppen mit 2 bis 6 Kohlenstoffatomen und einer Doppelbindung in einer beliebigen Position, welche (Alkenylthio) über ein Schwefelatom bzw. (Alkenylamino) ein Stickstoffatom an das Gerüst gebunden sind.
**Alkenylcarbonyl:** geradkettige oder verzweigte Alkenylgruppen mit 2 bis 10 Kohlenstoffatomen und einer Doppelbindung in einer beliebigen Position, welche über eine Carbonylgruppe (-CO-) an das Gerüst gebunden sind;
**Alkinyl:** geradkettige oder verzweigte Alkinylgruppen mit 2 bis 10 Kohlenstoffatomen und einer Dreifachbindung in einer beliebigen Position, z.B. C₂-C₆-Alkinyl wie Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
**Alkinyloxy bzw. Alkinylthio und Alkinylamino:** geradkettige oder verzweigte Alkinylgruppen mit 2 bis 6 Kohlenstoffatomen und einer Dreifachbindung in einer beliebigen Position, welche (Alkinyloxy) über ein Sauerstoffatom bzw. (Alkinylthio) über ein Schwefelatom oder (Alkinylamino) über ein Stickstoffatom an das Gerüst gebunden sind.
**Alkinylcarbonyl:** geradkettige oder verzweigte Alkinylgruppen mit 3 bis 10 Kohlenstoffatomen und einer Dreifachbindung in einer beliebigen Position, welche über eine Carbonylgruppe (-CO-) an das Gerüst gebunden sind;
**Cycloalkenyl bzw. Cycloalkenyloxy, Cycloalkenylthio und Cycloalkenylamino:** monocyclische Alkenylgruppen mit 3 bis 6 Kohlenstoffringgliedern, welche direkt bzw. (Cycloalkenyloxy) über ein Sauerstoffatom oder (Cycloalkenylthio) ein Schwefelatom oder Cycloalkenylamino) über ein Stickstoffatom an das Gerüst gebunden sind, z.B. Cyclopropenyl, Cyclobutenyl, Cyclopentenyl oder Cyclohexenyl.
**Cycloalkyloxy bzw. Cycloalkylthio und Cycloalkylamino:** monocyclische Alkylgruppen mit 3 bis 6 Kohlenstoffringgliedern, welche (Cycloalkyloxy) über ein Sauerstoffatom oder (Cycloalkylthio) ein Schwefelatom oder (Cycloalkylamino) über ein Stickstoffatom an das Gerüst gebunden sind, z.B. Cyclopropyl, Cyclobutyl, Cyclopentyl oder Cyclohexyl;
**Heterocyclyl bzw. Heterocyclyloxy, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Tri-azolidin-2-yl, 2,3-Dihydrofur-2-yl, 2,3-Dihydrofur-3-yl, 2,3-Dihydro-fur-4-yl, 2,3-Dihydro-fur-5-yl, 2,5-Dihydro-fur-2-yl, 2,5-Dihydro-fur-3-yl, 2,3-Dihydrothien-2-yl, 2,3-Dihydro-thien-3-yl, 2,3-Dihydrothien-4-yl, 2,3-Dihydrothien-5-yl, 2,5-Dihydrothien-2-yl, 2,5-Dihydrothien-3-yl, 2,3-Dihydro-pyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydro-pyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisothiazol-3-yl, 2,5-Dihydroisothiazol-4-yl, 2,5-Dihydroisothiazol-5-yl, 2,3 -Dihydroisopyrazol-3-yl, 2,3-Dihydroisopyrazol-4-yl, 2,3-Dihydroisopyrazol-5-yl, 4,5-Dihydroisopyrazol-3-yl, 4,5-Dihydroisopyrazol-4-yl, 4,5-Dihydroisopyrazol-5-yl, 2,5-Dihydroisopyrazol-3-yl, 2,5-Dihydroisopyrazol-4-yl, 2,5-Dihydroisopyrazol-5-yl, 2,3-Dihydrooxazol-3-yl, 2,3-Dihydrooxazol-4-yl, 2,3-Dihydrooxazol-5-yl, 4,5-Dihydrooxazol-3-yl, 4,5-Dihydrooxazol-4-yl, 4,5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl,
**Aryl bzw. Aryloxy, Arylthio, Arylcarbonyl und Arylsulfonyl:** aromatische mono- oder polycyclische Kohlenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;
**Arylamino:** aromatische mono- oder polycyclische Kohlenwasserstoffreste, welche über ein Stickstoffatom an das Gerüst gebunden sind.
**Heteroaryl bzw. Heteroaryloxy, Heteroarylthio, Heteroarylcarbonyl und Hetarylsulfonyl:** aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche direkt bzw. (Hetaryloxy) über ein Sauerstoffatom (-O-) oder (Hetarylthio) ein Schwefelatom (-S-), (Hetarylcarbonyl) über eine Carbonylgruppe (-CO-) oder (Hetarylsulfonyl) über eine Sulfonylgruppe (-SO₂-) an das Gerüst gebunden sind, z.B.
   - 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
   - 5-gliedriges Heteroaryl, enthaltend ein bis vier Stickstoffatome oder ein bis drei Stickstoffatome und ein Schwefel - oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
   - benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien- 1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
   - benzokondensiertes 6-gliedriges Heteroaryl, enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin,
   bzw. die entsprechenden Oxy-, Thio-, Carbonyl- oder Sulfonylgruppen.
**Heteroarylamino:** aromatische mono- oder polycyclische Reste, welche neben Kohlenstoffringgliedern zusätzlich ein bis vier Stickstoffatome oder ein bis drei Stickstoffatome und ein Sauerstoff-oder ein Schwefelatom enthalten können und welche über ein Stickstoffatom an das Gerüst gebunden sind.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome, wie vorstehend genannt, ersetzt sein können.

Die Angabe "gegebenenfalls substituiert" soll zum Ausdruck bringen, daß in den derart charakterisierten Gruppen die Wasserstoffatome zum Teil oder vollständig durch gleiche oder verschiedene beispielsweise derjenigen Gruppen ersetzt sein können, die unter den vorstehend ausgeführten Sammelbegriffen genannt sind.

Im Hinblick auf ihre biologische Wirkung sind Verbindungen der Formel I bevorzugt, in der die Variablen die folgenden Bedeutungen haben:
- X: NOCH₃, CHOCH₃ oder CHCH₃;
- Y: O oder NZ, wobei Z für Wasserstoff oder C₁-C₄-Alkyl steht;
- R¹: Wasserstoff oder C₁-C₄-Alkyl;
- R²: Cyano, Nitro, Halogen, C₁-C₄-Alkyl, Trifluormethyl oder C₁-C₄-Alkoxy;
- m: 0, 1 oder 2, wobei die Reste R² verschieden sein können, wenn m für 2 steht;
- R³: Wasserstoff, Cyano, C₁-C₄-Alkyl, C₁-C₄-Halogenalkyl, C₁-C₄-Alkoxy, Cyclopropyl;
- R⁴, R⁵, R⁶ unabhängig voneinander:: Wasserstoff;
C₁-C₆-Alkyl, C₂-C₆-Alkenyl, C₂-C₆-Alkinyl, wobei diese Reste partiell oder vollständig halogeniert sein und/oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Heteroaryl, Hetero-aryloxy, Heteroaryl-C₁-C₄-alkoxy, Heteroarylthio und Heteroaryl-C₁-C₄-alkylthio, wobei die cyclischen Gruppen ihrerseits partiell oder vollständig halogeniert sein und/oder einen bis drei der folgenden Substituenten tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio, C₃-C₆-Alkinyloxy, C₁-C₄-Alkylendioxy, welches halogeniert sein kann, und C(=NOR⁷)-Aₙ-R⁸, wobei A für Sauerstoff, Schwefel oder Stickstoff steht und wobei der Stickstoff Wasserstoff oder C₁-C₆-Alkyl trägt, n gleich 0 oder 1 ist, R⁷ Wasserstoff oder C₁-C₆-Alkyl und R⁸ Wasserstoff oder C₁-C₆-Alkyl bedeutet;
C₃-C₆-Cycloalkyl, C₃-C₆-Cycloalkenyl, Heterocyclyl, wobei diese Reste partiell oder vollständig halogeniert sein und/oder eine bis drei der folgenden Gruppen tragen können: Cyano, C₁-C₆-Alkyl, C₁-C₆-Halogenalkyl, C₂-C₆-Alkenyl, C₁-C₆-Alkoxy und C₁-C₆-Alkylthio;
Aryl oder Heteroaryl, wobei diese Reste partiell oder vollständig halogeniert sein und/oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio, C₃-C₆-Alkinyloxy, C₁-C₄-Alkylendioxy, welches halogeniert sein kann, und C(=NOR⁷)-Aₙ-R⁸,
wobei A für Sauerstoff, Schwefel oder Stickstoff steht und der Stickstoff Wasserstoff oder C₁-C₆-Alkyl trägt, n gleich 0 oder 1 ist, R⁷ Wasserstoff oder C₁-C₆-Alkyl und R⁸ Wasserstoff oder C₁-C₆-Alkyl bedeutet
und wobei die cyclischen Gruppen ihrerseits partiell oder vollständig halogeniert sein und/oder einen bis drei der folgenden Substituenten tragen können: Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy und C₁-C₄-Alkylendioxy, welches halogeniert sein kann;
- R⁵ und R⁶: zusammen mit dem Kohlenstoffatom, an das sie gebunden sind, ein gegebenenfalls substituierter gesättigter oder ungesättigter carbocyclischer oder heterocyclischer Ring, vorzugsweise gegebenenfalls wie folgt substituiert: partiell oder vollständig halogeniert und/oder eine bis drei der folgenden Gruppen tragend: Cyano, C₁-C₆-Alkyl, C₁-C₆-Halogenalkyl, C₂-C₆-Alkenyl, C₁-C₆-Alkoxy und C₁-C₆-Alkylthio,
sowie deren Salze.

Weiterhin sind Verbindungen der Formel I bevorzugt, in denen m für 0 steht.

Gleichermaßen bevorzugt sind Verbindungen der Formel I, in denen R¹ für Methyl steht.

Daneben werden Verbindungen I bevorzugt, in denen R³ für Wasserstoff, Cyclopropyl, Methyl, Ethyl, 1-Methylethyl, Trifluormethyl, Cyano oder Methoxy steht.

Besonders bevorzugt sind Verbindungen I, in denen R³ für Methyl steht.

Des weiteren werden Verbindungen I bevorzugt, in denen R³ für Trifluormethyl steht.

Besonders bevorzugt sind auch Verbindungen I, in denen R³ für Methoxy steht.

Des weiteren werden Verbindungen I bevorzugt, in denen R⁴ für gegebenenfalls substituiertes C₁-C₆-Alkyl, C₂-C₆-Alkenyl oder C₂-C₆-Alkinyl steht.

Außerdem werden Verbindungen I bevorzugt, in denen R⁴ für gegebenenfalls substituiertes Heterocyclyl oder gegebenenfalls substituiertes C₃-C₆-Cycloalkenyl steht.

Besonders bevorzugt werden Verbindungen I, in denen R⁴ für C₁-C₄-Alkyl steht.

Außerdem werden Verbindungen I bevorzugt, in denen R⁴ für gegebenenfalls substituiertes C₃-C₆-Cycloalkyl steht.

Des weiteren werden Verbindungen I bevorzugt, in denen R⁴ für gegebenenfalls substituiertes Aryl oder Hetaryl steht.

Des weiteren werden Verbindungen I bevorzugt, in denen R⁴ für ggf. subst. Pyridyl, Pyrimidyl, Pyrazinyl, Pyridazinyl oder Triazinyl steht.

Des weiteren werden Verbindungen I bevorzugt, in denen R⁴ für ggf. subst. Furyl, Thienyl oder Pyrrolyl steht.
Des weiteren werden Verbindungen I bevorzugt, in denen R⁴ für ggf. subst. Oxazolyl, Thiazolyl, Isoxazolyl, Isothiazolyl, Pyrazolyl oder Imidazolyl steht.

Des weiteren werden Verbindungen I bevorzugt, in denen R⁴ für ggf. subst. Oxdiazolyl, Thiadiazolyl oder Triazolyl steht.

Außerdem werden Verbindungen I bevorzugt, in denen R⁴ für Phenyl steht, welches unsubstituiert ist oder eine oder zwei der folgenden Gruppen trägt: Nitro, Cyano, Hydroxy, Amino, Aminocarbonyl, Aminothiocarbonyl, Halogen, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylamino, C₁-C₄-Alkylcarbonyl, Di-C₁-C₄-Alkylamino, C₁-C₄-Alkylsulfonyl, C₁-C₄-Alkoxycarbonyl, C₁-C₄-Alkylaminocarbonyl, Di-C₁-C₄-Alkylaminocarbonyl, C₁-C₄-Alkylthio, C₂-C₆-Alkenyl, C₂-C₆-Alkenyloxy, C₃-C₆-Alkinyloxy und C₁-C₄-Alkylendioxy, welches halogeniert sein kann.

Des weiteren werden Verbindungen I bevorzugt, in denen R⁵ für ggf. substituiertes C₁-C₆-Alkyl steht.

Außerdem werden Verbindungen I bevorzugt, in denen R⁵ für Methyl oder Ethyl steht.

Des weiteren werden Verbindungen I bevorzugt, in denen R⁵ für Wasserstoff steht.

Außerdem werden Verbindungen I bevorzugt, in denen R⁵ für ggf. substituiertes C₂-C₆-Alkenyl steht.

Ferner werden Verbindungen I bevorzugt, in denen R⁵ für ggf. substituiertes C₂-C₆-Alkinyl steht.

Außerdem werden Verbindungen I bevorzugt, in denen R⁵ für ggf. substituiertes Hetaryl steht.

Des weiteren werden Verbindungen I bevorzugt, in denen R⁵ für ggf. subst. Pyridyl, Pyrimidyl, Pyrazinyl, Pyridazinyl oder Triazinyl steht.

Des weiteren werden Verbindungen I bevorzugt, in denen R⁵ für ggf. subst. Furyl, Thienyl oder Pyrrolyl steht.

Des weiteren werden Verbindungen I bevorzugt, in denen R⁵ für ggf. subst. Oxazolyl, Thiazolyl, Isoxazolyl, Isothiazolyl, Pyrazolyl oder Imidazolyl steht.

Des weiteren werden Verbindungen I bevorzugt, in denen R⁵ für ggf. subst. Aryl steht.

Außerdem werden Verbindungen I bevorzugt, in denen R⁵ für Phenyl steht, welches unsubstituiert ist oder eine oder zwei der folgenden Gruppen trägt: Nitro, Cyano, Hydroxy, Amino, Aminocarbonyl, Aminothiocarbonyl, Halogen, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylamino, C₁-C₄-Alkylcarbonyl, Di-C₁-C₄-Alkylamino, C₁-C₄-Alkylsulfonyl, C₁-C₄-Alkoxycarbonyl, C₁-C₄-Alkylaminocarbonyl, Di-C₁-C₄-Alkylaminocarbonyl, C₁-C₄-Alkylthio, C₂-C₆-Alkenyl, C₂-C₆-Alkenyloxy, C₃-C₆-Alkinyloxy und C₁-C₄-Alkylendioxy, welches halogeniert sein kann.

Des weiteren werden Verbindungen I bevorzugt, in denen R⁶ für ggf. subst. C₁-C₆-Alkyl steht.

Außerdem werden Verbindungen I bevorzugt, in denen R⁶ für Methyl oder Ethyl steht.

Des weiteren werden Verbindungen I bevorzugt, in denen R⁶ für Wasserstoff steht.

Außerdem werden Verbindungen I bevorzugt, in denen R⁶ für ggf. subst. C₂-C₆-Alkenyl steht.

Außerdem werden Verbindungen I bevorzugt, in denen R⁶ für ggf. subst. C₂-C₆-Alkinyl steht.

Außerdem werden Verbindungen I bevorzugt, in denen R⁶ für gegebenenfalls substituiertes Hetaryl steht.

Des weiteren werden Verbindungen I bevorzugt, in denen R⁶ für ggf. subst. Pyridyl, Pyrimidyl, Pyrazinyl, Pyridazinyl oder Triazinyl steht.

Des weiteren werden Verbindungen I bevorzugt, in denen R⁶ für ggf. subst. Furyl, Thienyl oder Pyrrolyl steht.

Des weiteren werden Verbindungen I bevorzugt, in denen R⁶ für ggf. subst. Oxazolyl, Thiazolyl, Isoxazolyl, Isothiazolyl, Pyrazolyl oder Imidazolyl steht.

Des weiteren werden Verbindungen I bevorzugt, in denen R⁶ für ggf. subst. Aryl steht.

Außerdem werden Verbindungen I bevorzugt, in denen R⁶ für Phenyl steht, welches unsubstituiert ist oder eine oder zwei der folgenden Gruppen trägt: Nitro, Cyano, Hydroxy, Amino, Aminocarbonyl, Aminothiocarbonyl, Halogen, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylamino, C₁-C₄-Alkylcarbonyl, Di-C₁-C₄-Alkylamino, C₁-C₄-Alkylsulfonyl, C₁-C₄-Alkoxycarbonyl, C₁-C₄-Alkylaminocarbonyl, Di-C₁-C₄-Alkylaminocarbonyl, C₁-C₄-Alkylthio, C₂-C₆-Alkenyl, C₂-C₆-Alkenyloxy, C₃-C₆-Alkinyloxy und C₁-C₄-Alkylendioxy, welches halogeniert sein kann.

Daneben werden Verbindungen der Formel I bevorzugt, in denen X für NOCH₃ steht.

Daneben werden Verbindungen der Formel I bevorzugt, in denen X für CHOCH₃ steht.

Daneben werden Verbindungen der Formel I bevorzugt, in denen X für CHCH₃ steht.

Des weiteren werden Verbindungen der Formel I bevorzugt, in denen Y für O steht.

Außerdem werden Verbindungen der Formel I bevorzugt, in denen Y für NH steht.

Insbesondere sind im Hinblick auf ihre Verwendung die in den folgenden Tabellen zusammengestellten Verbindungen I bevorzugt.

Den folgenden Tabellen (1 bis 728) liegen die Formeln I.1, I.2, I.3 und I.4 zugrunde, wobei die mit "E" markierten Doppelbindungen E-Konfiguration aufweisen:

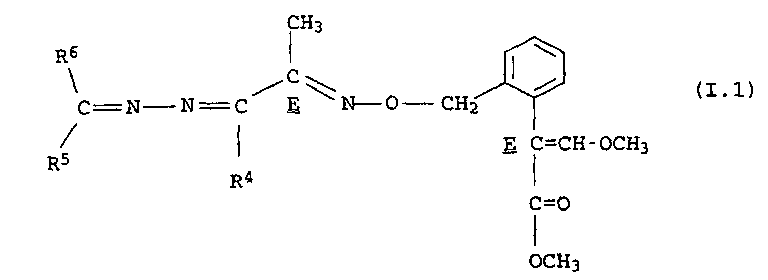

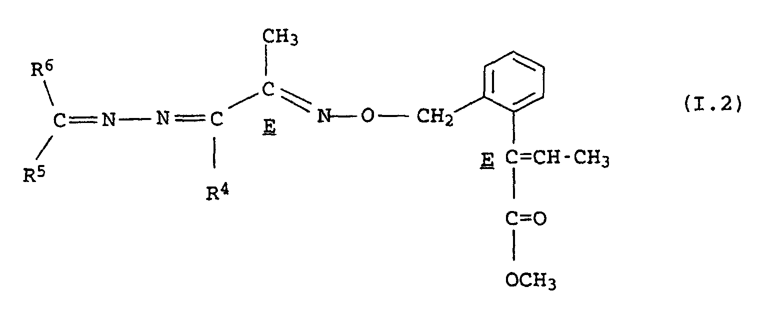

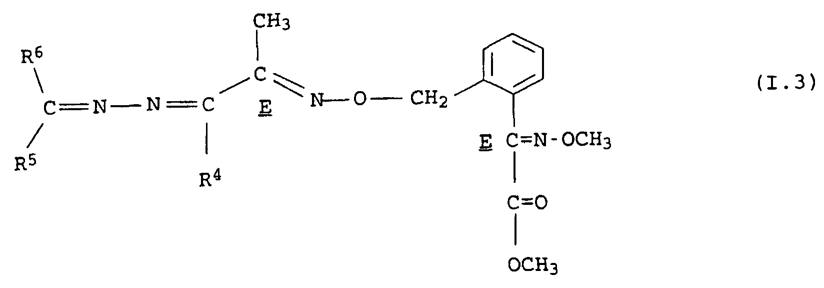

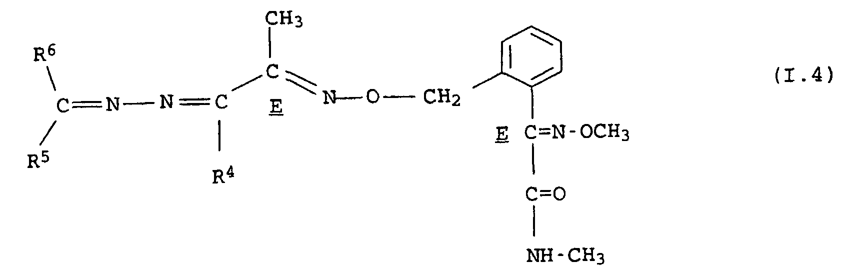

**Tabelle 1**

| Verbindungen der Formel I.1, mit | |
|---|---|
| R⁶ = | Wasserstoff; |
| R⁵ = | Wasserstoff; |
| R⁴ = | jeweils einer Zeile der Tabelle A. |

**Tabelle 2**

| Verbindungen der Formel I.2, mit | |
|---|---|
| R⁶ = | Wasserstoff; |
| R⁵ = | Wasserstoff; |
| R⁴ = | jeweils eine Zeile der Tabelle A. |

**Tabelle 3**

| Verbindungen der Formel I.3, mit | |
|---|---|
| R⁶ = | Wasserstoff; |
| R⁵ = | Wasserstoff; |
| R⁴ = | jeweils eine Zeile der Tabelle A. |

**Tabelle 4**

| Verbindungen der Formel I.4, mit | |
|---|---|
| R⁶ = | Wasserstoff; |
| R⁵ = | Wasserstoff; |
| R⁴ = | jeweils eine Zeile der Tabelle A. |

**Tabelle 5**

| Verbindungen der Formel I.1, mit | |
|---|---|
| R⁶ = | Wasserstoff; |
| R⁵ = | Methyl; |
| R⁴ = | jeweils eine Zeile der Tabelle A. |

**Tabelle 6**

| Verbindungen der Formel I.2, mit | |
|---|---|
| R⁶ = | Wasserstoff; |
| R⁵ = | Methyl; |
| R⁴ = | jeweils eine Zeile der Tabelle A. |

**Tabelle 7**

| Verbindungen der Formel I.3, mit | |
|---|---|
| R⁶ = | Wasserstoff; |
| R⁵ = | Methyl; |
| R⁴ = | jeweils eine Zeile der Tabelle A. |

**Tabelle 8**

| Verbindungen der Formel I.4, mit | |
|---|---|
| R⁶ = | Wasserstoff; |
| R⁵ = | Methyl; |
| R⁴ = | jeweils eine Zeile der Tabelle A. |

**Tabelle 9**

| Verbindungen der Formel I.1, mit | |
|---|---|
| R⁶ = | Wasserstoff; |
| R⁵ = | Ethyl; |
| R⁴ = | jeweils eine Zeile der Tabelle A. |

**Tabelle 10**

| Verbindungen der Formel I.2, mit | |
|---|---|
| R⁶ = | Wasserstoff; |
| R⁵ = | Ethyl; |
| R⁴ = | jeweils eine Zeile der Tabelle A. |

**Tabelle 11**

| Verbindungen der Formel I.3, mit | |
|---|---|
| R⁶ = | Wasserstoff; |
| R⁵ = | Ethyl; |
| R⁴ = | jeweils eine Zeile der Tabelle A. |

**Tabelle 12**

| Verbindungen der Formel I.4, mit | |
|---|---|
| R⁶ = | Wasserstoff; |
| R⁵ = | Ethyl; |
| R⁴ = | jeweils eine Zeile der Tabelle A. |

**Tabelle 13**

| Verbindungen der Formel I.1, mit | |
|---|---|
| R⁶ = | Wasserstoff; |
| R⁵ = | n-Propyl; |
| R⁴ = | jeweils eine Zeile der Tabelle A. |

**Tabelle 14**

| Verbindungen der Formel I.2, mit | |
|---|---|
| R⁶ = | Wasserstoff; |
| R⁵ = | n-Propyl; |
| R⁴ = | jeweils eine Zeile der Tabelle A. |

**Tabelle 15**

| Verbindungen der Formel I.3, mit | |
|---|---|
| R⁶ = | Wasserstoff; |
| R⁵ = | n-Propyl; |
| R⁴ = | jeweils eine Zeile der Tabelle A. |

**Tabelle 16**

| Verbindungen der Formel I.4, mit | |
|---|---|
| R⁶ = | Wasserstoff; |
| R⁵ = | n-Propyl; |
| R⁴ = | jeweils eine Zeile der Tabelle A. |

**Tabelle 17**

| Verbindungen der Formel I.1, mit | |
|---|---|
| R⁶ = | Wasserstoff; |
| R⁵ = | iso-Propyl; |
| R⁴ = | jeweils eine Zeile der Tabelle A. |

**Tabelle 18**

| Verbindungen der Formel I.2, mit | |
|---|---|
| R⁶ = | Wasserstoff; |
| R⁵ = | iso-Propyl; |
| R⁴ = | jeweils eine Zeile der Tabelle A. |

**Tabelle 19**

| Verbindungen der Formel I.3, mit | |
|---|---|
| R⁶ = | Wasserstoff; |
| R⁵ = | iso-Propyl; |
| R⁴ = | jeweils eine Zeile der Tabelle A. |

**Tabelle 20**

| Verbindungen der Formel I.4, mit | |
|---|---|
| R⁶ = | Wasserstoff; |
| R⁵ = | iso-Propyl; |
| R⁴ = | jeweils eine Zeile der Tabelle A. |

**Tabelle 21**

| Verbindungen der Formel I.1, mit | |
|---|---|
| R⁶ = | Wasserstoff; |
| R⁵ = | Cyclopropyl; |
| R⁴ = | jeweils eine Zeile der Tabelle A. |

**Tabelle 22**

| Verbindungen der Formel I.2, mit | |
|---|---|
| R⁶ = | Wasserstoff; |
| R⁵ = | Cyclopropyl; |
| R⁴ = | jeweils eine Zeile der Tabelle A. |

**Tabelle 23**

| Verbindungen der Formel I.3, mit | |
|---|---|
| R⁶ = | Wasserstoff; |
| R⁵ = | Cyclopropyl; |
| R⁴ = | jeweils eine Zeile der Tabelle A. |

**Tabelle 24**

| Verbindungen der Formel I.4, mit | |
|---|---|
| R⁶ = | Wasserstoff; |
| R⁵ = | Cyclopropyl; |
| R⁴ = | jeweils eine Zeile der Tabelle A. |

**Tabelle 25**

| Verbindungen der Formel I.1, mit | |
|---|---|
| R⁶ = | Wasserstoff; |
| R⁵ = | n-Butyl; |
| R⁴ = | jeweils eine Zeile der Tabelle A. |

**Tabelle 26**

| Verbindungen der Formel I.2, mit | |
|---|---|
| R⁶ = | Wasserstoff; |
| R⁵ = | n-Butyl; |
| R⁴ = | jeweils eine Zeile der Tabelle A. |

**Tabelle 27**

| Verbindungen der Formel 1.3, mit | |
|---|---|
| R⁶ = | Wasserstoff; |
| R⁵ = | n-Butyl; |
| R⁴ = | jeweils eine Zeile der Tabelle A. |

**Tabelle 28**

| Verbindungen der Formel I.4, mit | |
|---|---|
| R⁶ = | Wasserstoff; |
| R⁵ = | n-Butyl; |
| R⁴ = | jeweils eine Zeile der Tabelle A. |

**Tabelle 29**

| Verbindungen der Formel I.1, mit | |
|---|---|
| R⁶ = | Wasserstoff; |
| R⁵ = | iso-Butyl; |
| R⁴ = | jeweils eine Zeile der Tabelle A. |

**Tabelle 30**

| Verbindungen der Formel I.2, mit | |
|---|---|
| R⁶ = | Wasserstoff; |
| R⁵ = | iso-Butyl; |
| R⁴ = | jeweils eine Zeile der Tabelle A. |

**Tabelle 31**

| Verbindungen der Formel I.3, mit | |
|---|---|
| R⁶ = | Wasserstoff; |
| R⁵ = | iso-Butyl; |
| R⁴ = | jeweils eine Zeile der Tabelle A. |

**Tabelle 32**

| Verbindungen der Formel I.4, mit | |
|---|---|
| R⁶ = | Wasserstoff; |
| R⁵ = | iso-Butyl; |
| R⁴ = | jeweils eine Zeile der Tabelle A. |

**Tabelle 33**

| Verbindungen der Formel I.1, mit | |
|---|---|
| R⁶ = | Wasserstoff; |
| R⁵ = | sec-Butyl; |
| R⁴ = | jeweils eine Zeile der Tabelle A. |

**Tabelle 34**

| Verbindungen der Formel I.2, mit | |
|---|---|
| R⁶ = | Wasserstoff; |
| R⁵ = | sec-Butyl; |
| R⁴ = | jeweils eine Zeile der Tabelle A. |

**Tabelle 35**

| Verbindungen der Formel I.3, mit | |
|---|---|
| R⁶ = | Wasserstoff; |
| R⁵ = | sec-Butyl; |
| R⁴ = | jeweils eine Zeile der Tabelle A. |

**Tabelle 36**

| Verbindungen der Formel I.4, mit | |
|---|---|
| R⁶ = | Wasserstoff; |
| R⁵ = | sec-Butyl; |
| R⁴ = | jeweils eine Zeile der Tabelle A. |

**Tabelle 37**

| Verbindungen der Formel I.1, mit | |
|---|---|
| R⁶ = | Wasserstoff; |
| R⁵ = | t-Butyl; |
| R⁴ = | jeweils eine Zeile der Tabelle A. |

**Tabelle 38**

| Verbindungen der Formel I.2, mit | |
|---|---|
| R⁶ = | Wasserstoff; |
| R⁵ = | t-Butyl; |
| R⁴ = | jeweils eine Zeile der Tabelle A. |

**Tabelle 39**

| Verbindungen der Formel I.3, mit | |
|---|---|
| R⁶ = | Wasserstoff; |
| R⁵ = | t-Butyl; |
| R4 = | jeweils eine Zeile der Tabelle A. |

**Tabelle 40**

| Verbindungen der Formel I.4, mit | |
|---|---|
| R⁶ = | Wasserstoff; |
| R⁵ = | t-Butyl; |
| R⁴ = | jeweils eine Zeile der Tabelle A. |

**Tabelle 41**

| Verbindungen der Formel I.1, mit | |
|---|---|
| R⁶ = | Wasserstoff; |
| R⁵ = | Trifluormethyl; |
| R⁴ = | jeweils eine Zeile der Tabelle A. |

**Tabelle 42**

| Verbindungen der Formel I.2, mit | |
|---|---|
| R⁶ = | Wasserstoff; |
| R⁵ = | Trifluormethyl; |
| R⁴ = | jeweils eine Zeile der Tabelle A. |

**Tabelle 43**

| Verbindungen der Formel I.3, mit | |
|---|---|
| R⁶ = | Wasserstoff; |
| R⁵ = | Trifluormethyl; |
| R⁴ = | jeweils eine Zeile der Tabelle A. |

**Tabelle 44**

| Verbindungen der Formel I.4, mit | |
|---|---|
| R⁶ = | Wasserstoff; |
| R⁵ = | Trifluormethyl; |
| R⁴ = | jeweils eine Zeile der Tabelle A. |

**Tabelle 45**

| Verbindungen der Formel I.1, mit | |
|---|---|
| R⁶ = | Wasserstoff; |
| R⁵ = | Pentafluorethyl; |
| R⁴ = | jeweils eine Zeile der Tabelle A. |

**Tabelle 46**

| Verbindungen der Formel I.2, mit | |
|---|---|
| R⁶ = | Wasserstoff; |
| R⁵ = | Pentafluorethyl; |
| R⁴ = | jeweils eine Zeile der Tabelle A. |

**Tabelle 47**

| Verbindungen der Formel I.3, mit | |
|---|---|
| R⁶ = | Wasserstoff; |
| R⁵ = | Pentafluorethyl; |
| R⁴ = | jeweils eine Zeile der Tabelle A. |

**Tabelle 48**

| Verbindungen der Formel I.4, mit | |
|---|---|
| R⁶ = | Wasserstoff; |
| R⁵ = | Pentafluorethyl; |
| R⁴ = | jeweils eine Zeile der Tabelle A. |

**Tabelle 49**

| Verbindungen der Formel I.1, mit | |
|---|---|
| R⁶ = | Wasserstoff; |
| R⁵ = | Cyanomethyl; |
| R⁴ = | jeweils eine Zeile der Tabelle A. |

**Tabelle 50**

| Verbindungen der Formel I.2, mit | |
|---|---|
| R⁶ = | Wasserstoff; |
| R⁵ = | Cyanomethyl; |
| R⁴ = | jeweils eine Zeile der Tabelle A. |

**Tabelle 51**

| Verbindungen der Formel I.3, mit | |
|---|---|
| R⁶ = | Wasserstoff; |
| R⁵ = | Cyanomethyl; |
| R⁴ = | jeweils eine Zeile der Tabelle A. |

**Tabelle 52**

| Verbindungen der Formel I.4, mit | |
|---|---|
| R⁶ = | Wasserstoff; |
| R⁵ = | Cyanomethyl; |
| R⁴ = | jeweils eine Zeile der Tabelle A. |

**Tabelle 53**

| Verbindungen der Formel I.1, mit | |
|---|---|
| R⁶ = | Wasserstoff; |
| R⁵ = | Methoxymethyl; |
| R⁴ = | jeweils eine Zeile der Tabelle A. |

**Tabelle 54**

| Verbindungen der Formel I.2, mit | |
|---|---|
| R⁶ = | Wasserstoff; |
| R⁵ = | Methoxymethyl; |
| R⁴ = | jeweils eine Zeile der Tabelle A. |

**Tabelle 55**

| Verbindungen der Formel I.3, mit | |
|---|---|
| R⁶ = | Wasserstoff; |
| R⁵ = | Methoxymethyl; |
| R⁴ = | jeweils eine Zeile der Tabelle A. |

**Tabelle 56**

| Verbindungen der Formel I.4, mit | |
|---|---|
| R⁶ = | Wasserstoff; |
| R⁵ = | Methoxymethyl; |
| R⁴ = | jeweils eine Zeile der Tabelle A. |

**Tabelle 57**

| Verbindungen der Formel I.1, mit | |
|---|---|
| R⁶ = | Wasserstoff; |
| R⁵ = | Ethoxymethyl; |
| R⁴ = | jeweils einer Zeile der Tabelle A. |

**Tabelle 58**

| Verbindungen der Formel I.2, mit | |
|---|---|
| R⁶ = | Wasserstoff; |
| R⁵ = | Ethoxymethyl; |
| R⁴ = | jeweils eine Zeile der Tabelle A. |

**Tabelle 59**

| Verbindungen der Formel I.3, mit | |
|---|---|
| R⁶ = | Wasserstoff; |
| R⁵ = | Ethoxymethyl; |
| R⁴ = | jeweils eine Zeile der Tabelle A. |

**Tabelle 60**

| Verbindungen der Formel I.4, mit | |
|---|---|
| R⁶ = | Wasserstoff; |
| R⁵ = | Ethoxymethyl; |
| R⁴ = | jeweils eine Zeile der Tabelle A. |

**Tabelle 61**

| Verbindungen der Formel I.1, mit | |
|---|---|
| R⁶ = | Wasserstoff; |
| R⁵ = | Isopropoxymethyl; |
| R⁴ = | jeweils eine Zeile der Tabelle A. |

**Tabelle 62**

| Verbindungen der Formel I.2, mit | |
|---|---|
| R⁶ = | Wasserstoff; |
| R⁵ = | Isopropoxymethyl; |
| R⁴ = | jeweils eine Zeile der Tabelle A. |

**Tabelle 63**

| Verbindungen der Formel I.3, mit | |
|---|---|
| R⁶ = | Wasserstoff; |
| R⁵ = | Isopropoxymethyl; |
| R⁴ = | jeweils eine Zeile der Tabelle A. |

**Tabelle 64**

| Verbindungen der Formel I.4, mit | |
|---|---|
| R⁶ = | Wasserstoff; |
| R⁵ = | Isopropoxymethyl; |
| R⁴ = | jeweils eine Zeile der Tabelle A. |

**Tabelle 65**

| Verbindungen der Formel I.1, mit | |
|---|---|
| R⁶ = | Wasserstoff; |
| R⁵ = | Cyclopropylmethyl; |
| R⁴ = | jeweils eine Zeile der Tabelle A. |

**Tabelle 66**

| Verbindungen der Formel I.2, mit | |
|---|---|
| R⁶ = | Wasserstoff; |
| R⁵ = | Cyclopropylmethyl; |
| R⁴ = | jeweils eine Zeile der Tabelle A. |

**Tabelle 67**

| Verbindungen der Formel I.3, mit | |
|---|---|
| R⁶ = | Wasserstoff; |
| R⁵ = | Cyclopropylmethyl; |
| R⁴ = | jeweils eine Zeile der Tabelle A. |

**Tabelle 68**

| Verbindungen der Formel I.4, mit | |
|---|---|
| R⁶ = | Wasserstoff; |
| R⁵ = | Cyclopropylmethyl; |
| R⁴ = | jeweils eine Zeile der Tabelle A. |

**Tabelle 69**

| Verbindungen der Formel I.1, mit | |
|---|---|
| R⁶ = | Wasserstoff; |
| R⁵ = | Phenoxymethyl; |
| R⁴ = | jeweils eine Zeile der Tabelle A. |

**Tabelle 70**

| Verbindungen der Formel I.2, mit | |
|---|---|
| R⁶ = | Wasserstoff; |
| R⁵ = | Phenoxymethyl; |
| R⁴ = | jeweils eine Zeile der Tabelle A. |

**Tabelle 71**

| Verbindungen der Formel I.3, mit | |
|---|---|
| R⁶ = | Wasserstoff; |
| R⁵ = | Phenoxymethyl; |
| R⁴ = | jeweils eine Zeile der Tabelle A. |

**Tabelle 72**

| Verbindungen der Formel I.4, mit | |
|---|---|
| R⁶ = | Wasserstoff; |
| R⁵ = | Phenoxymethyl; |
| R⁴ = | jeweils eine Zeile der Tabelle A. |

**Tabelle 73**

| Verbindungen der Formel I.1, mit | |
|---|---|
| R⁶ = | Wasserstoff; |
| R⁵ = | 2-Chlorphenoxymethyl; |
| R⁴ = | jeweils eine Zeile der Tabelle A. |

**Tabelle 74**

| Verbindungen der Formel I.2, mit | |
|---|---|
| R⁶ = | Wasserstoff; |
| R⁵ = | 2-Chlorphenoxymethyl; |
| R⁴ = | jeweils eine Zeile der Tabelle A. |

**Tabelle 75**

| Verbindungen der Formel I.3, mit | |
|---|---|
| R⁶ = | Wasserstoff; |
| R⁵ = | 2-Chlorphenoxymethyl; |
| R4 = | jeweils eine Zeile der Tabelle A. |

**Tabelle 76**

| Verbindungen der Formel I.4, mit | |
|---|---|
| R⁶ = | Wasserstoff; |
| R⁵ = | 2-Chlorphenoxymethyl; |
| R⁴ = | jeweils eine Zeile der Tabelle A. |

**Tabelle 77**

| Verbindungen der Formel I.1, mit | |
|---|---|
| R⁶ = | Wasserstoff; |
| R⁵ = | 3-Chlorphenoxymethyl; |
| R⁴ = | jeweils eine Zeile der Tabelle A. |

**Tabelle 78**

| Verbindungen der Formel I.2, mit | |
|---|---|
| R⁶ = | Wasserstoff; |
| R⁵ = | 3-Chlorphenoxymethylf; |
| R⁴ = | jeweils eine Zeile der Tabelle A. |

**Tabelle 79**

| Verbindungen der Formel I.3, mit | |
|---|---|
| R⁶ = | Wasserstoff; |
| R⁵ = | 3-Chlorphenoxymethyl; |
| R⁴ = | jeweils eine Zeile der Tabelle A. |

**Tabelle 80**

| Verbindungen der Formel I.4, mit | |
|---|---|
| R⁶ = | Wasserstoff; |
| R⁵ = | 3-Chlorphenoxymethyl; |
| R⁴ = | jeweils eine Zeile der Tabelle A. |

**Tabelle 81**

| Verbindungen der Formel I.1, mit | |
|---|---|
| R⁶ = | Wasserstoff; |
| R⁵ = | 4-Chlorphenoxymethyl; |
| R⁴ = | jeweils eine Zeile der Tabelle A. |

**Tabelle 82**

| Verbindungen der Formel I.2, mit | |
|---|---|
| R⁶ = | Wasserstoff; |
| R⁵ = | 4-Chlorphenoxymethyl; |
| R⁴ = | jeweils eine Zeile der Tabelle A. |

**Tabelle 83**

| Verbindungen der Formel I.3, mit | |
|---|---|
| R⁶ = | Wasserstoff; |
| R⁵ = | 4-Chlorphenoxymethyl; |
| R⁴ = | jeweils eine Zeile der Tabelle A. |

**Tabelle 84**

| Verbindungen der Formel I.4, mit | |
|---|---|
| R⁶ = | Wasserstoff; |
| R⁵ = | 4-Chlorphenoxymethyl; |
| R⁴ = | jeweils eine Zeile der Tabelle A. |

**Tabelle 85**

| Verbindungen der Formel I.1, mit | |
|---|---|
| R⁶ = | Wasserstoff; |
| R⁵ = | 2-Cyanophenoxymethyl; |
| R⁴ = | jeweils eine Zeile der Tabelle A. |

**Tabelle 86**

| Verbindungen der Formel I.2, mit | |
|---|---|
| R⁶ = | Wasserstoff; |
| R⁵ = | 2-Cyanophenoxymethyl; |
| R⁴ = | jeweils eine Zeile der Tabelle A. |

**Tabelle 87**

| Verbindungen der Formel I.3, mit | |
|---|---|
| R⁶ = | Wasserstoff; |
| R⁵ = | 2-Cyanophenoxymethyl; |
| R⁴ = | jeweils eine Zeile der Tabelle A. |

**Tabelle 88**

| Verbindungen der Formel I.4, mit | |
|---|---|
| R⁶ = | Wasserstoff; |
| R⁵ = | 2-Cyanophenoxymethyl; |
| R⁴ = | jeweils eine Zeile der Tabelle A. |

**Tabelle 89**

| Verbindungen der Formel I.1, mit | |
|---|---|
| R⁶ = | Wasserstoff; |
| R⁵ = | 4-Methylphenoxymethyl; |
| R⁴ = | jeweils eine Zeile der Tabelle A. |

**Tabelle 90**

| Verbindungen der Formel I.2, mit | |
|---|---|
| R⁶ = | Wasserstoff; |
| R⁵ = | 4-Methylphenoxymethyl; |
| R⁴ = | jeweils eine Zeile der Tabelle A. |

**Tabelle 91**

| Verbindungen der Formel I.3, mit | |
|---|---|
| R⁶ = | Wasserstoff; |
| R⁵ = | 4-Methylphenoxymethyl; |
| R⁴ = | jeweils eine Zeile der Tabelle A. |

**Tabelle 92**

| Verbindungen der Formel I.4, mit | |
|---|---|
| R⁶ = | Wasserstoff; |
| R⁵ = | 4-Methylphenoxymethyl; |
| R⁴ = | jeweils eine Zeile der Tabelle A. |

**Tabelle 93**

| Verbindungen der Formel I.1, mit | |
|---|---|
| R⁶ = | Wasserstoff; |
| R⁵ = | 3-Methoxyphenoxymethyl; |
| R⁴ = | jeweils eine Zeile der Tabelle A. |

**Tabelle 94**

| Verbindungen der Formel I.2, mit | |
|---|---|
| R⁶ = | Wasserstoff; |
| R⁵ = | 3-Methoxyphenoxymethyl; |
| R⁴ = | jeweils eine Zeile der Tabelle A. |

**Tabelle 95**

| Verbindungen der Formel I.3, mit | |
|---|---|
| R⁶ = | Wasserstoff; |
| R⁵ = | 3-Methoxyphenoxymethyl; |
| R⁴ = | jeweils eine Zeile der Tabelle A. |

**Tabelle 96**

| Verbindungen der Formel I.4, mit | |
|---|---|
| R⁶ = | Wasserstoff; |
| R⁵ = | 3-Methoxyphenoxymethyl; |
| R⁴ = | jeweils eine Zeile der Tabelle A. |

**Tabelle 97**

| Verbindungen der Formel I.1, mit | |
|---|---|
| R⁶ = | Wasserstoff; |
| R⁵ = | 4-Trifluormethylphenoxymethyl; |
| R⁴ = | jeweils eine Zeile der Tabelle A. |

**Tabelle 98**

| Verbindungen der Formel I.2, mit | |
|---|---|
| R⁶ = | Wasserstoff; |
| R⁵ = | 4-Trifluormethylphenoxymethyl; |
| R⁴ = | jeweils eine Zeile der Tabelle A. |

**Tabelle 99**

| Verbindungen der Formel I.3, mit | |
|---|---|
| R⁶ = | Wasserstoff; |
| R⁵ = | 4-Trifluormethylphenoxymethyl; |
| R⁴ = | jeweils eine Zeile der Tabelle A. |

**Tabelle 100**

| Verbindungen der Formel I.4, mit | |
|---|---|
| R⁶ = | Wasserstoff; |
| R⁵ = | 4-Trifluormethylphenoxymethyl; |
| R⁴ = | jeweils eine Zeile der Tabelle A. |

**Tabelle 101**

| Verbindungen der Formel I.1, mit | |
|---|---|
| R⁶ = | Wasserstoff; |
| R⁵ = | 2-(3-Nitrophenoxy)eth-l-yl; |
| R⁴ = | jeweils eine Zeile der Tabelle A. |

**Tabelle 102**

| Verbindungen der Formel I.2, mit | |
|---|---|
| R⁶ = | Wasserstoff; |
| R⁵ = | 2-(3-Nitrophenoxy)eth-1-yl; |
| R⁴ = | jeweils eine Zeile der Tabelle A. |

**Tabelle 103**

| Verbindungen der Formel I.3, mit | |
|---|---|
| R⁶ = | Wasserstoff; |
| R⁵ = | 2-(3-Nitrophenoxy)eth-1-yl; |
| R⁴ = | jeweils eine Zeile der Tabelle A. |

**Tabelle 104**

| Verbindungen der Formel I.4, mit | |
|---|---|
| R⁶ = | Wasserstoff; |
| R⁵ = | 2-(3-Nitrophenoxy)eth-1-yl; |
| R⁴ = | jeweils eine Zeile der Tabelle A. |

**Tabelle 105**

| Verbindungen der Formel I.1, mit | |
|---|---|
| R⁶ = | Wasserstoff; |
| R⁵ = | 2-Pyridyloxymethyl; |
| R⁴ = | jeweils eine Zeile der Tabelle A. |

**Tabelle 106**

| Verbindungen der Formel I.2, mit | |
|---|---|
| R⁶ = | Wasserstoff; |
| R⁵ = | 2-Pyridyloxymethyl; |
| R⁴ = | jeweils eine Zeile der Tabelle A. |

**Tabelle 107**

| Verbindungen der Formel I.3, mit | |
|---|---|
| R⁶ = | Wasserstoff; |
| R⁵ = | 2-Pyridyloxymethyl; |
| R⁴ = | jeweils eine Zeile der Tabelle A. |

**Tabelle 108**

| Verbindungen der Formel I.4, mit | |
|---|---|
| R⁶ = | Wasserstoff; |
| R⁵ = | 2-Pyridyloxymethyl; |
| R⁴ = | jeweils eine Zeile der Tabelle A. |

**Tabelle 109**

| Verbindungen der Formel I.1, mit | |
|---|---|
| R⁶ = | Wasserstoff; |
| R⁵ = | 4 -Chlorpyrimidin-6-yloxymethyl; |
| R⁴ = | jeweils eine Zeile der Tabelle A. |

**Tabelle 110**

| Verbindungen der Formel I.2, mit | |
|---|---|
| R⁶ = | Wasserstoff; |
| R⁵ = | 4-Chlorpyrimidin-6-yloxymethyl; |
| R⁴ = | jeweils eine Zeile der Tabelle A. |

**Tabelle 111**

| Verbindungen der Formel I.3, mit | |
|---|---|
| R⁶ = | Wasserstoff; |
| R⁵ = | 4-Chlorpyrimidin-6-yloxymethyl; |
| R⁴ = | jeweils eine Zeile der Tabelle A. |

**Tabelle 112**

| Verbindungen der Formel I.4, mit | |
|---|---|
| R⁶ = | Wasserstoff; |
| R⁵ = | 4-Chlorpyrimidin-6-yloxymethyl; |
| R⁴ = | jeweils eine Zeile der Tabelle A. |

**Tabelle 113**

| Verbindungen der Formel I.1, mit | |
|---|---|
| R⁶ = | Wasserstoff; |
| R⁵ = | 5-Methylisoxazol-3-yloxymethyl; |
| R⁴ = | jeweils einer Zeile der Tabelle A. |

**Tabelle 114**

| Verbindungen der Formel I.2, mit | |
|---|---|
| R⁶ = | Wasserstoff; |
| R⁵ = | 5-Methylisoxazol-3-yloxymethyl; |
| R⁴ = | jeweils eine Zeile der Tabelle A. |

**Tabelle 115**

| Verbindungen der Formel I.3, mit | |
|---|---|
| R⁶ = | Wasserstoff; |
| R⁵ = | 5-Methylisoxazol-3-yloxymethyl; |
| R⁴ = | jeweils eine Zeile der Tabelle A. |

**Tabelle 116**

| Verbindungen der Formel I.4, mit | |
|---|---|
| R⁶ = | Wasserstoff; |
| R⁵ = | 5-Methylisoxazol-3-yloxymethyl; |
| R⁴ = | jeweils eine Zeile der Tabelle A. |

**Tabelle 117**

| Verbindungen der Formel I.1, mit | |
|---|---|
| R⁶ = | Wasserstoff; |
| R⁵ = | 1-Phenylpyrazol-3-yloxymethyl; |
| R⁴ = | jeweils eine Zeile der Tabelle A. |

**Tabelle 118**

| Verbindungen der Formel I.2, mit | |
|---|---|
| R⁶ = | Wasserstoff; |
| R⁵ = | 1-Phenylpyrazol-3-yloxymethyl; |
| R⁴ = | jeweils eine Zeile der Tabelle A. |

**Tabelle 119**

| Verbindungen der Formel I.3, mit | |
|---|---|
| R⁶ = | Wasserstoff; |
| R⁵ = | 1-Phenylpyrazol-3-yloxymethyl; |
| R⁴ = | jeweils eine Zeile der Tabelle A. |

**Tabelle 120**

| Verbindungen der Formel I.4, mit | |
|---|---|
| R⁶ = | Wasserstoff; |
| R⁵ = | 1-Phenylpyrazol-3-yloxymethyl; |
| R⁴ = | jeweils eine Zeile der Tabelle A. |

**Tabelle 121**

| Verbindungen der Formel I.1, mit | |
|---|---|
| R⁶ = | Wasserstoff; |
| R⁵ = | 3-Chlorpyrid-6-yloxymethyl; |
| R⁴ = | jeweils eine Zeile der Tabelle A. |

**Tabelle 122**

| Verbindungen der Formel I.2, mit | |
|---|---|
| R⁶ = | Wasserstoff; |
| R⁵ = | 3-Chlorpyrid-6-yloxymethyl; |
| R⁴ = | jeweils eine Zeile der Tabelle A. |

**Tabelle 123**

| Verbindungen der Formel I.3, mit | |
|---|---|
| R⁶ = | Wasserstoff; |
| R⁵ = | 3-Chlorpyrid-6-yloxymethyl; |
| R⁴ = | jeweils eine Zeile der Tabelle A. |

**Tabelle 124**

| Verbindungen der Formel I.4, mit | |
|---|---|
| R⁶ = | Wasserstoff; |
| R⁵ = | 3-Chlorpyrid-6-yloxymethyl; |
| R⁴ = | jeweils eine Zeile der Tabelle A. |

**Tabelle 125**

| Verbindungen der Formel I.1, mit | |
|---|---|
| R⁶ = | Wasserstoff; |
| R⁵ = | 2-iso-Propyloxazol-4-yloxymethyl; |
| R⁴ = | jeweils eine Zeile der Tabelle A. |

**Tabelle 126**

| Verbindungen der Formel I.2, mit | |
|---|---|
| R⁶ = | Wasserstoff; |
| R⁵ = | 2-iso-Propyloxazol-4-yloxymethyl; |
| R⁴ = | jeweils eine Zeile der Tabelle A. |

**Tabelle 127**

| Verbindungen der Formel I.3, mit | |
|---|---|
| R⁶ = | Wasserstoff; |
| R⁵ = | 2-iso-Propyloxazol-4-yloxymethyl; |
| R⁴ = | jeweils eine Zeile der Tabelle A. |

**Tabelle 128**

| Verbindungen der Formel I.4, mit | |
|---|---|
| R⁶ = | Wasserstoff; |
| R⁵ = | 2-iso-Propyloxazol-4-yloxymethyl; |
| R⁴ = | jeweils eine Zeile der Tabelle A. |

**Tabelle 129**

| Verbindungen der Formel I.1, mit | |
|---|---|
| R⁶ = | Wasserstoff; |
| R⁵ = | 3-Pyridazinyloxymethyl; |
| R⁴ = | jeweils eine Zeile der Tabelle A. |

**Tabelle 130**

| Verbindungen der Formel I.2, mit | |
|---|---|
| R⁶ = | Wasserstoff; |
| R⁵ = | 3-Pyridazinyloxymethyl; |
| R⁴ = | jeweils eine Zeile der Tabelle A. |

**Tabelle 131**

| Verbindungen der Formel I.3, mit | |
|---|---|
| R⁶ = | Wasserstoff; |
| R⁵ = | 3-Pyridazinyloxymethyl; |
| R⁴ = | jeweils eine Zeile der Tabelle A. |

**Tabelle 132**

| Verbindungen der Formel I.4, mit | |
|---|---|
| R⁶ = | Wasserstoff; |
| R⁵ = | 3-Pyridazinyloxymethyl; |
| R⁴ = | jeweils eine Zeile der Tabelle A. |

**Tabelle 133**

| Verbindungen der Formel I.1, mit | |
|---|---|
| R⁶ = | Wasserstoff; |
| R⁵ = | 5-Methylpyrimidin-2-yloxymethyl; |
| R⁴ = | jeweils eine Zeile der Tabelle A. |

**Tabelle 134**

| Verbindungen der Formel I.2, mit | |
|---|---|
| R⁶ = | Wasserstoff; |
| R⁵ = | 5-Methylpyrimidin-2-yloxymethyl; |
| R⁴ = | jeweils eine Zeile der Tabelle A. |

**Tabelle 135**

| Verbindungen der Formel I.3, mit | |
|---|---|
| R⁶ = | Wasserstoff; |
| R⁵ = | 5-Methylpyrimidin-2-yloxymethyl; |
| R⁴ = | jeweils eine Zeile der Tabelle A. |

**Tabelle 136**

| Verbindungen der Formel I.4, mit | |
|---|---|
| R⁶ = | Wasserstoff; |
| R⁵ = | 5-Methylpyrimidin-2-yloxymethyl; |
| R⁴ = | jeweils eine Zeile der Tabelle A. |

**Tabelle 137**

| Verbindungen der Formel I.1, mit | |
|---|---|
| R⁶ = | Wasserstoff; |
| R⁵ = | Ethenyl; |
| R⁴ = | jeweils eine Zeile der Tabelle A. |

**Tabelle 138**

| Verbindungen der Formel I.2, mit | |
|---|---|
| R⁶ = | Wasserstoff; |
| R⁵ = | Ethenyl; |
| R⁴ = | jeweils eine Zeile der Tabelle A. |

**Tabelle 139**

| Verbindungen der Formel I.3, mit | |
|---|---|
| R⁶ = | Wasserstoff; |
| R⁵ = | Ethenyl; |
| R⁴ = | jeweils eine Zeile der Tabelle A. |

**Tabelle 140**

| Verbindungen der Formel I.4, mit | |
|---|---|
| R⁶ = | Wasserstoff; |
| R⁵ = | Ethenyl; |
| R⁴ = | jeweils eine Zeile der Tabelle A. |

**Tabelle 141**

| Verbindungen der Formel I.1, mit | |
|---|---|
| R⁶ = | Wasserstoff; |
| R⁵ = | Prop-2-en-1-yl; |
| R⁴ = | jeweils eine Zeile der Tabelle A. |

**Tabelle 142**

| Verbindungen der Formel I.2, mit | |
|---|---|
| R⁶ = | Wasserstoff; |
| R⁵ = | Prop-2-en-1-yl; |
| R⁴ = | jeweils eine Zeile der Tabelle A. |

**Tabelle 143**

| Verbindungen der Formel 1.3, mit | |
|---|---|
| R⁶ = | Wasserstoff; |
| R⁵ = | Prop-2-en-1-yl; |
| R⁴ = | jeweils eine Zeile der Tabelle A. |

**Tabelle 144**

| Verbindungen der Formel I.4, mit | |
|---|---|
| R⁶ = | Wasserstoff; |
| R⁵ = | Prop-2-en-1-yl; |
| R⁴ = | jeweils eine Zeile der Tabelle A. |

**Tabelle 145**

| Verbindungen der Formel I.1, mit | |
|---|---|
| R⁶ = | Wasserstoff; |
| R⁵ = | E-3-Chlorprop-2-en-l-yl; |
| R⁴ = | jeweils eine Zeile der Tabelle A. |

**Tabelle 146**

| Verbindungen der Formel I.2, mit | |
|---|---|
| R⁶ = | Wasserstoff; |
| R⁵ = | E-3-Chlorprop-2-en-1-yl; |
| R⁴ = | jeweils eine Zeile der Tabelle A. |

**Tabelle 147**

| Verbindungen der Formel I.3, mit | |
|---|---|
| R⁶ = | Wasserstoff; |
| R⁵ = | E-3-Chlorprop-2-en-1-yl; |
| R⁴ = | jeweils eine Zeile der Tabelle A. |

**Tabelle 148**

| Verbindungen der Formel I.4, mit | |
|---|---|
| R⁶ = | Wasserstoff; |
| R⁵ = | E-3-Chlorprop-2-en-1-yl; |
| R⁴ = | jeweils eine Zeile der Tabelle A. |

**Tabelle 149**

| Verbindungen der Formel I.1, mit | |
|---|---|
| R⁶ = | Wasserstoff; |
| R⁵ = | E-But-2-en-1-yl; |
| R⁴ = | jeweils eine Zeile der Tabelle A. |

**Tabelle 150**

| Verbindungen der Formel I.2, mit | |
|---|---|
| R⁶ = | Wasserstoff; |
| R⁵ = | E-But-2-en-1-yl; |
| R⁴ = | jeweils eine Zeile der Tabelle A. |

**Tabelle 151**

| Verbindungen der Formel I.3, mit | |
|---|---|
| R⁶ = | Wasserstoff; |
| R⁵ = | E-But-2-en-1-yl; |
| R⁴ = | jeweils eine Zeile der Tabelle A. |

**Tabelle 152**

| Verbindungen der Formel I.4, mit | |
|---|---|
| R⁶ = | Wasserstoff; |
| R⁵ = | E-But-2-en-1-yl; |
| R⁴ = | jeweils eine Zeile der Tabelle A. |

**Tabelle 153**

| Verbindungen der Formel I.1, mit | |
|---|---|
| R⁶ = | Wasserstoff; |
| R⁵ = | Ethinyl; |
| R⁴ = | jeweils eine Zeile der Tabelle A. |

**Tabelle 154**

| Verbindungen der Formel I.2, mit | |
|---|---|
| R⁶ = | Wasserstoff; |
| R⁵ = | Ethinyl; |
| R⁴ = | jeweils eine Zeile der Tabelle A. |

**Tabelle 155**

| Verbindungen der Formel I.3, mit | |
|---|---|
| R⁶ = | Wasserstoff; |
| R⁵ = | Ethinyl; |
| R⁴ = | jeweils eine Zeile der Tabelle A. |

**Tabelle 156**

| Verbindungen der Formel I.4, mit | |
|---|---|
| R⁶ = | Wasserstoff; |
| R⁵ = | Ethinyl; |
| R⁴ = | jeweils eine Zeile der Tabelle A. |

**Tabelle 157**

| Verbindungen der Formel I.1, mit | |
|---|---|
| R⁶ = | Wasserstoff; |
| R⁵ = | Prop-2-in-1-yl; |
| R⁴ = | jeweils eine Zeile der Tabelle A. |

**Tabelle 158**

| Verbindungen der Formel I.2, mit | |
|---|---|
| R⁶ = | Wasserstoff; |
| R⁵ = | Prop-2-in-1-yl; |
| R⁴ = | jeweils eine Zeile der Tabelle A. |

**Tabelle 159**

| Verbindungen der Formel I.3, mit | |
|---|---|
| R⁶ = | Wasserstoff; |
| R⁵ = | Prop-2-in-1-yl; |
| R⁴ = | jeweils eine Zeile der Tabelle A. |

**Tabelle 160**

| Verbindungen der Formel I.4, mit | |
|---|---|
| R⁶ = | Wasserstoff; |
| R⁵ = | Prop-2-in-1-yl; |
| R⁴ = | jeweils eine Zeile der Tabelle A. |

**Tabelle 161**

| Verbindungen der Formel I.1, mit | |
|---|---|
| R⁶ = | Wasserstoff; |
| R⁵ = | Prop-1-in-1-yl; |
| R⁴ = | jeweils eine Zeile der Tabelle A. |

**Tabelle 162**

| Verbindungen der Formel I.2, mit | |
|---|---|
| R⁶ = | Wasserstoff; |
| R⁵ = | Prop-1-in-1-yl; |
| R⁴ = | jeweils eine Zeile der Tabelle A. |

**Tabelle 163**

| Verbindungen der Formel I.3, mit | |
|---|---|
| R⁶ = | Wasserstoff; |
| R⁵ = | Prop-1-in-1-yl; |
| R⁴ = | jeweils eine Zeile der Tabelle A. |

**Tabelle 164**

| Verbindungen der Formel I.4, mit | |
|---|---|
| R⁶ = | Wasserstoff; |
| R⁵ = | Prop-1-in-1-yl; |
| R⁴ = | jeweils eine Zeile der Tabelle A. |

**Tabelle 165**

| Verbindungen der Formel I.1, mit | |
|---|---|
| R⁶ = | Wasserstoff; |
| R⁵ = | Phenylethinyl; |
| R⁴ = | jeweils eine Zeile der Tabelle A. |

**Tabelle 166**

| Verbindungen der Formel I.2, mit | |
|---|---|
| R⁶ = | Wasserstoff; |
| R⁵ = | Phenylethinyl; |
| R⁴ = | jeweils eine Zeile der Tabelle A. |

**Tabelle 167**

| Verbindungen der Formel I.3, mit | |
|---|---|
| R⁶ = | Wasserstoff; |
| R⁵ = | Phenylethinyl; |
| R⁴ = | jeweils eine Zeile der Tabelle A. |

**Tabelle 168**

| Verbindungen der Formel I.4, mit | |
|---|---|
| R⁶ = | Wasserstoff; |
| R⁵ = | Phenylethinyl; |
| R⁴ = | jeweils eine Zeile der Tabelle A. |

**Tabelle 169**

| Verbindungen der Formel I.1, mit | |
|---|---|
| R⁶ = | Wasserstoff; |
| R⁵ = | Oxiran-2-yl; |
| R⁴ = | jeweils einer Zeile der Tabelle A. |

**Tabelle 170**

| Verbindungen der Formel I.2, mit | |
|---|---|
| R⁶ = | Wasserstoff; |
| R⁵ = | Oxiran-2-yl; |
| R⁴ = | jeweils eine Zeile der Tabelle A. |

**Tabelle 171**

| Verbindungen der Formel I.3, mit | |
|---|---|
| R⁶ = | Wasserstoff; |
| R⁵ = | Oxiran-2-yl; |
| R⁴ = | jeweils eine Zeile der Tabelle A. |

**Tabelle 172**

| Verbindungen der Formel I.4, mit | |
|---|---|
| R⁶ = | Wasserstoff; |
| R⁵ = | Oxiran-2-yl; |
| R⁴ = | jeweils eine Zeile der Tabelle A. |

**Tabelle 173**

| Verbindungen der Formel I.1, mit | |
|---|---|
| R⁶ = | Wasserstoff; |
| R⁵ = | 1,1-Dichlorcyclopropyl; |
| R⁴ = | jeweils eine Zeile der Tabelle A. |

**Tabelle 174**

| Verbindungen der Formel I.2, mit | |
|---|---|
| R⁶ = | Wasserstoff; |
| R⁵ = | 1,1-Dichlorcyclopropyl; |
| R⁴ = | jeweils eine Zeile der Tabelle A. |

**Tabelle 175**

| Verbindungen der Formel I.3, mit | |
|---|---|
| R⁶ = | Wasserstoff; |
| R⁵ = | 1,1-Dichlorcyclopropyl; |
| R⁴ = | jeweils eine Zeile der Tabelle A. |

**Tabelle 176**

| Verbindungen der Formel I.4, mit | |
|---|---|
| R⁶ = | Wasserstoff; |
| R⁵ = | 1,1-Dichlorcyclopropyl; |
| R⁴ = | jeweils eine Zeile der Tabelle A. |

**Tabelle 177**

| Verbindungen der Formel I.1, mit | |
|---|---|
| R⁶ = | Wasserstoff; |
| R⁵ = | Phenyl; |
| R⁴ = | jeweils eine Zeile der Tabelle A. |

**Tabelle 178**

| Verbindungen der Formel I.2, mit | |
|---|---|
| R⁶ = | Wasserstoff; |
| R⁵ = | Phenyl; |
| R⁴ = | jeweils eine Zeile der Tabelle A. |

**Tabelle 179**

| Verbindungen der Formel I.3, mit | |
|---|---|
| R⁶ = | Wasserstoff; |
| R⁵ = | Phenyl; |
| R⁴ = | jeweils eine Zeile der Tabelle A. |

**Tabelle 180**

| Verbindungen der Formel I.4, mit | |
|---|---|
| R⁶ = | Wasserstoff; |
| R⁵ = | Phenyl; |
| R⁴ = | jeweils eine Zeile der Tabelle A. |

**Tabelle 181**

| Verbindungen der Formel I.1, mit | |
|---|---|
| R⁶ = | Wasserstoff; |
| R⁵ = | 2-Naphthyl; |
| R⁴ = | jeweils eine Zeile der Tabelle A. |

**Tabelle 182**

| Verbindungen der Formel I.2, mit | |
|---|---|
| R⁶ = | Wasserstoff; |
| R⁵ = | 2-Naphthyl; |
| R⁴ = | jeweils eine Zeile der Tabelle A. |

**Tabelle 183**

| Verbindungen der Formel I.3, mit | |
|---|---|
| R⁶ = | Wasserstoff; |
| R⁵ = | 2-Naphthyl; |
| R⁴ = | jeweils eine Zeile der Tabelle A. |

**Tabelle 184**

| Verbindungen der Formel I.4, mit | |
|---|---|
| R⁶ = | Wasserstoff; |
| R⁵ = | 2-Naphthyl; |
| R⁴ = | jeweils eine Zeile der Tabelle A. |

**Tabelle 185**

| Verbindungen der Formel I.1, mit | |
|---|---|
| R⁶ = | Wasserstoff; |
| R⁵ = | 2-Chlorphenyl; |
| R⁴ = | jeweils eine Zeile der Tabelle A. |

**Tabelle 186**

| Verbindungen der Formel I.2, mit | |
|---|---|
| R⁶ = | Wasserstoff; |
| R⁵ = | 2-Chlorphenyl; |
| R⁴ = | jeweils eine Zeile der Tabelle A. |

**Tabelle 187**

| Verbindungen der Formel I.3, mit | |
|---|---|
| R⁶ = | Wasserstoff; |
| R⁵ = | 2-Chlorphenyl; |
| R⁴ = | jeweils eine Zeile der Tabelle A. |

**Tabelle 188**

| Verbindungen der Formel I.4, mit | |
|---|---|
| R⁶ = | Wasserstoff; |
| R⁵ = | 2-Chlorphenyl; |
| R⁴ = | jeweils eine Zeile der Tabelle A. |

**Tabelle 189**

| Verbindungen der Formel I.1, mit | |
|---|---|
| R⁶ = | Wasserstoff; |
| R⁵ = | 3-Chlorphenyl; |
| R⁴ = | jeweils eine Zeile der Tabelle A. |

**Tabelle 190**

| Verbindungen der Formel I.2, mit | |
|---|---|
| R⁶ = | Wasserstoff; |
| R⁵ = | 3-Chlorphenyl; |
| R⁴ = | jeweils eine Zeile der Tabelle A. |

**Tabelle 191**

| Verbindungen der Formel I.3, mit | |
|---|---|
| R⁶ = | Wasserstoff; |
| R⁵ = | 3-Chlorphenyl; |
| R⁴ = | jeweils eine Zeile der Tabelle A. |

**Tabelle 192**

| Verbindungen der Formel I.4, mit | |
|---|---|
| R⁶ = | Wasserstoff; |
| R⁵ = | 3-Chlorphenyl; |
| R⁴ = | jeweils eine Zeile der Tabelle A. |

**Tabelle 193**

| Verbindungen der Formel I.1, mit | |
|---|---|
| R⁶ = | Wasserstoff; |
| R⁵ = | 4-Chlorphenyl; |
| R⁴ = | jeweils eine Zeile der Tabelle A. |

**Tabelle 194**

| Verbindungen der Formel I.2, mit | |
|---|---|
| R⁶ = | Wasserstoff; |
| R⁵ = | 4-Chlorphenyl; |
| R⁴ = | jeweils eine Zeile der Tabelle A. |

**Tabelle 195**

| Verbindungen der Formel I.3, mit | |
|---|---|
| R⁶ = | Wasserstoff; |
| R⁵ = | 4-Chlorphenyl; |
| R⁴ = | jeweils eine Zeile der Tabelle A. |

**Tabelle 196**

| Verbindungen der Formel I.4, mit | |
|---|---|
| R⁶ = | Wasserstoff; |
| R⁵ = | 4-Chlorphenyl; |
| R⁴ = | jeweils eine Zeile der Tabelle A. |

**Tabelle 197**

| Verbindungen der Formel I.1, mit | |
|---|---|
| R⁶ = | Wasserstoff; |
| R⁵ = | 2-Nitrophenyl; |
| R⁴ = | jeweils eine Zeile der Tabelle A. |

**Tabelle 198**

| Verbindungen der Formel I.2, mit | |
|---|---|
| R⁶ = | Wasserstoff; |
| R⁵ = | 2-Nitrophenyl; |
| R⁴ = | jeweils eine Zeile der Tabelle A. |

**Tabelle 199**

| Verbindungen der Formel I.3, mit | |
|---|---|
| R⁶ = | Wasserstoff; |
| R⁵ = | 2-Nitrophenyl; |
| R⁴ = | jeweils eine Zeile der Tabelle A. |

**Tabelle 200**

| Verbindungen der Formel I.4, mit | |
|---|---|
| R⁶ = | Wasserstoff; |
| R⁵ = | 2-Nitrophenyl; |
| R⁴ = | jeweils eine Zeile der Tabelle A. |

**Tabelle 201**

| Verbindungen der Formel I.1, mit | |
|---|---|
| R⁶ = | Wasserstoff; |
| R⁵ = | 3-Cyanophenyl; |
| R⁴ = | jeweils eine Zeile der Tabelle A. |

**Tabelle 202**

| Verbindungen der Formel I.2, mit | |
|---|---|
| R⁶ = | Wasserstoff; |
| R⁵ = | 3-Cyanophenyl; |
| R⁴ = | jeweils eine Zeile der Tabelle A. |

**Tabelle 203**

| Verbindungen der Formel I.3, mit | |
|---|---|
| R⁶ = | Wasserstoff; |
| R⁵ = | 3-Cyanophenyl; |
| R⁴ = | jeweils eine Zeile der Tabelle A. |

**Tabelle 204**

| Verbindungen der Formel I.4, mit | |
|---|---|
| R⁶ = | Wasserstoff; |
| R⁵ = | 3-Cyanophenyl; |
| R⁴ = | jeweils eine Zeile der Tabelle A. |

**Tabelle 205**

| Verbindungen der Formel I.1, mit | |
|---|---|
| R⁶ = | Wasserstoff; |
| R⁵ = | 4-Methylphenyl; |
| R⁴ = | jeweils eine Zeile der Tabelle A. |

**Tabelle 206**

| Verbindungen der Formel I.2, mit | |
|---|---|
| R⁶ = | Wasserstoff; |
| R⁵ = | 4-Methylphenyl; |
| R⁴ = | jeweils eine Zeile der Tabelle A. |

**Tabelle 207**

| Verbindungen der Formel I.3, mit | |
|---|---|
| R⁶ = | Wasserstoff; |
| R⁵ = | 4-Methylphenyl; |
| R⁴ = | jeweils eine Zeile der Tabelle A. |

**Tabelle 208**

| Verbindungen der Formel I.4, mit | |
|---|---|
| R⁶ = | Wasserstoff; |
| R⁵ = | 4-Methylphenyl; |
| R⁴ = | jeweils eine Zeile der Tabelle A. |

**Tabelle 209**

| Verbindungen der Formel I.1, mit | |
|---|---|
| R⁶ = | Wasserstoff; |
| R⁵ = | 3-tert.-Butylphenyl; |
| R⁴ = | jeweils eine Zeile der Tabelle A. |

**Tabelle 210**

| Verbindungen der Formel I.2, mit | |
|---|---|
| R⁶ = | Wasserstoff; |
| R⁵ = | 3-tert.-Butylphenyl; |
| R⁴ = | jeweils eine Zeile der Tabelle A. |

**Tabelle 211**

| Verbindungen der Formel I.3, mit | |
|---|---|
| R⁶ = | Wasserstoff; |
| R⁵ = | 3-tert.-Butylphenyl; |
| R⁴ = | jeweils eine Zeile der Tabelle A. |

**Tabelle 212**

| Verbindungen der Formel I.4, mit | |
|---|---|
| R⁶ = | Wasserstoff; |
| R⁵ = | 3-tert.-Butylphenyl; |
| R⁴ = | jeweils eine Zeile der Tabelle A. |

**Tabelle 213**

| Verbindungen der Formel I.1, mit | |
|---|---|
| R⁶ = | Wasserstoff; |
| R⁵ = | 4-Trifluormethylphenyl; |
| R⁴ = | jeweils eine Zeile der Tabelle A. |

**Tabelle 214**

| Verbindungen der Formel I.2, mit | |
|---|---|
| R⁶ = | Wasserstoff; |
| R⁵ = | 4-Trifluormethylphenyl; |
| R⁴ = | jeweils eine Zeile der Tabelle A. |

**Tabelle 215**

| Verbindungen der Formel I.3, mit | |
|---|---|
| R⁶ = | Wasserstoff; |
| R⁵ = | 4-Trifluormethylphenyl; |
| R⁴ = | jeweils eine Zeile der Tabelle A. |

**Tabelle 216**

| Verbindungen der Formel I.4, mit | |
|---|---|
| R⁶ = | Wasserstoff; |
| R⁵ = | 4-Trifluormethylphenyl; |
| R⁴ = | jeweils eine Zeile der Tabelle A. |

**Tabelle 217**

| Verbindungen der Formel I.1, mit | |
|---|---|
| R⁶ = | Wasserstoff; |
| R⁵ = | 2-Methoxyphenyl; |
| R⁴ = | jeweils eine Zeile der Tabelle A. |

**Tabelle 218**

| Verbindungen der Formel I.2, mit | |
|---|---|
| R⁶ = | Wasserstoff; |
| R⁵ = | 2-Methoxyphenyl; |
| R⁴ = | jeweils eine Zeile der Tabelle A. |

**Tabelle 219**

| Verbindungen der Formel I.3, mit | |
|---|---|
| R⁶ = | Wasserstoff; |
| R⁵ = | 2-Methoxyphenyl; |
| R⁴ = | jeweils eine Zeile der Tabelle A. |

**Tabelle 220**

| Verbindungen der Formel I.4, mit | |
|---|---|
| R⁶ = | Wasserstoff; |
| R⁵ = | 2-Methoxyphenyl; |
| R⁴ = | jeweils eine Zeile der Tabelle A. |

**Tabelle 221**

| Verbindungen der Formel I.1, mit | |
|---|---|
| R⁶ = | Wasserstoff; |
| R⁵ = | 2,4-Dichlorphenyl; |
| R⁴ = | jeweils eine Zeile der Tabelle A. |

**Tabelle 222**

| Verbindungen der Formel I.2, mit | |
|---|---|
| R⁶ = | Wasserstoff; |
| R⁵ = | 2,4-Dichlorphenyl; |
| R⁴ = | jeweils eine Zeile der Tabelle A. |

**Tabelle 223**

| Verbindungen der Formel I.3, mit | |
|---|---|
| R⁶ = | Wasserstoff; |
| R⁵ = | 2,4-Dichlorphenyl; |
| R⁴ = | jeweils eine Zeile der Tabelle A. |

**Tabelle 224**

| Verbindungen der Formel I.4, mit | |
|---|---|
| R⁶ = | Wasserstoff; |
| R⁵ = | 2,4-Dichlorphenyl; |
| R⁴ = | jeweils eine Zeile der Tabelle A. |

**Tabelle 225**

| Verbindungen der Formel I.1, mit | |
|---|---|
| R⁶ = | Wasserstoff; |
| R⁵ = | 4-Fluorphenyl; |
| R⁴ = | jeweils einer Zeile der Tabelle A. |

**Tabelle 226**

| Verbindungen der Formel I.2, mit | |
|---|---|
| R⁶ = | Wasserstoff; |
| R⁵ = | 4-Fluorphenyl; |
| R⁴ = | jeweils eine Zeile der Tabelle A. |

**Tabelle 227**

| Verbindungen der Formel I.3, mit | |
|---|---|
| R⁶ = | Wasserstoff; |
| R⁵ = | 4-Fluorphenyl; |
| R⁴ = | jeweils eine Zeile der Tabelle A. |

**Tabelle 228**

| Verbindungen der Formel I.4, mit | |
|---|---|
| R⁶ = | Wasserstoff; |
| R⁵ = | 4-Fluorphenyl; |
| R⁴ = | jeweils eine Zeile der Tabelle A. |

**Tabelle 229**

| Verbindungen der Formel I.1, mit | |
|---|---|
| R⁶ = | Wasserstoff; |
| R⁵ = | 2-Pyridyl; |
| R⁴ = | jeweils eine Zeile der Tabelle A. |

**Tabelle 230**

| Verbindungen der Formel I.2, mit | |
|---|---|
| R⁶ = | Wasserstoff; |
| R⁵ = | 2-Pyridyl; |
| R⁴ = | jeweils eine Zeile der Tabelle A. |

**Tabelle 231**

| Verbindungen der Formel I.3, mit | |
|---|---|
| R⁶ = | Wasserstoff; |
| R⁵ = | 2-Pyridyl; |
| R⁴ = | jeweils eine Zeile der Tabelle A. |

**Tabelle 232**

| Verbindungen der Formel I.4, mit | |
|---|---|
| R⁶ = | Wasserstoff; |
| R⁵ = | 2-Pyridyl; |
| R⁴ = | jeweils eine Zeile der Tabelle A. |

**Tabelle 233**

| Verbindungen der Formel I.1, mit | |
|---|---|
| R⁶ = | Wasserstoff; |
| R⁵ = | 2-Chlorpyridin-6-yl; |
| R⁴ = | jeweils eine Zeile der Tabelle A. |

**Tabelle 234**

| Verbindungen der Formel I.2, mit | |
|---|---|
| R⁶ = | Wasserstoff; |
| R⁵ = | 2-Chlorpyridin-6-yl; |
| R⁴ = | jeweils eine Zeile der Tabelle A. |

**Tabelle 235**

| Verbindungen der Formel I.3, mit | |
|---|---|
| R⁶ = | Wasserstoff; |
| R⁵ = | 2-Chlorpyridin-6-yl; |
| R⁴ = | jeweils eine Zeile der Tabelle A. |

**Tabelle 236**

| Verbindungen der Formel I.4, mit | |
|---|---|
| R⁶ = | Wasserstoff; |
| R⁵ = | 2-Chlorpyridin-6-yl; |
| R⁴ = | jeweils eine Zeile der Tabelle A. |

**Tabelle 237**

| Verbindungen der Formel I.1, mit | |
|---|---|
| R⁶ = | Wasserstoff; |
| R⁵ = | 5-Methylpyrimidin-2-yl; |
| R⁴ = | jeweils eine Zeile der Tabelle A. |

**Tabelle 238**

| Verbindungen der Formel I.2, mit | |
|---|---|
| R⁶ = | Wasserstoff; |
| R⁵ = | 5-Methylpyrimidin-2-yl; |
| R⁴ = | jeweils eine Zeile der Tabelle A. |

**Tabelle 239**

| Verbindungen der Formel I.3, mit | |
|---|---|
| R⁶ = | Wasserstoff; |
| R⁵ = | 5-Methylpyrimidin-2-yl; |
| R⁴ = | jeweils eine Zeile der Tabelle A. |

**Tabelle 240**

| Verbindungen der Formel I.4, mit | |
|---|---|
| R⁶ = | Wasserstoff; |
| R⁵ = | 5-Methylpyrimidin-2-yl; |
| R⁴ = | jeweils eine Zeile der Tabelle A. |

**Tabelle 241**

| Verbindungen der Formel I.1, mit | |
|---|---|
| R⁶ = | Wasserstoff; |
| R⁵ = | 2-Chlorpyridin-5-yl; |
| R⁴ = | jeweils eine Zeile der Tabelle A. |

**Tabelle 242**

| Verbindungen der Formel I.2, mit | |
|---|---|
| R⁶ = | Wasserstoff; |
| R⁵ = | 2-Chlorpyridin-5-yl; |
| R⁴ = | jeweils eine Zeile der Tabelle A. |

**Tabelle 243**

| Verbindungen der Formel I.3, mit | |
|---|---|
| R⁶ = | Wasserstoff; |
| R⁵ = | 2-Chlorpyridin-5-yl; |
| R⁴ = | jeweils eine Zeile der Tabelle A. |

**Tabelle 244**

| Verbindungen der Formel I.4, mit | |
|---|---|
| R⁶ = | Wasserstoff; |
| R⁵ = | 2-Chlorpyridin-5-yl; |
| R4 = | jeweils eine Zeile der Tabelle A. |

**Tabelle 245**

| Verbindungen der Formel I.1, mit | |
|---|---|
| R⁶ = | Wasserstoff; |
| R⁵ = | 5-Cyanothien-2-yl; |
| R⁴ = | jeweils eine Zeile der Tabelle A. |

**Tabelle 246**

| Verbindungen der Formel I.2, mit | |
|---|---|
| R⁶ = | Wasserstoff; |
| R⁵ = | 5-Cyanothien-2-yl; |
| R⁴ = | jeweils eine Zeile der Tabelle A. |

**Tabelle 247**

| Verbindungen der Formel I.3, mit | |
|---|---|
| R⁶ = | Wasserstoff; |
| R⁵ = | 5-Cyanothien-2-yl; |
| R⁴ = | jeweils eine Zeile der Tabelle A. |

**Tabelle 248**

| Verbindungen der Formel I.4, mit | |
|---|---|
| R⁶ = | Wasserstoff; |
| R⁵ = | 5-Cyanothien-2-yl; |
| R⁴ = | jeweils eine Zeile der Tabelle A. |

**Tabelle 249**

| Verbindungen der Formel I.1, mit | |
|---|---|
| R⁶ = | Wasserstoff; |
| R⁵ = | 4-Chloroxazol-2-yl; |
| R⁴ = | jeweils eine Zeile der Tabelle A. |

**Tabelle 250**

| Verbindungen der Formel I.2, mit | |
|---|---|
| R⁶ = | Wasserstoff; |
| R⁵ = | 4-Chloroxazol-2-yl; |
| R⁴ = | jeweils eine Zeile der Tabelle A. |

**Tabelle 251**

| Verbindungen der Formel I.3, mit | |
|---|---|
| R⁶ = | Wasserstoff; |
| R⁵ = | 4-Chloroxazol-2-yl; |
| R⁴ = | jeweils eine Zeile der Tabelle A. |

**Tabelle 252**

| Verbindungen der Formel I.4, mit | |
|---|---|
| R⁶ = | Wasserstoff; |
| R⁵ = | 4-Chloroxazol-2-yl; |
| R⁴ = | jeweils eine Zeile der Tabelle A. |

**Tabelle 253**

| Verbindungen der Formel I.1, mit | |
|---|---|
| R⁶ = | Wasserstoff; |
| R⁵ = | 1-Methylpyrazol-3-yl; |
| R⁴ = | jeweils eine Zeile der Tabelle A. |

**Tabelle 254**

| Verbindungen der Formel I.2, mit | |
|---|---|
| R⁶ = | Wasserstoff; |
| R⁵ = | 1-Methylpyrazol-3-yl; |
| R⁴ = | jeweils eine Zeile der Tabelle A. |

**Tabelle 255**

| Verbindungen der Formel I.3, mit | |
|---|---|
| R⁶ = | Wasserstoff; |
| R⁵ = | l-Methylpyrazol-3-yl; |
| R⁴ = | jeweils eine Zeile der Tabelle A. |

**Tabelle 256**

| Verbindungen der Formel I.4, mit | |
|---|---|
| R⁶ = | Wasserstoff; |
| R⁵ = | 1-Methylpyrazol-3-yl; |
| R⁴ = | jeweils eine Zeile der Tabelle A. |

**Tabelle 257**

| Verbindungen der Formel I.1, mit | |
|---|---|
| R⁶ = | Wasserstoff; |
| R⁵ = | 5-Trifluormethyl-1,2,4-oxadiazol-3-yl; |
| R⁴ = | jeweils eine Zeile der Tabelle A. |

**Tabelle 258**

| Verbindungen der Formel I.2, mit | |
|---|---|
| R⁶ = | Wasserstoff; |
| R⁵ = | 5-Trifluormethyl-1,2,4-oxadiazol-3-yl; |
| R⁴ = | jeweils eine Zeile der Tabelle A. |

**Tabelle 259**

| Verbindungen der Formel I.3, mit | |
|---|---|
| R⁶ = | Wasserstoff; |
| R⁵ = | 5-Trifluormethyl-1,2,4-oxadiazol-3-yl; |
| R⁴ = | jeweils eine Zeile der Tabelle A. |

**Tabelle 260**

| Verbindungen der Formel I.4, mit | |
|---|---|
| R⁶ = | Wasserstoff; |
| R⁵ = | 5-Trifluormethyl-1,2,4-oxadiazol-3-yl; |
| R⁴ = | jeweils eine Zeile der Tabelle A. |

**Tabelle 261**

| Verbindungen der Formel I.1, mit | |
|---|---|
| R⁶ = | Methyl; |
| R⁵ = | n-Hexyl; |
| R⁴ = | jeweils einer Zeile der Tabelle A. |

**Tabelle 262**

| Verbindungen der Formel I.2, mit | |
|---|---|
| R⁶ = | Methyl; |
| R⁵ = | n-Hexyl; |
| R⁴ = | jeweils eine Zeile der Tabelle A. |

**Tabelle 263**

| Verbindungen der Formel I.3, mit | |
|---|---|
| R⁶ = | Methyl; |
| R⁵ = | n-Hexyl; |
| R⁴ = | jeweils eine Zeile der Tabelle A. |

**Tabelle 264**

| Verbindungen der Formel I.4, mit | |
|---|---|
| R⁶ = | Methyl; |
| R⁵ = | n-Hexyl; |
| R⁴ = | jeweils eine Zeile der Tabelle A. |

**Tabelle 265**

| Verbindungen der Formel I.1, mit | |
|---|---|
| R⁶ = | Methyl; |
| R⁵ = | Methyl; |
| R⁴ = | jeweils eine Zeile der Tabelle A. |

**Tabelle 266**

| Verbindungen der Formel I.2, mit | |
|---|---|
| R⁶ = | Methyl; |
| R⁵ = | Methyl; |
| R⁴ = | jeweils eine Zeile der Tabelle A. |

**Tabelle 267**

| Verbindungen der Formel I.3, mit | |
|---|---|
| R⁶ = | Methyl; |
| R⁵ = | Methyl; |
| R⁴ = | jeweils eine Zeile der Tabelle A. |

**Tabelle 268**

| Verbindungen der Formel I.4, mit | |
|---|---|
| R⁶ = | Methyl; |
| R⁵ = | Methyl; |
| R⁴ = | jeweils eine Zeile der Tabelle A. |

**Tabelle 269**

| Verbindungen der Formel I.1, mit | |
|---|---|
| R⁶ = | Methyl; |
| R⁵ = | Ethyl; |
| R⁴ = | jeweils eine Zeile der Tabelle A. |

**Tabelle 270**

| Verbindungen der Formel I.2, mit | |
|---|---|
| R⁶ = | Methyl; |
| R⁵ = | Ethyl; |
| R⁴ = | jeweils eine Zeile der Tabelle A. |

**Tabelle 271**

| Verbindungen der Formel I.3, mit | |
|---|---|
| R⁶ = | Methyl; |
| R⁵ = | Ethyl; |
| R⁴ = | jeweils eine Zeile der Tabelle A. |

**Tabelle 272**

| Verbindungen der Formel I.4, mit | |
|---|---|
| R⁶ = | Methyl; |
| R⁵ = | Ethyl; |
| R⁴ = | jeweils eine Zeile der Tabelle A. |

**Tabelle 273**

| Verbindungen der Formel I.1, mit | |
|---|---|
| R⁶ = | Methyl; |
| R⁵ = | n-Propyl; |
| R⁴ = | jeweils eine Zeile der Tabelle A. |

**Tabelle 274**

| Verbindungen der Formel I.2, mit | |
|---|---|
| R⁶ = | Methyl; |
| R⁵ = | n-Propyl; |
| R⁴ = | jeweils eine Zeile der Tabelle A. |

**Tabelle 275**

| Verbindungen der Formel I.3, mit | |
|---|---|
| R⁶ = | Methyl; |
| R⁵ = | n-Propyl; |
| R⁴ = | jeweils eine Zeile der Tabelle A. |

**Tabelle 276**

| Verbindungen der Formel I.4, mit | |
|---|---|
| R⁶ = | Methyl; |
| R⁵ = | n-Propyl; |
| R⁴ = | jeweils eine Zeile der Tabelle A. |

**Tabelle 277**

| Verbindungen der Formel I.1, mit | |
|---|---|
| R⁶ = | Methyl; |
| R⁵ = | iso-Propyl; |
| R⁴ = | jeweils eine Zeile der Tabelle A. |

**Tabelle 278**

| Verbindungen der Formel I.2, mit | |
|---|---|
| R⁶ = | Methyl; |
| R⁵ = | iso-Propyl; |
| R⁴ = | jeweils eine Zeile der Tabelle A. |

**Tabelle 279**

| Verbindungen der Formel I.3, mit | |
|---|---|
| R⁶ = | Methyl; |
| R⁵ = | iso-Propyl; |
| R⁴ = | jeweils eine Zeile der Tabelle A. |

**Tabelle 280**

| Verbindungen der Formel I.4, mit | |
|---|---|
| R⁶ = | Methyl; |
| R⁵ = | iso-Propyl; |
| R⁴ = | jeweils eine Zeile der Tabelle A. |

**Tabelle 281**

| Verbindungen der Formel I.1, mit | |
|---|---|
| R⁶ = | Methyl; |
| R⁵ = | Cyclopropyl; |
| R⁴ = | jeweils eine Zeile der Tabelle A. |

**Tabelle 282**

| Verbindungen der Formel I.2, mit | |
|---|---|
| R⁶ = | Methyl; |
| R⁵ = | Cyclopropyl; |
| R⁴ = | jeweils eine Zeile der Tabelle A. |

**Tabelle 283**

| Verbindungen der Formel I.3, mit | |
|---|---|
| R⁶ = | Methyl; |
| R⁵ = | Cyclopropyl; |
| R⁴ = | jeweils eine Zeile der Tabelle A. |

**Tabelle 284**

| Verbindungen der Formel I.4, mit | |
|---|---|
| R⁶ = | Methyl; |
| R⁵ = | Cyclopropyl; |
| R⁴ = | jeweils eine Zeile der Tabelle A. |

**Tabelle 285**

| Verbindungen der Formel I.1, mit | |
|---|---|
| R⁶ = | Methyl; |
| R⁵ = | n-Butyl; |
| R⁴ = | jeweils eine Zeile der Tabelle A. |

**Tabelle 286**

| Verbindungen der Formel I.2, mit | |
|---|---|
| R⁶ = | Methyl; |
| R⁵ = | n-Butyl; |
| R⁴ = | jeweils eine Zeile der Tabelle A. |

**Tabelle 287**

| Verbindungen der Formel I.3, mit | |
|---|---|
| R⁶ = | Methyl; |
| R⁵ = | n-Butyl; |
| R⁴ = | jeweils eine Zeile der Tabelle A. |

**Tabelle 288**

| Verbindungen der Formel I.4, mit | |
|---|---|
| R⁶ = | Methyl; |
| R⁵ = | n-Butyl; |
| R⁴ = | jeweils eine Zeile der Tabelle A. |

**Tabelle 289**

| Verbindungen der Formel I.1, mit | |
|---|---|
| R⁶ = | Methyl; |
| R⁵ = | iso-Butyl; |
| R⁴ = | jeweils eine Zeile der Tabelle A. |

**Tabelle 290**

| Verbindungen der Formel I.2, mit | |
|---|---|
| R⁶ = | Methyl; |
| R⁵ = | iso-Butyl; |
| R⁴ = | jeweils eine Zeile der Tabelle A. |

**Tabelle 291**

| Verbindungen der Formel I.3, mit | |
|---|---|
| R⁶ = | Methyl; |
| R⁵ = | iso-Butyl; |
| R⁴ = | jeweils eine Zeile der Tabelle A. |

**Tabelle 292**

| Verbindungen der Formel I.4, mit | |
|---|---|
| R⁶ = | Methyl; |
| R⁵ = | iso-Butyl; |
| R⁴ = | jeweils eine Zeile der Tabelle A. |

**Tabelle 293**

| Verbindungen der Formel I.1, mit | |
|---|---|
| R⁶ = | Methyl; |
| R⁵ = | sec-Butyl; |
| R⁴ = | jeweils eine Zeile der Tabelle A. |

**Tabelle 294**

| Verbindungen der Formel I.2, mit | |
|---|---|
| R⁶ = | Methyl; |
| R⁵ = | sec-Butyl; |
| R⁴ = | jeweils eine Zeile der Tabelle A. |

**Tabelle 295**

| Verbindungen der Formel I.3, mit | |
|---|---|
| R⁶ = | Methyl; |
| R⁵ = | sec-Butyl; |
| R⁴ = | jeweils eine Zeile der Tabelle A. |

**Tabelle 296**

| Verbindungen der Formel I.4, mit | |
|---|---|
| R⁶ = | Methyl; |
| R⁵ = | sec-Butyl; |
| R⁴ = | jeweils eine Zeile der Tabelle A. |

**Tabelle 297**

| Verbindungen der Formel I.1, mit | |
|---|---|
| R⁶ = | Methyl; |
| R⁵ = | tert.-Butyl; |
| R⁴ = | jeweils eine Zeile der Tabelle A. |

**Tabelle 298**

| Verbindungen der Formel I.2, mit | |
|---|---|
| R⁶ = | Methyl; |
| R⁵ = | tert.-Butyl; |
| R⁴ = | jeweils eine Zeile der Tabelle A. |

**Tabelle 299**

| Verbindungen der Formel I.3, mit | |
|---|---|
| R⁶ = | Methyl; |
| R⁵ = | tert.-Butyl; |
| R⁴ = | jeweils eine Zeile der Tabelle A. |

**Tabelle 300**

| Verbindungen der Formel I.4, mit | |
|---|---|
| R⁶ = | Methyl; |
| R⁵ = | tert.-Butyl; |
| R⁴ = | jeweils eine Zeile der Tabelle A. |

**Tabelle 301**

| Verbindungen der Formel I.1, mit | |
|---|---|
| R⁶ = | Methyl; |
| R⁵ = | Trifluormethyl; |
| R⁴ = | jeweils eine Zeile der Tabelle A. |

**Tabelle 302**

| Verbindungen der Formel I.2, mit | |
|---|---|
| R⁶ = | Methyl; |
| R⁵ = | Trifluormethyl; |
| R⁴ = | jeweils eine Zeile der Tabelle A. |

**Tabelle 303**

| Verbindungen der Formel I.3, mit | |
|---|---|
| R⁶ = | Methyl; |
| R⁵ = | Trifluormethyl; |
| R⁴ = | jeweils eine Zeile der Tabelle A. |

**Tabelle 304**

| Verbindungen der Formel I.4, mit | |
|---|---|
| R⁶ = | Methyl; |
| R⁵ = | Trifluormethyl; |
| R⁴ = | jeweils eine Zeile der Tabelle A. |

**Tabelle 305**

| Verbindungen der Formel I.1, mit | |
|---|---|
| R⁶ = | Methyl; |
| R⁵ = | Pentafluorethyl; |
| R⁴ = | jeweils eine Zeile der Tabelle A. |

**Tabelle 306**

| Verbindungen der Formel I.2, mit | |
|---|---|
| R⁶ = | Methyl; |
| R⁵ = | Pentafluorethyl; |
| R⁴ = | jeweils eine Zeile der Tabelle A. |

**Tabelle 307**

| Verbindungen der Formel I.3, mit | |
|---|---|
| R⁶ = | Methyl; |
| R⁵ = | Pentafluorethyl; |
| R⁴ = | jeweils eine Zeile der Tabelle A. |

**Tabelle 308**

| Verbindungen der Formel I.4, mit | |
|---|---|
| R⁶ = | Methyl; |
| R⁵ = | Pentafluorethyl; |
| R⁴ = | jeweils eine Zeile der Tabelle A. |

**Tabelle 309**

| Verbindungen der Formel I.1, mit | |
|---|---|
| R⁶ = | Methyl; |
| R⁵ = | Cyanomethyl; |
| R⁴ = | jeweils eine Zeile der Tabelle A. |

**Tabelle 310**

| Verbindungen der Formel I.2, mit | |
|---|---|
| R⁶ = | Methyl; |
| R⁵ = | Cyanomethyl; |
| R⁴ = | jeweils eine Zeile der Tabelle A. |

**Tabelle 311**

| Verbindungen der Formel I.3, mit | |
|---|---|
| R⁶ = | Methyl; |
| R⁵ = | Cyanomethyl; |
| R⁴ = | jeweils eine Zeile der Tabelle A. |

**Tabelle 312**

| Verbindungen der Formel I.4, mit | |
|---|---|
| R⁶ = | Methyl; |
| R⁵ = | Cyanomethyl; |
| R⁴ = | jeweils eine Zeile der Tabelle A. |

**Tabelle 313**

| Verbindungen der Formel I.1, mit | |
|---|---|
| R⁶ = | Methyl; |
| R⁵ = | Methoxymethyl; |
| R⁴ = | jeweils eine Zeile der Tabelle A. |

**Tabelle 314**

| Verbindungen der Formel I.2, mit | |
|---|---|
| R⁶ = | Methyl; |
| R⁵ = | Methoxymethyl; |
| R⁴ = | jeweils eine Zeile der Tabelle A. |

**Tabelle 315**

| Verbindungen der Formel I.3, mit | |
|---|---|
| R⁶ = | Methyl; |
| R⁵ = | Methoxymethyl; |
| R⁴ = | jeweils eine Zeile der Tabelle A. |

**Tabelle 316**

| Verbindungen der Formel I.4, mit | |
|---|---|
| R⁶ = | Methyl; |
| R⁵ = | Methoxymethyl; |
| R⁴ = | jeweils eine Zeile der Tabelle A. |

**Tabelle 317**

| Verbindungen der Formel I.1, mit | |
|---|---|
| R⁶ = | Methyl; |
| R⁵ = | Ethoxymethyl; |
| R⁴ = | jeweils einer Zeile der Tabelle A. |

**Tabelle 318**

| Verbindungen der Formel I.2, mit | |
|---|---|
| R⁶ = | Methyl; |
| R⁵ = | Ethoxymethyl; |
| R⁴ = | jeweils eine Zeile der Tabelle A. |

**Tabelle 319**

| Verbindungen der Formel I.3, mit | |
|---|---|
| R⁶ = | Methyl; |
| R⁵ = | Ethoxymethyl; |
| R⁴ = | jeweils eine Zeile der Tabelle A. |

**Tabelle 320**

| Verbindungen der Formel I.4, mit | |
|---|---|
| R⁶ = | Methyl; |
| R⁵ = | Ethoxymethyl; |
| R⁴ = | jeweils eine Zeile der Tabelle A. |

**Tabelle 321**

| Verbindungen der Formel I.1, mit | |
|---|---|
| R⁶ = | Methyl; |
| R⁵ = | Isopropoxymethyl; |
| R⁴ = | jeweils eine Zeile der Tabelle A. |

**Tabelle 322**

| Verbindungen der Formel I.2, mit | |
|---|---|
| R⁶ = | Methyl; |
| R⁵ = | Isopropoxymethyl; |
| R⁴ = | jeweils eine Zeile der Tabelle A. |

**Tabelle 323**

| Verbindungen der Formel I.3, mit | |
|---|---|
| R⁶ = | Methyl; |
| R⁵ = | Isopropoxymethyl; |
| R⁴ = | jeweils eine Zeile der Tabelle A. |

**Tabelle 324**

| Verbindungen der Formel I.4, mit | |
|---|---|
| R⁶ = | Methyl; |
| R⁵ = | Isopropoxymethyl; |
| R⁴ = | jeweils eine Zeile der Tabelle A. |

**Tabelle 325**

| Verbindungen der Formel I.1, mit | |
|---|---|
| R⁶ = | Methyl; |
| R⁵ = | Cyclopropylmethyl; |
| R⁴ = | jeweils eine Zeile der Tabelle A. |

**Tabelle 326**

| Verbindungen der Formel I.2, mit | |
|---|---|
| R⁶ = | Methyl; |
| R⁵ = | Cyclopropylmethyl; |
| R⁴ = | jeweils eine Zeile der Tabelle A. |

**Tabelle 327**

| Verbindungen der Formel I.3, mit | |
|---|---|
| R⁶ = | Methyl; |
| R⁵ = | Cyclopropylmethyl; |
| R⁴ = | jeweils eine Zeile der Tabelle A. |

**Tabelle 328**

| Verbindungen der Formel I.4, mit | |
|---|---|
| R⁶ = | Methyl; |
| R⁵ = | Cyclopropylmethyl; |
| R⁴ = | jeweils eine Zeile der Tabelle A. |

**Tabelle 329**

| Verbindungen der Formel I.1, mit | |
|---|---|
| R⁶ = | Methyl; |
| R⁵ = | Phenoxymethyl; |
| R⁴ = | jeweils eine Zeile der Tabelle A. |

**Tabelle 330**

| Verbindungen der Formel I.2, mit | |
|---|---|
| R⁶ = | Methyl; |
| R⁵ = | Phenoxymethyl; |
| R⁴ = | jeweils eine Zeile der Tabelle A. |

**Tabelle 331**

| Verbindungen der Formel I.3, mit | |
|---|---|
| R⁶ = | Methyl; |
| R⁵ = | Phenoxymethyl; |
| R⁴ = | jeweils eine Zeile der Tabelle A. |

**Tabelle 332**

| Verbindungen der Formel I.4, mit | |
|---|---|
| R⁶ = | Methyl; |
| R⁵ = | Phenoxymethyl; |
| R⁴ = | jeweils eine Zeile der Tabelle A. |

**Tabelle 333**

| Verbindungen der Formel I.1, mit | |
|---|---|
| R⁶ = | Methyl; |
| R⁵ = | 2-Chlorphenoxymethyl; |
| R⁴ = | jeweils eine Zeile der Tabelle A. |

**Tabelle 334**

| Verbindungen der Formel I.2, mit | |
|---|---|
| R⁶ = | Methyl; |
| R⁵ = | 2-Chlorphenoxymethyl; |
| R⁴ = | jeweils eine Zeile der Tabelle A. |

**Tabelle 335**

| Verbindungen der Formel I.3, mit | |
|---|---|
| R⁶ = | Methyl; |
| R⁵ = | 2-Chlorphenoxymethyl; |
| R⁴ = | jeweils eine Zeile der Tabelle A. |

**Tabelle 336**

| Verbindungen der Formel I.4, mit | |
|---|---|
| R⁶ = | Methyl; |
| R⁵ = | 2-Chlorphenoxymethyl; |
| R⁴ = | jeweils eine Zeile der Tabelle A. |

**Tabelle 337**

| Verbindungen der Formel I.1, mit | |
|---|---|
| R⁶ = | Methyl; |
| R⁵ = | 3-Chlorphenoxymethyl; |
| R⁴ = | jeweils eine Zeile der Tabelle A. |

**Tabelle 338**

| Verbindungen der Formel I.2, mit | |
|---|---|
| R⁶ = | Methyl; |
| R⁵ = | 3-Chlorphenoxymethyl; |
| R⁴ = | jeweils eine Zeile der Tabelle A. |

**Tabelle 339**

| Verbindungen der Formel I.3, mit | |
|---|---|
| R⁶ = | Methyl; |
| R⁵ = | 3-Chlorphenoxymethyl; |
| R⁴ = | jeweils eine Zeile der Tabelle A. |

**Tabelle 340**

| Verbindungen der Formel I.4, mit | |
|---|---|
| R⁶ = | Methyl; |
| R⁵ = | 3-Chlorphenoxymethyl; |
| R⁴ = | jeweils eine Zeile der Tabelle A. |

**Tabelle 341**

| Verbindungen der Formel I.1, mit | |
|---|---|
| R⁶ = | Methyl; |
| R⁵ = | 4-Chlorphenoxymethyl; |
| R⁴ = | jeweils eine Zeile der Tabelle A. |

**Tabelle 342**

| Verbindungen der Formel I.2, mit | |
|---|---|
| R⁶ = | Methyl; |
| R⁵ = | 4-Chlorphenoxymethyl; |
| R⁴ = | jeweils eine Zeile der Tabelle A. |

**Tabelle 343**

| Verbindungen der Formel I.3, mit | |
|---|---|
| R⁶ = | Methyl; |
| R⁵ = | 4-Chlorphenoxymethyl; |
| R⁴ = | jeweils eine Zeile der Tabelle A. |

**Tabelle 344**

| Verbindungen der Formel I.4, mit | |
|---|---|
| R⁶ = | Methyl; |
| R⁵ = | 4-Chlorphenoxymethyl; |
| R⁴ = | jeweils eine Zeile der Tabelle A. |

**Tabelle 345**

| Verbindungen der Formel I.1, mit | |
|---|---|
| R⁶ = | Methyl; |
| R⁵ = | 2-Cyanophenoxymethyl; |
| R⁴ = | jeweils eine Zeile der Tabelle A. |

**Tabelle 346**

| Verbindungen der Formel I.2, mit | |
|---|---|
| R⁶ = | Methyl; |
| R⁵ = | 2-Cyanophenoxymethyl; |
| R⁴ = | jeweils eine Zeile der Tabelle A. |

**Tabelle 347**

| Verbindungen der Formel I.3, mit | |
|---|---|
| R⁶ = | Methyl; |
| R⁵ = | 2-Cyanophenoxymethyl; |
| R⁴ = | jeweils eine Zeile der Tabelle A. |

**Tabelle 348**

| Verbindungen der Formel I.4, mit | |
|---|---|
| R⁶ = | Methyl; |
| R⁵ = | 2-Cyanophenoxymethyl; |
| R⁴ = | jeweils eine Zeile der Tabelle A. |

**Tabelle 349**

| Verbindungen der Formel I.1, mit | |
|---|---|
| R⁶ = | Methyl; |
| R⁵ = | 4-Methylphenoxymethyl; |
| R⁴ = | jeweils eine Zeile der Tabelle A. |

**Tabelle 350**

| Verbindungen der Formel I.2, mit | |
|---|---|
| R⁶ = | Methyl; |
| R⁵ = | 4-Methylphenoxymethyl; |
| R⁴ = | jeweils eine Zeile der Tabelle A. |

**Tabelle 351**

| Verbindungen der Formel I.3, mit | |
|---|---|
| R⁶ = | Methyl; |
| R⁵ = | 4-Methylphenoxymethyl; |
| R⁴ = | jeweils eine Zeile der Tabelle A. |

**Tabelle 352**

| Verbindungen der Formel I.4, mit | |
|---|---|
| R⁶ = | Methyl; |
| R⁵ = | 4-Methylphenoxymethyl; |
| R⁴ = | jeweils eine Zeile der Tabelle A. |

**Tabelle 353**

| Verbindungen der Formel I.1, mit | |
|---|---|
| R⁶ = | Methyl; |
| R⁵ = | 3-Methoxyphenoxymethyl; |
| R⁴ = | jeweils eine Zeile der Tabelle A. |

**Tabelle 354**

| Verbindungen der Formel I.2, mit | |
|---|---|
| R⁶ = | Methyl; |
| R⁵ = | 3-Methoxyphenoxymethyl; |
| R⁴ = | jeweils eine Zeile der Tabelle A. |

**Tabelle 355**

| Verbindungen der Formel I.3, mit | |
|---|---|
| R⁶ = | Methyl; |
| R⁵ = | 3-Methoxyphenoxymethyl; |
| R⁴ = | jeweils eine Zeile der Tabelle A. |

**Tabelle 356**

| Verbindungen der Formel I.4, mit | |
|---|---|
| R⁶ = | Methyl; |
| R⁵ = | 3-Methoxyphenoxymethyl; |
| R⁴ = | jeweils eine Zeile der Tabelle A. |

**Tabelle 357**

| Verbindungen der Formel I.1, mit | |
|---|---|
| R⁶ = | Methyl; |
| R⁵ = | 4-Trifluormethylphenoxymethyl; |
| R⁴ = | jeweils eine Zeile der Tabelle A. |

**Tabelle 358**

| Verbindungen der Formel I.2, mit | |
|---|---|
| R⁶ = | Methyl; |
| R⁵ = | 4-Trifluormethylphenoxymethyl; |
| R⁴ = | jeweils eine Zeile der Tabelle A. |

**Tabelle 359**

| Verbindungen der Formel I.3, mit | |
|---|---|
| R⁶ = | Methyl; |
| R⁵ = | 4-Trifluormethylphenoxymethyl; |
| R⁴ = | jeweils eine Zeile der Tabelle A. |

**Tabelle 360**

| Verbindungen der Formel I.4, mit | |
|---|---|
| R⁶ = | Methyl; |
| R⁵ = | 4-Trifluormethylphenoxymethyl; |
| R⁴ = | jeweils eine Zeile der Tabelle A. |

**Tabelle 361**

| Verbindungen der Formel I.1, mit | |
|---|---|
| R⁶ = | Methyl; |
| R⁵ = | 2-(3-Nitrophenoxy)eth-1-yl; |
| R⁴ = | jeweils eine Zeile der Tabelle A. |

**Tabelle 362**

| Verbindungen der Formel I.2, mit | |
|---|---|
| R⁶ = | Methyl; |
| R⁵ = | 2-(3-Nitrophenoxy)eth-1-yl; |
| R⁴ = | jeweils eine Zeile der Tabelle A. |

**Tabelle 363**

| Verbindungen der Formel I.3, mit | |
|---|---|
| R⁶ = | Methyl; |
| R⁵ = | 2-(3-Nitrophenoxy)eth-1-yl; |
| R⁴ = | jeweils eine Zeile der Tabelle A. |

**Tabelle 364**

| Verbindungen der Formel I.4, mit | |
|---|---|
| R⁶ = | Methyl; |
| R⁵ = | 2-(3-Nitrophenoxy)eth-1-yl; |
| R⁴ = | jeweils eine Zeile der Tabelle A. |

**Tabelle 365**

| Verbindungen der Formel I.1, mit | |
|---|---|
| R⁶ = | Methyl; |
| R⁵ = | 2-Pyridyloxymethyl; |
| R⁴ = | jeweils eine Zeile der Tabelle A. |

**Tabelle 366**

| Verbindungen der Formel I.2, mit | |
|---|---|
| R⁶ = | Methyl; |
| R⁵ = | 2-Pyridyloxymethyl; |
| R⁴ = | jeweils eine Zeile der Tabelle A. |

**Tabelle 367**

| Verbindungen der Formel I.3, mit | |
|---|---|
| R⁶ = | Methyl; |
| R⁵ = | 2-Pyridyloxymethyl; |
| R⁴ = | jeweils eine Zeile der Tabelle A. |

**Tabelle 368**

| Verbindungen der Formel I.4, mit | |
|---|---|
| R⁶ = | Methyl; |
| R⁵ = | 2-Pyridyloxymethyl; |
| R⁴ = | jeweils eine Zeile der Tabelle A. |

**Tabelle 369**

| Verbindungen der Formel I.1, mit | |
|---|---|
| R⁶ = | Methyl; |
| R⁵ = | 4-Chlorpyrimidin-6-yloxymethyl; |
| R⁴ = | jeweils eine Zeile der Tabelle A. |

**Tabelle 370**

| Verbindungen der Formel I.2, mit | |
|---|---|
| R⁶ = | Methyl; |
| R⁵ = | 4-Chlorpyrimidin-6 -yloxymethyl; |
| R⁴ = | jeweils eine Zeile der Tabelle A. |

**Tabelle 371**

| Verbindungen der Formel I.3, mit | |
|---|---|
| R⁶ = | Methyl; |
| R⁵ = | 4-Chlorpyrimidin-6-yloxymethyl; |
| R⁴ = | jeweils eine Zeile der Tabelle A. |

**Tabelle 372**

| Verbindungen der Formel I.4, mit | |
|---|---|
| R⁶ = | Methyl; |
| R⁵ = | 4-Chlorpyrimidin-6-yloxymethyl; |
| R⁴ = | jeweils eine Zeile der Tabelle A. |

**Tabelle 373**

| Verbindungen der Formel I.1, mit | |
|---|---|
| R⁶ = | Methyl; |
| R⁵ = | 5-Methylisoxazol-3-yloxymethyl; |
| R⁴ = | jeweils einer Zeile der Tabelle A. |

**Tabelle 374**

| Verbindungen der Formel I.2, mit | |
|---|---|
| R⁶ = | Methyl; |
| R⁵ = | 5-Methylisoxazol-3-yloxymethyl; |
| R⁴ = | jeweils eine Zeile der Tabelle A. |

**Tabelle 375**

| Verbindungen der Formel I.3, mit | |
|---|---|
| R⁶ = | Methyl; |
| R⁵ = | 5-Methylisoxazol-3-yloxymethyl; |
| R⁴ = | jeweils eine Zeile der Tabelle A. |

**Tabelle 376**

| Verbindungen der Formel I.4, mit | |
|---|---|
| R⁶ = | Methyl; |
| R⁵ = | 5-Methylisoxazol-3-yloxymethyl; |
| R⁴ = | jeweils eine Zeile der Tabelle A. |

**Tabelle 377**

| Verbindungen der Formel I.1, mit | |
|---|---|
| R⁶ = | Methyl; |
| R⁵ = | 1-Phenylpyrazol-3-yloxymethyl; |
| R⁴ = | jeweils eine Zeile der Tabelle A. |

**Tabelle 378**

| Verbindungen der Formel I.2, mit | |
|---|---|
| R⁶ = | Methyl; |
| R⁵ = | 1-Phenylpyrazol-3-yloxymethyl; |
| R⁴ = | jeweils eine Zeile der Tabelle A. |

**Tabelle 379**

| Verbindungen der Formel I.3, mit | |
|---|---|
| R⁶ = | Methyl; |
| R⁵ = | 1-Phenylpyrazol-3-yloxymethyl; |
| R⁴ = | jeweils eine Zeile der Tabelle A. |

**Tabelle 380**

| Verbindungen der Formel I.4, mit | |
|---|---|
| R⁶ = | Methyl; |
| R⁵ = | 1-Phenylpyrazol-3-yloxymethyl; |
| R⁴ = | jeweils eine Zeile der Tabelle A. |

**Tabelle 381**

| Verbindungen der Formel I.1, mit | |
|---|---|
| R⁶ = | Methyl; |
| R⁵ = | 3-Chlorpyrid-6-yloxymethyl; |
| R⁴ = | jeweils eine Zeile der Tabelle A. |

**Tabelle 382**

| Verbindungen der Formel I.2, mit | |
|---|---|
| R⁶ = | Methyl; |
| R⁵ = | 3-Chlorpyrid-6-yloxymethyl; |
| R⁴ = | jeweils eine Zeile der Tabelle A. |

**Tabelle 383**

| Verbindungen der Formel I.3, mit | |
|---|---|
| R⁶ = | Methyl; |
| R⁵ = | 3-Chlorpyrid-6-yloxymethyl; |
| R⁴ = | jeweils eine Zeile der Tabelle A. |

**Tabelle 384**

| Verbindungen der Formel I.4, mit | |
|---|---|
| R⁶ = | Methyl; |
| R⁵ = | 3-Chlorpyrid-6-yloxymethyl; |
| R⁴ = | jeweils eine Zeile der Tabelle A. |

**Tabelle 385**

| Verbindungen der Formel I.1, mit | |
|---|---|
| R⁶ = | Methyl; |
| R⁵ = | 2-iso-Propyloxazol-4-yloxymethyl; |
| R⁴ = | jeweils eine Zeile der Tabelle A. |

**Tabelle 386**

| Verbindungen der Formel I.2, mit | |
|---|---|
| R⁶ = | Methyl; |
| R⁵ = | 2-iso-Propyloxazol-4-yloxymethyl; |
| R⁴ = | jeweils eine Zeile der Tabelle A. |

**Tabelle 387**

| Verbindungen der Formel I.3, mit | |
|---|---|
| R⁶ = | Methyl; |
| R⁵ = | 2-iso-Propyloxazol-4-yloxymethyl; |
| R⁴ = | jeweils eine Zeile der Tabelle A. |

**Tabelle 388**

| Verbindungen der Formel I.4, mit | |
|---|---|
| R⁶ = | Methyl; |
| R⁵ = | 2-iso-Propyloxazol-4-yloxymethyl; |
| R⁴ = | jeweils eine Zeile der Tabelle A. |

**Tabelle 389**

| Verbindungen der Formel I.1, mit | |
|---|---|
| R⁶ = | Methyl; |
| R⁵ = | 3-Pyridazinyloxymethyl; |
| R⁴ = | jeweils eine Zeile der Tabelle A. |

**Tabelle 390**

| Verbindungen der Formel I.2, mit | |
|---|---|
| R⁶ = | Methyl; |
| R⁵ = | 3-Pyridazinyloxymethyl; |
| R⁴ = | jeweils eine Zeile der Tabelle A. |

**Tabelle 391**

| Verbindungen der Formel I.3, mit | |
|---|---|
| R⁶ = | Methyl; |
| R⁵ = | 3-Pyridazinyloxymethyl; |
| R⁴ = | jeweils eine Zeile der Tabelle A. |

**Tabelle 392**

| Verbindungen der Formel I.4, mit | |
|---|---|
| R⁶ = | Methyl; |
| R⁵ = | 3-Pyridazinyloxymethyl; |
| R⁴ = | jeweils eine Zeile der Tabelle A. |

**Tabelle 393**

| Verbindungen der Formel I.1, mit | |
|---|---|
| R⁶ = | Methyl; |
| R⁵ = | 5-Methylpyrimidin-2-yloxymethyl; |
| R⁴ = | jeweils eine Zeile der Tabelle A. |

**Tabelle 394**

| Verbindungen der Formel I.2, mit | |
|---|---|
| R⁶ = | Methyl; |
| R⁵ = | 5-Methylpyrimidin-2-yloxymethyl; |
| R⁴ = | jeweils eine Zeile der Tabelle A. |

**Tabelle 395**

| Verbindungen der Formel I.3, mit | |
|---|---|
| R⁶ = | Methyl; |
| R⁵ = | 5-Methylpyrimidin-2-yloxymethyl; |
| R⁴ = | jeweils eine Zeile der Tabelle A. |

**Tabelle 396**

| Verbindungen der Formel I.4, mit | |
|---|---|
| R⁶ = | Methyl; |
| R⁵ = | 5-Methylpyrimidin-2-yloxymethyl; |
| R⁴ = | jeweils eine Zeile der Tabelle A. |

**Tabelle 397**

| Verbindungen der Formel I.1, mit | |
|---|---|
| R⁶ = | Methyl; |
| R⁵ = | Ethenyl; |
| R⁴ = | jeweils eine Zeile der Tabelle A. |

**Tabelle 398**

| Verbindungen der Formel I.2, mit | |
|---|---|
| R⁶ = | Methyl; |
| R⁵ = | Ethenyl; |
| R⁴ = | jeweils eine Zeile der Tabelle A. |

**Tabelle 399**

| Verbindungen der Formel I.3, mit | |
|---|---|
| R⁶ = | Methyl; |
| R⁵ = | Ethenyl; |
| R⁴ = | jeweils eine Zeile der Tabelle A. |

**Tabelle 400**

| Verbindungen der Formel I.4, mit | |
|---|---|
| R⁶ = | Methyl; |
| R⁵ = | Ethenyl; |
| R⁴ = | jeweils eine Zeile der Tabelle A. |

**Tabelle 401**

| Verbindungen der Formel I.1, mit | |
|---|---|
| R⁶ = | Methyl; |
| R⁵ = | Prop-2-en-1-yl; |
| R⁴ = | jeweils eine Zeile der Tabelle A. |

**Tabelle 402**

| Verbindungen der Formel I.2, mit | |
|---|---|
| R⁶ = | Methyl; |
| R⁵ = | Prop-2-en-1-yl; |
| R⁴ = | jeweils eine Zeile der Tabelle A. |

**Tabelle 403**

| Verbindungen der Formel I.3, mit | |
|---|---|
| R⁶ = | Methyl; |
| R⁵ = | Prop-2-en-1-yl; |
| R⁴ = | jeweils eine Zeile der Tabelle A. |

**Tabelle 404**

| Verbindungen der Formel I.4, mit | |
|---|---|
| R⁶ = | Methyl; |
| R⁵ = | Prop-2-en-1-yl; |
| R⁴ = | jeweils eine Zeile der Tabelle A. |

**Tabelle 405**

| Verbindungen der Formel I.1, mit | |
|---|---|
| R⁶ = | Methyl; |
| R⁵ = | E-3-Chlorprop-2-en-1-yl; |
| R⁴ = | jeweils eine Zeile der Tabelle A. |

**Tabelle 406**

| Verbindungen der Formel I.2, mit | |
|---|---|
| R⁶ = | Methyl; |
| R⁵ = | E-3-Chlorprop-2-en-1-yl; |
| R⁴ = | jeweils eine Zeile der Tabelle A. |

**Tabelle 407**

| Verbindungen der Formel I.3, mit | |
|---|---|
| R⁶ = | Methyl; |
| R⁵ = | E-3-Chlorprop-2-en-1-yl; |
| R⁴ = | jeweils eine Zeile der Tabelle A. |

**Tabelle 408**

| Verbindungen der Formel I.4, mit | |
|---|---|
| R⁶ = | Methyl; |
| R⁵ = | E-3-Chlorprop-2-en-1-yl; |
| R⁴ = | jeweils eine Zeile der Tabelle A. |

**Tabelle 409**

| Verbindungen der Formel I.1, mit | |
|---|---|
| R⁶ = | Methyl; |
| R⁵ = | E-But-2-en-1-yl; |
| R⁴ = | jeweils eine Zeile der Tabelle A. |

**Tabelle 410**

| Verbindungen der Formel I.2, mit | |
|---|---|
| R⁶ = | Methyl; |
| R⁵ = | E-But-2-en-1-yl; |
| R⁴ = | jeweils eine Zeile der Tabelle A. |

**Tabelle 411**

| Verbindungen der Formel I.3, mit | |
|---|---|
| R⁶ = | Methyl; |
| R⁵ = | E-But-2-en-1-yl; |
| R⁴ = | jeweils eine Zeile der Tabelle A. |

**Tabelle 412**

| Verbindungen der Formel I.4, mit | |
|---|---|
| R⁶ = | Methyl; |
| R⁵ = | E-But-2-en-1-yl; |
| R⁴ = | jeweils eine Zeile der Tabelle A. |

**Tabelle 413**

| Verbindungen der Formel I.1, mit | |
|---|---|
| R⁶ = | Methyl; |
| R⁵ = | Ethinyl; |
| R⁴ = | jeweils eine Zeile der Tabelle A. |

**Tabelle 414**

| Verbindungen der Formel I.2, mit | |
|---|---|
| R⁶ = | Methyl; |
| R⁵ = | Ethinyl; |
| R⁴ = | jeweils eine Zeile der Tabelle A. |

**Tabelle 415**

| Verbindungen der Formel I.3, mit | |
|---|---|
| R⁶ = | Methyl; |
| R⁵ = | Ethinyl; |
| R⁴ = | jeweils eine Zeile der Tabelle A. |

**Tabelle 416**

| Verbindungen der Formel I.4, mit | |
|---|---|
| R⁶ = | Methyl; |
| R⁵ = | Ethinyl; |
| R⁴ = | jeweils eine Zeile der Tabelle A. |

**Tabelle 417**

| Verbindungen der Formel I.1, mit | |
|---|---|
| R⁶ = | Methyl; |
| R⁵ = | Prop-2-in-1-yl; |
| R⁴ = | jeweils eine Zeile der Tabelle A. |

**Tabelle 418**

| Verbindungen der Formel I.2, mit | |
|---|---|
| R⁶ = | Methyl; |
| R⁵ = | Prop-2-in-1-yl; |
| R⁴ = | jeweils eine Zeile der Tabelle A. |

**Tabelle 419**

| Verbindungen der Formel I.3, mit | |
|---|---|
| R⁶ = | Methyl; |
| R⁵ = | Prop-2-in-1-yl; |
| R⁴ = | jeweils eine Zeile der Tabelle A. |

**Tabelle 420**

| Verbindungen der Formel I.4, mit | |
|---|---|
| R⁶ = | Methyl; |
| R⁵ = | Prop-2-in-1-yl; |
| R⁴ = | jeweils eine Zeile der Tabelle A. |

**Tabelle 421**

| Verbindungen der Formel I.1, mit | |
|---|---|
| R⁶ = | Methyl; |
| R⁵ = | Prop-1-in-1-yl; |
| R⁴ = | jeweils eine Zeile der Tabelle A. |

**Tabelle 422**

| Verbindungen der Formel I.2, mit | |
|---|---|
| R⁶ = | Methyl; |
| R⁵ = | Prop-1-in-1-yl; |
| R⁴ = | jeweils eine Zeile der Tabelle A. |

**Tabelle 423**

| Verbindungen der Formel I.3, mit | |
|---|---|
| R⁶ = | Methyl; |
| R⁵ = | Prop-1-in-1-yl; |
| R⁴ = | jeweils eine Zeile der Tabelle A. |

**Tabelle 424**

| Verbindungen der Formel I.4, mit | |
|---|---|
| R⁶ = | Methyl; |
| R⁵ = | Prop-1-in-1-yl; |
| R⁴ = | jeweils eine Zeile der Tabelle A. |

**Tabelle 425**

| Verbindungen der Formel I.1, mit | |
|---|---|
| R⁶ = | Methyl; |
| R⁵ = | Phenylethinyl; |
| R⁴ = | jeweils eine Zeile der Tabelle A. |

**Tabelle 426**

| Verbindungen der Formel I.2, mit | |
|---|---|
| R⁶ = | Methyl; |
| R⁵ = | Phenylethinyl; |
| R⁴ = | jeweils eine Zeile der Tabelle A. |

**Tabelle 427**

| Verbindungen der Formel I.3, mit | |
|---|---|
| R⁶ = | Methyl; |
| R⁵ = | Phenylethinyl; |
| R⁴ = | jeweils eine Zeile der Tabelle A. |

**Tabelle 428**

| Verbindungen der Formel I.4, mit | |
|---|---|
| R⁶ = | Methyl; |
| R⁵ = | Phenylethinyl; |
| R⁴ = | jeweils eine Zeile der Tabelle A. |

**Tabelle 429**

| Verbindungen der Formel I.1, mit | |
|---|---|
| R⁶ = | Methyl; |
| R⁵ = | Oxiran-2-yl; |
| R⁴ = | jeweils einer Zeile der Tabelle A. |

**Tabelle 430**

| Verbindungen der Formel I.2, mit | |
|---|---|
| R⁶ = | Methyl; |
| R⁵ = | Oxiran-2-yl; |
| R⁴ = | jeweils eine Zeile der Tabelle A. |

**Tabelle 431**

| Verbindungen der Formel I.3, mit | |
|---|---|
| R⁶ = | Methyl; |
| R⁵ = | Oxiran-2-yl; |
| R⁴ = | jeweils eine Zeile der Tabelle A. |

**Tabelle 432**

| Jerbindungen der Formel I.4, mit | |
|---|---|
| R⁶ = | Methyl; |
| R⁵ = | Oxiran-2-yl; |
| R⁴ = | jeweils eine Zeile der Tabelle A. |

**Tabelle 433**

| Verbindungen der Formel I.1, mit | |
|---|---|
| R⁶ = | Methyl; |
| R⁵ = | 1,1-Dichlorcyclopropyl; |
| R⁴ = | jeweils eine Zeile der Tabelle A. |

**Tabelle 434**

| Verbindungen der Formel I.2, mit | |
|---|---|
| R⁶ = | Methyl; |
| R⁵ = | 1,1-Dichlorcyclopropyl; |
| R⁴ = | jeweils eine Zeile der Tabelle A. |

**Tabelle 435**

| Verbindungen der Formel I.3, mit | |
|---|---|
| R⁶ = | Methyl; |
| R⁵ = | 1,1-Dichlorcyclopropyl; |
| R⁴ = | jeweils eine Zeile der Tabelle A. |

**Tabelle 436**

| Verbindungen der Formel I.4, mit | |
|---|---|
| R⁶ = | Methyl; |
| R⁵ = | 1,1-Dichlorcyclopropyl; |
| R⁴ = | jeweils eine Zeile der Tabelle A. |

**Tabelle 437**

| Verbindungen der Formel I.1, mit | |
|---|---|
| R⁶ = | Methyl; |
| R⁵ = | Phenyl; |
| R⁴ = | jeweils eine Zeile der Tabelle A. |

**Tabelle 438**

| Verbindungen der Formel I.2, mit | |
|---|---|
| R⁶ = | Methyl; |
| R⁵ = | Phenyl; |
| R⁴ = | jeweils eine Zeile der Tabelle A. |

**Tabelle 439**

| Verbindungen der Formel I.3, mit | |
|---|---|
| R⁶ = | Methyl; |
| R⁵ = | Phenyl; |
| R⁴ = | jeweils eine Zeile der Tabelle A. |

**Tabelle 440**

| Verbindungen der Formel I.4, mit | |
|---|---|
| R⁶ = | Methyl; |
| R⁵ = | Phenyl; |
| R⁴ = | jeweils eine Zeile der Tabelle A. |

**Tabelle 441**

| Verbindungen der Formel I.1, mit | |
|---|---|
| R⁶ = | Methyl; |
| R⁵ = | 2-Naphthyl; |
| R⁴ = | jeweils eine Zeile der Tabelle A. |

**Tabelle 442**

| Verbindungen der Formel I.2, mit | |
|---|---|
| R⁶ = | Methyl; |
| R⁵ = | 2-Naphthyl; |
| R⁴ = | jeweils eine Zeile der Tabelle A. |

**Tabelle 443**

| Verbindungen der Formel I.3, mit | |
|---|---|
| R⁶ = | Methyl; |
| R⁵ = | 2-Naphthyl; |
| R⁴ = | jeweils eine Zeile der Tabelle A. |

**Tabelle 444**

| Verbindungen der Formel I.4, mit | |
|---|---|
| R⁶ = | Methyl; |
| R⁵ = | 2-Naphthyl; |
| R⁴ = | jeweils eine Zeile der Tabelle A. |

**Tabelle 445**

| Verbindungen der Formel I.1, mit | |
|---|---|
| R⁶ = | Methyl; |
| R⁵ = | 2-Chlorphenyl; |
| R⁴ = | jeweils eine Zeile der Tabelle A. |

**Tabelle 446**

| Verbindungen der Formel I.2, mit | |
|---|---|
| R⁶ = | Methyl; |
| R⁵ = | 2-Chlorphenyl; |
| R⁴ = | jeweils eine Zeile der Tabelle A. |

**Tabelle 447**

| Verbindungen der Formel I.3, mit | |
|---|---|
| R⁶ = | Methyl; |
| R⁵ = | 2-Chlorphenyl; |
| R⁴ = | jeweils eine Zeile der Tabelle A. |

**Tabelle 448**

| Verbindungen der Formel I.4, mit | |
|---|---|
| R⁶ = | Methyl; |
| R⁵ = | 2-Chlorphenyl; |
| R⁴ = | jeweils eine Zeile der Tabelle A. |

**Tabelle 449**

| Verbindungen der Formel I.1, mit | |
|---|---|
| R⁶ = | Methyl; |
| R⁵ = | 3-Chlorphenyl; |
| R⁴ = | jeweils eine Zeile der Tabelle A. |

**Tabelle 450**

| Verbindungen der Formel I.2, mit | |
|---|---|
| R⁶ = | Methyl; |
| R⁵ = | 3-Chlorphenyl; |
| R⁴ = | jeweils eine Zeile der Tabelle A. |

**Tabelle 451**

| Verbindungen der Formel I.3, mit | |
|---|---|
| R⁶ = | Methyl; |
| R⁵ = | 3-Chlorphenyl; |
| R⁴ = | jeweils eine Zeile der Tabelle A. |

**Tabelle 452**

| Verbindungen der Formel I.4, mit | |
|---|---|
| R⁶ = | Methyl; |
| R⁵ = | 3-Chlorphenyl; |
| R⁴ = | jeweils eine Zeile der Tabelle A. |

**Tabelle 453**

| Verbindungen der Formel I.1, mit | |
|---|---|
| R⁶ = | Methyl; |
| R⁵ = | 4-Chlorphenyl; |
| R⁴ = | jeweils eine Zeile der Tabelle A. |

**Tabelle 454**

| Verbindungen der Formel I.2, mit | |
|---|---|
| R⁶ = | Methyl; |
| R⁵ = | 4-Chlorphenyl; |
| R⁴ = | jeweils eine Zeile der Tabelle A. |

**Tabelle 455**

| Verbindungen der Formel I.3, mit | |
|---|---|
| R⁶ = | Methyl; |
| R⁵ = | 4-Chlorphenyl; |
| R⁴ = | jeweils eine Zeile der Tabelle A. |

**Tabelle 456**

| Verbindungen der Formel I.4, mit | |
|---|---|
| R⁶ = | Methyl; |
| R⁵ = | 4-Chlorphenyl; |
| R⁴ = | jeweils eine Zeile der Tabelle A. |

**Tabelle 457**

| Verbindungen der Formel I.1, mit | |
|---|---|
| R⁶ = | Methyl; |
| R⁵ = | 2-Nitrophenyl; |
| R⁴ = | jeweils eine Zeile der Tabelle A. |

**Tabelle 458**

| Verbindungen der Formel I.2, mit | |
|---|---|
| R⁶ = | Methyl; |
| R⁵ = | 2-Nitrophenyl; |
| R⁴ = | jeweils eine Zeile der Tabelle A. |

**Tabelle 459**

| Verbindungen der Formel I.3, mit | |
|---|---|
| R⁶ = | Methyl; |
| R⁵ = | 2-Nitrophenyl; |
| R⁴ = | jeweils eine Zeile der Tabelle A. |

**Tabelle 460**

| Verbindungen der Formel I.4, mit | |
|---|---|
| R⁶ = | Methyl; |
| R⁵ = | 2-Nitrophenyl; |
| R⁴ = | jeweils eine Zeile der Tabelle A. |

**Tabelle 461**

| Verbindungen der Formel I.1, mit | |
|---|---|
| R⁶ = | Methyl; |
| R⁵ = | 3-Cyanophenyl; |
| R⁴ = | jeweils eine Zeile der Tabelle A. |

**Tabelle 462**

| Verbindungen der Formel I.2, mit | |
|---|---|
| R⁶ = | Methyl; |
| R⁵ = | 3-Cyanophenyl; |
| R⁴ = | jeweils eine Zeile der Tabelle A. |

**Tabelle 463**

| Verbindungen der Formel I.3, mit | |
|---|---|
| R⁶ = | Methyl; |
| R⁵ = | 3-Cyanophenyl; |
| R⁴ = | jeweils eine Zeile der Tabelle A. |

**Tabelle 464**

| Verbindungen der Formel I.4, mit | |
|---|---|
| R⁶ = | Methyl; |
| R⁵ = | 3-Cyanophenyl; |
| R⁴ = | jeweils eine Zeile der Tabelle A. |

**Tabelle 465**

| Verbindungen der Formel I.1, mit | |
|---|---|
| R⁶ = | Methyl; |
| R⁵ = | 4-Methylphenyl; |
| R⁴ = | jeweils eine Zeile der Tabelle A. |

**Tabelle 466**

| Verbindungen der Formel I.2, mit | |
|---|---|
| R⁶ = | Methyl; |
| R⁵ = | 4-Methylphenyl; |
| R⁴ = | jeweils eine Zeile der Tabelle A. |

**Tabelle 467**

| Verbindungen der Formel I.3, mit | |
|---|---|
| R⁶ = | Methyl; |
| R⁵ = | 4-Methylphenyl; |
| R⁴ = | jeweils eine Zeile der Tabelle A. |

**Tabelle 468**

| Verbindungen der Formel I.4, mit | |
|---|---|
| R⁶ = | Methyl; |
| R⁵ = | 4-Methylphenyl; |
| R⁴ = | jeweils eine Zeile der Tabelle A. |

**Tabelle 469**

| Verbindungen der Formel I.1, mit | |
|---|---|
| R⁶ = | Methyl; |
| R⁵ = | 3-tert.-Butylphenyl; |
| R⁴ = | jeweils eine Zeile der Tabelle A. |

**Tabelle 470**

| Verbindungen der Formel I.2, mit | |
|---|---|
| R⁶ = | Methyl; |
| R⁵ = | 3-tert.-Butylphenyl; |
| R⁴ = | jeweils eine Zeile der Tabelle A. |

**Tabelle 471**

| Verbindungen der Formel I.3, mit | |
|---|---|
| R⁶ = | Methyl; |
| R⁵ = | 3-tert.-Butylphenyl; |
| R⁴ = | jeweils eine Zeile der Tabelle A. |

**Tabelle 472**

| Verbindungen der Formel I.4, mit | |
|---|---|
| R⁶ = | Methyl; |
| R⁵ = | 3-tert.-Butylphenyl; |
| R⁴ = | jeweils eine Zeile der Tabelle A. |

**Tabelle 473**

| Verbindungen der Formel I.1, mit | |
|---|---|
| R⁶ = | Methyl; |
| R⁵ = | 4-Trifluormethylphenyl; |
| R⁴ = | jeweils eine Zeile der Tabelle A. |

**Tabelle 474**

| Verbindungen der Formel I.2, mit | |
|---|---|
| R⁶ = | Methyl; |
| R⁵ = | 4-Trifluormethylphenyl; |
| R⁴ = | jeweils eine Zeile der Tabelle A. |

**Tabelle 475**

| Verbindungen der Formel I.3, mit | |
|---|---|
| R⁶ = | Methyl; |
| R⁵ = | 4-Trifluormethylphenyl; |
| R⁴ = | jeweils eine Zeile der Tabelle A. |

**Tabelle 476**

| Verbindungen der Formel I.4, mit | |
|---|---|
| R⁶ = | Methyl; |
| R⁵ = | 4-Trifluormethylphenyl; |
| R⁴ = | jeweils eine Zeile der Tabelle A. |

**Tabelle 477**

| Verbindungen der Formel I.1, mit | |
|---|---|
| R⁶ = | Methyl; |
| R⁵ = | 2-Methoxyphenyl; |
| R⁴ = | jeweils eine Zeile der Tabelle A. |

**Tabelle 478**

| Verbindungen der Formel I.2, mit | |
|---|---|
| R⁶ = | Methyl; |
| R⁵ = | 2-Methoxyphenyl; |
| R⁴ = | jeweils eine Zeile der Tabelle A. |

**Tabelle 479**

| Verbindungen der Formel I.3, mit | |
|---|---|
| R⁶ = | Methyl; |
| R⁵ = | 2-Methoxyphenyl; |
| R⁴ = | jeweils eine Zeile der Tabelle A. |

**Tabelle 480**

| Verbindungen der Formel I.4, mit | |
|---|---|
| R⁶ = | Methyl; |
| R⁵ = | 2-Methoxyphenyl; |
| R⁴ = | jeweils eine Zeile der Tabelle A. |

**Tabelle 481**

| Verbindungen der Formel I.1, mit | |
|---|---|
| R⁶ = | Methyl; |
| R⁵ = | 2,4-Dichlorphenyl; |
| R⁴ = | jeweils eine Zeile der Tabelle A. |

**Tabelle 482**

| Verbindungen der Formel I.2, mit | |
|---|---|
| R⁶ = | Methyl; |
| R⁵ = | 2,4-Dichlorphenyl; |
| R⁴ = | jeweils eine Zeile der Tabelle A. |

**Tabelle 483**

| Verbindungen der Formel I.3, mit | |
|---|---|
| R⁶ = | Methyl; |
| R⁵ = | 2,4-Dichlorphenyl; |
| R⁴ = | jeweils eine Zeile der Tabelle A. |

**Tabelle 484**

| Verbindungen der Formel I.4, mit | |
|---|---|
| R⁶ = | Methyl; |
| R⁵ = | 2,4-Dichlorphenyl; |
| R⁴ = | jeweils eine Zeile der Tabelle A. |

**Tabelle 485**

| Verbindungen der Formel I.1, mit | |
|---|---|
| R⁶ = | Methyl; |
| R⁵ = | 4-Fluorphenyl; |
| R⁴ = | jeweils einer Zeile der Tabelle A. |

**Tabelle 486**

| Verbindungen der Formel I.2, mit | |
|---|---|
| R⁶ = | Methyl; |
| R⁵ = | 4-Fluorphenyl; |
| R⁴ = | jeweils eine Zeile der Tabelle A. |

**Tabelle 487**

| Verbindungen der Formel I.3, mit | |
|---|---|
| R⁶ = | Methyl; |
| R⁵ = | 4-Fluorphenyl; |
| R⁴ = | jeweils eine Zeile der Tabelle A. |

**Tabelle 488**

| Verbindungen der Formel I.4, mit | |
|---|---|
| R⁶ = | Methyl; |
| R⁵ = | 4-Fluorphenyl; |
| R⁴ = | jeweils eine Zeile der Tabelle A. |

**Tabelle 489**

| Verbindungen der Formel I.1, mit | |
|---|---|
| R⁶ = | Methyl; |
| R⁵ = | 2-Pyridyl; |
| R⁴ = | jeweils eine Zeile der Tabelle A. |

**Tabelle 490**

| Verbindungen der Formel I.2, mit | |
|---|---|
| R⁶ = | Methyl; |
| R⁵ = | 2-Pyridyl; |
| R⁴ = | jeweils eine Zeile der Tabelle A. |

**Tabelle 491**

| Verbindungen der Formel I.3, mit | |
|---|---|
| R⁶ = | Methyl; |
| R⁵ = | 2-Pyridyl; |
| R⁴ = | jeweils eine Zeile der Tabelle A. |

**Tabelle 492**

| Verbindungen der Formel I.4, mit | |
|---|---|
| R⁶ = | Methyl; |
| R⁵ = | 2-Pyridyl; |
| R⁴ = | jeweils eine Zeile der Tabelle A. |

**Tabelle 493**

| Verbindungen der Formel I.1, mit | |
|---|---|
| R⁶ = | Methyl; |
| R⁵ = | 2-Chlorpyridin-6-yl; |
| R⁴ = | jeweils eine Zeile der Tabelle A. |

**Tabelle 494**

| Verbindungen der Formel I.2, mit | |
|---|---|
| R⁶ = | Methyl; |
| R⁵ = | 2-Chlorpyridin-6-yl; |
| R⁴ = | jeweils eine Zeile der Tabelle A. |

**Tabelle 495**

| Verbindungen der Formel I.3, mit | |
|---|---|
| R⁶ = | Methyl; |
| R⁵ = | 2-Chlorpyridin-6-yl; |
| R⁴ = | jeweils eine Zeile der Tabelle A. |

**Tabelle 496**

| Verbindungen der Formel I.4, mit | |
|---|---|
| R⁶ = | Methyl; |
| R⁵ = | 2-Chlorpyridin-6-yl; |
| R⁴ = | jeweils eine Zeile der Tabelle A. |

**Tabelle 497**

| Verbindungen der Formel I.1, mit | |
|---|---|
| R⁶ = | Methyl; |
| R⁵ = | 5-Methylpyrimidin-2-yl; |
| R⁴ = | jeweils eine Zeile der Tabelle A. |

**Tabelle 498**

| Verbindungen der Formel I.2, mit | |
|---|---|
| R⁶ = | Methyl; |
| R⁵ = | 5-Methylpyrimidin-2-yl; |
| R⁴ = | jeweils eine Zeile der Tabelle A. |

**Tabelle 499**

| Verbindungen der Formel I.3, mit | |
|---|---|
| R⁶ = | Methyl; |
| R⁵ = | 5-Methylpyrimidin-2-yl; |
| R⁴ = | jeweils eine Zeile der Tabelle A. |

**Tabelle 500**

| Verbindungen der Formel I.4, mit | |
|---|---|
| R⁶ = | Methyl; |
| R⁵ = | 5-Methylpyrimidin-2-yl; |
| R⁴ = | jeweils eine Zeile der Tabelle A. |

**Tabelle 501**

| Verbindungen der Formel I.1, mit | |
|---|---|
| R⁶ = | Methyl; |
| R⁵ = | 2-Chlorpyridin-5-yl; |
| R⁴ = | jeweils eine Zeile der Tabelle A. |

**Tabelle 502**

| Verbindungen der Formel I.2, mit | |
|---|---|
| R⁶ = | Methyl; |
| R⁵ = | 2-Chlorpyridin-5-yl; |
| R⁴ = | jeweils eine Zeile der Tabelle A. |

**Tabelle 503**

| Verbindungen der Formel I.3, mit | |
|---|---|
| R⁶ = | Methyl; |
| R⁵ = | 2-Chlorpyridin-5-yl; |
| R⁴ = | jeweils eine Zeile der Tabelle A. |

**Tabelle 504**

| Verbindungen der Formel I.4, mit | |
|---|---|
| R⁶ = | Methyl; |
| R⁵ = | 2-Chlorpyridin-5-yl; |
| R⁴ = | jeweils eine Zeile der Tabelle A. |

**Tabelle 505**

| Verbindungen der Formel I.1, mit | |
|---|---|
| R⁶ = | Methyl; |
| R⁵ = | 5-Cyanothien-2-yl; |
| R⁴ = | jeweils eine Zeile der Tabelle A. |

**Tabelle 506**

| Verbindungen der Formel I.2, mit | |
|---|---|
| R⁶ = | Methyl; |
| R⁵ = | 5-Cyanothien-2-yl; |
| R⁴ = | jeweils eine Zeile der Tabelle A. |

**Tabelle 507**

| Verbindungen der Formel I.3, mit | |
|---|---|
| R⁶ = | Methyl; |
| R⁵ = | 5-Cyanothien-2-yl; |
| R⁴ = | jeweils eine Zeile der Tabelle A. |

**Tabelle 508**

| Verbindungen der Formel I.4, mit | |
|---|---|
| R⁶ = | Methyl; |
| R⁵ = | 5-Cyanothien-2-yl; |
| R⁴ = | jeweils eine Zeile der Tabelle A. |

**Tabelle 509**

| Verbindungen der Formel I.1, mit | |
|---|---|
| R⁶ = | Methyl; |
| R⁵ = | 4-Chloroxazol-2-yl; |
| R⁴ = | jeweils eine Zeile der Tabelle A. |

**Tabelle 510**

| Verbindungen der Formel I.2, mit | |
|---|---|
| R⁶ = | Methyl; |
| R⁵ = | 4-Chloroxazol-2-yl; |
| R⁴ = | jeweils eine Zeile der Tabelle A. |

**Tabelle 511**

| Verbindungen der Formel I.3, mit | |
|---|---|
| R⁶ = | Methyl; |
| R⁵ = | 4-Chloroxazol-2-yl; |
| R⁴ = | jeweils eine Zeile der Tabelle A. |

**Tabelle 512**

| Verbindungen der Formel I.4, mit | |
|---|---|
| R⁶ = | Methyl; |
| R⁵ = | 4-Chloroxazol-2-yl; |
| R⁴ = | jeweils eine Zeile der Tabelle A. |

**Tabelle 513**

| Verbindungen der Formel I.1, mit | |
|---|---|
| R⁶ = | Methyl; |
| R⁵ = | 1-Methoxypyrazol-3-yl; |
| R⁴ = | jeweils eine Zeile der Tabelle A. |

**Tabelle 514**

| Verbindungen der Formel I.2, mit | |
|---|---|
| R⁶ = | Methyl; |
| R⁵ = | 1-Methoxypyrazol-3-yl; |
| R⁴ = | jeweils eine Zeile der Tabelle A. |

**Tabelle 515**

| Verbindungen der Formel I.3, mit | |
|---|---|
| R⁶ = | Methyl; |
| R⁵ = | 1-Methoxypyrazol-3-yl; |
| R⁴ = | jeweils eine Zeile der Tabelle A. |

**Tabelle 516**

| Verbindungen der Formel I.4, mit | |
|---|---|
| R⁶ = | Methyl; |
| R⁵ = | 1-Methoxypyrazol-3-yl; |
| R⁴ = | jeweils eine Zeile der Tabelle A. |

**Tabelle 517**

| Verbindungen der Formel I.1, mit | |
|---|---|
| R⁶ = | Methyl; |
| R⁵ = | 5-Trifluormethyl-1,2,4-oxadiazol-3-yl; |
| R⁴ = | jeweils eine Zeile der Tabelle A. |

**Tabelle 518**

| Verbindungen der Formel I.2, mit | |
|---|---|
| R⁶ = | Methyl; |
| R⁵ = | 5-Trifluormethyl-1,2,4-oxadiazol-3-yl; |
| R⁴ = | jeweils eine Zeile der Tabelle A. |

**Tabelle 519**

| Verbindungen der Formel I.3, mit | |
|---|---|
| R⁶ = | Methyl; |
| R⁵ = | 5-Trifluormethyl-1,2,4-oxadiazol-3-yl; |
| R⁴ = | jeweils eine Zeile der Tabelle A. |

**Tabelle 520**

| Verbindungen der Formel I.4, mit | |
|---|---|
| R⁶ = | Methyl; |
| R⁵ = | 5-Trifluormethyl-1,2,4-oxadiazol-3-yl; |
| R⁴ = | jeweils eine Zeile der Tabelle A. |

**Tabelle 521**

| Verbindungen der Formel I.1, mit | |
|---|---|
| R⁶ = | Ethyl; |
| R⁵ = | Ethyl; |
| R⁴ = | jeweils eine Zeile der Tabelle A. |

**Tabelle 522**

| Verbindungen der Formel I.2, mit | |
|---|---|
| R⁶ = | Ethyl; |
| R⁵ = | Ethyl; |
| R⁴ = | jeweils eine Zeile der Tabelle A. |

**Tabelle 523**

| Verbindungen der Formel I.3, mit | |
|---|---|
| R⁶ = | Ethyl; |
| R⁵ = | Ethyl; |
| R⁴ = | jeweils eine Zeile der Tabelle A. |

**Tabelle 524**

| Verbindungen der Formel I.4, mit | |
|---|---|
| R⁶ = | Ethyl; |
| R⁵ = | Ethyl; |
| R⁴ = | jeweils eine Zeile der Tabelle A. |

**Tabelle 525**

| Verbindungen der Formel I.1, mit | |
|---|---|
| R⁶ = | Ethyl; |
| R⁵ = | n-Propyl; |
| R⁴ = | jeweils eine Zeile der Tabelle A. |

**Tabelle 526**

| Verbindungen der Formel I.2, mit | |
|---|---|
| R⁶ = | Ethyl; |
| R⁵ = | n-Propyl; |
| R⁴ = | jeweils eine Zeile der Tabelle A. |

**Tabelle 527**

| Verbindungen der Formel I.3, mit | |
|---|---|
| R⁶ = | Ethyl; |
| R⁵ = | n-Propyl; |
| R⁴ = | jeweils eine Zeile der Tabelle A. |

**Tabelle 528**

| Verbindungen der Formel I.4, mit | |
|---|---|
| R⁶ = | Ethyl; |
| R⁵ = | n-Propyl; |
| R⁴ = | jeweils eine Zeile der Tabelle A. |

**Tabelle 529**

| Verbindungen der Formel I.1, mit | |
|---|---|
| R⁶ = | Ethyl; |
| R⁵ = | iso-Propyl; |
| R⁴ = | jeweils eine Zeile der Tabelle A. |

**Tabelle 530**

| Verbindungen der Formel I.2, mit | |
|---|---|
| R⁶ = | Ethyl; |
| R⁵ = | iso-Propyl; |
| R⁴ = | jeweils eine Zeile der Tabelle A. |

**Tabelle 531**

| Verbindungen der Formel I.3, mit | |
|---|---|
| R⁶ = | Ethyl; |
| R⁵ = | iso-Propyl; |
| R⁴ = | jeweils eine Zeile der Tabelle A. |

**Tabelle 532**

| Verbindungen der Formel I.4, mit | |
|---|---|
| R⁶ = | Ethyl; |
| R⁵ = | iso-Propyl; |
| R⁴ = | jeweils eine Zeile der Tabelle A. |

**Tabelle 533**

| Verbindungen der Formel I.1, mit | |
|---|---|
| R⁶ = | Ethyl; |
| R⁵ = | Cyclopropyl; |
| R⁴ = | jeweils eine Zeile der Tabelle A. |

**Tabelle 534**

| Verbindungen der Formel I.2, mit | |
|---|---|
| R⁶ = | Ethyl; |
| R⁵ = | Cyclopropyl; |
| R⁴ = | jeweils eine Zeile der Tabelle A. |

**Tabelle 535**

| Verbindungen der Formel I.3, mit | |
|---|---|
| R⁶ = | Ethyl; |
| R⁵ = | Cyclopropyl; |
| R⁴ = | jeweils eine Zeile der Tabelle A. |

**Tabelle 536**

| Verbindungen der Formel I.4, mit | |
|---|---|
| R⁶ = | Ethyl; |
| R⁵ = | Cyclopropyl; |
| R⁴ = | jeweils eine Zeile der Tabelle A. |

**Tabelle 537**

| Verbindungen der Formel I.1, mit | |
|---|---|
| R⁶ = | Ethyl; |
| R⁵ = | tert.-Butyl; |
| R⁴ = | jeweils eine Zeile der Tabelle A. |

**Tabelle 538**

| Verbindungen der Formel I.2, mit | |
|---|---|
| R⁶ = | Ethyl; |
| R⁵ = | tert.-Butyl; |
| R⁴ = | jeweils eine Zeile der Tabelle A. |

**Tabelle 539**

| Verbindungen der Formel I.3, mit | |
|---|---|
| R⁶ = | Ethyl; |
| R⁵ = | tert.-Butyl; |
| R⁴ = | jeweils eine Zeile der Tabelle A. |

**Tabelle 540**

| Verbindungen der Formel I.4, mit | |
|---|---|
| R⁶ = | Ethyl; |
| R⁵ = | tert.-Butyl; |
| R⁴ = | jeweils eine Zeile der Tabelle A. |

**Tabelle 541**

| Verbindungen der Formel I.1, mit | |
|---|---|
| R⁶ = | Ethyl; |
| R⁵ = | Trifluormethyl; |
| R⁴ = | jeweils eine Zeile der Tabelle A. |

**Tabelle 542**

| Verbindungen der Formel I.2, mit | |
|---|---|
| R⁶ = | Ethyl; |
| R⁵ = | Trifluormethyl; |
| R⁴ = | jeweils eine Zeile der Tabelle A. |

**Tabelle 543**

| Verbindungen der Formel I.3, mit | |
|---|---|
| R⁶ = | Ethyl; |
| R⁵ = | Trifluormethyl; |
| R⁴ = | jeweils eine Zeile der Tabelle A. |

**Tabelle 544**

| Verbindungen der Formel I.4, mit | |
|---|---|
| R⁶ = | Ethyl; |
| R⁵ = | Trifluormethyl; |
| R⁴ = | jeweils eine Zeile der Tabelle A. |

**Tabelle 545**

| Verbindungen der Formel I.1, mit | |
|---|---|
| R⁶ = | Ethyl; |
| R⁵ = | Phenoxymethyl; |
| R⁴ = | jeweils eine Zeile der Tabelle A. |

**Tabelle 546**

| Verbindungen der Formel I.2, mit | |
|---|---|
| R⁶ = | Ethyl; |
| R⁵ = | Phenoxymethyl; |
| R⁴ = | jeweils eine Zeile der Tabelle A. |

**Tabelle 547**

| Verbindungen der Formel I.3, mit | |
|---|---|
| R⁶ = | Ethyl; |
| R⁵ = | Phenoxymethyl; |
| R⁴ = | jeweils eine Zeile der Tabelle A. |

**Tabelle 548**

| Verbindungen der Formel I.4, mit | |
|---|---|
| R⁶ = | Ethyl; |
| R⁵ = | Phenoxymethyl; |
| R⁴ = | jeweils eine Zeile der Tabelle A. |

**Tabelle 549**

| Verbindungen der Formel I.1, mit | |
|---|---|
| R⁶ = | Ethyl; |
| R⁵ = | 2-Pyridyloxymethyl; |
| R⁴ = | jeweils eine Zeile der Tabelle A. |

**Tabelle 550**

| Verbindungen der Formel I.2, mit | |
|---|---|
| R⁶ = | Ethyl; |
| R⁵ = | 2-Pyridyloxymethyl; |
| R⁴ = | jeweils eine Zeile der Tabelle A. |

**Tabelle 551**

| Verbindungen der Formel I.3, mit | |
|---|---|
| R⁶ = | Ethyl; |
| R⁵ = | 2-Pyridyloxymethyl; |
| R⁴ = | jeweils eine Zeile der Tabelle A. |

**Tabelle 552**

| Verbindungen der Formel I.4, mit | |
|---|---|
| R⁶ = | Ethyl; |
| R⁵ = | 2-Pyridyloxymethyl; |
| R⁴ = | jeweils eine Zeile der Tabelle A. |

**Tabelle 553**

| Verbindungen der Formel I.1, mit | |
|---|---|
| R⁶ = | Ethyl; |
| R⁵ = | 5-Methylpyrazol-3-yloxymethyl; |
| R⁴ = | jeweils eine Zeile der Tabelle A. |

**Tabelle 554**

| Verbindungen der Formel I.2, mit | |
|---|---|
| R⁶ = | Ethyl; |
| R⁵ = | 5-Methylpyrazol-3-yloxymethyl; |
| R⁴ = | jeweils eine Zeile der Tabelle A. |

**Tabelle 555**

| Verbindungen der Formel I.3, mit | |
|---|---|
| R⁶ = | Ethyl; |
| R⁵ = | 5-Methylpyrazol-3-yloxymethyl; |
| R⁴ = | jeweils eine Zeile der Tabelle A. |

**Tabelle 556**

| Verbindungen der Formel I.4, mit | |
|---|---|
| R⁶ = | Ethyl; |
| R⁵ = | 5-Methylpyrazol-3-yloxymethyl; |
| R⁴ = | jeweils eine Zeile der Tabelle A. |

**Tabelle 557**

| Verbindungen der Formel I.1, mit | |
|---|---|
| R⁶ = | Ethyl; |
| R⁵ = | Ethenyl; |
| R⁴ = | jeweils eine Zeile der Tabelle A. |

**Tabelle 558**

| Verbindungen der Formel I.2, mit | |
|---|---|
| R⁶ = | Ethyl; |
| R⁵ = | Ethenyl; |
| R⁴ = | jeweils eine Zeile der Tabelle A. |

**Tabelle 559**

| Verbindungen der Formel I.3, mit | |
|---|---|
| R⁶ = | Ethyl; |
| R⁵ = | Ethenyl; |
| R⁴ = | jeweils eine Zeile der Tabelle A. |

**Tabelle 560**

| Verbindungen der Formel I.4, mit | |
|---|---|
| R⁶ = | Ethyl; |
| R⁵ = | Ethenyl; |
| R⁴ = | jeweils eine Zeile der Tabelle A. |

**Tabelle 561**

| Verbindungen der Formel I.1, mit | |
|---|---|
| R⁶ = | Ethyl; |
| R⁵ = | Ethinyl; |
| R⁴ = | jeweils eine Zeile der Tabelle A. |

**Tabelle 562**

| Verbindungen der Formel I.2, mit | |
|---|---|
| R⁶ = | Ethyl; |
| R⁵ = | Ethinyl; |
| R⁴ = | jeweils eine Zeile der Tabelle A. |

**Tabelle 563**

| Verbindungen der Formel I.3, mit | |
|---|---|
| R⁶ = | Ethyl; |
| R⁵ = | Ethinyl; |
| R⁴ = | jeweils eine Zeile der Tabelle A. |

**Tabelle 564**

| Verbindungen der Formel I.4, mit | |
|---|---|
| R⁶ = | Ethyl; |
| R⁵ = | Ethinyl; |
| R⁴ = | jeweils eine Zeile der Tabelle A. |

**Tabelle 565**

| Verbindungen der Formel I.1, mit | |
|---|---|
| R⁶ = | Ethyl; |
| R⁵ = | Phenyl; |
| R⁴ = | jeweils eine Zeile der Tabelle A. |

**Tabelle 566**

| Verbindungen der Formel I.2, mit | |
|---|---|
| R⁶ = | Ethyl; |
| R⁵ = | Phenyl; |
| R⁴ = | jeweils eine Zeile der Tabelle A. |

**Tabelle 567**

| Verbindungen der Formel I.3, mit | |
|---|---|
| R⁶ = | Ethyl; |
| R⁵ = | Phenyl; |
| R⁴ = | jeweils eine Zeile der Tabelle A. |

**Tabelle 568**

| Verbindungen der Formel I.4, mit | |
|---|---|
| R⁶ = | Ethyl; |
| R⁵ = | Phenyl; |
| R⁴ = | jeweils eine Zeile der Tabelle A. |

**Tabelle 569**

| Verbindungen der Formel I.1, mit | |
|---|---|
| R⁶ = | Ethyl; |
| R⁵ = | 4-Chlorphenyl; |
| R⁴ = | jeweils eine Zeile der Tabelle A. |

**Tabelle 570**

| Verbindungen der Formel I.2, mit | |
|---|---|
| R⁶ = | Ethyl; |
| R⁵ = | 4-Chlorphenyl; |
| R⁴ = | jeweils eine Zeile der Tabelle A. |

**Tabelle 571**

| Verbindungen der Formel I.3, mit | |
|---|---|
| R⁶ = | Ethyl; |
| R⁵ = | 4-Chlorphenyl; |
| R⁴ = | jeweils eine Zeile der Tabelle A. |

**Tabelle 572**

| Verbindungen der Formel I.4, mit | |
|---|---|
| R⁶ = | Ethyl; |
| R⁵ = | 4-Chlorphenyl; |
| R⁴ = | jeweils eine Zeile der Tabelle A. |

**Tabelle 573**

| Verbindungen der Formel I.1, mit | |
|---|---|
| R⁶ = | Ethyl; |
| R⁵ = | 4-Fluorphenyl; |
| R⁴ = | jeweils eine Zeile der Tabelle A. |

**Tabelle 574**

| Verbindungen der Formel I.2, mit | |
|---|---|
| R⁶ = | Ethyl; |
| R⁵ = | 4-Fluorphenyl; |
| R⁴ = | jeweils eine Zeile der Tabelle A. |

**Tabelle 575**

| Verbindungen der Formel I.3, mit | |
|---|---|
| R⁶ = | Ethyl; |
| R⁵ = | 4-Fluorphenyl; |
| R⁴ = | jeweils eine Zeile der Tabelle A. |

**Tabelle 576**

| Verbindungen der Formel I.4, mit | |
|---|---|
| R⁶ = | Ethyl; |
| R⁵ = | 4-Fluorphenyl; |
| R⁴ = | jeweils eine Zeile der Tabelle A. |

**Tabelle 577**

| Verbindungen der Formel I.1, mit | |
|---|---|
| R⁶ = | Ethyl; |
| R⁵ = | 2-Chlorpyrid-6-yl; |
| R⁴ = | jeweils eine Zeile der Tabelle A. |

**Tabelle 578**

| Verbindungen der Formel I.2, mit | |
|---|---|
| R⁶ = | Ethyl; |
| R⁵ = | 2-Chlorpyrid-6-yl; |
| R⁴ = | jeweils eine Zeile der Tabelle A. |

**Tabelle 579**

| Verbindungen der Formel I.3, mit | |
|---|---|
| R⁶ = | Ethyl; |
| R⁵ = | 2-Chlorpyrid-6-yl; |
| R⁴ = | jeweils eine Zeile der Tabelle A. |

**Tabelle 580**

| Verbindungen der Formel I.4, mit | |
|---|---|
| R⁶ = | Ethyl; |
| R⁵ = | 2-Chlorpyrid-6-yl; |
| R⁴ = | jeweils eine Zeile der Tabelle A. |

**Tabelle 581**

| Verbindungen der Formel I.1, mit | |
|---|---|
| R⁶ = | iso-Propyl; |
| R⁵ = | n-Propyl; |
| R⁴ = | jeweils eine Zeile der Tabelle A. |

**Tabelle 582**

| Verbindungen der Formel I.2, mit | |
|---|---|
| R⁶ = | iso-Propyl; |
| R⁵ = | n-Propyl; |
| R⁴ = | jeweils eine Zeile der Tabelle A. |

**Tabelle 583**

| Verbindungen der Formel I.3, mit | |
|---|---|
| R⁶ = | iso-Propyl; |
| R⁵ = | n-Propyl; |
| R⁴ = | jeweils eine Zeile der Tabelle A. |

**Tabelle 584**

| Verbindungen der Formel I.4, mit | |
|---|---|
| R⁶ = | iso-Propyl; |
| R⁵ = | n-Propyl; |
| R⁴ = | jeweils eine Zeile der Tabelle A. |

**Tabelle 585**

| Verbindungen der Formel I.1, mit | |
|---|---|
| R⁶ = | iso-Propyl; |
| R⁵ = | iso-Propyl; |
| R⁴ = | jeweils eine Zeile der Tabelle A. |

**Tabelle 586**

| Verbindungen der Formel I.2, mit | |
|---|---|
| R⁶ = | iso-Propyl; |
| R⁵ = | iso-Propyl; |
| R⁴ = | jeweils eine Zeile der Tabelle A. |

**Tabelle 587**

| Verbindungen der Formel I.3, mit | |
|---|---|
| R⁶ = | iso-Propyl; |
| R⁵ = | iso-Propyl; |
| R⁴ = | jeweils eine Zeile der Tabelle A. |

**Tabelle 588**

| Verbindungen der Formel I.4, mit | |
|---|---|
| R⁶ = | iso-Propyl; |
| R⁵ = | iso-Propyl; |
| R⁴ = | jeweils eine Zeile der Tabelle A. |

**Tabelle 589**

| Verbindungen der Formel I.1, mit | |
|---|---|
| R⁶ = | iso-Propyl; |
| R⁵ = | Trifluormethyl; |
| R⁴ = | jeweils eine Zeile der Tabelle A. |

**Tabelle 590**

| Verbindungen der Formel I.2, mit | |
|---|---|
| R⁶ = | iso-Propyl; |
| R⁵ = | Trifluormethyl; |
| R⁴ = | jeweils eine Zeile der Tabelle A. |

**Tabelle 591**

| Verbindungen der Formel I.3, mit | |
|---|---|
| R⁶ = | iso-Propyl; |
| R⁵ = | Trifluormethyl; |
| R⁴ = | jeweils eine Zeile der Tabelle A. |

**Tabelle 592**

| Verbindungen der Formel I.4, mit | |
|---|---|
| R⁶ = | iso-Propyl; |
| R⁵ = | Trifluormethyl; |
| R⁴ = | jeweils eine Zeile der Tabelle A. |

**Tabelle 593**

| Verbindungen der Formel I.1, mit | |
|---|---|
| R⁶ = | iso-Propyl; |
| R⁵ = | Phenoxymethyl; |
| R⁴ = | jeweils eine Zeile der Tabelle A. |

**Tabelle 594**

| Verbindungen der Formel I.2, mit | |
|---|---|
| R⁶ = | iso-Propyl; |
| R⁵ = | Phenoxymethyl; |
| R⁴ = | jeweils eine Zeile der Tabelle A. |

**Tabelle 595**

| Verbindungen der Formel I.3, mit | |
|---|---|
| R⁶ = | iso-Propyl; |
| R⁵ = | Phenoxymethyl; |
| R⁴ = | jeweils eine Zeile der Tabelle A. |

**Tabelle 596**

| Verbindungen der Formel I.4, mit | |
|---|---|
| R⁶ = | iso-Propyl; |
| R⁵ = | Phenoxymethyl; |
| R⁴ = | jeweils eine Zeile der Tabelle A. |

**Tabelle 597**

| Verbindungen der Formel I.1, mit | |
|---|---|
| R⁶ = | iso-Propyl; |
| R⁵ = | 4-Fluorphenoxymethyl; |
| R⁴ = | jeweils eine Zeile der Tabelle A. |

**Tabelle 598**

| Verbindungen der Formel I.2, mit | |
|---|---|
| R⁶ = | iso-Propyl; |
| R⁵ = | 4-Fluorphenoxymethyl; |
| R⁴ = | jeweils eine Zeile der Tabelle A. |

**Tabelle 599**

| Verbindungen der Formel I.3, mit | |
|---|---|
| R⁶ = | iso-Propyl; |
| R⁵ = | 4-Fluorphenoxymethyl; |
| R⁴ = | jeweils eine Zeile der Tabelle A. |

**Tabelle 600**

| Verbindungen der Formel I.4, mit | |
|---|---|
| R⁶ = | iso-Propyl; |
| R⁵ = | 4-Fluorphenoxymethyl; |
| R⁴ = | jeweils eine Zeile der Tabelle A. |

**Tabelle 601**

| Verbindungen der Formel I.1, mit | |
|---|---|
| R⁶ = | iso-Propyl; |
| R⁵ = | 2-Pyridyloxymethyl; |
| R⁴ = | jeweils eine Zeile der Tabelle A. |

**Tabelle 602**

| Verbindungen der Formel I.2, mit | |
|---|---|
| R⁶ = | iso-Propyl; |
| R⁵ = | 2-Pyridyloxymethyl; |
| R⁴ = | jeweils eine Zeile der Tabelle A. |

**Tabelle 603**

| Verbindungen der Formel I.3, mit | |
|---|---|
| R⁶ = | iso-Propyl; |
| R⁵ = | 2-Pyridyloxymethyl; |
| R⁴ = | jeweils eine Zeile der Tabelle A. |

**Tabelle 604**

| Verbindungen der Formel I.4, mit | |
|---|---|
| R⁶ = | iso-Propyl; |
| R⁵ = | 2-Pyridyloxymethyl; |
| R⁴ = | jeweils eine Zeile der Tabelle A. |

**Tabelle 605**

| Verbindungen der Formel I.1, mit | |
|---|---|
| R⁶ = | iso-Propyl; |
| R⁵ = | Ethenyl; |
| R⁴ = | jeweils eine Zeile der Tabelle A. |

**Tabelle 606**

| Verbindungen der Formel I.2, mit | |
|---|---|
| R⁶ = | iso-Propyl; |
| R⁵ = | Ethenyl; |
| R⁴ = | jeweils eine Zeile der Tabelle A. |

**Tabelle 607**

| Verbindungen der Formel I.3, mit | |
|---|---|
| R⁶ = | iso-Propyl; |
| R⁵ = | Ethenyl; |
| R⁴ = | jeweils eine Zeile der Tabelle A. |

**Tabelle 608**

| Verbindungen der Formel I.4, mit | |
|---|---|
| R⁶ = | iso-Propyl; |
| R⁵ = | Ethenyl; |
| R⁴ = | jeweils eine Zeile der Tabelle A. |

**Tabelle 609**

| Verbindungen der Formel I.1, mit | |
|---|---|
| R⁶ = | iso-Propyl; |
| R⁵ = | Prop-1-in-1-yl; |
| R⁴ = | jeweils eine Zeile der Tabelle A. |

**Tabelle 610**

| Verbindungen der Formel I.2, mit | |
|---|---|
| R⁶ = | iso-Propyl; |
| R⁵ = | Prop-1-in-1-yl; |
| R⁴ = | jeweils eine Zeile der Tabelle A. |

**Tabelle 611**

| Verbindungen der Formel I.3, mit | |
|---|---|
| R⁶ = | iso-Propyl; |
| R⁵ = | Prop-1-in-1-yl; |
| R⁴ = | jeweils eine Zeile der Tabelle A. |

**Tabelle 612**

| Verbindungen der Formel I.4, mit | |
|---|---|
| R⁶ = | iso-Propyl; |
| R⁵ = | Prop-1-in-1-yl; |
| R⁴ = | jeweils eine Zeile der Tabelle A. |

**Tabelle 613**

| Verbindungen der Formel I.1, mit | |
|---|---|
| R⁶ = | iso-Propyl; |
| R⁵ = | Phenyl; |
| R⁴ = | jeweils eine Zeile der Tabelle A. |

**Tabelle 614**

| Verbindungen der Formel I.2, mit | |
|---|---|
| R⁶ = | iso-Propyl; |
| R⁵ = | Phenyl; |
| R⁴ = | jeweils eine Zeile der Tabelle A. |

**Tabelle 615**

| Verbindungen der Formel I.3, mit | |
|---|---|
| R⁶ = | iso-Propyl; |
| R⁵ = | Phenyl; |
| R⁴ = | jeweils eine Zeile der Tabelle A. |

**Tabelle 616**

| Verbindungen der Formel I.4, mit | |
|---|---|
| R⁶ = | iso-Propyl; |
| R⁵ = | Phenyl; |
| R⁴ = | jeweils eine Zeile der Tabelle A. |

**Tabelle 617**

| Verbindungen der Formel I.1, mit | |
|---|---|
| R⁶ = | iso-Propyl; |
| R⁵ = | 3-Cyanophenyl; |
| R⁴ = | jeweils eine Zeile der Tabelle A. |

**Tabelle 618**

| Verbindungen der Formel I.2, mit | |
|---|---|
| R⁶ = | iso-Propyl; |
| R⁵ = | 3-Cyanophenyl; |
| R⁴ = | jeweils eine Zeile der Tabelle A. |

**Tabelle 619**

| Verbindungen der Formel I.3, mit | |
|---|---|
| R⁶ = | iso-Propyl; |
| R⁵ = | 3-Cyanophenyl; |
| R⁴ = | jeweils eine Zeile der Tabelle A. |

**Tabelle 620**

| Verbindungen der Formel I.4, mit | |
|---|---|
| R⁶ = | iso-Propyl; |
| R⁵ = | 3-Cyanophenyl; |
| R⁴ = | jeweils eine Zeile der Tabelle A. |

**Tabelle 621**

| Verbindungen der Formel I.1, mit | |
|---|---|
| R⁶ = | iso-Propyl; |
| R⁵ = | 2-Chlorpyrid-6-yl; |
| R⁴ = | jeweils eine Zeile der Tabelle A. |

**Tabelle 622**

| Verbindungen der Formel I.2, mit | |
|---|---|
| R⁶ = | iso-Propyl; |
| R⁵ = | 2-Chlorpyrid-6-yl; |
| R⁴ = | jeweils eine Zeile der Tabelle A. |

**Tabelle 623**

| Verbindungen der Formel I.3, mit | |
|---|---|
| R⁶ = | iso-Propyl; |
| R⁵ = | 2-Chlorpyrid-6-yl; |
| R⁴ = | jeweils eine Zeile der Tabelle A. |

**Tabelle 624**

| Verbindungen der Formel I.4, mit | |
|---|---|
| R⁶ = | iso-Propyl; |
| R⁵ = | 2-Chlorpyrid-6-yl; |
| R⁴ = | jeweils eine Zeile der Tabelle A. |

**Tabelle 625**

| Verbindungen der Formel I.1, mit | |
|---|---|
| R⁶ = | Cyclopropyl; |
| R⁵ = | Cyclopropyl; |
| R⁴ = | jeweils eine Zeile der Tabelle A. |

**Tabelle 626**

| Verbindungen der Formel I.2, mit | |
|---|---|
| R⁶ = | Cyclopropyl; |
| R⁵ = | Cyclopropyl; |
| R⁴ = | jeweils eine Zeile der Tabelle A. |

**Tabelle 627**

| Verbindungen der Formel I.3, mit | |
|---|---|
| R⁶ = | Cyclopropyl; |
| R⁵ = | Cyclopropyl; |
| R⁴ = | jeweils eine Zeile der Tabelle A. |

**Tabelle 628**

| Verbindungen der Formel I.4, mit | |
|---|---|
| R⁶ = | Cyclopropyl; |
| R⁵ = | Cyclopropyl; |
| R⁴ = | jeweils eine Zeile der Tabelle A. |

**Tabelle 629**

| Verbindungen der Formel I.1, mit | |
|---|---|
| R⁶ = | Cyclopropyl; |
| R⁵ = | Trifluormethyl; |
| R⁴ = | jeweils eine Zeile der Tabelle A. |

**Tabelle 630**

| Verbindungen der Formel I.2, mit | |
|---|---|
| R⁶ = | Cyclopropyl; |
| R⁵ = | Trifluormethyl; |
| R⁴ = | jeweils eine Zeile der Tabelle A. |

**Tabelle 631**

| Verbindungen der Formel I.3, mit | |
|---|---|
| R⁶ = | Cyclopropyl; |
| R⁵ = | Trifluormethyl; |
| R⁴ = | jeweils eine Zeile der Tabelle A. |

**Tabelle 632**

| Verbindungen der Formel I.4, mit | |
|---|---|
| R⁶ = | Cyclopropyl; |
| R⁵ = | Trifluormethyl; |
| R⁴ = | jeweils eine Zeile der Tabelle A. |

**Tabelle 633**

| Verbindungen der Formel I.1, mit | |
|---|---|
| R⁶ = | Cyclopropyl; |
| R⁵ = | Phenoxymethyl; |
| R⁴ = | jeweils eine Zeile der Tabelle A. |

**Tabelle 634**

| Verbindungen der Formel I.2, mit | |
|---|---|
| R⁶ = | Cyclopropyl; |
| R⁵ = | Phenoxymethyl; |
| R⁴ = | jeweils eine Zeile der Tabelle A. |

**Tabelle 635**

| Verbindungen der Formel I.3, mit | |
|---|---|
| R⁶ = | Cyclopropyl; |
| R⁵ = | Phenoxymethyl; |
| R⁴ = | jeweils eine Zeile der Tabelle A. |

**Tabelle 636**

| Verbindungen der Formel I.4, mit | |
|---|---|
| R⁶ = | Cyclopropyl; |
| R⁵ = | Phenoxymethyl; |
| R⁴ = | jeweils eine Zeile der Tabelle A. |

**Tabelle 637**

| Verbindungen der Formel I.1, mit | |
|---|---|
| R⁶ = | Cyclopropyl; |
| R⁵ = | 2-Pyridinyloxymethyl; |
| R⁴ = | jeweils eine Zeile der Tabelle A. |

**Tabelle 638**

| Verbindungen der Formel I.2, mit | |
|---|---|
| R⁶ = | Cyclopropyl; |
| R⁵ = | 2-Pyridinyloxymethyl; |
| R⁴ = | jeweils eine Zeile der Tabelle A. |

**Tabelle 639**

| Verbindungen der Formel I.3, mit | |
|---|---|
| R⁶ = | Cyclopropyl; |
| R⁵ = | 2-Pyridinyloxymethyl; |
| R⁴ = | jeweils eine Zeile der Tabelle A. |

**Tabelle 640**

| Verbindungen der Formel I.4, mit | |
|---|---|
| R⁶ = | Cyclopropyl; |
| R⁵ = | 2-Pyridinyloxymethyl; |
| R⁴ = | jeweils eine Zeile der Tabelle A. |

**Tabelle 641**

| Verbindungen der Formel I.1, mit | |
|---|---|
| R⁶ = | Cyclopropyl; |
| R⁵ = | Ethenyl; |
| R⁴ = | jeweils eine Zeile der Tabelle A. |

**Tabelle 642**

| Verbindungen der Formel I.2, mit | |
|---|---|
| R⁶ = | Cyclopropyl; |
| R⁵ = | Ethenyl; |
| R⁴ = | jeweils eine Zeile der Tabelle A. |

**Tabelle 643**

| Verbindungen der Formel I.3, mit | |
|---|---|
| R⁶ = | Cyclopropyl; |
| R⁵ = | Ethenyl; |
| R⁴ = | jeweils eine Zeile der Tabelle A. |

**Tabelle 644**

| Verbindungen der Formel I.4, mit | |
|---|---|
| R⁶ = | Cyclopropyl; |
| R⁵ = | Ethenyl; |
| R⁴ = | jeweils eine Zeile der Tabelle A. |

**Tabelle 645**

| Verbindungen der Formel I.1, mit | |
|---|---|
| R⁶ = | Cyclopropyl; |
| R⁵ = | Ethinyl; |
| R⁴ = | jeweils eine Zeile der Tabelle A. |

**Tabelle 646**

| Verbindungen der Formel I.2, mit | |
|---|---|
| R⁶ = | Cyclopropyl; |
| R⁵ = | Ethinyl; |
| R⁴ = | jeweils eine Zeile der Tabelle A. |

**Tabelle 647**

| Verbindungen der Formel I.3, mit | |
|---|---|
| R⁶ = | Cyclopropyl; |
| R⁵ = | Ethinyl; |
| R⁴ = | jeweils eine Zeile der Tabelle A. |

**Tabelle 648**

| Verbindungen der Formel I.4, mit | |
|---|---|
| R⁶ = | Cyclopropyl; |
| R⁵ = | Ethinyl; |
| R⁴ = | jeweils eine Zeile der Tabelle A. |

**Tabelle 649**

| Verbindungen der Formel I.1, mit | |
|---|---|
| R⁶ = | Cyclopropyl; |
| R⁵ = | Phenyl; |
| R⁴ = | jeweils eine Zeile der Tabelle A. |

**Tabelle 650**

| Verbindungen der Formel I.2, mit | |
|---|---|
| R⁶ = | Cyclopropyl; |
| R⁵ = | Phenyl; |
| R⁴ = | jeweils eine Zeile der Tabelle A. |

**Tabelle 651**

| Verbindungen der Formel I.3, mit | |
|---|---|
| R⁶ = | Cyclopropyl; |
| R⁵ = | Phenyl; |
| R⁴ = | jeweils eine Zeile der Tabelle A. |

**Tabelle 652**

| Verbindungen der Formel I.4, mit | |
|---|---|
| R⁶ = | Cyclopropyl; |
| R⁵ = | Phenyl; |
| R⁴ = | jeweils eine Zeile der Tabelle A. |

**Tabelle 653**

| Verbindungen der Formel I.1, mit | |
|---|---|
| R⁶ = | Cyclopropyl; |
| R⁵ = | 4-Chlorphenyl; |
| R⁴ = | jeweils eine Zeile der Tabelle A. |

**Tabelle 654**

| Verbindungen der Formel I.2, mit | |
|---|---|
| R⁶ = | Cyclopropyl; |
| R⁵ = | 4-Chlorphenyl; |
| R⁴ = | jeweils eine Zeile der Tabelle A. |

**Tabelle 655**

| Verbindungen der Formel I.3, mit | |
|---|---|
| R⁶ = | Cyclopropyl; |
| R⁵ = | 4-Chlorphenyl; |
| R⁴ = | jeweils eine Zeile der Tabelle A. |

**Tabelle 656**

| Verbindungen der Formel I.4, mit | |
|---|---|
| R⁶ = | Cyclopropyl; |
| R⁵ = | 4-Chlorphenyl; |
| R⁴ = | jeweils eine Zeile der Tabelle A. |

**Tabelle 657**

| Verbindungen der Formel I.1, mit | |
|---|---|
| R⁶ = | Cyclopropyl; |
| R⁵ = | 4-Fluorphenyl; |
| R⁴ = | jeweils eine Zeile der Tabelle A. |

**Tabelle 658**

| Verbindungen der Formel I.2, mit | |
|---|---|
| R⁶ = | Cyclopropyl; |
| R⁵ = | 4-Fluorphenyl; |
| R⁴ = | jeweils eine Zeile der Tabelle A. |

**Tabelle 659**

| Verbindungen der Formel I.3, mit | |
|---|---|
| R⁶ = | Cyclopropyl; |
| R⁵ = | 4-Fluorphenyl; |
| R⁴ = | jeweils eine Zeile der Tabelle A. |

**Tabelle 660**

| Verbindungen der Formel I.4, mit | |
|---|---|
| R⁶ = | Cyclopropyl; |
| R⁵ = | 4-Fluorphenyl; |
| R⁴ = | jeweils eine Zeile der Tabelle A. |

**Tabelle 661**

| Verbindungen der Formel I.1, mit | |
|---|---|
| R⁶ = | Cyclopropyl; |
| R⁵ = | 2-Methylpyrid-6-yl; |
| R⁴ = | jeweils eine Zeile der Tabelle A. |

**Tabelle 662**

| Verbindungen der Formel I.2, mit | |
|---|---|
| R⁶ = | Cyclopropyl; |
| R⁵ = | 2-Methylpyrid-6-yl; |
| R⁴ = | jeweils eine Zeile der Tabelle A. |

**Tabelle 663**

| Verbindungen der Formel I.3, mit | |
|---|---|
| R⁶ = | Cyclopropyl; |
| R⁵ = | 2-Methylpyrid-6-yl; |
| R⁴ = | jeweils eine Zeile der Tabelle A. |

**Tabelle 664**

| Verbindungen der Formel I.4, mit | |
|---|---|
| R⁶ = | Cyclopropyl; |
| R⁵ = | 2-Methylpyrid-6-yl; |
| R⁴ = | jeweils eine Zeile der Tabelle A. |

**Tabelle 665**

| Verbindungen der Formel I.1, mit | |
|---|---|
| R⁶ = | Trifluormethyl; |
| R⁵ = | n-Propyl; |
| R⁴ = | jeweils eine Zeile der Tabelle A. |

**Tabelle 666**

| Verbindungen der Formel I.2, mit | |
|---|---|
| R⁶ = | Trifluormethyl; |
| R⁵ = | n-Propyl; |
| R⁴ = | jeweils eine Zeile der Tabelle A. |

**Tabelle 667**

| Verbindungen der Formel I.3, mit | |
|---|---|
| R⁶ = | Trifluormethyl; |
| R⁵ = | n-Propyl; |
| R⁴ = | jeweils eine Zeile der Tabelle A. |

**Tabelle 668**

| Verbindungen der Formel I.4, mit | |
|---|---|
| R⁶ = | Trifluormethyl; |
| R⁵ = | n-Propyl; |
| R⁴ = | jeweils eine Zeile der Tabelle A. |

**Tabelle 669**

| Verbindungen der Formel I.1, mit | |
|---|---|
| R⁶ = | Trifluormethyl; |
| R⁵ = | tert.-Butyl; |
| R⁴ = | jeweils eine Zeile der Tabelle A. |

**Tabelle 670**

| Verbindungen der Formel I.2, mit | |
|---|---|
| R⁶ = | Trifluormethyl; |
| R⁵ = | tert.-Butyl; |
| R⁴ = | jeweils eine Zeile der Tabelle A. |

**Tabelle 671**

| Verbindungen der Formel I.3, mit | |
|---|---|
| R⁶ = | Trifluormethyl; |
| R⁵ = | tert.-Butyl; |
| R⁴ = | jeweils eine Zeile der Tabelle A. |

**Tabelle 672**

| Verbindungen der Formel I.4, mit | |
|---|---|
| R⁶ = | Trifluormethyl; |
| R⁵ = | tert.-Butyl; |
| R⁴ = | jeweils eine Zeile der Tabelle A. |

**Tabelle 673**

| Verbindungen der Formel I.1, mit | |
|---|---|
| R⁶ = | Trifluormethyl; |
| R⁵ = | Trifluormethyl; |
| R⁴ = | jeweils eine Zeile der Tabelle A. |

**Tabelle 674**

| Verbindungen der Formel I.2, mit | |
|---|---|
| R⁶ = | Trifluormethyl; |
| R⁵ = | Trifluormethyl; |
| R⁴ = | jeweils eine Zeile der Tabelle A. |

**Tabelle 675**

| Verbindungen der Formel I.3, mit | |
|---|---|
| R⁶ = | Trifluormethyl; |
| R⁵ = | Trifluormethyl; |
| R⁴ = | jeweils eine Zeile der Tabelle A. |

**Tabelle 676**

| Verbindungen der Formel I.4, mit | |
|---|---|
| R⁶ = | Trifluormethyl; |
| R⁵ = | Trifluormethyl; |
| R⁴ = | jeweils eine Zeile der Tabelle A. |

**Tabelle 677**

| Verbindungen der Formel I.1, mit | |
|---|---|
| R⁶ = | Trifluormethyl; |
| R⁵ = | Phenoxymethyl; |
| R⁴ = | jeweils eine Zeile der Tabelle A. |

**Tabelle 678**

| Verbindungen der Formel I.2, mit | |
|---|---|
| R⁶ = | Trifluormethyl; |
| R⁵ = | Phenoxymethyl; |
| R⁴ = | jeweils eine Zeile der Tabelle A. |

**Tabelle 679**

| Verbindungen der Formel I.3, mit | |
|---|---|
| R⁶ = | Trifluormethyl; |
| R⁵ = | Phenoxymethyl; |
| R⁴ = | jeweils eine Zeile der Tabelle A. |

**Tabelle 680**

| Verbindungen der Formel I.4, mit | |
|---|---|
| R⁶ = | Trifluormethyl; |
| R⁵ = | Phenoxymethyl; |
| R⁴ = | jeweils eine Zeile der Tabelle A. |

**Tabelle 681**

| Verbindungen der Formel I.1, mit | |
|---|---|
| R⁶ = | Trifluormethyl; |
| R⁵ = | Phenyl; |
| R⁴ = | jeweils eine Zeile der Tabelle A. |

**Tabelle 682**

| Verbindungen der Formel I.2, mit | |
|---|---|
| R⁶ = | Trifluormethyl; |
| R⁵ = | Phenyl; |
| R⁴ = | jeweils eine Zeile der Tabelle A. |

**Tabelle 683**

| Verbindungen der Formel I.3, mit | |
|---|---|
| R⁶ = | Trifluormethyl; |
| R⁵ = | Phenyl; |
| R⁴ = | jeweils eine Zeile der Tabelle A. |

**Tabelle 684**

| Verbindungen der Formel I.4, mit | |
|---|---|
| R⁶ = | Trifluormethyl; |
| R⁵ = | Phenyl; |
| R⁴ = | jeweils eine Zeile der Tabelle A. |

**Tabelle 685**

| Verbindungen der Formel I.1, mit | |
|---|---|
| R⁶ = | Trifluormethyl; |
| R⁵ = | 4-Fluorphenyl; |
| R⁴ = | jeweils eine Zeile der Tabelle A. |

**Tabelle 686**

| Verbindungen der Formel I.2, mit | |
|---|---|
| R⁶ = | Trifluormethyl; |
| R⁵ = | 4-Fluorphenyl; |
| R⁴ = | jeweils eine Zeile der Tabelle A. |

**Tabelle 687**

| Verbindungen der Formel I.3, mit | |
|---|---|
| R⁶ = | Trifluormethyl; |
| R⁵ = | 4-Fluorphenyl; |
| R⁴ = | jeweils eine Zeile der Tabelle A. |

**Tabelle 688**

| Verbindungen der Formel I.4, mit | |
|---|---|
| R⁶ = | Trifluormethyl; |
| R⁵ = | 4 -Fluorphenyl; |
| R⁴ = | jeweils eine Zeile der Tabelle A. |

**Tabelle 689**

| Verbindungen der Formel I.1, mit | |
|---|---|
| R⁶ = | Trifluormethyl; |
| R⁵ = | 4-Chlorphenyl; |
| R⁴ = | jeweils eine Zeile der Tabelle A. |

**Tabelle 690**

| Verbindungen der Formel I.2, mit | |
|---|---|
| R⁶ = | Trifluormethyl; |
| R⁵ = | 4-chlorphenyl; |
| R⁴ = | jeweils eine Zeile der Tabelle A. |

**Tabelle 691**

| Verbindungen der Formel I.3, mit | |
|---|---|
| R⁶ = | Trifluormethyl; |
| R⁵ = | 4-Chlorphenyl; |
| R⁴ = | jeweils eine Zeile der Tabelle A. |

**Tabelle 692**

| Verbindungen der Formel I.4, mit | |
|---|---|
| R⁶ = | Trifluormethyl; |
| R⁵ = | 4-Chlorphenyl; |
| R⁴ = | jeweils eine Zeile der Tabelle A. |

**Tabelle 693**

| Verbindungen der Formel I.1, mit | |
|---|---|
| R⁶ = | Trifluormethyl; |
| R⁵ = | 3-Methoxyphenyl; |
| R⁴ = | jeweils eine Zeile der Tabelle A. |

**Tabelle 694**

| Verbindungen der Formel I.2, mit | |
|---|---|
| R⁶ = | Trifluormethyl; |
| R⁵ = | 3-Methoxyphenyl; |
| R⁴ = | jeweils eine Zeile der Tabelle A. |

**Tabelle 695**

| Verbindungen der Formel I.3, mit | |
|---|---|
| R⁶ = | Trifluormethyl; |
| R⁵ = | 3-Methoxyphenyl; |
| R⁴ = | jeweils eine Zeile der Tabelle A. |

**Tabelle 696**

| Verbindungen der Formel I.4, mit | |
|---|---|
| R⁶ = | Trifluormethyl; |
| R⁵ = | 3-Methoxyphenyl; |
| R⁴ = | jeweils eine Zeile der Tabelle A. |

**Tabelle 697**

| Verbindungen der Formel I.1, mit | |
|---|---|
| R⁶ = | Trifluormethyl; |
| R⁵ = | 2-Chlorpyrid-6-yl; |
| R⁴ = | jeweils eine Zeile der Tabelle A. |

**Tabelle 698**

| Verbindungen der Formel I.2, mit | |
|---|---|
| R⁶ = | Trifluormethyl; |
| R⁵ = | 2-Chlorpyrid-6-yl; |
| R⁴ = | jeweils eine Zeile der Tabelle A. |

**Tabelle 699**

| Verbindungen der Formel I.3, mit | |
|---|---|
| R⁶ = | Trifluormethyl; |
| R⁵ = | 2-Chlorpyrid-6-yl; |
| R⁴ = | jeweils eine Zeile der Tabelle A. |

**Tabelle 700**

| Verbindungen der Formel I.4, mit | |
|---|---|
| R⁶ = | Trifluormethyl; |
| R⁵ = | 2-Chlorpyrid-6-yl; |
| R⁴ = | jeweils eine Zeile der Tabelle A. |

**Tabelle 701**

| Verbindungen der Formel I.1, mit | |
|---|---|
| R⁶ = | Phenyl; |
| R⁵ = | Phenyl; |
| R⁴ = | jeweils eine Zeile der Tabelle A. |

**Tabelle 702**

| Verbindungen der Formel I.2, mit | |
|---|---|
| R⁶ = | Phenyl; |
| R⁵ = | Phenyl; |
| R⁴ = | jeweils eine Zeile der Tabelle A. |

**Tabelle 703**

| Verbindungen der Formel I.3, mit | |
|---|---|
| R⁶ = | Phenyl; |
| R⁵ = | Phenyl; |
| R⁴ = | jeweils eine Zeile der Tabelle A. |

**Tabelle 704**

| Verbindungen der Formel I.4, mit | |
|---|---|
| R⁶ = | Phenyl; |
| R⁵ = | Phenyl; |
| R⁴ = | jeweils eine Zeile der Tabelle A. |

**Tabelle 705**

| Verbindungen der Formel I.1, mit | |
|---|---|
| R⁶ = | Phenyl; |
| R⁵ = | 4-Fluorphenyl; |
| R⁴ = | jeweils eine Zeile der Tabelle A. |

**Tabelle 706**

| Verbindungen der Formel I.2, mit | |
|---|---|
| R⁶ = | Phenyl; |
| R⁵ = | 4-Fluorphenyl; |
| R⁴ = | jeweils eine Zeile der Tabelle A. |

**Tabelle 707**

| Verbindungen der Formel I.3, mit | |
|---|---|
| R⁶ = | Phenyl; |
| R⁵ = | 4-Fluorphenyl; |
| R⁴ = | jeweils eine Zeile der Tabelle A. |

**Tabelle 708**

| Verbindungen der Formel I.4, mit | |
|---|---|
| R⁶ = | Phenyl; |
| R⁵ = | 4-Fluorphenyl; |
| R⁴ = | jeweils eine Zeile der Tabelle A. |

**Tabelle 709**

| Verbindungen der Formel I.1, mit | |
|---|---|
| R⁶ = | Methoxymethyl; |
| R⁵ = | Phenyl; |
| R⁴ = | jeweils eine Zeile der Tabelle A. |

**Tabelle 710**

| Verbindungen der Formel I.2, mit | |
|---|---|
| R⁶ = | Methoxymethyl; |
| R⁵ = | Phenyl; |
| R⁴ = | jeweils eine Zeile der Tabelle A. |

**Tabelle 711**

| Verbindungen der Formel I.3, mit | |
|---|---|
| R⁶ = | Methoxymethyl; |
| R⁵ = | Phenyl; |
| R⁴ = | jeweils eine Zeile der Tabelle A. |

**Tabelle 712**

| Verbindungen der Formel I.4, mit | |
|---|---|
| R⁶ = | Methoxymethyl; |
| R⁵ = | Phenyl; |
| R⁴ = | jeweils eine Zeile der Tabelle A. |

**Tabelle 713**

| Verbindungen der Formel I.1, mit | |
|---|---|
| R⁶ = | Methoxymethyl; |
| R⁵ = | 4-Chlorphenyl; |
| R⁴ = | jeweils eine Zeile der Tabelle A. |

**Tabelle 714**

| Verbindungen der Formel I.2, mit | |
|---|---|
| R⁶ = | Methoxymethyl; |
| R⁵ = | 4-Chlorphenyl; |
| R⁴ = | jeweils eine Zeile der Tabelle A. |

**Tabelle 715**

| Verbindungen der Formel I.3, mit | |
|---|---|
| R⁶ = | Methoxymethyl; |
| R⁵ = | 4-Chlorphenyl; |
| R⁴ = | jeweils eine Zeile der Tabelle A. |

**Tabelle 716**

| Verbindungen der Formel I.4, mit | |
|---|---|
| R⁶ = | Methoxymethyl; |
| R⁵ = | 4-Chlorphenyl; |
| R⁴ = | jeweils eine Zeile der Tabelle A. |

**Tabelle 717**

| Verbindungen der Formel I.1, mit | |
|---|---|
| R⁶ = | 2-Pyrimidinyl; |
| R⁵ = | Phenyl; |
| R⁴ = | jeweils eine Zeile der Tabelle A. |

**Tabelle 718**

| Verbindungen der Formel I.2, mit | |
|---|---|
| R⁶ = | 2-Pyrimidinyl; |
| R⁵ = | Phenyl; |
| R⁴ = | jeweils eine Zeile der Tabelle A. |

**Tabelle 719**

| Verbindungen der Formel I.3, mit | |
|---|---|
| R⁶ = | 2-Pyrimidinyl; |
| R⁵ = | Phenyl; |
| R⁴ = | jeweils eine Zeile der Tabelle A. |

**Tabelle 720**

| Verbindungen der Formel I.4, mit | |
|---|---|
| R⁶ = | 2-Pyrimidinyl; |
| R⁵ = | Phenyl; |
| R⁴ = | jeweils eine Zeile der Tabelle A. |

**Tabelle 721**

| Verbindungen der Formel I.1, mit | |
|---|---|
| R⁶ = | Cyanomethyl; |
| R⁵ = | Phenyl; |
| R⁴ = | jeweils eine Zeile der Tabelle A. |

**Tabelle 722**

| Verbindungen der Formel I.2, mit | |
|---|---|
| R⁶ = | Cyanomethyl; |
| R⁵ = | Phenyl; |
| R⁴ = | jeweils eine Zeile der Tabelle A. |

**Tabelle 723**

| Verbindungen der Formel I.3, mit | |
|---|---|
| R⁶ = | Cyanomethyl; |
| R⁵ = | Phenyl; |
| R⁴ = | jeweils eine Zeile der Tabelle A. |

**Tabelle 724**

| Verbindungen der Formel I.4, mit | |
|---|---|
| R⁶ = | Cyanomethyl; |
| R⁵ = | Phenyl; |
| R⁴ = | jeweils eine Zeile der Tabelle A. |

**Tabelle 725**

| Verbindungen der Formel I.1, mit | |
|---|---|
| R⁶ = | n-Propyl; |
| R⁵ = | Phenyl; |
| R⁴ = | jeweils eine Zeile der Tabelle A. |

**Tabelle 726**

| Verbindungen der Formel I.2, mit | |
|---|---|
| R⁶ = | n-Propyl; |
| R⁵ = | Phenyl; |
| R⁴ = | jeweils eine Zeile der Tabelle A. |

**Tabelle 727**

| Verbindungen der Formel I.3, mit | |
|---|---|
| R⁶ = | n-Propyl; |
| R⁵ = | Phenyl; |
| R⁴ = | jeweils eine Zeile der Tabelle A. |

**Tabelle 728**

| Verbindungen der Formel I.4, mit | |
|---|---|
| R⁶ = | n-Propyl; |
| R⁵ = | Phenyl; |
| R⁴ = | jeweils eine Zeile der Tabelle A. |

**Tabelle A**

| Nr. | R⁴ |
|---|---|
| 1 | H |
| 2 | CH₃ |
| 3 | C₂H₅ |
| 4 | n-C₃H₇ |
| 5 | i-C₃H₇ |
| 6 | Cyclopropyl |
| 7 | n-C₄H₉ |
| 8 | s-C₄H₉ |
| 9 | i-C₄H₉ |
| 10 | t-C₄H₉ |
| 11 | n-C₅H₁₁ |
| 12 | i-C₅H₁₁ |
| 13 | neo-C₅H₁₁ |
| 14 | Cyclopentyl |
| 15 | n-C₆H₁₃ |
| 16 | Cyclohexyl |
| 17 | Cyclobutyl |
| 18 | CH₂CH₂Cl |
| 19 | (CH₂)₄Cl |
| 20 | CH₂CN |
| 21 | CH₂CH₂CN |
| 22 | (CH₂)₃CN |
| 23 | (CH₂)₄CN |
| 24 | (CH₂)₆CN |
| 25 | Cyclohexylmethyl |
| 26 | 2-Cyclohexyleth-1-yl |
| 27 | Cyclopropylmethyl |
| 28 | 2-Cyclopropyleth-1-yl |
| 29 | 2-Methoxyeth-1-yl |
| 30 | 2-Ethoxyeth-1-yl |
| 31 | 2-Isopropoxyeth-1-yl |
| 32 | 3-Methoxyprop-1-yl |
| 33 | 3-Ethoxyprop-1-yl |
| 34 | 3-Isopropoxyprop-1-yl |
| 35 | 4-Methoxybut-1-yl |
| 36 | 4 -Isopropoxybut-1-yl |
| 37 | Prop-2-en-1-yl |
| 38 | But-2-en-1-yl |
| 39 | 3-Methylbut-2-en-1-yl |
| 40 | 2-Vinyloxyeth-1-yl |
| 41 | Allyloxyeth-1-yl |
| 42 | 2-Trifluormethoxyeth-1-yl |
| 43 | 3-Trifluormethoxyprop-1-yl |
| 44 | 4-Difluormethoxybut-1-yl |
| 45 | Hydroxycarbonylmethyl |
| 46 | Methoxycarbonylmethyl |
| 47 | Aminocarbonylmethyl |
| 48 | N-Methylaminocarbonylmethyl |
| 49 | N,N-Dimethylaminocarbonyl-methyl |
| 50 | 2-Hydroxycarbonyleth-1-yl |
| 51 | 2-Methoxycarbonyleth-1-yl |
| 52 | 2-Aminocarbonyleth-1-yl |
| 53 | 2-N-Methylaminocarbonyleth-1-yl |
| 54 | 2-Dimethylaminocarbonyleth-1-yl |
| 55 | 2-Aminoeth-1-yl |
| 56 | 2-Aminoprop-1-yl |
| 57 | 4-Aminobut-1-yl |
| 58 | 3-Dimethylaminoprop-1-yl |
| 59 | 4-Aminothiocarbonylbut-1-yl |
| 60 | E-3-Chlorprop-2-en-1-yl |
| 61 | Z-3-Chlorprop-2-en-1-yl |
| 62 | Prop-2-in-1-yl |
| 63 | But-2-in-1-yl |
| 64 | But-3-in-1-yl |
| 65 | 3-Chlorprop-2-in-1-yl |
| 66 | 6-Aminocarbonylhex-1-yl |
| 67 | 3-Aminothiocarbonylprop-1-yl |
| 68 | 2-Aminothiocarbonyleth-1-yl |
| 69 | Aminothiocarbonylmethyl |
| 70 | 4-(N,N-Dimethylamino)but-1-yl |
| 71 | 2-(Methylthio)eth-1-yl |
| 72 | 2-(Methylsulfonyl)eth-1-yl |
| 73 | 4-(Methylthio)prop-1-yl |
| 74 | 4-(Methylsulfonyl)prop-1-yl |
| 75 | Benyzl |
| 76 | 2-F-C₆H₄-CH₂ |
| 77 | 3-F-C₆H₄-CH₂ |
| 78 | 4-F-C₆H₄-CH₂ |
| 79 | 2,3-F₂-C₆H₃-CH₂ |
| 80 | 2,4-F₂-C₆H₃-CH₂ |
| 81 | 2,5-F₂-C₆H₃-CH₂ |
| 82 | 2,6-F₂-C₆H₃-CH₂ |
| 83 | 3,4-F₂-C₆H₃-CH₂ |
| 84 | 3,5-F₂-C₆H₃-CH₂ |
| 85 | 2-Cl-C₆H₄-CH₂ |
| 86 | 3-Cl-C₆H₄-CH₂ |
| 87 | 4-Cl-C₆H₄-CH₂ |
| 88 | 2,3-Cl₂-C₆H₃-CH₂ |
| 89 | 2,4-Cl₂-C₆H₃-CH₂ |
| 90 | 2,5-Cl₂-C₆H₃-CH₂ |
| 91 | 2,6-Cl₂-C₆H₃-CH₂ |
| 92 | 3,4-Cl₂-C₆H₃-CH₂ |
| 93 | 3,5-Cl₂-C₆H₃-CH₂ |
| 94 | 2,3,4-Cl₃-C₆H₂-CH₂ |
| 95 | 2,3,5-Cl₃-C₆H₂-CH₂ |
| 96 | 2,3,6-Cl₃-C₆H₂-CH₂ |
| 97 | 2,4,5-Cl₃-C₆H₂-CH₂ |
| 98 | 2,4,6-Cl₃-C₆H₂-CH₂ |
| 99 | 3,4,5-Cl₃-C₆H₂-CH₂ |
| 100 | 2-Br-C₆H₄-CH₂ |
| 101 | 3-Br-C₆H₄-CH₂ |
| 102 | 4-Br-C₆H₄-CH₂ |
| 103 | 2,3-Br₂-C₆H₃-CH₂ |
| 104 | 2,4-Br₂-C₆H₃-CH₂ |
| 105 | 2,5-Br₂-C₆H₃-CH₂ |
| 106 | 2,6-Br₂-C₆H₃-CH₂ |
| 107 | 3,4-Br₂-C₆H₃-CH₂ |
| 108 | 3,5-Br₂-C₆H₃-CH₂ |
| 109 | 2-F, 3-Cl-C₆H₃-CH₂ |
| 110 | 2-F, 4-Cl-C₆H₃-CH₂ |
| 111 | 2-F, 5-Cl-C₆H₃-CH₂ |
| 112 | 2-F, 3-Br-C₆H₃-CH₂ |
| 113 | 2-F, 4-Br-C₆H₃-CH₂ |
| 114 | 2-F, 5-Br-C₆H₃-CH₂ |
| 115 | 2-Cl, 3-Br-C₆H₃-CH₂ |
| 116 | 2-Cl, 4-Br-C₆H₃-CH₂ |
| 117 | 2-Cl, 5-Br-C₆H₃-CH₂ |
| 118 | 3-F, 4-Cl-C₆H₃-CH₂ |
| 119 | 3-F, 5-Cl-C₆H₃-CH₂ |
| 120 | 3-F, 6-Cl-C₆H₃-CH₂ |
| 121 | 3-F, 4-Br-C₆H₃-CH₂ |
| 122 | 3-F, 5-Br-C₆H₃-CH₂ |
| 123 | 3-F, 6-Br-C₆H₃-CH₂ |
| 124 | 3-Cl, 4-Br-C₆H₃-CH₂ |
| 125 | 3-Cl, 5-Br-C₆H₃-CH₂ |
| 126 | 3-Cl, 6-Br-C₆H₃-CH₂ |
| 127 | 4-F, 5-Cl-C₆H₃-CH₂ |
| 128 | 4-F, 6-Cl-C₆H₃-CH₂ |
| 129 | 4-F, 5-Br-C₆H₃-CH₂ |
| 130 | 4-F, 6-Br-C₆H₃-CH₂ |
| 131 | 4-Cl, 5-Br-C₆H₃-CH₂ |
| 132 | 5-F, 6-Cl-C₆H₃-CH₂ |
| 133 | 5-F, 6-Br-C₆H₃-CH₂ |
| 134 | 5-Cl, 6-Br-C₆H₃-CH₂ |
| 135 | 3-Br, 4-Cl, 5-Br-C₆H₂-CH₂ |
| 136 | 2-CN-C₆H₄-CH₂ |
| 137 | 3-CN-C₆H₄-CH₂ |
| 138 | 4-CN-C₆H₄-CH₂ |
| 139 | 2-NO₂-C₆H₄-CH₂ |
| 140 | 3-NO₂-C₆H₄-CH₂ |
| 141 | 4-NO₂-C₆H₄-CH₂ |
| 142 | 2-CH₃-C₆H₄-CH₂ |
| 143 | 3-CH₃-C₆H₄-CH₂ |
| 144 | 4-CH₃-C₆H₄-CH₂ |
| 145 | 2,3-(CH₃)₂-C₆H₃-CH₂ |
| 146 | 2,4-(CH₃)₂-C₆H₃-CH₂ |
| 147 | 2,5-(CH₃)₂-C₆H₃-CH₂ |
| 148 | 2,6-(CH₃)₂-C₆H₃-CH₂ |
| 149 | 3,4-(CH₃)₂-C₆H₃-CH₂ |
| 150 | 3,5-(CH₃)₂-C₆H₃-CH₂ |
| 151 | 2-C₂H₅-C₆H₄-CH₂ |
| 152 | 3-C₂H₅-C₆H₄-CH₂ |
| 153 | 4-C₂H₅-C₆H₄-CH₂ |
| 154 | 2-i-C₃H₇-C₆H₄-CH₂ |
| 155 | 3-i-C₃H₇-C₆H₄-CH₂ |
| 156 | 4-i-C₃H₇-C₆H₄-CH₂ |
| 157 | 2-Cyclohexyl-C₆H₄-CH₂ |
| 158 | 3-Cyclohexyl-C₆H₄-CH₂ |
| 159 | 4-Cyclohexyl-C₆H₄-CH₂ |
| 160 | 2-Vinyl-C₆H₄-CH₂ |
| 161 | 3-Vinyl-C₆H₄-CH₂ |
| 162 | 4-Vinyl-C₆H₄-CH₂ |
| 163 | 2-Allyl-C₆H₄-CH₂ |
| 164 | 3-Allyl-C₆H₄-CH₂ |
| 165 | 4-Allyl-C₆H₄-CH₂ |
| 166 | 2-C₆H₅-C₆H₄-CH₂ |
| 167 | 3-C₆H₅-C₆H₄-CH₂ |
| 168 | 4-C₆H₅-C₆H₄-CH₂ |
| 169 | 3-CH₃, 5-t-C₄H₉-C₆H₃-CH₂ |
| 170 | 2-OH-C₆H₄-CH₂ |
| 171 | 3-OH-C₆H₄-CH₂ |
| 172 | 4-OH-C₆H₄-CH₂ |
| 173 | 2-OCH₃-C₆H₄-CH₂ |
| 174 | 3-OCH₃-C₆H₄-CH₂ |
| 175 | 4-OCH₃-C₆H₄-CH₂ |
| 176 | 2,3-(OCH₃)₂-C₆H₃-CH₂ |
| 177 | 2,4-(OCH₃)₂-C₆H₃-CH₂ |
| 178 | 2,5-(OCH₃)₂-C₆H₃-CH₂ |
| 179 | 3,4-(OCH₃)₂-C₆H₃-CH₂ |
| 180 | 3,5-(OCH₃)₂-C₆H₃-CH₂ |
| 181 | 3,4,5-(OCH₃)₃-C₆H₂-CH₂ |
| 182 | 2-OC₂H₅-C₆H₄-CH₂ |
| 183 | 3-OC₂H₅-C₆H₄-CH₂ |
| 184 | 4-OC₂H₅-C₆H₄-CH₂ |
| 185 | 2-O-(n-C₃H₇)-C₆H₄-CH₂ |
| 186 | 3-O-(n-C₃H₇)-C₆H₄-CH₂ |
| 187 | 4-O-(n-C₃H₇)-C₆H₄-CH₂ |
| 188 | 2-O-(i-C₃H₇)-C₆H₄-CH₂ |
| 189 | 3-O-(i-C₃H₇)-C₆H₄-CH₂ |
| 190 | 4-O-(i-C₃H₇)-C₆H₄-CH₂ |
| 191 | 4-O-(n-C₄H₉)-C₆H₄-CH₂ |
| 192 | 3-O-(t-C₄H₉)-C₆H₄-CH₂ |
| 193 | 4-O-(n-C₆H₁₃)-C₆H₄-CH₂ |
| 194 | 2-O-Allyl-C₆H₄-CH₂ |
| 195 | 3-O-Allyl-C₆H₄-CH₂ |
| 196 | 4-O-Allyl-C₆H₄-CH₂ |
| 197 | 2-CF₃-C₆H₄-CH₂ |
| 198 | 3-CF₃-C₆H₄-CH₂ |
| 199 | 4-CF₃-C₆H₄-CH₂ |
| 200 | 2-Acetyl-C₆H₄-CH₂ |
| 201 | 3-Acetyl-C₆H₄-CH₂ |
| 202 | 4-Acetyl-C₆H₄-CH₂ |
| 203 | 2-Methoxycarbonyl-C₆H₄-CH₂ |
| 204 | 3-Methoxycarbonyl-C₆H₄-CH₂ |
| 205 | 4-Methoxycarbonyl-C₆H₄-CH₂ |
| 206 | 2-Aminocarbonyl-C₆H₄-CH₂ |
| 207 | 3-Aminocarbonyl-C₆H₄-CH₂ |
| 208 | 4-Aminocarbonyl-C₆H₄-CH₂ |
| 209 | 2-Dimethylaminocarbonyl-C₆H₄-CH₂ |
| 210 | 3-Dimethylaminocarbonyl-C₆H₄-CH₂ |
| 211 | 4-Dimethylaminocarbonyl-C₆H₄-CH₂ |
| 212 | 2-(N-Methylaminocarbonyl) -C₆H₄-CH₂ |
| 213 | 3-(N-Methylaminocarbonyl)-C₆H₄-CH₂ |
| 214 | 4-(N-Methylaminocarbonyl) -C₆H₄-CH₂ |
| 215 | 2-H₂N-C₆H₄-CH₂ |
| 216 | 3-H₂N-C₆H₄-CH₂ |
| 217 | 4-H₂N-C₆H₄-CH₂ |
| 218 | 2-Aminothiocarbonyl-C₆H₄-CH₂ |
| 219 | 3-Aminothiocarbonyl-C₆H₄-CH₂ |
| 220 | 4-Aminothiocarbonyl-C₆H₄-CH₂ |
| 221 | 2-Methoxyiminomethyl-C₆H₄-CH₂ |
| 222 | 3-Methoxyiminomethyl-C₆H₄-CH₂ |
| 223 | 4-Methoxyiminomethyl-C₆H₄-CH₂ |
| 224 | 3,4-Methylendioxy-C₆H₃-CH₂ |
| 225 | 3,4-Difluormethylendioxy-C₆H₃-CH₂ |
| 226 | 2,3-Methylendioxy-C₆H₃-CH₂ |
| 227 | 2-(1'-Methoxyiminoeth-1'-yl)-C₆H₄-CH₂ |
| 228 | 3-(1'-Methoxyiminoeth-1'-yl)-C₆H₄-CH₂ |
| 229 | 4-(1'-Methoxyiminoeth-1'-yl)-C₆H₄-CH₂ |
| 230 | 2-SCH₃-C₆H₄-CH₂ |
| 231 | 3-SCH₃-C₆H₄-CH₂ |
| 232 | 4-SCH₃-C₆H₄-CH₂ |
| 233 | 2-SO₂CH₃-C₆H₄-CH₂ |
| 234 | 3-SO₂CH₃-C₆H₄-CH₂ |
| 235 | 4-SO₂CH₃-C₆H₄-CH₂ |
| 236 | 2-OCF₃-C₆H₄-CH₂ |
| 237 | 3-OCF₃-C₆H₄-CH₂ |
| 238 | 4-OCF₃-C₆H₄-CH₂ |
| 239 | 2-OCHF₂-C₆H₄-CH₂ |
| 240 | 3-OCHF₂-C₆H₄-CH₂ |
| 241 | 4-OCHF₂-C₆H₄-CH₂ |
| 242 | 3-CF₃, 4-OCF₃-C₆H₃-CH₂ |
| 243 | 1-Naphthyl-CH₂ |
| 244 | 2-Naphthyl-CH₂ |
| 245 | 2-Phenoxyeth-1-yl |
| 246 | 2-(2'-Chlorphenoxy)eth-1-yl |
| 247 | 2-(3'-Chlorphenoxy)eth-1-yl |
| 248 | 2- (4'-Chlorphenoxy)eth-1-yl |
| 249 | 2-(3',5'-Dichlorphenoxy)eth-1-yl |
| 250 | 2-(2'-Cyanophenoxy)eth-1-yl |
| 251 | 2- (3'-Cyanophenoxy)eth-1-yl |
| 252 | 2-(4'-Cyanophenoxy)eth-1-yl |
| 253 | 2-(2'-Methylphenoxy)eth-1-yl |
| 254 | 2-(3'-Methylphenoxy)eth-1-yl |
| 255 | 2-(4'-Methylphenoxy)eth-1-yl |
| 256 | 2-(3'-t-Butylphenoxy)eth-1-yl |
| 257 | 2- (4'-t-Butylphenoxy)eth-1-yl |
| 258 | 2- (2'-Nitrophenoxy)eth-1-yl |
| 259 | 2-(3'-Nitrophenoxy)eth-1-yl |
| 260 | 2- (4'-Nitrophenoxy)eth-1-yl |
| 261 | 2-(2'-Methoxyphenoxy)eth-1-yl |
| 262 | 2-(3'-Methoxyphenoxy)eth-1-yl |
| 263 | 2- (4'-Methoxyphenoxy)eth-1-yl |
| 264 | 2- (2'-Trifluormethylphenoxy)eth-1-yl |
| 265 | 2-(3'-Trifluormethylphenoxy)eth-1-yl |
| 266 | 2-(4'-Trifluormethylphenoxy)eth-1-yl |
| 267 | 2-(2'-Acetylphenoxy)eth-1-yl |
| 268 | 2-(3'-Acetylphenoxy)eth-1-yl |
| 269 | 2-(4'-Acetylphenoxy)eth-1-yl |
| 270 | 2-(2'-Methoxycarbonyl)eth-1-yl |
| 271 | 2-(3'-Methoxycarbonyl)eth-1-yl |
| 272 | 2-(4'-Methoxycarbonyl)eth-1-yl |
| 273 | 2- (2'-Dimethylaminocarbonyl)eth-1-yl |
| 274 | 2-(3'-Dimethylaminocarbonyl)eth-1-yl |
| 275 | 2-(4'-Dimethylaminocarbonyl)eth-1-yl |
| 276 | 2-(2'-Aminothiocarbonyl)eth-1-yl |
| 277 | 2-(3'-Aminothiocarbonyl)eth-1-yl |
| 278 | 2-(4'-Aminothiocarbonyl)eth-1-yl |
| 279 | 2- (2'-Methylsulfonyl)eth-1-yl |
| 280 | 2- (3'-Methylsulfonyl)eth-1-yl |
| 281 | 2-(4'-Methylsulfonyl)eth-1-yl |
| 282 | 3-Phenoxyprop-1-yl |
| 283 | 3-(2'-Chlorphenoxy)prop-1-yl |
| 284 | 3-(3'-Chlorphenoxy)prop-1-yl |
| 285 | 3-(4'-Chlorphenoxy)prop-1-yl |
| 286 | 3-(3',5',Dichlorphenoxy)prop-1-yl |
| 287 | 3-(2'-Cyanophenoxy)prop-1-yl |
| 288 | 3-(3'-Cyanophenoxy)prop-1-yl |
| 289 | 3-(4'-Cyanophenoxy)prop-1-yl |
| 290 | 3- (2'-Methylphenoxy)prop-1-yl |
| 291 | 3-(3'-Methylphenoxy)prop-1-yl |
| 292 | 3-(4'-Methylphenoxy)prop-1-yl |
| 293 | 3-(2'-Methoxyphenoxy)prop-1-yl |
| 294 | 3-(3'-Methoxyphenoxy)prop-1-yl |
| 295 | 3-(4'-Methoxyphenoxy)prop-1-yl |
| 296 | 3-(2'-Trifluormethylphenoxy)prop-1-yl |
| 297 | 3-(3'-Trifluormethylphenoxy)prop-1-yl |
| 298 | 3-(4'-Trifluormethylphenoxy)prop-1-yl |
| 299 | 4-Phenoxybut-1-yl |
| 300 | 2-Phenyleth-1-yl |
| 301 | 2-(2'-Chlorphenyl)eth-1-yl |
| 302 | 2-(3'-Chlorphenyl)eth-1-yl |
| 303 | 2- (4'-Chlorphenyl)ether-1-yl |
| 304 | 2- (3',5'-Dichlorphenyl)eth-1-yl |
| 305 | 2- (2'-Cyanophenyl)eth-1-yl |
| 306 | 2- (3'-Cyanophenyl)eth-1-yl |
| 307 | 2- (4'-Cyanophenyl)eth-1-yl |
| 308 | 2- (2'-Methylphenyl)eth-1-yl |
| 309 | 2- (3'-Methylphenyl)eth-1-yl |
| 310 | 2-(4'-Methylphenyl)eth-1-yl |
| 311 | 2- (2'-Methoxyphenyl)eth-1-yl |
| 312 | 2- (3'-Methoxyphenyl)eth-1-yl |
| 313 | 2- (4'-Methoxyphenyl)eth-1-yl |
| 314 | 2- (2'-Trifluormethylphenyl)eth-1-yl |
| 315 | 2- (3'-Trifluormethylphenyl)eth-1-yl |
| 316 | 2- (4'-Trifluormethylphenyl)eth-1-yl |
| 317 | 3-Phenylprop-1-yl |
| 318 | 3-(2'-Chlorphenyl)prop-1-yl |
| 319 | 3- (3'-Chlorphenyl)prop-1-yl |
| 320 | 3- (4'-Chlorphenyl)prop-1-yl |
| 321 | 3- (2'-Cyanophenyl)prop-1-yl |
| 322 | 3- (3'-Cyanophenyl)prop-1-yl |
| 323 | 3-(4'-Cyanophenyl)prop-1-yl |
| 324 | 3-(2'-Trifluormethylphenyl)prop-1-yl |
| 325 | 4-Phenylbut-1-yl |
| 326 | 4-(4'-Chlorphenyl)but-1-yl |
| 327 | 6-(4'-Chlorphenyl)hex-1-yl |
| 328 | 2-Pyridylmethyl |
| 329 | 3-Pyridylmethyl |
| 330 | 4-Pyridylmethyl |
| 331 | 4-Chlorpyridin-2-ylmethyl |
| 332 | 5-Chlorpyridin-2-ylmethyl |
| 333 | 6-Chlorpyridin-2-ylmethyl |
| 334 | 5-Chlorpyridin-3-ylmethyl |
| 335 | 6-Chlorpyridin-3-ylmethyl |
| 336 | 2-Chlorpyridin-4-ylmethyl |
| 337 | 2-Pyrimidinylmethyl |
| 338 | 4-Chlorpyrimidin-2-ylmethyl |
| 339 | 5-Chlorpyrimidin-2-ylmethyl |
| 340 | 2-Chlorpyrimidin-4-ylmethyl |
| 341 | 6-Chlorpyrimidin-4-ylmethyl |
| 342 | 2-Chlorpyrimidin-5-ylmethyl |
| 343 | 4-Pyridazinylmethyl |
| 344 | 2-Pyrazinylmethyl |
| 345 | 5-Chlorpyrazin-2-ylmethyl |
| 346 | 6-Chlorpyrazin-2-ylmethyl |
| 347 | 3-Pyridazinylmethyl |
| 348 | 6-Chlorpyridazin-3-ylmethyl |
| 349 | 1,3,5-Triazinylmethyl |
| 350 | 2-Furylmethyl |
| 351 | 3-Furylmethyl |
| 352 | 4-Bromfur-2-ylmethyl |
| 353 | 5-Chlorfur-2-ylmethyl |
| 354 | 2-Thienylmethyl |
| 355 | 3-Thienylmethyl |
| 356 | 5-Methylthien-3-ylmethyl |
| 357 | 5-Chlorthien-2-ylmethyl |
| 358 | 2-Chlorthien-4-ylmethyl |
| 359 | 2-Pyrrolylmethyl |
| 360 | 3-Pyrrolylmethyl |
| 361 | 2-Oxazolylmethyl |
| 362 | 4-Methyloxazol-2-ylmethyl |
| 363 | 5-Methyloxazol-2-ylmethyl |
| 364 | 4-Chloroxazol-2-ylmethyl |
| 365 | 5-Chloroxazol-2-ylmethyl |
| 366 | 4-Oxazolylmethyl |
| 367 | 2-Methyloxazol-4-ylmethyl |
| 368 | 5-Methyloxazol-4-ylmethyl |
| 369 | 2-Chloroxazol-4-ylmethyl |
| 370 | 5-Chloroxazol-4-ylmethyl |
| 371 | 5-Oxazolylmethyl |
| 372 | 2-Methyloxazol-5-ylmethyl |
| 373 | 4-Methyloxazol-5-ylmethyl |
| 374 | 2-Chloroxazol-5-ylmethyl |
| 375 | 4-Chloroxazol-5-ylmethyl |
| 376 | 2-Thiazolylmethyl |
| 377 | 4-Methylthiazol-2-ylmethyl |
| 378 | 5-Methylthiazol-2-ylmethyl |
| 379 | 4-Chlorthiazol-2-ylmethyl |
| 380 | 5-Chlorthiazol-2-ylmethyl |
| 381 | 4-Thiazolylmethyl |
| 382 | 2-Methylthiazol-4-ylmethyl |
| 383 | 5-Methylthiazol-4-ylmethyl |
| 384 | 2-Chlorthiazol-4-ylmethyl |
| 385 | 5-Chlorthiazol-4-ylmethyl |
| 386 | 5-Thiazolylmethyl |
| 387 | 2-Methylthiazol-5-ylmethyl |
| 388 | 4-Methylthiazol-5-ylmethyl |
| 389 | 2-Chlorthiazol-5-ylmethyl |
| 390 | 4-Chlorthiazol-5-ylmethyl |
| 391 | 3-Isoxazolylmethyl |
| 392 | 4-Methylisoxazol-3-ylmethyl |
| 393 | 5-Methylisoxazol-3-ylmethyl |
| 394 | 4-Chlorisoxazol-3-ylmethyl |
| 395 | 5-Chlorisoxazol-3-ylmethyl |
| 396 | 4-Isoxazolylmethyl |
| 397 | 3-Methylisoxazol-4-ylmethyl |
| 398 | 5-Methylisoxazol-4-ylmethyl |
| 399 | 3-Chlorisoxazol-4-ylmethyl |
| 400 | 5-Chlorisoxazol-4-ylmethyl |
| 401 | 5-Isoxazolylmethyl |
| 402 | 3-Methylisoxazol-5-ylmethyl |
| 403 | 4-Methylisoxazol-5-ylmethyl |
| 404 | 3-Chlorisoxazol-5-ylmethyl |
| 405 | 4-Chlorisoxazol-5-ylmethyl |
| 406 | 3-Isothiazolylmethyl |
| 407 | 4-Methylisothiazol-3-ylmethyl |
| 408 | 5-Methylisothiazol-3-ylmethyl |
| 409 | 4-Chlorisothiazol-3-ylmethyl |
| 410 | 5-Chlorisothiazol-3-ylmethyl |
| 411 | 4-Isothiazolylmethyl |
| 412 | 3-Methylisothiazol-4-ylmethyl |
| 413 | 5-Methylisothiazol-4-ylmethyl |
| 414 | 3-Chlorisothiazol-4-ylmethyl |
| 415 | 5-Chlorisothiazol-4-ylmethyl |
| 416 | 5-Isothiazolylmethyl |
| 417 | 3-Methylisothiazol-5-ylmethyl |
| 418 | 4-Methylisothiazol-5-ylmethyl |
| 419 | 3-Chlorisothiazol-5-ylmethyl |
| 420 | 4-Chlorisothiazol-5-ylmethyl |
| 421 | 4-Imidazolylmethyl |
| 422 | 1-Phenylpyrazol-3-ylmethyl |
| 423 | 1-Methylimidazol-4-ylmethyl |
| 424 | 1-Phenyl-1,2,4-triazol-3-ylmethyl |
| 425 | 1,2,4-Oxadiazol-3-ylmethyl |
| 426 | 5-Chlor-1,2,4-oxadiazol-3-ylmethyl |
| 427 | 5-Methyl-1,2,4-oxadiazol-3-ylmethyl |
| 428 | 5-Trifluormethyl-1,2,4-oxadiazol-3-ylmethyl |
| 429 | 1,3,4-Oxadiazol-2-ylmethyl |
| 430 | 5-Chlor-1,3,4-oxadiazol-2-ylmethyl |
| 431 | 5-Methyl-1,3,4-oxadiazol-2-ylmethyl |
| 432 | 5-Methoxy-1,3,4-oxadiazol-2-ylmethyl |
| 433 | 1,2,4-Thiadiazol-3-ylmethyl |
| 434 | 5-Chlor-1,2,4-thiadiazol-3-ylmethyl |
| 435 | 5-Methyl-1,2,4-thiadiazol-3-ylmethyl |
| 436 | 1,3,4-Thiadiazol-2-ylmethyl |
| 437 | 5-Chlor-1,3,4-thiadiazol-2-ylmethyl |
| 438 | 5-Methyl-1,3,4-thiadiazol-2-ylmethyl |
| 439 | 5-Cyano-1,3,4-thiadiazol-2-ylmethyl |
| 440 | 2-(2'-Pyridinyloxy)eth-1-yl |
| 441 | 2-(3'-Pyridinyloxy)eth-1-yl |
| 442 | 2-(4'-Pyridinyloxy)eth-1-yl |
| 443 | 2-(2'-Pyrimidinyloxy)eth-1-yl |
| 444 | 2-(4'-Pyrimidinyloxy)eth-1-yl |
| 445 | 2-(5'-Pyrimidinyloxy)eth-1-yl |
| 446 | 2-(2'-Pyrazinyloxy)eth-1-yl |
| 447 | 2-(2'-Pyridazinyloxy)eth-1-yl |
| 448 | 2-(3'-Pyridazinyloxy)eth-1-yl |
| 449 | 2-(1',3',5'-Triazinyloxy)eth-1-yl |
| 450 | 2-(5'-Methylisoxazol-3'-yloxy)eth-1-yl |
| 451 | 2-(5'-Chlorisoxazol-3'-yloxy)eth-1-yl |
| 452 | 2-(2'-Methoxythiazol-4'-yloxy)eth-1-yl |
| 453 | 2-(4'-Chloroxazol-2'-yloxy)eth-1-yl |
| 454 | 2-(1'-Phenyl-1'H-1',2',4'-triazol-3'-yloxy)eth-1-yl |
| 455 | 2-(1'-Phenylpyrazol-3'-yloxy)eth-1-yl |
| 456 | C₆H₅ |
| 457 | 2-F-C₆H₄ |
| 458 | 3-F-C₆H₄ |
| 459 | 4-F-C₆H₄ |
| 460 | 2,3-F₂-C₆H₃ |
| 461 | 2,4-F₂-C₆H₃ |
| 462 | 2,5-F₂-C₆H₃ |
| 463 | 2,6-F₂-C₆H₃ |
| 464 | 3,4-F₂-C₆H₃ |
| 465 | 3,5-F₂-C₆H₃ |
| 466 | 2-Cl-C₆H₄ |
| 467 | 3-Cl-C₆H₄ |
| 468 | 4-Cl-C₆H₄ |
| 469 | 2,3-Cl₂-C₆H₃ |
| 470 | 2,4-Cl₂-C₆H₃ |
| 471 | 2,5-Cl₂-C₆H₃ |
| 472 | 2,6-Cl₂-C₆H₃ |
| 473 | 3,4-Cl₂-C₆H₃ |
| 474 | 3,5-Cl₂-C₆H₃ |
| 475 | 2,3,4-Cl₃-C₆H₂ |
| 476 | 2,3,5-Cl₃-C₆H₂ |
| 477 | 2,3,6-Cl₃-C₆H₂ |
| 478 | 2,4,5-Cl₃-C₆H₂ |
| 479 | 2,4,6-Cl₃-C₆H₂ |
| 480 | 3,4,5-Cl₃-C₆H₂ |
| 481 | 2-Br-C₆H₄ |
| 482 | 3-Br-C₆H₄ |
| 483 | 4-Br-C₆H₄ |
| 484 | 2,3-Br₂-C₆H₃ |
| 485 | 2,4-Br₂-C₆H₃ |
| 486 | 2,5-Br₂-C₆H₃ |
| 487 | 2,6-Br₂-C₆H₃ |
| 488 | 3,4-Br₂-C₆H₃ |
| 489 | 3,5-Br₂-C₆H₃ |
| 490 | 2-F, 3-Cl-C₆H₃ |
| 491 | 2-F, 4-Cl-C₆H₃ |
| 492 | 2-F, 5-Cl-C₆H₃ |
| 493 | 2-F, 3-Br-C₆H₃ |
| 494 | 2-F, 4-Br-C₆H₃ |
| 495 | 2-F, 5-Br-C₆H₃ |
| 496 | 2-Cl, 3-Br-C₆H₃ |
| 497 | 2-Cl, 4-Br-C₆H₃ |
| 498 | 2-Cl, 5-Br-C₆H₃ |
| 499 | 3-F, 4-Cl-C₆H₃ |
| 500 | 3-F, 5-Cl-C₆H₃ |
| 501 | 3-F, 6-Cl-C₆H₃ |
| 502 | 3-F, 4-Br-C₆H₃ |
| 503 | 3-F, 5-Br-C₆H₃ |
| 504 | 3-F, 6-Br-C₆H₃ |
| 505 | 3-Cl, 4-Br-C₆H₃ |
| 506 | 3-Cl, 5-Br-C₆H₃ |
| 507 | 3-Cl, 6-Br-C₆H₃ |
| 508 | 4-F, 5-Cl-C₆H₃ |
| 509 | 4-F, 6-Cl-C₆H₃ |
| 510 | 4-F, 5-Br-C₆H₃ |
| 511 | 4-F, 6-Br-C₆H₃ |
| 512 | 4-Cl, 5-Br-C₆H₃ |
| 513 | 5-F, 6-Cl-C₆H₃ |
| 514 | 5-F, 6-Br-C₆H₃ |
| 515 | 5-Cl, 6-Br-C₆H₃ |
| 516 | 3-Br, 4-Cl, 5-Br-C₆H₂ |
| 517 | 2-CN-C₆H₄ |
| 518 | 3-CN-C₆H₄ |
| 519 | 4-CN-C₆H₄ |
| 520 | 2-NO₂-C₆H₄ |
| 521 | 3-NO₂-C₆H₄ |
| 522 | 4-NO₂-C₆H₄ |
| 523 | 2-CH₃-C₆H₄ |
| 524 | 3-CH₃-C₆H₄ |
| 525 | 4-CH₃-C₆H₄ |
| 526 | 2,3-(CH₃)₂-C₆H₃ |
| 527 | 2,4-(CH₃)₂-C₆H₃ |
| 528 | 2,5-(CH₃)₂-C₆H₃ |
| 529 | 2,6-(CH₃)₂-C₆H₃ |
| 530 | 3,4-(CH₃)₂-C₆H₃ |
| 531 | 3,5-(CH₃)₂-C₆H₃ |
| 532 | 2-C₂H₅-C₆H₄ |
| 533 | 3-C₂H₅-C₆H₄ |
| 534 | 4-C₂H₅-C₆H₄ |
| 535 | 2-i-C₃H₇-C₆H₄ |
| 536 | 3-i-C₃H₇-C₆H₄ |
| 537 | 4-i-C₃H₇-C₆H₄ |
| 538 | 3-tert.-C₄H₉-C₆H₄ |
| 539 | 4-tert.-C₄H₉-C₆H₄ |
| 540 | 2-Vinyl-C₆H₄ |
| 541 | 3-Vinyl-C₆H₄ |
| 542 | 4-Vinyl-C₆H₄ |
| 543 | 2-Allyl-C₆H₄ |
| 544 | 3-Allyl-C₆H₄ |
| 545 | 4-Allyl-C₆H₄ |
| 546 | 2-C₆H₅-C₆H₄ |
| 547 | 3-C₆H₅-C₆H₄ |
| 548 | 4-C₆H₅-C₆H₄ |
| 549 | 3-CH₃, 5-tert.-C₄H₉-C₆H₃ |
| 550 | 2-OH-C₆H₄ |
| 551 | 3-OH-C₆H₄ |
| 552 | 4-OH-C₆H₄ |
| 553 | 2-OCH₃-C₆H₄ |
| 554 | 3-OCH₃-C₆H₄ |
| 555 | 4-OCH₃-C₆H₄ |
| 556 | 2,3-(OCH₃)₂-C₆H₃ |
| 557 | 2,4-(OCH₃)₂-C₆H₃ |
| 558 | 2,5-(OCH₃)₂-C₆H₃ |
| 559 | 3,4-(OCH₃)₂-C₆H₃ |
| 560 | 3,5-(OCH₃)₂-C₆H₃ |
| 561 | 3,4,5-(OCH₃)₃-C₆H₂ |
| 562 | 2-OC₂H₅-C₆H₄ |
| 563 | 3-OC₂H₅-C₆H₄ |
| 564 | 4-OC₂H₅-C₆H₄ |
| 565 | 2-O-(n-C₃H₇)-C₆H₄ |
| 566 | 3-O-(n-C₃H₇)-C₆H₄ |
| 567 | 4-O-(n-C₃H₇)-C₆H₄ |
| 568 | 2-O-(i-C₃H₇)-C₆H₄ |
| 569 | 3-O-(i-C₃H₇)-C₆H₄ |
| 570 | 4-O-(i-C₃H₇)-C₆H₄ |
| 571 | 4-O-(n-C₄H₉)-C₆H₄ |
| 572 | 3-O-(t-C₄H₉)-C₆H₄ |
| 573 | 4-O-(t-C₄H₉)-C₆H₄ |
| 574 | 2-O-Allyl-C₆H₄ |
| 575 | 3-O-Allyl-C₆H₄ |
| 576 | 4-O-Allyl-C₆H₄ |
| 577 | 2-CF₃-C₆H₄ |
| 578 | 3-CF₃-C₆H₄ |
| 579 | 4-CF₃-C₆H₄ |
| 580 | 2-Acetyl-C₆H₄ |
| 581 | 3-Acetyl-C₆H₄ |
| 582 | 4-Acetyl-C₆H₄ |
| 583 | 2-Methoxycarbonyl-C₆H₄ |
| 584 | 3-Methoxycarbonyl-C₆H₄ |
| 585 | 4-Methoxycarbonyl-C₆H₄ |
| 586 | 2-Aminocarbonyl-C₆H₄ |
| 587 | 3-Aminocarbonyl-C₆H₄ |
| 588 | 4-Aminocarbonyl-C₆H₄ |
| 589 | 2-Dimethylaminocarbonyl-C₆H₄ |
| 590 | 3-Dimethylaminocarbonyl-C₆H₄ |
| 591 | 4-Dimethylaminocarbonyl-C₆H₄ |
| 592 | 2- (N-Methylaminocarbonyl)-C₆H₄ |
| 593 | 3-(N-Methylaminocarbonyl)-C₆H₄ |
| 594 | 4-(N-Methylaminocarbonyl)-C₆H₄ |
| 595 | 2-H₂N-C₆H₄ |
| 596 | 3-H₂N-C₆H₄ |
| 597 | 4-H₂N-C₆H₄ |
| 598 | 2-Aminothiocarbonyl-C₆H₄ |
| 599 | 3-Aminothiocarbonyl-C₆H₄ |
| 600 | 4-Aminothiocarbonyl-C₆H₄ |
| 601 | 2-Methoxyiminomethyl-C₆H₄ |
| 602 | 3-Methoxyiminomethyl-C₆H₄ |
| 603 | 4-Methoxyiminomethyl-C₆H₄ |
| 604 | 3,4-Methylendioxy-C₆H₃ |
| 605 | 3,4-Difluormethylendioxy-C₆H₃ |
| 606 | 2,3-Methylendioxy-C₆H₃ |
| 607 | 2-(1'-Methoxyiminoeth-1'-yl) -C₆H₄ |
| 608 | 3-(1'-Methoxyiminoeth-1'-yl)-C₆H₄ |
| 609 | 4-(1'-Methoxyiminoeth-1'-yl)-C₆H₄ |
| 610 | 2-SCH₃-C₆H₄ |
| 611 | 3-SCH₃-C₆H₄ |
| 612 | 4-SCH₃-C₆H₄ |
| 613 | 2-SO₂CH₃-C₆H₄ |
| 614 | 3-SO₂CH₃-C₆H₄ |
| 615 | 4-SO₂CH₃-C₆H₄ |
| 616 | 2-OCF₃-C₆H₄ |
| 617 | 3-OCF₃-C₆H₄ |
| 618 | 4-OCF₃-C₆H₄ |
| 619 | 2-OCHF₂-C₆H₄ |
| 620 | 3-OCHF₂-C₆H₄ |
| 621 | 4-OCHF₂-C₆H₄ |
| 622 | 3-CF₃, 4-OCF₃-C₆H₃ |
| 623 | 2-NHCH₃-C₆H₄ |
| 624 | 3-NHCH₃-C₆H₄ |
| 625 | 4-NHCH₃-C₆H₄ |
| 626 | 2-N(CH₃)₂-C₆H₄ |
| 627 | 3-N(CH₃)₂-C₆H₄ |
| 628 | 4-N(CH₃)₂-C₆H₄ |
| 629 | 2-Ethoxycarbonyl-C₆H₄ |
| 630 | 3-Ethoxycarbonyl-C₆H₄ |
| 631 | 4-Ethoxycarbonyl-C₆H₄ |
| 632 | 2-CH₂CH₂F-C₆H₄ |
| 633 | 3-CH₂CH₂F-C₆H₄ |
| 634 | 4-CH₂CH₂F-C₆H₄ |
| 635 | 2-CH₂CF₃-C₆H₄ |
| 636 | 3-CH₂CF₃-C₆H₄ |
| 637 | 4-CH₂CF₃-C₆H₄ |
| 638 | 2-CF₂CHF₂-C₆H₄ |
| 639 | 3-CF₂CHF₂-C₆H₄ |
| 640 | 4-CF₂CHF₂-C₆H₄ |
| 641 | 2-CHF₂-C₆H₄ |
| 642 | 3-CHF₂-C₆H₄ |
| 643 | 4-CHF₂-C₆H₄ |
| 644 | 2-(1'-Oxo-n-prop-1-yl) -C₆H₄ |
| 645 | 3-(1'-Oxo-n-prop-1-yl)-C₆H₄ |
| 646 | 4-(1'-Oxo-n-prop-1-yl)-C₆H₄ |
| 647 | 2-(1'-Oxo-iso-prop-1-yl)-C₆H₄ |
| 648 | 3-(1'-Oxo-iso-prop-1-yl)-C₆H₄ |
| 649 | 4-(1'-Oxo-iso-prop-1-yl)-C₆H₄ |
| 650 | 3-Cyclopropyl-C₆H₄ |
| 651 | 4-Cyclopropyl-C₆H₄ |
| 652 | 4-Cyclohexyl-C₆H₄ |
| 653 | 1-Naphthyl |
| 654 | 2-Naphthyl |
| 655 | 2-Pyridyl |
| 656 | 3-Pyridyl |
| 657 | 4-Pyridyl |
| 658 | 5-CH₃-Pyridin-2-yl |
| 659 | 5-Cl-Pyridin-2-yl |
| 660 | 6-Cl-Pyridin-2-yl |
| 661 | 3,5-Cl₂-Pyridin-2-yl |
| 662 | 6-OCH₃-Pyridin-2-yl |
| 663 | 6-CH₃-Pyridin-2-yl |
| 664 | 6-Cl-Pyridin-3-yl |
| 665 | 6-CH₃-Pyridin-3-yl |
| 666 | 6-OCH₃-Pyridin-3-yl |
| 667 | 2-Pyrimidinyl |
| 668 | 4-OCH₃-Pyrimidin-2-yl |
| 669 | 4-OC₂H₅-Pyrimidin-2-yl |
| 670 | 4-Cl-Pyrimidin-2-yl |
| 671 | 4-CH₃-Pyrimidin-2-yl |
| 672 | 5-CH₃-Pyrimidin-2-yl |
| 673 | 5-Cl-Pyrimidin-2-yl |
| 674 | 5-OCH₃-Pyrimidin-2-yl |
| 675 | 5-OC₂H₅-Pyrimidin-2-yl |
| 676 | 4-Pyrimidinyl |
| 677 | 2-Cl-Pyrimidin-4-yl |
| 678 | 2-OCH₃-Pyrimidin-4-yl |
| 679 | 2-CH₃-Pyrimidin-4-yl |
| 680 | 6-Cl-Pyrimidin-4-yl |
| 681 | 6-CH₃-Pyrimidin-4-yl |
| 682 | 6-OCH₃-Pyrimidin-4-yl |
| 683 | 5-Pyrimidinyl |
| 684 | 2-CH₃-Pyrimidin-5-yl |
| 685 | 2-Cl-Pyrimidin-5-yl |
| 686 | 2-OCH₃-Pyrimidin-5-yl |
| 687 | 2-OC₂H₅-Pyrimidin-5-yl |
| 688 | 2-Furyl |
| 689 | 4-C₂H₅-Fur-2-yl |
| 690 | 4-CH₃-Fur-2-yl |
| 691 | 4-Cl-Fur-2-yl |
| 692 | 4-CN-Fur-2-yl |
| 693 | 5-CH₃-Fur-2-yl |
| 694 | 5-Cl-Fur-2-yl |
| 695 | 5-CN-Fur-2-yl |
| 696 | 3-Furyl |
| 697 | 5-CH₃-Fur-3-yl |
| 698 | 5-Cl-Fur-3-yl |
| 699 | 5-CN-Fur-3-yl |
| 700 | 2-Thienyl |
| 701 | 4-CH₃-Thien-2-yl |
| 702 | 4-Cl-Thien-2-yl |
| 703 | 4-CN-Thien-2-yl |
| 704 | 5-CH₃-Thien-2-yl |
| 705 | 5-Cl-Thien-2-yl |
| 706 | 5-CN-Thien-2-yl |
| 707 | 3-Thienyl |
| 708 | 5-CH₃-Thien-3-yl |
| 709 | 5-Cl-Thien-3-yl |
| 710 | 5-CN-Thien-3-yl |
| 711 | 4-CH₃-Thien-3-yl |
| 712 | 5-F-Thien-3-yl |
| 713 | 2-Oxazolyl |
| 714 | 4-CH₃-Oxazol-2-yl |
| 715 | 4-Cl-Oxazol-2-yl |
| 716 | 4-CN-Oxazol-2-yl |
| 717 | 5-CH₃-Oxazol-2-yl |
| 718 | 5-Cl-Oxazol-2-yl |
| 719 | 5-CN-Oxazol-2-yl |
| 720 | 4-Oxazolyl |
| 721 | 2-CH₃-Oxazol-4-yl |
| 722 | 2-Cl-Oxazol-4-yl |
| 723 | 2-CN-Oxazol-4-yl |
| 724 | 5-Oxazolyl |
| 725 | 2-CH₃-Oxazol-5-yl |
| 726 | 2-Cl-Oxazol-5-yl |
| 727 | 2-CN-Oxazol-5-yl |
| 728 | 3-Isoxazolyl |
| 729 | 5-CH₃-Isoxazol-3-yl |
| 730 | 5-Cl-Isoxazol-3-yl |
| 731 | 5-CN-Isoxazol-3-yl |
| 732 | 5-Isoxxazolyl |
| 733 | 3-CH₃-Isoxazol-5-yl |
| 734 | 3-Cl-Isoxazol-5-yl |
| 735 | 3-CN-Isoxazol-5-yl |
| 736 | 2-Thiazolyl |
| 737 | 4-CH₃-Thiazol-2-yl |
| 738 | 4-Cl-Thiazol-2-yl |
| 739 | 4-CN-Thiazol-2-yl |
| 740 | 5-CH₃-Thiazol-2-yl |
| 741 | 5-Cl-Thiazol-2-yl |
| 742 | 5-CN-Thiazol-2-yl |
| 743 | 4-Thiazolyl |
| 744 | 2-CH₃-Thiazol-4-yl |
| 745 | 2-Cl-Thiazol-4-yl |
| 746 | 2-CN-Thiazol-4-yl |
| 747 | 2-SCH₃-Thiazol-4-yl |
| 748 | 5-Thiazolyl |
| 749 | 2-CH₃-Thiazol-5-yl |
| 750 | 2-Cl-Thiazol-5-yl |
| 751 | 2-CN-Thiazol-5-yl |
| 752 | 3-Isothiazolyl |
| 753 | 5-CH₃-Isothiazol-3-yl |
| 754 | 5-Cl-Isothiazol-3-yl |
| 755 | 5-CN-Isothiazol-3-yl |
| 756 | 5-Isothiazolyl |
| 757 | 3-CH₃-Isothiazol-5-yl |
| 758 | 3-Cl-Isothiazol-5-yl |
| 759 | 3-CN-Isothiazol-5-yl |
| 760 | 2-Imidazolyl |
| 761 | 4-CH₃-Imidazol-2-yl |
| 762 | 4-Cl-Imidazol-2-yl |
| 763 | 4-CN-Imidazol-2-yl |
| 764 | 1-CH₃-Imidazol-2-yl |
| 765 | 1-CH₃, 4-Cl-Imidazol-2-yl |
| 766 | 1,4-(CH₃)₂-Imidazol-2-yl |
| 767 | 1-CH₃, 5-Cl-Imidazol-2-yl |
| 768 | 1,5-(CH₃)₂-Imidazol-2-yl |
| 769 | 4-Imidazolyl |
| 770 | 2-CH₃-Imidazol-4-yl |
| 771 | 2-Cl-Imidazol-4-yl |
| 772 | 1-CH₃-Imidazol-4-yl |
| 773 | 1,2-(CH₃)₂-Imidazol-4-yl |
| 774 | 1-CH₃, 2-Cl-Imidazol-4-yl |
| 775 | 1-CH₃-Imidazol-5-yl |
| 776 | 1-CH₃, 3-Cl-Imidazol-5-yl |
| 777 | 1,2- (CH₃)₂-Imidazol-5-yl |
| 778 | 3-Pyrazolyl |
| 779 | 5-CH₃-Pyrazol-3-yl |
| 780 | 5-Cl-Pyrazol-3-yl |
| 781 | 5-CN-Pyrazol-3-yl |
| 782 | 1-CH₃-Pyrazol-3-yl |
| 783 | 1-CH₃, 4-Cl-Pyrazol-3-yl |
| 784 | 1-CH₃, 5-Cl-Pyrazol-3-yl |
| 785 | 1,5-(CH₃)₂-Pyrazol-3-yl |
| 786 | 1-CH₃-Pyrazol-5-yl |
| 787 | 1-CH₃, 3-Cl-Pyrazol-5-yl |
| 788 | 1,3-(CH₃)₂-Pyrazol-5-yl |
| 789 | 4-Pyrazolyl |
| 790 | 3-Cl-Pyrazol-4-yl |
| 791 | 3-CH₃-Pyrazol-4-yl |
| 792 | 1-CH₃-Pyrazol-4-yl |
| 793 | 1-CH₃, 3-Cl-Pyrazol-4-yl |
| 794 | 1,3-(CH₃)₂-Pyrazol-4-yl |
| 795 | 1,3,4-Oxadiazol-5-yl |
| 796 | 2-CH₃-1,3,4-Oxadiazol-5-yl |
| 797 | 2-Cl-1,3,4-Oxadiazol-5-yl |
| 798 | 2-CF₃-1,3,4-Oxadiazol-5-yl |
| 799 | 2-i-C₃H₇-1,3,4-Oxadiazol-5-yl |
| 800 | 2-OCH₃-1,3,4-Oxadiazol-5-yl |
| 801 | 1,2,4-Oxadiazol-3-yl |
| 802 | 5-CH₃-1,2,4-Oxadiazol-3-yl |
| 803 | 5-i-C₃H₇-1,2,4-Oxadiazol-3-yl |
| 804 | 5-Cl-1,2,4-Oxadiazol-3-yl |
| 805 | 5-CF₃-1,2,4-Oxadiazol-3-yl |
| 806 | 1,2,4-Triazol-3-yl |
| 807 | 1-CH₃-1,2,4-Triazol-3-yl |
| 808 | 3-Fluorpyridin-2-yl |
| 809 | 3-Chlorpyridin-2-yl |
| 810 | 3-Brompyridin-2-yl |
| 811 | 3-Methylpyridin-2-yl |
| 812 | 3-Trifluormethylpyridin-2-yl |
| 813 | 3-Methoxypyridin-2-yl |
| 814 | 4-Fluorpyridin-2-yl |
| 815 | 4-Chlorpyridin-2-yl |
| 816 | 4-Brompyridin-2-yl |
| 817 | 4-Methylpyridin-2-yl |
| 818 | 4-Trifluormethylpyridin-2-yl |
| 819 | 4-Methoxypyridin-2-yl |
| 820 | 5-Fluorpyridin-2-yl |
| 821 | 5-Brompyridin-2-yl |
| 822 | 6-Trifluormethylpyridin-2-yl |
| 823 | 2-Fluorpyridin-3-yl |
| 824 | 2-Chlorpyridin-3-yl |
| 825 | 2-Brompyridin-3-yl |
| 826 | 2-Methylpyridin-3-yl |
| 827 | 2-Trifluormethylpyridin-3-yl |
| 828 | 3-Methoxypyridin-3-yl |
| 829 | 4-Fluorpyridin-3-yl |
| 830 | 4-Chlorpyridin-3-yl |
| 831 | 4-Brompyridin-3-yl |
| 832 | 4-Methylpyridin-3-yl |
| 833 | 4-Trifluormethylpyridin-3-yl |
| 834 | 4-Methoxypyridin-3-yl |
| 835 | 5-Fluorpyridin-3-yl |
| 836 | 5-Chlorpyridin-3-yl |
| 837 | 5-Brompyridin-3-yl |
| 838 | 5-Methylpyridin-3-yl |
| 839 | 5-Trifluormethylpyridin-3-yl |
| 840 | 5-Methoxypyridin-3-yl |
| 841 | 6-Fluorpyridin-3-yl |
| 842 | 6-Brompyridin-3-yl |
| 843 | 6-Trifluormethylpyridin-3-yl |
| 844 | 2-Fluorpyridin-4-yl |
| 845 | 2-Chlorpyridin-4-yl |
| 846 | 2-Brompyridin-4-yl |
| 847 | 2-Methylpyridin-4-yl |
| 848 | 2-Trifluormethylpyridin-4-yl |
| 849 | 2-Methoxypyridin-4-yl |
| 850 | 3-Fluorpyridin-4-yl |
| 851 | 3-Chlorpyridin-4-yl |
| 852 | 3-Brompyridin-4-yl |
| 853 | 3-Methylpyridin-4-yl |
| 854 | 3-Trifluormethylpyridin-4-yl |
| 855 | 3-Methoxypyridin-4-yl |
| 856 | 4-Fluorpyrimidin-2-yl |
| 857 | 4-Brompyrimidin-2-yl |
| 858 | 4 -Trifluormethylpyrimidin-2-yl |
| 859 | 5-Fluorpyrimidin-2-yl |
| 860 | 5-Brompyrimidin-2-yl |
| 861 | 5-Trifluormethylpyrimidin-2-yl |
| 862 | 2-Fluorpyrimidin-4-yl |
| 863 | 2-Brompyrimidin-4-yl |
| 864 | 2 -Trifluormethylpyrimidin-4-yl |
| 865 | 2-Trifluormethoxypyrimidin-4-yl |
| 866 | 5-Fluorpyrimidin-4-yl |
| 867 | 5-Chlorpyrimidin-4-yl |
| 868 | 5-Brompyrimidin-4-yl |
| 869 | 5-Methoxypyrimidin-4-yl |
| 870 | 5-Trifluormethylpyrimidin-4-yl |
| 871 | 5-Methoxypyrimidim-4-yl |
| 872 | 6-Fluorpyrimidin-4-yl |
| 873 | 6-Brompyrimidin-4-yl |
| 874 | 6-Trifluormethylpyrimidin-4-yl |
| 875 | 2-Fluorpyrimidin-5-yl |
| 876 | 2-Brompyrimidin-5-yl |
| 877 | 2-Trifluormethylpyrimidin-5-yl |
| 878 | 4-Fluorpyrimidin-5-yl |
| 879 | 4-Chlorpyrimidin-5-yl |
| 880 | 4-Brompyrimidin-5-yl |
| 881 | 4-Methylpyrimidin-5-yl |
| 882 | 4-Trifluormethylpyrimidin-5-yl |
| 883 | 3-Fluor-5-trifluormethylpyridin-2-yl |
| 884 | 3,6-Dichlor-5-trifluormethylpyridin-2-yl |
| 885 | 5,6-Dichlor-3-trifluormethylpyridin-2-yl |
| 886 | 5-Chlor-3-trifluormethylpyridin-2-yl |
| 887 | 3-Chlor-5-trifluormethylpyridin-2-yl |
| 888 | 6-Chlor-4-cyanopyridin-2-yl |
| 889 | 3-Cyano-5-nitropyridin-2-yl |
| 890 | 2-Chlor-6-fluorpyridin-4-yl |
| 891 | 6-Chlor-4-fluorpyridin-2-yl |
| 892 | 4,6-Difluorpyridin-2-yl |
| 893 | 3,5-Dichlor-6-fluorpyridin-2-yl |
| 894 | 6-Methoxy-3-nitropyridin-2-yl |
| 895 | 4-Cyano-6-fluorpyridin-2-yl |
| 896 | 6-Chlor-5-cyanopyridin-2-yl |
| 897 | 6-Chlor-3-cyanopyridin-2-yl |
| 898 | 4-Cyano-3,5,6-trifluorpyridin-2-yl |
| 899 | 6-Chlor-5-nitropyridin-2-yl |
| 900 | 6-Chlor-3-nitropyridin-2-yl |
| 901 | 5-Cyano-6-fluorpyridin-2-yl |
| 902 | 3-Cyano-6-fluorpyridin-2-yl |
| 903 | 4,6-Dicyanopyridin-2-yl |
| 904 | 5-Trichlormethylpyridin-2-yl |
| 905 | 5-Cyanopyridin-2-yl |
| 906 | 5-Brom-4-trifluormethylpyridin-2-yl |
| 907 | 3-Nitro-5-trifluormethylpyridin-2-yl |
| 908 | 5-Aminopyridin-2-yl |
| 909 | 2,3,5,6-Tetrafluorpyridin-4-yl |
| 910 | 5-Nitropyridin-2-yl |
| 911 | 4-Methyl-5-nitroypridin-2-yl |
| 912 | 5-Difluormethylpyridin-2-yl |
| 913 | 5-Fluormethylpyridin-2-yl |
| 914 | 4,6-Difluorpyrimidin-2-yl |
| 915 | 2,6-Difluorpyrimidin-4-yl |
| 916 | 2-Chlor-6-trichlormethylpyrimidin-4-yl |
| 917 | 2,6-Dichlorpyrimidin-4-yl |
| 918 | 5-Methoxycarbonylpyridin-2-yl |
| 919 | 5-Chlor-6-fluorpyridin-2-yl |
| 920 | 5-Chlor-6-hydroxypyridin-2-yl |
| 921 | 5-Chlor-6-methoxypyridin-2-yl |
| 922 | 5-Chlor-6-cyanopyridin-2-yl |
| 923 | 5,6-Dichlorpyridin-2-yl |
| 924 | 6-Brom-5-chlorpyridin-2-yl |
| 925 | 5-Brom-6-fluorpyridin-2-yl |
| 926 | 5-Brom-6-chlorpyridin-2-yl |
| 927 | 5-Brom-6-cyanopyridin-2-yl |
| 928 | 5-Brom-6-hydroxypyridin-2-yl |
| 929 | 5-Brom-6-methoxypyridin-2-yl |
| 930 | 5,6-Dibrompyridin-2-yl |
| 931 | 4-Cyanopyridin-2-yl |
| 932 | 6-Cyanopyridin-2-yl |
| 933 | 4-Chlor-6-methylpyrimidin-2-yl |
| 934 | 2-Choro-6-fluorpyridin-4-yl |
| 935 | 5-Brom-4-trifluormethylpyridin-2-yl |
| 936 | 4,5-Dichlorpyridin-2-yl |
| 937 | 4,5-Dibrompyridin-2-yl |
| 938 | 5,6-Dichlorpyridin-2-yl |
| 939 | 4,6-Dichlorpyridin-2-yl |
| 940 | 4,6-Dibrompyridin-2-yl |
| 941 | 5,6-Dibrompyridin-2-yl |
| 942 | 4-Brom-5-chlorpyridin-2-yl |
| 943 | 6-Brom-5-chlorpyridin-2-yl |
| 944 | 5-Brom-4-chlorpyridin-2-yl |
| 945 | 5-Brom-4-chlorpyridin-2-yl |
| 946 | 6-Brom-4-chlorpyridin-2-yl |
| 947 | 4-Brom-6-chlorpyridin-2-yl |
| 948 | 6-Chlor-4-methoxypyridin-2-yl |
| 949 | 6-Brom-4-methoxypyridin-2-yl |
| 950 | 6-Chlorchinazolin-2-yl |
| 951 | Chinazolin-2-yl |
| 952 | 4-Cyanopyridin-2-yl |
| 953 | 6-Cyanopyridin-2-yl |
| 954 | 5-Hydroxymethylpyridin-2-yl |
| 955 | 6-Chlor-4-trifluormethylpyridin-2-yl |
| 956 | 6-Chlor-4-trifluormethylpyridin-2-yl |
| 957 | 6-Chlor-4-methylpyridin-2-yl |
| 958 | 2,5-Dichlor-6-cyanopyridin-2-yl |
| 959 | 2,5-Dichlor-6-carboxypyridin-2-yl |
| 960 | 2,5-Dichlor-6-methoxycarbonyl-pyridin-2-yl |
| 961 | 6-Trifluormethylpyridin-2-yl |
| 962 | 6-Methoxycarbonylpyridin-2-yl |
| 963 | 6-Carboxypyridin-2-yl |
| 964 | 4-Phenoxypyridin-2-yl |
| 965 | 5-Phenoxypyridin-2-yl |
| 966 | 6-Phenoxypyridin-2-yl |
| 967 | 4-Phenoxypyrimidin-4-yl |
| 968 | 4-(4-Methylphenoxy)pyrimidin-4-yl |
| 969 | 4-Phenoxypyrimidin-2-yl |
| 970 | 4-(2-Fluorphenoxy)-pyrimidin-2-yl |
| 971 | 4-Phenoxypyrimidin-6-yl |
| 972 | 4-(4-Chlorphenoxy)pyrimidin-6-yl |
| 973 | 4-(2-Pyridyloxy)pyrimidin-6-yl |
| 974 | 4-(6-Chlor-2-pyridyloxy)pyrimidin-6-yl |
| 975 | 4-(3-Pyridyloxy)pyrimidin-6-yl |
| 976 | 4-(2-Methyl-3-pyridyloxy)pyrimidin-6-yl |
| 977 | 4-(4-Pyridyloxy)pyrimidin-6-yl |
| 978 | 5-Brom-2-thienyl |
| 979 | 5-Nitro-2-thienyl |
| 980 | 2-Chlor-3-thienyl |
| 981 | 2-Brom-3-thienyl |
| 982 | 1-Methyl-3-pyrrolyl |
| 983 | 1-Methyl-2-pyrrolyl |
| 984 | 1-Benzofuran-2-yl |
| 985 | 1-Benzofuran-3-yl |
| 986 | 1-Benzothiophen-2-yl |
| 987 | 1-Benzothiophen-3-yl |
| 988 | 3-Pyrrolyl |
| 989 | 2-Pyrrolyl |
| 990 | 3-Indolyl |
| 991 | 2-Indolyl |
| 992 | 1-Methyl-3-indolyl |
| 993 | 1-Methyl-2-indolyl |
| 994 | Isoxazol-4-yl |
| 995 | Isothiazol-4-yl |
| 996 | 1,2-Benzisoxazol-3-yl |
| 997 | 1,2-Benzisothiazol-3-yl |
| 998 | 1-Methylindazol-3-yl |
| 999 | Benzoxazol-2-yl |
| 1000 | 5-Chlorbenzoxazol-2-yl |
| 1001 | 6-Fluorbenzoxazol-2-yl |
| 1002 | Benzthiazol-2-yl |
| 1003 | 5-Fluorbenzthiazol-2-yl |
| 1004 | 6-Fluorbenzthiazol-2-yl |
| 1005 | Pyrido[3,2-d]thiazol-2-yl |
| 1006 | (6-Chlor-pyrido)[3,2-d]thiazol-2-yl |
| 1007 | 1-Methyl-1,2,3-triazol-5-yl |
| 1008 | 1-Methyl-1,2,3-triazol-4-yl |
| 1009 | 1-Methyl-1,2,4-triazol-5-yl |
| 1010 | 1-Methyl-1,2,3,4-tetrazol-5-yl |
| 1011 | 2-Methyl-1,2,3,4-tetrazol-5-yl |
| 1012 | 5-Trifluormethyl-1,3,4-thiadiazol-2-yl |
| 1013 | 6-Chlorbenzoxazol-2-yl |
| 1014 | 5-Fluorbenzoxazol-2-yl |
| 1015 | 5-Nitrothiazol-2-yl |
| 1016 | 1-CH(CH₃)₂-pyrrol-3-yl |
| 1017 | 1-C(CH₃)₃-pyrrol-3-yl |
| 1018 | 1-cyclopropyl-pyrrol-3-yl |
| 1019 | 1-C₆H₅-pyrrol-3-yl |
| 1020 | 1-(2-CH₃-C₆H₄)-pyrrol-3-yl |
| 1021 | 1-(3-CH₃-C₆H₄)-pyrrol-3-yl |
| 1022 | 1-(4-CH₃-C₆H₄)-pyrrol-3-yl |
| 1023 | 1-(3-OCH₃-C₆H₄)-pyrrol-3-yl |
| 1024 | 1-(4-OCH₃-C₆H₄)-pyrrol-3-yl |
| 1025 | 1-(4-NO₂-C₆H₄)-pyrrol-3-yl |
| 1026 | 1-(3-NO₂-C₆H₄)-pyrrol-3-yl |
| 1027 | 1-(4-CN-C₆H₄)-pyrrol-3-yl |
| 1028 | 1-(3-CN-C₆H₄)-pyrrol-3-yl |
| 1029 | 1-(3-CF₃-C₆H₄)-pyrrol-3-yl |
| 1030 | 1-(4-CF₃-C₆H₄)-pyrrol-3-yl |
| 1031 | 1-(4-C(CH₃)₃-C₆H₄)-pyrrol-3-yl |
| 1032 | 1-(2-Cl-C₆H₄)-pyrrol-3-yl |
| 1033 | 1-(3-Cl-C₆H₄)-pyrrol-3-yl |
| 1034 | 1-(4-Cl-C₆H₄)-pyrrol-3-yl |
| 1035 | 1-(2-Br-C₆H₄)-pyrrol-3-yl |
| 1036 | 1-(3-Br-C₆H₄)-pyrrol-3-yl |
| 1037 | 1-(4-Br-C₆H₄)-pyrrol-3-yl |
| 1038 | 1-(2-F-C₆H₄)-pyrrol-3-yl |
| 1039 | 1-(3-F-C₆H₄)-pyrrol-3-yl |
| 1040 | 1-(4-F-C₆H₄)-pyrrol-3-yl |
| 1041 | 1-(2,4-Cl₂-C₆H₃)-pyrrol-3-yl |
| 1042 | 1-(2,5-Cl₂-C₆H₃)-pyrrol-3-yl |
| 1043 | 1-(2,6-Cl₂-C₆H₃)-pyrrol-3-yl |
| 1044 | 1-(3,4-Cl₂-C₆H₃)-pyrrol-3-yl |
| 1045 | 1-(2,4-F₂-C₆H₃)-pyrrol-3-yl |
| 1046 | 1- (2,5-F₂-C₆H₃)-pyrrol-3-yl |
| 1047 | 1-(2,6-F₂-C₆H₃)-pyrrol-3-yl |
| 1048 | 1-(3,4-F₂-C₆H₃)-pyrrol-3-yl |
| 1049 | 1-(2-Cl, 5-OCH₃-C₆H₃)-pyrrol-3-yl |
| 1050 | 1-(2-Cl, 5-CH₃-C₆H₃)-pyrrol-3-yl |
| 1051 | 1-(5-Cl, 2-OCH₃-C₆H₃)-Pyrrol-3-yl |
| 1052 | 1-(5-Cl, 2-CH₃-C₆H₃)-pyrrol-3-yl |
| 1053 | 1-[2,5-[CH₃)₂-C₆H₃]-pyrrol-3-yl |
| 1054 | 1-CH(CH₃)₂-pyrrol-2-yl |
| 1055 | 1-C(CH₃)₃-pyrrol-2-yl |
| 1056 | 1-cyclopropyl-pyrrol-2-yl |
| 1057 | 1-C₆H₅-pyrrol-2-yl |
| 1058 | 1-(2-CH₃-C₆H₄)-pyrrol-2-yl |
| 1059 | 1-(3-CH₃-C₆H₄)-pyrrol-2-yl |
| 1060 | 1-(4-CH₃-C₆H₄)-pyrrol-2-yl |
| 1061 | 1-(3-OCH₃-C₆H₄)-pyrrol-2-yl |
| 1062 | 1-(4-OCH₃-C₆H₄)-pyrrol-2-yl |
| 1063 | 1-(4-NO₂-C₆H₄)-pyrrol-2-yl |
| 1064 | 1-(3-NO₂-C₆H₄)-pyrrol-2-yl |
| 1065 | 1-(4-CN-C₆H₄)-pyrrol-2-yl |
| 1066 | 1-(3-CN-C₆H₄)-pyrrol-2-yl |
| 1067 | 1-(3-CF₃-C₆H₄)-pyrrol-2-yl |
| 1068 | 1-(4-CF₃-C₆H₄)-pyrrol-2-yl |
| 1069 | 1-(4-C(CH₃)₃-C₆H₄)-pyrrol-2-yl |
| 1070 | 1-(2-Cl-C₆H₄)-pyrrol-2-yl |
| 1071 | 1-(3-Cl-C₆H₄)-pyrrol-2-yl |
| 1072 | 1-(4-Cl-C₆H₄)-pyrrol-2-yl |
| 1073 | 1-(2-Br-C₆H₄)-pyrrol-2-yl |
| 1074 | 1-(3-Br-C₆H₄)-pyrrol-2-yl |
| 1075 | 1-(4-Br-C₆H₄)-pyrrol-2-yl |
| 1076 | 1-(2-F-C₆H₄)-pyrrol-2-yl |
| 1077 | 1-(3-F-C₆H₄)-pyrrol-2-yl |
| 1078 | 1-(4-F-C₆H₄)-pyrrol-2-yl |
| 1079 | 1-(2,4-Cl₂-C₆H₃)-pyrrol-2-yl |
| 1080 | 1-(2,5-Cl₂-C₆H₃)-pyrrol-2-yl |
| 1081 | 1-(2,6-Cl₂-C₆H₃)-pyrrol-2-yl |
| 1082 | 1-(3,4-Cl₂-C₆H₃)-pyrrol-2-yl |
| 1083 | 1-(2,4-F₂-C₆H₃)-pyrrol-2-yl |
| 1084 | 1-(2,5-F₂-C₆H₃)-pyrrol-2-yl |
| 1085 | 1-(2,6-F₂-C₆H₃)-pyrrol-2-yl |
| 1086 | 1-(3,4-F₂-C₆H₃)-pyrrol-2-yl |
| 1087 | 1-(2-Cl, 5-OCH₃-C₆H₃)-pyrrol-2-yl |
| 1088 | 1-(2-Cl, 5-CH₃-C₆H₃)-pyrrol-2-yl |
| 1089 | 1-(5-Cl, 2-OCH₃-C₆H₃)-pyrrol-2-yl |
| 1090 | 1-(5-Cl, 2-CH₃-C₆H₃)-pyrrol-2-yl |
| 1091 | 1-[2,5(CH₃)₂-C₆H₃]-pyrrol-2-yl |
| 1092 | 5-CH(CH₃)₂-furan-2-yl |
| 1093 | 5-C(CH₃)₃-furan-2-yl |
| 1094 | 5-cyclopropyl-furan-2-yl |
| 1095 | 5-C₆H₅-furan-2-yl |
| 1096 | 5-(2-CH₃-C₆H₄)-furan-2-yl |
| 1097 | 5-(3-CH₃-C₆H₄)-furan-2-yl |
| 1098 | 5-(4-CH₃-C₆H₄)-furan-2-yl |
| 1099 | 5-(3-OCH₃-C₆H₄)-furan-2-yl |
| 1100 | 5-(4-OCH₃-C₆H₄)-furan-2-yl |
| 1101 | 5-(4-NO₂-C₆H₄)-furan-2-yl |
| 1102 | 5-(3-NO₂-C₆H₄)-furan-2-yl |
| 1103 | 5-(4-CN-C₆H₄)-furan-2-yl |
| 1104 | 5-(3-CN-C₆H₄)-furan-2-yl |
| 1105 | 5-(3-CF₃-C₆H₄)-furan-2-yl |
| 1106 | 5-(4-CF₃-C₆H₄)-furan-2-yl |
| 1107 | 5-(4-C(CH₃)₃-C₆H₄)-furan-2-yl |
| 1108 | 5-(4-C₆H₅-C₆H₄)-furan-2-yl |
| 1109 | 5-(2-Cl-C₆H₄)-furan-2-yl |
| 1110 | 5-(3-Cl-C₆H₄)-furan-2-yl |
| 1111 | 5-(4-Cl-C₆H₄)-furan-2-yl |
| 1112 | 5-(2-Br-C₆H₄)-furan-2-yl |
| 1113 | 5-(3-Br-C₆H₄)-furan-2-yl |
| 1114 | 5-(4-Br-C₆H₄)-furan-2-yl |
| 1115 | 5-(2-F-C₆H₄)-furan-2-yl |
| 1116 | 5-(3-F-C₆H₄)-furan-2-yl |
| 1117 | 5-(4-F-C₆H₄)-furan-2-yl |
| 1118 | 5-(2,4-Cl₂-C₆H₃)-furan-2-yl |
| 1119 | 5-(2,5-Cl₂-C₆H₃)-furan-2-yl |
| 1120 | 5-(2,6-Cl₂-C₆H₃)-furan-2-yl |
| 1121 | 5-(3,4-Cl₂-C₆H₃)-furan-2-yl |
| 1122 | 5-(2,4-F₂-C₆H₃)-furan-2-yl |
| 1123 | 5-(2,5-F₂-C₆H₃)-furan-2-yl |
| 1124 | 5-(2,6-F₂-C₆H₃)-furan-2-yl |
| 1125 | 5-(3,4-F₂-C₆H₃)-furan-2-yl |
| 1126 | 5-(2-Cl, 5-OCH₃-C₆H₃)-furan-2-yl |
| 1127 | 5-(2-Cl, 5-CH₃-C₆H₃)-furan-2-yl |
| 1128 | 5-(5-Cl, 2-OCH₃-C₆H₃)-furan-2-yl |
| 1129 | 5-(5-Cl, 2-CH₃-C₆H₃)-furan-2-yl |
| 1130 | 5-[2,5-(CH₃)₂-C₆H₃]-furan-2-yl |
| 1131 | 4-CH(CH₃)₂-furan-2-yl |
| 1132 | 4-C(CH₃)₃-furan-2-yl |
| 1133 | 4-cyclopropyl-furan-2-yl |
| 1134 4 | 4-C₆H₅-furan-2-yl |
| 1135 | 4-(2-CH₃-C₆H₄)-furan-2-yl |
| 1136 | 4-(3-CH₃-C₆H₄)-furan-2-yl |
| 1137 | 4-(4-CH₃-C₆H₄)-furan-2-yl |
| 1138 | 4-(3-OCH₃-C₆H₄)-furan-2-yl |
| 1139 | 4-(4-OCH₃-C₆H₄)-furan-2-yl |
| 1140 | 4-(4-NO₂-C₆H₄)-furan-2-yl |
| 1141 | 4-(3-NO₂-C₆H₄)-furan-2-yl |
| 1142 | 4-(4-CN-C₆H₄)-furan-2-yl |
| 1143 | 4-(3-CN-C₆H₄)-furan-2-yl |
| 1144 | 4-(3-CF₃-C₆H₄)-furan-2-yl |
| 1145 | 4-(4-CF₃-C₆H₄)-furan-2-yl |
| 1146 | 4- (4-C(CH₃)₃-C₆H₄)-furan-2-yl |
| 1147 | 4-(2-Cl-C₆H₄)-furan-2-yl |
| 1148 | 4-(3-Cl-C₆H₄)-furan-2-yl |
| 1149 | 4-(4-Cl-C₆H₄)-furan-2-yl |
| 1150 | 4-(2-Br-C₆H₄)-furan-2-yl |
| 1151 | 4-(3-Br-C₆H₄)-furan-2-yl |
| 1152 | 4-(4-Br-C₆H₄)-furan-2-yl |
| 1153 | 4-(2-F-C₆H₄)-furan-2-yl |
| 1154 | 4-(3-F-C₆H₄)-furan-2-yl |
| 1155 | 4-(4-F-C₆H₄)-furan-2-yl |
| 1156 | 4-(2,4-Cl₂-C₆H₃)-furan-2-yl |
| 1157 | 4-(2,5-Cl₂-C₆H₃)-furan-2-yl |
| 1158 | 4-(2,6-Cl₂-C₆H₃)-furan-2-yl |
| 1159 | 4-(3,4-Cl₂-C₆H₃)-furan-2-yl |
| 1160 | 4-(2,4-F₂-C₆H₃)-furan-2-yl |
| 1161 | 4-(2,5-F₂-C₆H₃)-furan-2-yl |
| 1162 | 4-(2,6-F₂-C₆H₃)-furan-2-yl |
| 1163 | 4-(3,4-F₂-C₆H₃)-furan-2-yl |
| 1164 | 4-(2-Cl, 5-OCH₃-C₆H₃)-furan-2-yl |
| 1165 | 4-(2-Cl, 5-CH₃-C₆H₃)-furan-2-yl |
| 1166 | 4-(5-Cl, 2-OCH₃-C₆H₃)-furan-2-yl |
| 1167 | 4-(5-Cl, 2-CH₃-C₆H₃)-furan-2-yl |
| 1168 | 4-[2,5-(CH₃)₂-C₆H₃]-furan-2-yl |
| 1169 | 5-CH(CH₃)₂-thien-2-yl |
| 1170 | 5-C(CH₃)₃-thien-2-yl |
| 1171 | 5-cyclopropyl-thien-2-yl |
| 1172 | 5-C₆H₅-thien-2-yl |
| 1173 | 5-(2-CH₃-C₆H₄)-thien-2-yl |
| 1174 | 5-(3-CH₃-C₆H₄)-thien-2-yl |
| 1175 | 5-(4-CH₃-C₆H₄)-thien-2-yl |
| 1176 | 5-(3-OCH₃-C₆H₄)-thien-2-yl |
| 1177 | 5-(4-OCH₃-C₆H₄)-thien-2-yl |
| 1178 | 5-(4-NO₂-C₆H₄)-thien-2-yl |
| 1179 | 5-(3-NO₂-C₆H₄)-thien-2-yl |
| 1180 | 5-(4-CN-C₆H₄)-thien-2-yl |
| 1181 | 5-(3-CN-C₆H₄)-thien-2-yl |
| 1182 | 5-(3-CF₃-C₆H₄)-thien-2-yl |
| 1183 | 5-(4-CF₃-C₆H₄)-thien-2-yl |
| 1184 | 5-(4-C(CH₃)₃-C₆H₄)-thien-2-yl |
| 1185 | 5-(2-Cl-C₆H₄)-thien-2-yl |
| 1186 | 5-(3-Cl-C₆H₄)-thien-2-yl |
| 1187 | 5-(4-Cl-C₆H₄)-thien-2-yl |
| 1188 | 5-(2-Br-C₆H₄)-thien-2-yl |
| 1189 | 5-(3-Br-C₆H₄)-thien-2-yl |
| 1190 | 5-(4-Br-C₆H₄)-thien-2-yl |
| 1191 | 5-(2-F-C₆H₄)-thien-2-yl |
| 1192 | 5-(3-F-C₆H₄)-thien-2-yl |
| 1193 | 5-(4-F-C₆H₄)-thien-2-yl |
| 1194 | 5-(2,4-Cl₂-C₆H₃)-thien-2-yl |
| 1195 | 5-(2,5-Cl₂-C₆H₃)-thien-2-yl |
| 1196 | 5-(2,6-Cl₂-C₆H₃)-thien-2-yl |
| 1197 | 5-(3,4-Cl₂-C₆H₃)-thien-2-yl |
| 1198 | 5-(2,4-F₂-C₆H₃)-thien-2-yl |
| 1199 | 5-(2,5-F₂-C₆H₃)-thien-2-yl |
| 1200 | 5-(2,6-F₂-C₆H₃)-thien-2-yl |
| 1201 | 5-(3,4-F₂-C₆H₃)-thien-2-yl |
| 1202 | 5-(2-Cl, 5-OCH₃-C₆H₃)-thien-2-yl |
| 1203 | 5-(2-Cl, 5-CH₃-C₆H₃)-thien-2-yl |
| 1204 | 5-(5-Cl, 2-OCH₃-C₆H₃)-thien-2-yl |
| 1205 | 5-(5-Cl, 2-CH₃-C₆H₃)-thien-2-yl |
| 1206 | 5-[2,5-(CH₃)₂-C₆H₃)-thien-2-yl |
| 1207 | 4-CH(CH₃)₂-thien-2-yl |
| 1208 | 4-C(CH₃)₃-thien-2-yl |
| 1209 | 4-cyclopropyl-thien-2-yl |
| 1210 | 4-C₆H₅-thien-2-yl |
| 1211 | 4-(2-CH₃-C₆H₄)-thien-2-yl |
| 1212 | 4-(3-CH₃-C₆H₄)-thien-2-yl |
| 1213 | 4-(4-CH₃-C₆H₄)-thien-2-yl |
| 1214 | 4-(3-OCH₃-C₆H₄)-thien-2-yl |
| 1215 | 4-(4-OCH₃-C₆H₄)-thien-2-yl |
| 1216 | 4-(4-NO₂-C₆H₄)-thien-2-yl |
| 1217 | 4-(3-NO₂-C₆H₄)-thien-2-yl |
| 1218 | 4-(4-CN-C₆H₄)-thien-2-yl |
| 1219 | 4-(3-CN-C₆H₄)-thien-2-yl |
| 1220 | 4-(3-CF₃-C₆H₄)-thien-2-yl |
| 1221 | 4-(4-CF₃-C₆H₄)-thien-2-yl |
| 1222 | 4-(4-C(CH₃)₃-C₆H₄)-thien-2-yl |
| 1223 | 4-(2-Cl-C₆H₄)-thien-2-yl |
| 1224 | 4-(3-Cl-C₆H₄)-thien-2-yl |
| 1225 | 4-(4-Cl-C₆H₄)-thien-2-yl |
| 1226 | 4-(2-Br-C₆H₄)-thien-2-yl |
| 1227 | 4-(3-Br-C₆H₄)-thien-2-yl |
| 1228 | 4-(4-Br-C₆H₄)-thien-2-yl |
| 1229 | 4-(2-F-C₆H₄)-thien-2-yl |
| 1230 | 4-(3-F-C₆H₄)-thien-2-yl |
| 1231 | 4-(4-F-C₆H₄)-thien-2-yl |
| 1232 | 4-(2,4-Cl₂-C₆H₃)-thien-2-yl |
| 1233 | 4-(2,5-Cl₂-C₆H₃)-thien-2-yl |
| 1234 | 4-(2,6-Cl₂-C₆H₃)-thien-2-yl |
| 1235 | 4-(3,4-Cl₂-C₆H₃)-thien-2-yl |
| 1236 | 4-(2,4-F₂-C₆H₃)-thien-2-yl |
| 1237 | 4-(2,5-F₂-C₆H₃)-thien-2-yl |
| 1238 | 4-(2,6-F₂-C₆H₃)-thien-2-yl |
| 1239 | 4-(3,4-F₂-C₆H₃)-thien-2-yl |
| 1240 | 4-(2-Cl, 5-OCH₃-C₆H₃)-thien-2-yl |
| 1241 | 4-(2-Cl, 5-CH₃-C₆H₃)-thien-2-yl |
| 1242 | 4-(5-Cl, 2-OCH₃-C₆H₃)-thien-2-yl |
| 1243 | 4-(5-C1, 2-CH₃-C₆H₃)-thien-2-yl |
| 1244 | 4-[2,5-(CH₃)₂-C₆H₃]-thien-2-yl |
| 1245 | 2-CH₃-thien-4-yl |
| 1246 | 2-CH(CH₃)₂-thien-4-yl |
| 1247 | 2-C(CH₃)₃-thien-4-yl |
| 1248 | 2-cyclopropyl-thien-4-yl |
| 1249 | 2-C₆H₅-thien-4-yl |
| 1250 | 2-(2-CH₃-C₆H₄)-thien-4-yl |
| 1251 | 2-(3-CH₃-C₆H₄)-thien-4-yl |
| 1252 | 2-(4-CH₃-C₆H₄)-thien-4-yl |
| 1253 | 2-(3-OCH₃-C₆H₄)-thien-4-yl |
| 1254 | 2-(4-OCH₃-C₆H₄)-thien-4-yl |
| 1255 | 2-(4-NO₂-C₆H₄)-thien-4-yl |
| 1256 | 2-(3-NO₂-C₆H₄)-thien-4-yl |
| 1257 | 2-(4-CN-C₆H₄)-thien-4-yl |
| 1258 | 2-(3-CN-C₆H₄)-thien-4-yl |
| 1259 | 2-(3-CF3-C₆H₄)-thien-4-yl |
| 1260 | 2-(4-CF₃-C₆H₄)-thien-4-yl |
| 1261 | 2-(4-C(CH₃)₃-C₆H₄)-thien-4-yl |
| 1262 | 2-(2-Cl-C₆H₄)-thien-4-yl |
| 1263 | 2-(3-Cl-C₆H₄)-thien-4-yl |
| 1264 | 2-(4-Cl-C₆H₄)-thien-4-yl |
| 1265 | 2-(2-Br-C₆H₄)-thien-4-yl |
| 1266 | 2-(3-Br-C₆H₄)-thien-4-yl |
| 1267 | 2-(4-Br-C₆H₄)-thien-4-yl |
| 1268 | 2-(2-F-C₆H₄)-thien-4-yl |
| 1269 | 2-(3-F-C₆H₄)-thien-4-yl |
| 1270 | 2-(4-F-C₆H₄)-thien-4-yl |
| 1271 | 2- (2,4-Cl₂-C₆H₃)-thien-4-yl |
| 1272 | 2-(2,5-Cl₂-C₆H₃)-thien-4-yl |
| 1273 | 2-(2,6-Cl₂-C₆H₃)-thien-4-yl |
| 1274 | 2-(3,4-Cl₂-C₆H₃)-thien-4-yl |
| 1275 | 2-(2,4-F₂-C₆H₃)-thien-4-yl |
| 1276 | 2-(2,5-F₂-C₆H₃)-thien-4-yl |
| 1277 | 2-(2,6-F₂-C₆H₃)-thien-4-yl |
| 1278 | 2-(3,4-F₂-C₆H₃)-thien-4-yl |
| 1279 | 2-(2-Cl, 5-OCH₃-C₆H₃)-thien-4-yl |
| 1280 | 2-(2-Cl, 5-CH₃-C₆H₃)-thien-4-yl |
| 1281 | 2-(5-Cl, 2-OCH₃-C₆H₃)-thien-4-yl |
| 1282 | 2-(5-Cl, 2-CH₃-C₆H₃)-thien-4-yl |
| 1283 | 2-[2,5-(CH₃)₂-C₆H₃] -thien-4-yl |
| 1284 | 1-CH(CH₃)₂-pyrazol-4-yl |
| 1285 | 1-C(CH₃)₃-pyrazol-4-yl |
| 1286 | 1-cyclopropyl-pyrazol-4-yl |
| 1287 | 1-C₆H₅-pyrazol-4-yl |
| 1288 | 1-(2-CH₃-C₆H₄)-pyrazol-4-yl |
| 1289 | 1-(3-CH₃-C₆H₄)-pyrazol-4-yl |
| 1290 | 1-(4-CH₃-C₆H₄)-pyrazol-4-yl |
| 1291 | 1-(3-OCH₃-C₆H₄)-pyrazol-4-yl |
| 1292 | 1-(4-OCH₃-C₆H₄)-pyrazol-4-yl |
| 1293 | 1-(4-NO₂-C₆H₄)-pyrazol-4-yl |
| 1294 | 1-(3-NO₂-C₆H₄)-pyrazol-4-yl |
| 1295 | 1-(4-CN-C₆H₄)-pyrazol-4-yl |
| 1296 | 1-(3-CN-C₆H₄)-pyrazol-4-yl |
| 1297 | 1-(3-CF₃-C₆H₄)-pyrazol-4-yl |
| 1298 | 1-(4-CF₃-C₆H₄)-pyrazol-4-yl |
| 1299 | 1-(4-C(CH₃)₃-C₆H₄)-pyrazol-4-yl |
| 1300 | 1-(2-Cl-C₆H₄)-pyrazol-4-yl |
| 1301 | 1-(3-Cl-C₆H₄)-pyrazol-4-yl |
| 1302 | 1-(4-Cl-C₆H₄)-pyrazol-4-yl |
| 1303 | 1-(2-Br-C₆H₄)-pyrazol-4-yl |
| 1304 | 1-(3-Br-C₆H₄)-pyrazol-4-yl |
| 1305 | 1-(4-Br-C₆H₄)-pyrazol-4-yl |
| 1306 | 1-(2-F-C₆H₄)-pyrazol-4-yl |
| 1307 | 1-(3-F-C₆H₄)-pyrazol-4-yl |
| 1308 | 1-(4-F-C₆H₄)-pyrazol-4-yl |
| 1309 | 1-(2,4-Cl₂-C₆H₃)-pyrazol-4-yl |
| 1310 | 1-(2, 5-Cl₂-C₆H₃)-Pyrazol-4-yl |
| 1311 | 1-(2,6-Cl₂-C₆H₃)-Pyrazol-4-yl |
| 1312 | 1-(3,4-Cl₂-C₆H₃)-Pyrazol-4-yl |
| 1313 | 1-(2,4-F₂-C₆H₃)-pyrazol-4-yl |
| 1314 | 1-(2, 5-F₂-C₆H₃)-pyrazol-4-yl |
| 1315 | 1-(2,6-F₂-C₆H₃)-pyrazol-4-yl |
| 1316 | 1-(3,4-F₂-C₆H₃)-pyrazol-4-yl |
| 1317 | 1-(2-Cl, 5-OCH₃-C₆H₃)-pyrazol-4-yl |
| 1318 | 1-(2-Cl, 5-CH₃-C₆H₃)-pyrazol-4-yl |
| 1319 | 1-(5-Cl, 2-OCH₃-C₆H₃)-pyrazol-4-yl |
| 1320 | 1-(5-Cl, 2-CH₃-C₆H₃)-pyrazol-4-yl |
| 1321 | 1-[2,5-(CH₃)₂-C₆H₃]-pyrazol-4-yl |
| 1322 | 1-CH(CH₃)₂-pyrazol-3-yl |
| 1323 | 1-C(CH₃)₃-pyrazol-3-yl |
| 1324 | 1-cyclopropyl-pyrazol-3-yl |
| 1325 | 1-C₆H₅-pyrazol-3-yl |
| 1326 | 1-(2-CH₃-C₆H₄)-pyrazol-3-yl |
| 1327 | 1-(3-CH₃-C₆H₄)-pyrazol-3-yl |
| 1328 | 1-(4-CH₃-C₆H₄)-pyrazol-3-yl |
| 1329 | 1-(3-OCH₃-C₆H₄)-pyrazol-3-yl |
| 1330 | 1-(4-OCH₃-C₆H₄)-pyrazol-3-yl |
| 1331 | 1-(4-NO₂-C₆H₄)-pyrazol-3-yl |
| 1332 | 1-(3-NO₂-C₆H₄)-pyrazol-3-yl |
| 1333 | 1-(4-CN-C₆H₄)-pyrazol-3-yl |
| 1334 | 1-(3-CN-C₆H₄)-pyrazol-3-yl |
| 1335 | 1-(3-CF₃-C₆H₄)-pyrazol-3-yl |
| 1336 | 1-(4-CF₃-C₆H₄)-pyrazol-3-yl |
| 1337 | 1-(4-C(CH₃)₃-C₆H₄)-pyrazol-3-yl |
| 1338 | 1-(2-Cl-C₆H₄)-pyrazol-3-yl |
| 1339 | 1-(3-Cl-C₆H₄)-pyrazol-3-yl |
| 1340 | 1-(4-Cl-C₆H₄) -pyrazol-3-yl |
| 1341 | 1-(2-Br-C₆H₄)-pyrazol-3-yl |
| 1342 | 1-(3-Br-C₆H₄)-pyrazol-3-yl |
| 1343 | 1-(4-Br-C₆H₄) -pyrazol-3-yl |
| 1344 | 1-(2-F-C₆H₄)-pyrazol-3-yl |
| 1345 | 1-(3-F-C₆H₄)-pyrazol-3-yl |
| 1346 | 1-(4-F-C₆H₄)-pyrazol-3-yl |
| 1347 | 1-(2,4-Cl₂-C₆H₃)-pyrazol-3-yl |
| 1348 | 1-(2,5-Cl₂-C₆H₃)-pyrazol-3-yl |
| 1349 | 1-(2,6-Cl₂-C₆H₃)-pyrazol-3-yl |
| 1350 | 1-(3,4-Cl₂-C₆H₃)-pyrazol-3-yl |
| 1351 | 1-(2,4-F₂-C₆H₃)-pyrazol-3-yl |
| 1352 | 1-(2,5-F₂-C₆H₃)-pyrazol-3-yl |
| 1353 | 1-(2,6-F₂-C₆H₃)-pyrazol-3-yl |
| 1354 | 1-(3,4-F₂-C₆H₃)-pyrazol-3-yl |
| 1355 | 1-(2-Cl, 5-OCH₃-C₆H₃)-pyrazol-3-yl |
| 1356 | 1-(2-Cl, 5-CH₃-C₆H₃)-pyrazol-3-yl |
| 1357 | 1-(5-Cl, 2-OCH₃-C₆H₃)-pyrazol-3-yl |
| 1358 | 1-(5-Cl, 2-CH₃-C₆H₃)-pyrazol-3-yl |
| 1359 | 1-[2,5- (CH₃)₂-C₆H₃]-pyrazol-3-yl |
| 1360 | 3-CH(CH₃)₂-isoxazol-5-yl |
| 1361 | 3-C(CH₃)₃-isoxazol-5-yl |
| 1362 | 3-cyclopropyl-isoxazol-5-yl |
| 1363 | 3-C₆H₅-isoxazol-5-yl |
| 1364 | 3-(2-CH₃-C₆H₄)-isoxazol-5-yl |
| 1365 | 3-(3-CH₃-C₆H₄)-isoxazol-5-yl |
| 1366 | 3-(4-CH₃-C₆H₄)-isoxazol-5-yl |
| 1367 | 3-(3-OCH₃-C₆H₄)-isoxazol-5-yl |
| 1368 | 3- (4-OCH₃-C₆H₄)-isoxazol-5-yl |
| 1369 | 3-(4-NO₂-C₆H₄)-isoxazol-5-yl |
| 1370 | 3-(3-NO₂-C₆H₄)-isoxazol-5-yl |
| 1371 | 3-(4-CN-C₆H₄)-isoxazol-5-yl |
| 1372 | 3-(3-CN-C₆H₄)-isoxazol-5-yl |
| 1373 | 3-(3-CF₃-C₆H₄)-isoxazol-5-yl |
| 1374 | 3-(4-CF₃-C₆H₄)-isoxazol-5-yl |
| 1375 | 3-(4-C(CH₃)₃-C₆H₄)-isoxazol-5-yl |
| 1376 | 3-(2-Cl-C₆H₄)-isoxazol-5-yl |
| 1377 | 3-(3-Cl-C₆H₄)-isoxazol-5-yl |
| 1378 | 3-(4-Cl-C₆H₄)-isoxazol-5-yl |
| 1379 | 3-(2-Br-C₆H₄)-isoxazol-5-yl |
| 1380 | 3-(3-Br-C₆H₄)-isoxazol-5-yl |
| 1381 | 3-(4-Br-C₆H₄)-isoxazol-5-yl |
| 1382 | 3-(2-F-C₆H₄)-isoxazol-5-yl |
| 1383 | 3-(3-F-C₆H₄)-isoxazol-5-yl |
| 1384 | 3-(4-F-C₆H₄)-isoxazol-5-yl |
| 1385 | 3- (2,4-Cl₂-C₆H₃)-isoxazol-5-yl |
| 1386 | 3-(2,5-Cl₂-C₆H₃)-isoxazol-5-yl |
| 1387 | 3-(2,6-Cl₂-C₆H₃)-isoxazol-5-yl |
| 1388 | 3-(3,4-Cl₂-C₆H₃)-isoxazol-5-yl |
| 1389 | 3-(2,4-F₂-C₆H₃)-isoxazol-5-yl |
| 1390 | 3-(2,5-F₂-C₆H₃)-isoxazol-5-yl |
| 1391 | 3-(2,6-F₂-C₆H₃)-isoxazol-5-yl |
| 1392 | 3-(3,4-F₂-C₆H₃)-isoxazol-5-yl |
| 1393 | 3-(2-Cl, 5-OCH₃-C₆H₃)-isoxazol-5-yl |
| 1394 | 3-(2-Cl, 5-CH₃-C₆H₃)-isoxazol-5-yl |
| 1395 | 3-(5-Cl, 2-OCH₃-C₆H₃)-isoxazol-5-yl |
| 1396 | 3-(5-Cl, 2-CH₃-C₆H₃)-isoxazol-5-yl |
| 1397 | 3- [2,5-(CH₃)₂-C₆H₃)-isoxazol-5-yl |
| 1398 | 5-CH(CH₃)₂-isoxazol-3-yl |
| 1399 | 5-C(CH₃)₃-isoxazol-3-yl |
| 1400 | 5-cyclopropyl-isoxazol-3-yl |
| 1401 | 5-C₆H₅-isoxazol-3-yl |
| 1402 | 5-(2-CH₃-C₆H₄)-isoxazol-3-yl |
| 1403 | 5-(3-CH₃-C₆H₄)-isoxazol-3-yl |
| 1404 | 5-(4-CH₃-C₆H₄)-isoxazol-3-yl |
| 1405 | 5-(3-OCH₃-C₆H₄)-isoxazol-3-yl |
| 1406 | 5-(4-OCH₃-C₆H₄)-isoxazol-3-yl |
| 1407 | 5-(4-NO₂-C₆H₄)-isoxazol-3-yl |
| 1408 | 5-(3-NO₂-C₆H₄)-isoxazol-3-yl |
| 1409 | 5-(4-CN-C₆H₄)-isoxazol-3-yl |
| 1410 | 5-(3-CN-C₆H₄)-isoxazol-3-yl |
| 1411 | 5-(3-CF₃-C₆H₄)-isoxazol-3-yl |
| 1412 | 5-(4-CF₃-C₆H₄)-isoxazol-3-yl |
| 1413 | 5-(4-C(CH₃)₃-C₆H₄)-isoxazol-3-yl |
| 1414 | 5-(2-Cl-C₆H₄)-isoxazol-3-yl |
| 1415 | 5-(3-Cl-C₆H₄)-isoxazol-3-yl |
| 1416 | 5-(4-Cl-C₆H₄)-isoxazol-3-yl |
| 1417 | 5-(2-Br-C₆H₄)-isoxazol-3-yl |
| 1418 | 5-(3-Br-C₆H₄)-isoxazol-3-yl |
| 1419 | 5-(4-Br-C₆H₄)-isoxazol-3-yl |
| 1420 | 5-(2-F-C₆H₄)-isoxazol-3-yl |
| 1421 | 5-(3-F-C₆H₄)-isoxazol-3-yl |
| 1422 | 5-(4-F-C₆H₄)-isoxazol-3-yl |
| 1423 | 5-(2,4-Cl₂-C₆H₃)-isoxazol-3-yl |
| 1424 | 5-(2,5-Cl₂-C₆H₃)-isoxazol-3-yl |
| 1425 | 5-(2,6-Cl₂-C₆H₃)-isoxazol-3-yl |
| 1426 | 5-(3,4-Cl₂-C₆H₃)-isoxazol-3-yl |
| 1427 | 5-(2,4-F₂-C₆H₃)-isoxazol-3-yl |
| 1428 | 5-(2,5-F₂-C₆H₃)-isoxazol-3-yl |
| 1429 | 5-(2,6-F₂-C₆H₃)-isoxazol-3-yl |
| 1430 | 5-(3,4-F₂-C₆H₃)-isoxazol-3-yl |
| 1431 | 5-(2-Cl, 5-OCH₃-C₆H₃)-isoxazol-3-yl |
| 1432 | 5-(2-Cl, 5-CH₃-C₆H₃)-isoxazol-3-yl |
| 1433 | 5-(5-Cl, 2-OCH₃-C₆H₃)-isoxazol-3-yl |
| 1434 | 5-(5-Cl, 2-CH₃-C₆H₃)-isoxazol-3-yl |
| 1435 | 5-[2,5-(CH₃)₂-C₆H₃]-isoxazol-3-yl |
| 1436 | 3-CH(CH₃)₂-isothiazol-5-yl |
| 1437 | 3-C(CH₃)₃-isothiazol-5-yl |
| 1438 | 3-cyclopropyl-isothiazol-5-yl |
| 1439 | 3-C₆H₅-isothiazol-5-yl |
| 1440 | 3- (2-CH₃-C₆H₄)-isothiazol-5-yl |
| 1441 | 3- (3-CH₃-C₆H₄)-isothiazol-5-yl |
| 1442 | 3-(4-CH₃-C₆H₄)-isothiazol-5-yl |
| 1443 | 3- (3-OCH₃-C₆H₄)-isothiazol-5-yl |
| 1444 | 3-(4-OCH₃-C₆H₄)-isothiazol-5-yl |
| 1445 | 3- (4-NO₂-C₆H₄)-isothiazol-5-yl |
| 1446 | 3- (3-NO₂-C₆H₄)-isothiazol-5-yl |
| 1447 | 3-(4-CN-C₆H₄)-isothiazol-5-yl |
| 1448 | 3-(3-CN-C₆H₄)-isothiazol-5-yl |
| 1449 | 3- (3-CF₃-C₆H₄)-isothiazol-5-yl |
| 1450 | 3-(4-CF₃-C₆H₄)-isothiazol-5-yl |
| 1451 | 3-(4-C(CH₃)₃-C₆H₄)-isothiazol-5-yl |
| 1452 | 3-(2-Cl-C₆H₄)-isothiazol-5-yl |
| 1453 | 3-(3-Cl-C₆H₄)-isothiazol-5-yl |
| 1454 | 3-(4-Cl-C₆H₄)-isothiazol-5-yl |
| 1455 | 3-(2-Br-C₆H₄)-isothiazol-5-yl |
| 1456 | 3-(3-Br-C₆H₄)-isothiazol-5-yl |
| 1457 | 3-(4-Br-C₆H₄)-isothiazol-5-yl |
| 1458 | 3-(2-F-C₆H₄)-isothiazol-5-yl |
| 1459 | 3-(3-F-C₆H₄)-isothiazol-5-yl |
| 1460 | 3- (4-F-C₆H₄)-isothiazol-5-yl |
| 1461 | 3-(2,4-Cl₂-C₆H₃)-isothiazol-5-yl |
| 1462 | 3-(2,5-Cl₂-C₆H₃)-isothiazol-5-yl |
| 1463 | 3-(2,6-Cl₂-C₆H₃)-isothiazol-5-yl |
| 1464 | 3- (3,4-Cl₂-C₆H₃)-isothiazol-5-yl |
| 1465 | 3-(2,4-F₂-C₆H₃)-isothiazol-5-yl |
| 1466 | 3-(2,5-F₂-C₆H₃)-isothiazol-5-yl |
| 1467 | 3-(2,6-F₂-C₆H₃)-isothiazol-5-yl |
| 1468 | 3-(3,4-F₂-C₆H₃)-isothiazol-5-yl |
| 1469 | 3-(2-Cl, 5-OCH₃-C₆H₃)-isothiazol-5-yl |
| 1470 | 3-(2-Cl, 5-CH₃-C₆H₃)-isothiazol-5-yl |
| 1471 | 3-(5-Cl, 2-OCH₃-C₆H₃)-isothiazol-5-yl |
| 1472 | 3-(5-Cl, 2-CH₃-C₆H₃)-isothiazol-5-yl |
| 1473 | 3-[2,5-(CH₃)₂-C₆H₃]-isothiazol-5-yl |
| 1474 | 2-CH(CH₃)₂-oxazol-4-yl |
| 1475 | 2-C(CH₃)₃-oxazol-4-yl |
| 1476 | 2-cyclopropyl-oxazol-4-yl |
| 1477 | 2-C₆H₅-oxazol-4-yl |
| 1478 | 2-(2-CH₃-C₆H₄)-oxazol-4-yl |
| 1479 | 2-(3-CH₃-C₆H₄)-oxazol-4-yl |
| 1480 | 2-(4-CH₃-C₆H₄)-oxazol-4-yl |
| 1481 | 2-(3-OCH₃-C₆H₄)-oxazol-4-yl |
| 1482 | 2-(4-OCH₃-C₆H₄)-oxazol-4-yl |
| 1483 | 2-(4-NO₂-C₆H₄)-oxazol-4-yl |
| 1484 | 2-(3-NO₂-C₆H₄)-oxazol-4-yl |
| 1485 | 2-(4-CN-C₆H₄)-oxazol-4-yl |
| 1486 | 2-(3-CN-C₆H₄)-oxazol-4-yl |
| 1487 | 2-(3-CF₃-C₆H₄)-oxazol-4-yl |
| 1488 | 2-(4-CF₃-C₆H₄)-oxazol-4-yl |
| 1489 | 2-(4-C(CH₃)₃-C₆H₄)-oxazol-4-yl |
| 1490 | 2-(2-Cl-C₆H₄)-oxazol-4-yl |
| 1491 | 2-(3-Cl-C₆H₄)-oxazol-4-yl |
| 1492 | 2-(4-Cl-C₆H₄)-oxazol-4-yl |
| 1493 | 2-(2-Br-C₆H₄)-oxazol-4-yl |
| 1494 | 2-(3-Br-C₆H₄)-oxazol-4-yl |
| 1495 | 2-(4-Br-C₆H₄)-oxazol-4-yl |
| 1496 | 2-(2-F-C₆H₄)-oxazol-4-yl |
| 1497 | 2-(3-F-C₆H₄)-oxazol-4-yl |
| 1498 | 2-(4-F-C₆H₄)-oxazol-4-yl |
| 1499 | 2-(2,4-Cl₂-C₆H₃)-oxazol-4-yl |
| 1500 | 2-(2,5-Cl₂-C₆H₃)-oxazol-4-yl |
| 1501 | 2-(2,6-Cl₂-C₆H₃)-oxazol-4-yl |
| 1502 | 2-(3,4-Cl₂-C₆H₃)-oxazol-4-yl |
| 1503 | 2-(2,4-F₂-C₆H₃)-oxazol-4-yl |
| 1504 | 2-(2,5-F₂-C₆H₃)-oxazol-4-yl |
| 1505 | 2-(2,6-F₂-C₆H₃)-oxazol-4-yl |
| 1506 | 2-(3,4-F₂-C₆H₃)-oxazol-4-yl |
| 1507 | 2-(2-Cl, 5-OCH₃-C₆H₃)-oxazol-4-yl |
| 1508 | 2-(2-Cl, 5-CH₃-C₆H₃)-oxazol-4-yl |
| 1509 | 2-(5-Cl, 2-OCH₃-C₆H₃)-oxazol-4-yl |
| 1510 | 2-(5-Cl, 2-CH₃-C₆H₃)-oxazol-4-yl |
| 1511 | 2-[2,5-(CH₃)₂-C₆H₃]-oxazol-4-yl |
| 1512 | 2-CH(CH₃)₂-thiazol-4-yl |
| 1513 | 2-C(CH₃)₃-thiazol-4-yl |
| 1514 | 2-cyclopropyl-thiazol-4-yl |
| 1515 | 2-C₆H₅-thiazol-4-yl |
| 1516 | 2-(2-CH₃-C₆H₄)-thiazol-4-yl |
| 1517 | 2-(3-CH₃-C₆H₄)-thiazol-4-yl |
| 1518 | 2-(4-CH₃-C₆H₄) -thiazol-4-yl |
| 1519 | 2-(3-OCH₃-C₆H₄)-thiazol-4-yl |
| 1520 | 2-(4-OCH₃-C₆H₄)-thiazol-4-yl |
| 1521 | 2-(4-NO₂-C₆H₄)-thiazol-4-yl |
| 1522 | 2-(3-NO₂-C₆H₄)-thiazol-4-yl |
| 1523 | 2-(4-CN-C₆H₄)-thiazol-4-yl |
| 1524 | 2-(3-CN-C₆H₄)-thiazol-4-yl |
| 1525 | 2-(3-CF₃-C₆H₄)-thiazol-4-yl |
| 1526 | 2-(4-CF₃-C₆H₄)-thiazol-4-yl |
| 1527 | 2-(4-C(CH₃)₃-C₆H₄)-thiazol-4-yl |
| 1528 | 2-(2-Cl-C₆H₄)-thiazol-4-yl |
| 1529 | 2-(3-Cl-C₆H₄)-thiazol-4-yl |
| 1530 | 2-(4-Cl-C₆H₄)-thiazol-4-yl |
| 1531 | 2-(2-Br-C₆H₄)-thiazol-4-yl |
| 1532 | 2-(3-Br-C₆H₄)-thiazol-4-yl |
| 1533 | 2-(4-Br-C₆H₄)-thiazol-4-yl |
| 1534 | 2-(2-F-C₆H₄)-thiazol-4-yl |
| 1535 | 2-(3-F-C₆H₄)-thiazol-4-yl |
| 1536 | 2-(4-F-C₆H₄)-thiazol-4-yl |
| 1537 | 2-(2,4-Cl₂-C₆H₃)-thiazol-4-yl |
| 1538 | 2-(2,5-Cl₂-C₆H₃)-thiazol-4-yl |
| 1539 | 2-(2,6-Cl₂-C₆H₃)-thiazol-4-yl |
| 1540 | 2-(3,4-Cl₂-C6H3)-thiazol-4-yl |
| 1541 | 2-(2,4-F₂-C₆H₃)-thiazol-4-yl |
| 1542 | 2-(2,5-F₂-C₆H₃)-thiazol-4-yl |
| 1543 | 2-(2,6-F₂-C₆H₃)-thiazol-4-yl |
| 1544 | 2-(3,4-F₂-C₆H₃)-thiazol-4-yl |
| 1545 | 2-(2-Cl, 5-OCH₃-C₆H₃)-thiazol-4-yl |
| 1546 | 2-(2-Cl, 5-CH₃-C₆H₃)-thiazol-4-yl |
| 1547 | 2-(5-Cl, 2-OCH₃-C₆H₃)-thiazol-4-yl |
| 1548 | 2-(5-Cl, 2-CH₃-C₆H₃)-thiazol-4-yl |
| 1549 | 2-[2,5-(CH₃)₂-C₆H₃]-thiazol-4-yl |
| 1550 | 1,3-(CH₃)₂-1,2,4-triazol-5-yl |
| 1551 | 1-CH(CH₃)₂-1,2,4-triazol-3-yl |
| 1552 | 1-C(CH₃)₃-1,2,4-triazol-3-yl |
| 1553 | 1-cyclopropyl-1,2,4-triazol-3-yl |
| 1554 | 1-C₆H₅-1,2,4-triazol-3-yl |
| 1555 | 1-(2-CH₃-C₆H₄)-1,2,4-triazol-3-yl |
| 1556 | 1-(3-CH₃-C₆H₄)-1,2,4-triazol-3-yl |
| 1557 | 1-(4-CH₃-C₆H₄)-1,2,4-triazol-3-yl |
| 1558 | 1-(3-OCH₃-C₆H₄)-1,2,4-triazol-3-yl |
| 1559 | 1-(4-OCH₃-C₆H₄)-1,2,4-triazol-3-yl |
| 1560 | 1-(4-NO₂-C₆H₄)-1,2,4-triazol-3-yl |
| 1561 | 1-(3-NO₂-C₆H₄)-1,2,4-triazol-3-yl |
| 1562 | 1-(4-CN-C₆H₄)-1,2,4-triazol-3-yl |
| 1563 | 1-(3-CN-C₆H₄)-1,2,4-triazol-3-yl |
| 1564 | 1-(3-CF₃-C₆H₄)-1,2,4-triazol-3-yl |
| 1565 | 1-(4-CF₃-C₆H₄)-1,2,4-triazol-3-yl |
| 1566 | 1-(4-C(CH₃)₃-C₆H₄)-1,2,4-triazol-3-yl |
| 1567 | 1-(4-C₆H₅-C₆H₄)-1,2,4-triazol-3-yl |
| 1568 | 1-(2-Cl-C₆H₄)-1,2,4-triazol-3-yl |
| 1569 | 1-(3-Cl-C₆H₄)-1,2,4-triazol-3-yl |
| 1570 | 1-(4-Cl-C₆H₄)-1,2,4-triazol-3-yl |
| 1571 | 1-(2-Br-C₆H₄)-1,2,4-triazol-3-yl |
| 1572 | 1-(3-Br-C₆H₄)-1,2,4-triazol-3-yl |
| 1573 | 1-(4-Br-C₆H₄)-1,2,4-triazol-3-yl |
| 1574 | 1-(2-F-C₆H₄)-1,2,4-triazol-3-yl |
| 1575 | 1-(3-F-C₆H₄)-1,2,4-triazol-3-yl |
| 1576 | 1-(4-F-C₆H₄)-1,2,4-triazol-3-yl |
| 1577 | 1-(2,4-Cl₂-C₆H₃)-1,2,4-triazol-3-yl |
| 1578 | 1-(2,5-Cl₂-C₆H₃)-1,2,4-triazol-3-yl |
| 1579 | 1-(2,6-Cl₂-C₆H₃)-1,2,4-triazol-3-yl |
| 1580 | 1-(3,4-Cl₂-C₆H₃)-1,2,4-triazol-3-yl |
| 1581 | 1-(2,4-F₂-C₆H₃)-1,2,4-triazol-3-yl |
| 1582 | 1-(2,5-F₂-C₆H₃)-1,2,4-triazol-3-yl |
| 1583 | 1-(2,6-F₂-C₆H₃)-1,2,4-triazol-3-yl |
| 1584 | 1-(3,4-F₂-C₆H₃)-1,2,4-triazol-3-yl |
| 1585 | 1-(2-Cl, 5-OCH₃-C₆H₃)-1,2,4-triazol-3-yl |
| 1586 | 1-(2-Cl, 5-CH₃-C₆H₃)-1,2,4-triazol-3-yl |
| 1587 | 1-(5-Cl, 2-OCH₃-C₆H₃)-1,2,4-triazol-3-yl |
| 1588 | 1-(5-Cl, 2-CH₃-C₆H₃)-1,2,4-triazol-3-yl |
| 1589 | 1-[2,5-(CH₃)₂-C₆H₃]-1,2,4-triazol-3-yl |
| 1590 | 5-C(CH₃)₃-1,3,4-oxadiazol-2-yl |
| 1591 | 5-cyclopropyl-1,3,4-oxadiazol-2-yl |
| 1592 | 5-C₆H₅-1,3,4-oxadiazol-2-yl |
| 1593 | 5-(2-CH₃-C₆H₄)-1,3,4-oxadiazol-2-yl |
| 1594 | 5-(3-CH₃-C₆H₄)-1,3,4-oxadiazol-2-yl |
| 1595 | 5-(4-CH₃-C₆H₄)-1,3,4-oxadiazol-2-yl |
| 1596 | 5-(3-OCH₃-C₆H₄)-1,3,4-oxadiazol-2-yl |
| 1597 | 5-(4-OCH₃-C₆H₄)-1,3,4-oxadiazol-2-yl |
| 1598 | 5-(4-NO₂-C₆H₄)-1,3,4-oxadiazol-2-yl |
| 1599 | 5-(3-NO₂-C₆H₄)-1,3,4-oxadiazol-2-yl |
| 1600 | 5-(4-CN-C₆H₄)-1,3,4-oxadiazol-2-yl |
| 1601 | 5-(3-CN-C₆H₄)-1,3,4-oxadiazol-2-yl |
| 1602 | 5-(3-CF₃-C₆H₄)-1,3,4-oxadiazol-2-yl |
| 1603 | 5-(4-CF₃-C₆H₄)-1,3,4-oxadiazol-2-yl |
| 1604 | 5-(4-C(CH₃)₃-C₆H₄)-1,3,4-oxadiazol-2-yl |
| 1605 | 5-(2-Cl-C₆H₄)-1,3,4-oxadiazol-2-yl |
| 1606 | 5-(3-Cl-C₆H₄)-1,3,4-oxadiazol-2-yl |
| 1607 | 5-(4-Cl-C₆H₄)-1,3,4-oxadiazol-2-yl |
| 1608 | 5-(2-Br-C₆H₄)-1,3,4-oxadiazol-2-yl |
| 1609 | 5-(3-Br-C₆H₄)-1,3,4-oxadiazol-2-yl |
| 1610 | 5-(4-Br-C₆H₄)-1,3,4-oxadiazol-2-yl |
| 1611 | 5-(2-F-C₆H₄)-1,3,4-oxadiazol-2-yl |
| 1612 | 5-(3-F-C₆H₄)-1,3,4-oxadiazol-2-yl |
| 1613 | 5-(4-F-C₆H₄)-1,3,4-oxadiazol-2-yl |
| 1614 | 5-(2,4-Cl₂-C₆H₃)-1,3,4-oxadiazol-2-yl |
| 1615 | 5-(2,5-Cl₂-C₆H₃)-1,3,4-oxadiazol-2-yl |
| 1616 | 5-(2,6-Cl₂-C₆H₃)-1,3,4-oxadiazol-2-yl |
| 1617 | 5-(3,4-Cl₂-C₆H₃)-1,3,4-oxadiazol-2-yl |
| 1618 | 5-(2,4-F₂-C₆H₃)-1,3,4-oxadiazol-2-yl |
| 1619 | 5-(2,5-F₂-C₆H₃)-1,3,4-oxadiazol-2-yl |
| 1620 | 5-(2,6-F₂-C₆H₃)-1,3,4-oxadiazol-2-yl |
| 1621 | 5-(3,4-F₂-C₆H₃)-1,3,4-oxadiazol-2-yl |
| 1622 | 5-(2-Cl, 5-OCH₃-C₆H₃)-1,3,4-oxadiazol-2-yl |
| 1623 | 5-(2-Cl, 5-CH₃-C₆H₃)-1,3,4-oxadiazol-2-yl |
| 1624 | 5-(5-Cl, 2-OCH₃-C₆H₃)-1,3,4-oxadiazol-2-yl |
| 1625 | 5-(5-Cl, 2-CH₃-C₆H₃)-1,3,4-oxadiazol-2-yl |
| 1626 | 5-[2,5-(CH₃)₂-C₆H₃]-1,3,4-oxadiazol-2-yl |
| 1627 | 5-C(CH₃)₃-1,2,4-oxadiazol-3-yl |
| 1628 | 5-cyclopropyl-1,2,4-oxadiazol-3-yl |
| 1629 | 5-C₆H₅-1,2,4-oxadiazol-3-yl |
| 1630 | 5-(2-CH₃-C₆H₄)-1,2,4-oxadiazol-3-yl |
| 1631 | 5-(3-CH₃-C₆H₄)-1,2,4-oxadiazol-3-yl |
| 1632 | 5-(4-CH₃-C₆H₄)-1,2,4-oxadiazol-3-yl |
| 1633 | 5-(3-OCH₃-C₆H₄)-1,2,4-oxadiazol-3-yl |
| 1634 | 5-(4-OCH₃-C₆H₄)-1,2,4-oxadiazol-3-yl |
| 1635 | 5-(4-NO₂-C₆H₄)-1,2,4-oxadiazol-3-yl |
| 1636 | 5-(3-NO₂-C₆H₄)-1,2,4-oxadiazol-3-yl |
| 1637 | 5-(4-CN-C₆H₄)-1,2,4-oxadiazol-3-yl |
| 1638 | 5-(3-CN-C₆H₄)-1,2,4-oxadiazol-3-yl |
| 1639 | 5-(3-CF₃-C₆H₄)-1,2,4-oxadiazol-3-yl |
| 1640 | 5-(4-CF₃-C₆H₄)-1,2,4-oxadiazol-3-yl |
| 1641 | 5-(4-C(CH₃)₃-C₆H₄)-1,2,4-oxadiazol-3-yl |
| 1642 | 5-(2-Cl-C₆H₄)-1,2,4-oxadiazol-3-yl |
| 1643 | 5-(3-Cl-C₆H₄)-1,2,4-oxadiazol-3-yl |
| 1644 | 5-(4-Cl-C₆H₄)-1,2,4-oxadiazol-3-yl |
| 1645 | 5-(2-Br-C₆H₄)-1,2,4-oxadiazol-3-yl |
| 1646 | 5-(3-Br-C₆H₄)-1,2,4-oxadiazol-3-yl |
| 1647 | 5-(4-Br-C₆H₄)-1,2,4-oxadiazol-3-yl |
| 1648 | 5-(2-F-C₆H₄)-1,2,4-oxadiazol-3-yl |
| 1649 | 5-(3-F-C₆H₄)-1,2,4-oxadiazol-3-yl |
| 1650 | 5-(4-F-C₆H₄)-1,2,4-oxadiazol-3-yl |
| 1651 | 5-(2,4-Cl₂-C₆H₃)-1,2,4-oxadiazol-3-yl |
| 1652 | 5- (2,5-Cl₂-C₆H₃)-1,2,4-oxadiazol-3-yl |
| 1653 | 5- (2,6-Cl₂-C₆H₃)-1,2,4-oxadiazol-3-yl |
| 1654 | 5-(3,4-Cl₂-C₆H₃)-1,2,4-oxadiazol-3-yl |
| 1655 | 5-(2,4-F₂-C₆H₃)-1,2,4-oxadiazol-3-yl |
| 1656 | 5-(2,5-F₂-C₆H₃)-1,2,4-oxadiazol-3-yl |
| 1657 | 5- (2,6-F₂-C₆H₃)-1,2,4-oxadiazol-3-yl |
| 1658 | 5-(3,4-F₂-C₆H₃)-1,2,4-oxadiazol-3-yl |
| 1659 | 5-(2-Cl, 5-OCH₃-C₆H₃)-1,2,4-oxadiazol-3-yl |
| 1660 | 5-(2-Cl, 5-CH₃-C₆H₃)-1,2,4-oxadiazol-3-yl |
| 1661 | 5-(5-Cl, 2-OCH₃-C₆H₃)-1,2,4-oxadiazol-3-yl |
| 1662 | 5-(5-Cl, 2-CH₃-C₆H₃)-1,2,4-oxadiazol-3-yl |
| 1663 | 5-[2,5-(CH₃)₂-C₆H₃]-1,2,4-oxadiazol-3-yl |
| 1664 | 3-CH₃-1,2,4-oxadiazol-5-yl |
| 1665 | 3-CH(CH₃)₂-1,2,4-oxadiazol-5-yl |
| 1666 | 3-C(CH₃)₃-1,2,4-oxadiazol-5-yl |
| 1667 | 3-cyclopropyl-1,2,4-oxadiazol-5-yl |
| 1668 | 3-C₆H₅-1,2,4-oxadiazol-5-yl |
| 1669 | 3-(2-CH₃-C₆H₄)-1,2,4-oxadiazol-5-yl |
| 1670 | 3-(3-CH₃-C₆H₄)-1,2,4-oxadiazol-5-yl |
| 1671 | 3-(4-CH₃-C₆H₄)-1,2,4-oxadiazol-5-yl |
| 1672 | 3-(3-OCH₃-C₆H₄)-1,2,4-oxadiazol-5-yl |
| 1673 | 3-(4-OCH₃-C₆H₄)-1,2,4-oxadiazol-5-yl |
| 1674 | 3-(4-NO₂-C₆H₄)-1,2,4-oxadiazol-5-yl |
| 1675 | 3-(3-NO₂-C₆H₄)-1,2,4-oxadiazol-5-yl |
| 1676 | 3-(4-CN-C₆H₄)-1,2,4-oxadiazol-5-yl |
| 1677 | 3-(3-CN-C₆H₄)-1,2,4-oxadiazol-5-yl |
| 1678 | 3-(3-CF₃-C₆H₄)-1,2,4-oxadiazol-5-yl |
| 1679 | 3-(4-CF₃-C₆H₄)-1,2,4-oxadiazol-5-yl |
| 1680 | 3-(4-C(CH₃)₃-C₆H₄)-1,2,4-oxadiazol-5-yl |
| 1681 | 3-(2-Cl-C₆H₄)-1,2,4-oxadiazol-5-yl |
| 1682 | 3-(3-Cl-C₆H₄)-1,2,4-oxadiazol-5-yl |
| 1683 | 3-(4-Cl-C₆H₄)-1,2,4-oxadiazol-5-yl |
| 1684 | 3-(2-Br-C₆H₄)-1,2,4-oxadiazol-5-yl |
| 1685 | 3-(3-Br-C₆H₄)-1,2,4-oxadiazol-5-yl |
| 1686 | 3-(4-Br-C₆H₄)-1,2,4-oxadiazol-5-yl |
| 1687 | 3-(2-F-C₆H₄)-1,2,4-oxadiazol-5-yl |
| 1688 | 3-(3-F-C₆H₄)-1,2,4-oxadiazol-5-yl |
| 1689 | 3-(4-F-C₆H₄)-1,2,4-oxadiazol-5-yl |
| 1690 | 3-(2,4-Cl₂-C₆H₃)-1,2,4-oxadiazol-5-yl |
| 1691 | 3-(2,5-Cl₂-C₆H₃)-1,2,4-oxadiazol-5-yl |
| 1692 | 3-(2,6-Cl₂-C₆H₃)-1,2,4-oxadiazol-5-yl |
| 1693 | 3-(3,4-Cl₂-C₆H₃)-1,2,4-oxadiazol-5-yl |
| 1694 | 3-(2,4-F₂-C₆H₃)-1,2,4-oxadiazol-5-yl |
| 1695 | 3-(2,5-F₂-C₆H₃)-1,2,4-oxadiazol-5-yl |
| 1696 | 3-(2,6-F₂-C₆H₃)-1,2,4-oxadiazol-5-yl |
| 1697 | 3-(3,4-F₂-C₆H₃)-1,2,4-oxadiazol-5-yl |
| 1698 | 3-(2-Cl, 5-OCH₃-C₆H₃)-1,2,4-oxadiazol-5-yl |
| 1699 | 3-(2-Cl, 5-CH₃-C₆H₃)-1,2,4-oxadiazol-5-yl |
| 1700 | 3-(5-Cl, 2-OCH₃-C₆H₃)-1,2,4-oxadiazol-5-yl |
| 1701 | 3-(5-Cl, 2-CH₃-C₆H₃)-1,2,4-oxadiazol-5-yl |
| 1702 | 3-[2,5-(CH₃)₂-C₆H₃]-1,2,4-oxadiazol-5-yl |
| 1703 | 5-CH₃-1,2,4-thiadiazol-3-yl |
| 1704 | 5-CH(CH₃)₂-1,2,4-thiadiazol-3-yl |
| 1705 | 5-C(CH₃)₃-1,2,4-thiadiazol-3-yl |
| 1706 | 5-cyclopropyl-1,2,4-thiadiazol-3-yl |
| 1707 | 5-C₆H₅-1,2,4-thiadiazol-3-yl |
| 1708 | 5-(2-CH₃-C₆H₄)-1,2,4-thiadiazol-3-yl |
| 1709 | 5-(3-CH₃-C₆H₄)-1,2,4-thiadiazol-3-yl |
| 1710 | 5-(4-CH₃-C₆H₄)-1,2,4-thiadiazol-3-yl |
| 1711 | 5-(3-OCH₃-C₆H₄)-1,2,4-thiadiazol-3-yl |
| 1712 | 5-(4-OCH₃-C₆H₄)-1,2,4-thiadiazol-3-yl |
| 1713 | 5-(4-NO₂-C₆H₄)-1,2,4-thiadiazol-3-yl |
| 1714 | 5-(3-NO₂-C₆H₄)-1,2,4-thiadiazol-3-yl |
| 1715 | 5-(4-CN-C₆H₄)-1,2,4-thiadiazol-3-yl |
| 1716 | 5-(3-CN-C₆H₄)-1,2,4-thiadiazol-3-yl |
| 1717 | 5-(3-CF₃-C₆H₄)-1,2,4-thiadiazol-3-yl |
| 1718 | 5-(4-CF₃-C₆H₄)-1,2,4-thiadiazol-3-yl |
| 1719 | 5-(4-C(CH₃)₃-C₆H₄)-1,2,4-thiadiazol-3-yl |
| 1720 | 5-(2-Cl-C₆H₄)-1,2,4-thiadiazol-3-yl |
| 1721 | 5-(3-Cl-C₆H₄)-1,2,4-thiadiazol-3-yl |
| 1722 | 5-(4-Cl-C₆H₄)-1,2,4-thiadiazol-3-yl |
| 1723 | 5-(2-Br-C₆H₄)-1,2,4-thiadiazol-3-yl |
| 1724 | 5-(3-Br-C₆H₄)-1,2,4-thiadiazol-3-yl |
| 1725 | 5-(4-Br-C₆H₄)-1,2,4-thiadiazol-3-yl |
| 1726 | 5-(2-F-C₆H₄)-1,2,4-thiadiazol-3-yl |
| 1727 | 5-(3-F-C₆H₄)-1,2,4-thiadiazol-3-yl |
| 1728 | 5-(4-F-C₆H₄)-1,2,4-thiadiazol-3-yl |
| 1729 | 5-(2,4-Cl₂-C₆H₃)-1,2,4-thiadiazol-3-yl |
| 1730 | 5-(2,5-Cl₂-C₆H₃)-1,2,4-thiadiazol-3-yl |
| 1731 | 5-(2,6-Cl₂-C₆H₃)-1,2,4-thiadiazol-3-yl |
| 1732 | 5-(3,4-Cl₂-C₆H₃)-1,2,4-thiadiazol-3-yl |
| 1733 | 5-(2,4-F₂-C₆H₃)-1,2,4-thiadiazol-3-yl |
| 1734 | 5-(2,5-F₂-C₆H₃)-1,2,4-thiadiazol-3-yl |
| 1735 | 5-(2,6-F₂-C₆H₃)-1,2,4-thiadiazol-3-yl |
| 1736 | 5-(3,4-F₂-C₆H₃)-1,2,4-thiadiazol-3-yl |
| 1737 | 5-(2-Cl, 5-OCH₃-C₆H₃)-1,2,4-thiadiazol-3-yl |
| 1738 | 5-(2-Cl, 5-CH₃-C₆H₃)-1,2,4-thiadiazol-3-yl |
| 1739 | 5-(5-Cl, 2-OCH₃-C₆H₃)-1,2,4-thiadiazol-3-yl |
| 1740 | 5-(5-Cl, 2-CH₃-C₆H₃)-1,2,4-thiadiazol-3-yl |
| 1741 | 5-[2,5-(CH₃)₂-C₆H₃]-1,2,4-thiadiazol-3-yl |
| 1742 | 5-CH₃-1,3,4-thiadiazol-2-yl |
| 1743 | 5-CH(CH₃)₂-1,3,4-thiadiazol-2-yl |
| 1744 | 5-C(CH₃)₃-1,3,4-thiadiazol-2-yl |
| 1745 | 5-cyclopropyl-1,3,4-thiadiazol-2-yl |
| 1746 | 5-C₆H₅-1,3,4-thiadiazol-2-yl |
| 1747 | 5-(2-CH₃-C₆H₄)-1,3,4-thiadiazol-2-yl |
| 1748 | 5-(3-CH₃-C₆H₄)-1,3,4-thiadiazol-2-yl |
| 1749 | 5-(4-CH₃-C₆H₄)-1,3,4-thiadiazol-2-yl |
| 1750 | 5-(3-OCH₃-C₆H₄)-1,3,4-thiadiazol-2-yl |
| 1751 | 5-(4-OCH₃-C₆H₄)-1,3,4-thiadiazol-2-yl |
| 1752 | 5-(4-NO₂-C₆H₄)-1,3,4-thiadiazol-2-yl |
| 1753 | 5-(3-NO₂-C₆H₄)-1,3,4-thiadiazol-2-yl |
| 1754 | 5-(4-CN-C₆H₄)-1,3,4-thiadiazol-2-yl |
| 1755 | 5-(3-CN-C₆H₄)-1,3,4-thiadiazol-2-yl |
| 1756 | 5-(3-CF₃-C₆H₄)-1,3,4-thiadiazol-2-yl |
| 1757 | 5-(4-CF₃-C₆H₄)-1,3,4-thiadiazol-2-yl |
| 1758 | 5-(4-C(CH₃)₃-C₆H₄)-1,3,4-thiadiazol-2-yl |
| 1759 | 5-(2-Cl-C₆H₄)-1,3,4-thiadiazol-2-yl |
| 1760 | 5-(3-Cl-C₆H₄)-1,3,4-thiadiazol-2-yl |
| 1761 | 5-(4-Cl-C₆H₄)-1,3,4-thiadiazol-2-yl |
| 1762 | 5-(2-Br-C₆H₄)-1,3,4-thiadiazol-2-yl |
| 1763 | 5-(3-Br-C₆H₄)-1,3,4-thiadiazol-2-yl |
| 1764 | 5-(4-Br-C₆H₄)-1,3,4-thiadiazol-2-yl |
| 1765 | 5-(2-F-C₆H₄)-1,3,4-thiadiazol-2-yl |
| 1766 | 5-(3-F-C₆H₄)-1,3,4-thiadiazol-2-yl |
| 1767 | 5-(4-F-C₆H₄)-1,3,4-thiadiazol-2-yl |
| 1768 | 5-(2,4-Cl₂-C₆H₃)-1,3,4-thiadiazol-2-yl |
| 1769 | 5-(2,5-Cl₂-C₆H₃)-1,3,4-thiadiazol-2-yl |
| 1770 | 5- (2,6-Cl₂-C₆H₃)-1,3,4-thiadiazol-2-yl |
| 1771 | 5-(3,4-Cl₂-C₆H₃)-1,3,4-thiadiazol-2-yl |
| 1772 | 5-(2,4-F₂-C₆H₃)-1,3,4-thiadiazol-2-yl |
| 1773 | 5-(2,5-F₂-C₆H₃)-1,3,4-thiadiazol-2-yl |
| 1774 | 5-(2,6-F₂-C₆H₃)-1,3,4-thiadiazol-2-yl |
| 1775 | 5-(3,4-F₂-C₆H₃)-1,3,4-thiadiazol-2-yl |
| 1776 | 5-(2-Cl, 5-OCH₃-C₆H₃)-1,3,4-thiadiazol-2-yl |
| 1777 | 5-(2-Cl, 5-CH₃-C₆H₃)-1,3,4-thiadiazol-2-yl |
| 1778 | 5-(5-Cl, 2-OCH₃-C₆H₃)-1,3,4-thiadiazol-2-yl |
| 1779 | 5-(5-Cl, 2-CH₃-C₆H₃)-1,3,4-thiadiazol-2-yl |
| 1780 | 5-[2,5-(CH₃)₂-C₆H₃]-1,3,4-thiadiazol-2-yl |
| 1781 | 1-CH(CH₃)₂-imidazol-4-yl |
| 1782 | 1-C(CH₃)₃-imidazol-4-yl |
| 1783 | 1-cyclopropyl-imidazol-4-yl |
| 1784 | 1-C₆H₅-imidazol-4-yl |
| 1785 | 1-(2-CH₃-C₆H₄)-imidazol-4-yl |
| 1786 | 1-(3-CH₃-C₆H₄)-imidazol-4-yl |
| 1787 | 1-(4-CH₃-C₆H₄)-imidazol-4-yl |
| 1788 | 1-(3-OCH₃-C₆H₄)-imidazol-4-y1 |
| 1789 | 1-(4-OCH₃-C₆H₄)-imidazol-4-yl |
| 1790 | 1-(4-NO₂-C₆H₄)-imidazol-4-yl |
| 1791 | 1-(3-NO₂-C₆H₄)-imidazol-4-yl |
| 1792 | 1-(4-CN-C₆H₄)-imidazol-4-yl |
| 1793 | 1-(3-CN-C₆H₄)-imidazol-4-yl |
| 1794 | 1-(3-CF₃-C₆H₄)-imidazol-4-yl |
| 1795 | 1-(4-CF₃-C₆H₄)-imidazol-4-yl |
| 1796 | 1-(4-C(CH₃)₃-C₆H₄)-imidazol-4-yl |
| 1797 | 1-(2-Cl-C₆H₄)-imidazol-4-yl |
| 1798 | 1-(3-Cl-C₆H₄)-imidazol-4-yl |
| 1799 | 1-(4-Cl-C₆H₄)-imidazol-4-yl |
| 1800 | 1-(2-Br-C₆H₄)-imidazol-4-yl |
| 1801 | 1-(3-Br-C₆H₄)-imidazol-4-y1 |
| 1802 | 1-(4-Br-C₆H₄)-imidazol-4-yl |
| 1803 | 1-(2-F-C₆H₄)-imidazol-4-yl |
| 1804 | 1-(3-F-C₆H₄)-imidazol-4-yl |
| 1805 | 1-(4-F-C₆H₄)-imidazol-4-yl |
| 1806 | 1-(2,4-Cl₂-C₆H₃)-imidazol-4-yl |
| 1807 | 1-(2,5-Cl₂-C₆H₃)-imidazol-4-yl |
| 1808 | 1-(2,6-Cl₂-C₆H₃)-imidazol-4-yl |
| 1809 | 1-(3,4-Cl₂-C₆H₃)-imidazol-4-yl |
| 1810 | 1-(2,4-F₂-C₆H₃)-imidazol-4-yl |
| 1811 | 1-(2,5-F₂-C₆H₃)-imidazol-4-yl |
| 1812 | 1-(2,6-F₂-C₆H₃)-imidazol-4-yl |
| 1813 | 1-(3,4-F₂-C₆H₃)-imidazol-4-yl |
| 1814 | 1-(2-Cl, 5-OCH₃-C₆H₃)-imidazol-4-yl |
| 1815 | 1-(2-Cl, 5-CH₃-C₆H₃)-imidazol-4-yl |
| 1816 | 1-(5-Cl, 2-OCH₃-C₆H₃)-imidazol-4-yl |
| 1817 | 1-(5-Cl, 2-CH₃-C₆H₃)-imidazol-4-yl |
| 1818 | 1-[2,5-(CH₃)₂-C₆H₃]-imidazol-4-yl |

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten, Deuteromyceten, Phycomyceten und Basidiomyceten, aus. Sie sind zum Teil systemisch wirksam und können als Blatt- und Bodenfungizide eingesetzt werden. Besondere Bedeutung haben sie für die Bekämpfung einer Vielzahl von Pilzen an verschiedenen Kulturpflanzen wie Weizen, Roggen, Gerste, Hafer, Bananen, Reis, Mais, Gras, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Gemüse und Zierpflanzen, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Pseudoperonospora-Arten in Hopfen und Gurken, Alternaria-Arten an Gemüse und Obst sowie Mycosphaerella-Arten in Bananen.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können mit an sich bekannten Formulierungshilfsmitteln in die üblichen Formulierungen (Mittel) überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der Verbindungen I gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylen-diamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis- (4-Fluorphenyl)-methylsilyl)-methyl)-1H-1,2,4-triazol.

Strobilurine wie Methyl-E-methoximino-[α-(o-tolyloxy)-o-tolyl]-acetat, Methyl-E-2-{2-[6-(2-cyanophenoxy)-pyrimidin-4-yloxy]-phenyl}-3-methoxyacrylat, Methyl-E-methoximino-[α-(2-phenoxyphenyl)]-acetamid, Methyl-E-methoximino-[α-(2,5-dimethylphenoxy)-o-tolyl]-acetamid.

Anilinopyrimidine wie N-(4,6-Dimethylpyrimidin-2-yl)-anilin, N-[4-Methyl-6-(1-propinyl)-pyrimidin-2-yl] -anilin, N-(4-Methyl-6-cyclopropyl-pyrimidin-2-yl)-anilin.

Phenylpyrrole wie 4-(2,2-difluor-1,3-benzodioxol-4-yl)-pyrrol-3-carbonitril, Zimtsäureamide wie 3-(4-Chlorphenyl)-3-(3,4-dimethoxyphenyl)-acrylsäuremorpholid,
(2RS,3SR)-1-[3-(2-Chlorphenyl)-2-[4-fluorphenyl]oxiran-2-yl-methyl]-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Verbindungen I können als solche, in Form ihrer unter Verwendung an sich bekannter Formulierungshilfsmittel gewonnener Formulierungen (Mittel) oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwekken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methyl-pyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile einer erfindungsgemäßen Verbindung I werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile einer erfindungsgemäßen Verbindung I werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile einer erfindungsgemäßen Verbindung I werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile einer erfindungsgemäßen Verbindung I werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile einer erfindungsgemäßen Verbindung I werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile einer erfindungsgemäßen Verbindung I mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile einer erfindungsgemäßen Verbindung I werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII.20 Gew.-Teile einer erfindungsgemäßen Verbindung I werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die chemischen Verschiebungen (in ppm) der ¹H-NMR-Spektren wurde gemessen gegen Tetramethylsilan (br = breites Signal, s = Singulett, d = Dublett, m = Multiplett).

### Beispiel 1

### Darstellung der Verbindung S1

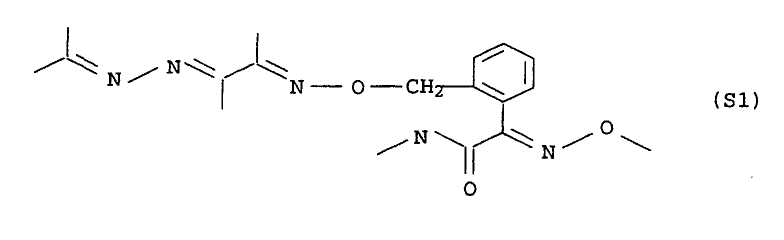

9,2 g (0,03 mol) (E,E)-2-Methoxyimino-2-(2'-[{1"-methyl,1"-acetyl}iminooxymethyl]phenyl)essigsäuremethylester (vgl. WO-A 95/21153) wurden in 200 ml Tetrahydrofuran gelöst, mit 50 ml 40 gew.-%iger wäßriger Monomethylamin-Lösung versetzt und 2 Stunden bei Raumtemperatur stehengelassen. Anschließend wurde mit 2 N Salzsäure versetzt, mit Methyl-tert.-butylether extrahiert, die organische Phase mit Wasser gewaschen, über Natriumsulfat getrocknet und das Lösungsmittel entfernt. Als Rückstand verblieben 7,7 g (E,E)-2-Methoxyimino-2-(2"-[{1"-methyl,1"-acetyl}iminooxymethyl]phenyl)essigsäuremonomethylamid als farblose Kristalle vom Schmelzpunkt 89-92°C. Eine Lösung von 3,7 g (0,012 mol) dieser Verbindung in 50 ml Tetrahydrofuran gab man zu einer Lösung von 2,4 g (0,048 mol) Hydrazinhydrat in 50 ml Tetrahydrofuran und ließ 60 Stunden bei Raumtemperatur rühren. Anschließend wurde mit Wasser versetzt, mit Methyl-tert.-butylether extrahiert, die organische Phase mit Wasser gewaschen, über Natriumsulfat getrocknet und das Lösungsmittel entfernt. Als Rückstand verblieben 3,5 g (E,E,E)-2-Methoxyimino-2-(2'-[{1''-methyl,1"-{1'''-hydrazonoethyl}}iminooxymethyl]phenyl)essigsäuremonomethylamid als farbloses Öl.
¹H-NMR (CDCl₃): 1,89 (s, 3H); 1,99 (s, 3H); 2,9 (d, 3H); 3,94 (s, 3H); 5,05 (s, 2H); 5,44 (s, br, 2H); 6,71 (s, br, 1H); 7,15-7,47 (m, 4H)

1,6 g (0,005 mol) dieser Verbindung wurden in 100 ml Aceton gelöst. Nach Zugabe von 1 Tropfen Essigsäure wurde der Reaktionsansatz 60 Stunden bei Raumtemperatur stehengelassen. Anschließend wurde das Aceton abgezogen, der Rückstand in Methyl-tert.-butylether und Wasser aufgenommen, mit Methyl-tert.-butylether extrahiert, die organische Phase mit Wasser gewaschen und über Natriumsulfat getrocknet. Danach Wurde das Lösungsmittel aus der organischen Phase entfernt. Als Rückstand verblieben 1,7 g der Verbindung "S1" als farbloses Öl.
¹H-NMR (CDCl₃): 1,8 (s, 3H); 1,94 (s, 3H); 2,05 (s, 6H); 2,9 (d, 3H); 3,96 (s, 3H); 5,1 (s, 2H); 6,75 (s, br, 1H); 7,17-7,5 (m, 4H)

### Beispiel 2: (nicht erfindungsgemäß)

### Darstellung der Verbindung S2

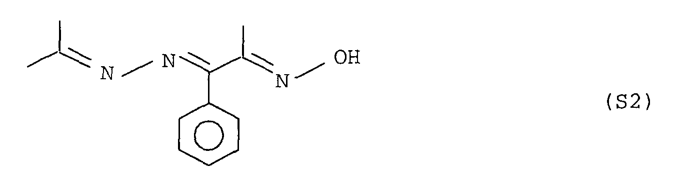

Eine Lösung von 20 g (0,11 mol) 1-Hydrazono-1-phenyl-propan-2-onoxim [J. Med. Chem. 21, 623-8, (1978)] in 300 ml Aceton wurde 1 Stunde bei Raumtemperatur gerührt. Nach Abrotieren des Lösungsmittels wurde der Rückstand aus Methanol kristallisiert. Man erhielt so 9,5 g (40 % Ausbeute) der Verbindung S2 als farblose Kristalle vom Schmelzpunkt 127-130°C.
¹H-NMR (CDCl₃): δ =1,87 (s,6H); 2,18 (s,3H); 6,98-7,36 (m,5H); 8,56 (s,br,1H)

### Beispiel 3:

### Darstellung der Verbindung S3

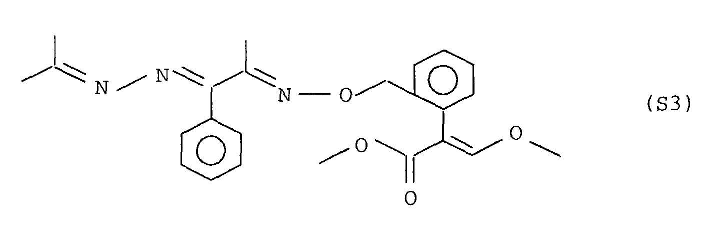

Zu 0,29 g (12 mmol) Natriumhydrid in 50 ml Dimethylformamid gab man 2,5 g (11,5 mmol) 1-(Isopropylidenhydrazono)-1-phenyl-propan-2-on-oxim (Verbindung S2), gelöst in 50 ml Dimethylformamid. Man ließ 30 min nachrühren, gab anschließend 3,3 g (11,5 mmol) (E)-3-Methoxy-2-[2'-brommethyl)phenyl] acrylsäuremethylester zu und ließ 16 Stunden bei Raumtemperatur rühren. Das Reaktionsgemisch wurde auf Wasser gegossen und mit tert.-Butylmethylether extrahiert. Die organische Phase wurde mit Wasser gewaschen, getrocknet und eingeengt. Nach Säulenchromatographie an Kieselgel (tert.-Butylmethylether/Cyclohexan/2 % Triethylamin) erhielt man 1,7 g (35 % Ausbeute) der Verbindung S3 als farbloses Öl.
IR (Film): 2940, 1709, 1634, 1435, 1284, 1256, 1191, 1130, 1111, 1056, 1009 cm⁻¹

### Beispiel 4:

### Darstellung der Verbindung S4

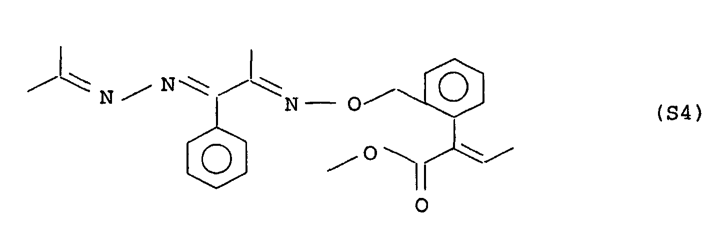

Zu 0,29 g (12 mmol) Natriumhydrid in 50 ml Dimethylformamid gab man 2,5 g (11,5 mmol) 1-(Isopropylidenhydrazono)-1-phenyl-propan-2-on-oxim (Verbindung S2), gelöst in 50 ml Dimethylformamid. Man ließ 30 min nachrühren, gab anschließend 3,1 g (11,5 mmol) (E)-3-Methyl-2-[2'-brommethyl)phenyl]acrylsäuremethylester zu und ließ 16 Stunden bei Raumtemperatur rühren. Das Reaktionsgemisch wurde auf Wasser gegossen und mit tert.-Butylmethylether extrahiert. Die organische Phase wurde mit Wasser gewaschen, getrocknet und eingeengt. Nach Säulenchromatographie an Kieselgel (tert.-Butylmethylether/Cyclohexan( 2 % Triethylamin) erhielt man 2,3 g (49 % Ausbeute) der Verbindung S4 als farbloses Öl.
¹H-NMR (CDCl₃): δ =1,49 (d,3H); 1,89 (s,6H); 2,21 (s,3H); 3,64 (s,3H); 4,87-4,93 (m,2H); 6,98-7,36 (m,9H)

### Beispiel 5:

### Darstellung der Verbindung S5

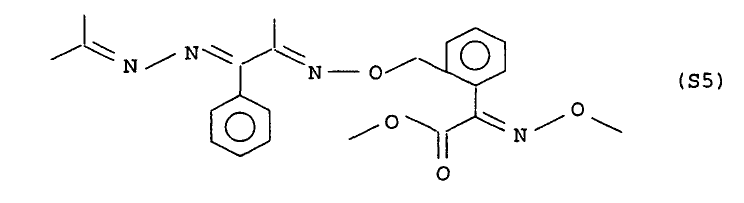

Zu 0,5 g (21 mmol) Natriumhydrid in 50 ml Dimethylformamid gab man 4,3 g (20 mmol) 1-(Isopropylidenhydrazono)-1-phenyl-propan-2-on-oxim (Verbindung S2), gelöst in 50 ml Dimethylformamid. Man ließ 30 min nachrühren, gab anschließend 5,7 g (20 mmol) (E)-2-Methoxyimino-2-[2'-brommethyl)phenyl]essigsäuremethylester zu und ließ 16 Stunden bei Raumtemperatur rühren. Das Reaktionsgemisch wurde auf Wasser gegossen und mit tert.-Butylmethylether extrahiert. Die organische Phase wurde mit Wasser gewaschen, getrocknet und eingeengt. Nach Säulenchromatographie an Kieselgel (tert.-Butylmethylether/Cyclohexan/2 % Triethylamin) erhielt man 4,2 g (50 % Ausbeute) der Verbindung S3 als farbloses Öl.
¹H-NMR (CDCl₃): δ = 1,89 (s,6H); 2,17 (s,3H); 3,82 (s,3H); 3,97 (s,3H); 4,93 (s,2H); 7,00-7,40 (m,9H)

### Beispiel 6:

### Darstellung der Verbindung S6

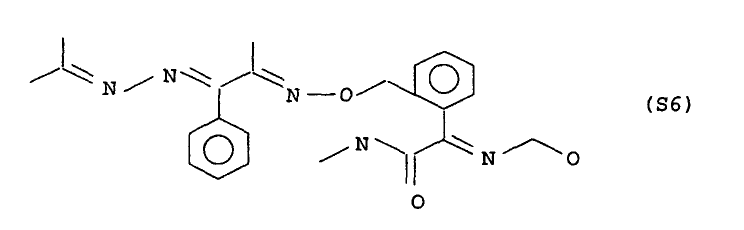

3,0 g (7,1 mmol) der Verbindung S5 werden in 100 ml Tetrahydrofuran gelöst, mit 20 ml 40%-iger wäßriger Monomethylaminlösung versetzt und 1 Stunde bei Raumtemperatur gerührt. Anschließend wurde mit Wasser versetzt, mit tert.-Butylmethylether extrahiert und die organische Phase mit Wasser gewaschen, über natriumsulfat getrocknet und einrotiert. Man erhielt so 3,0 g der Verbindung S6 als farbloses Öl.
¹H-NMR (CDCl₃) : δ = 1,89 (s,6H); 2,18 (s,3H); 2,85 (d,3H); 3,88 (s,3H); 4,94 (s,2H); 6,64 (s,br,1H); 6,98-7,38 (m,9H)

In analoger Weise wie vorstehend für die Verbindungen S1 bis S6 beschrieben, lassen sich die Verbindungen der Formel I ganz allgemein herstellen.

Die nachstehende Tabelle I zeigt die physikalischen Daten weiteter hergestellter erfindungsgemäßer Verbindungen. In die Tabelle wurden auch die Verbindungen S1, S3, S4, S5 und S6 mit aufgenommen.

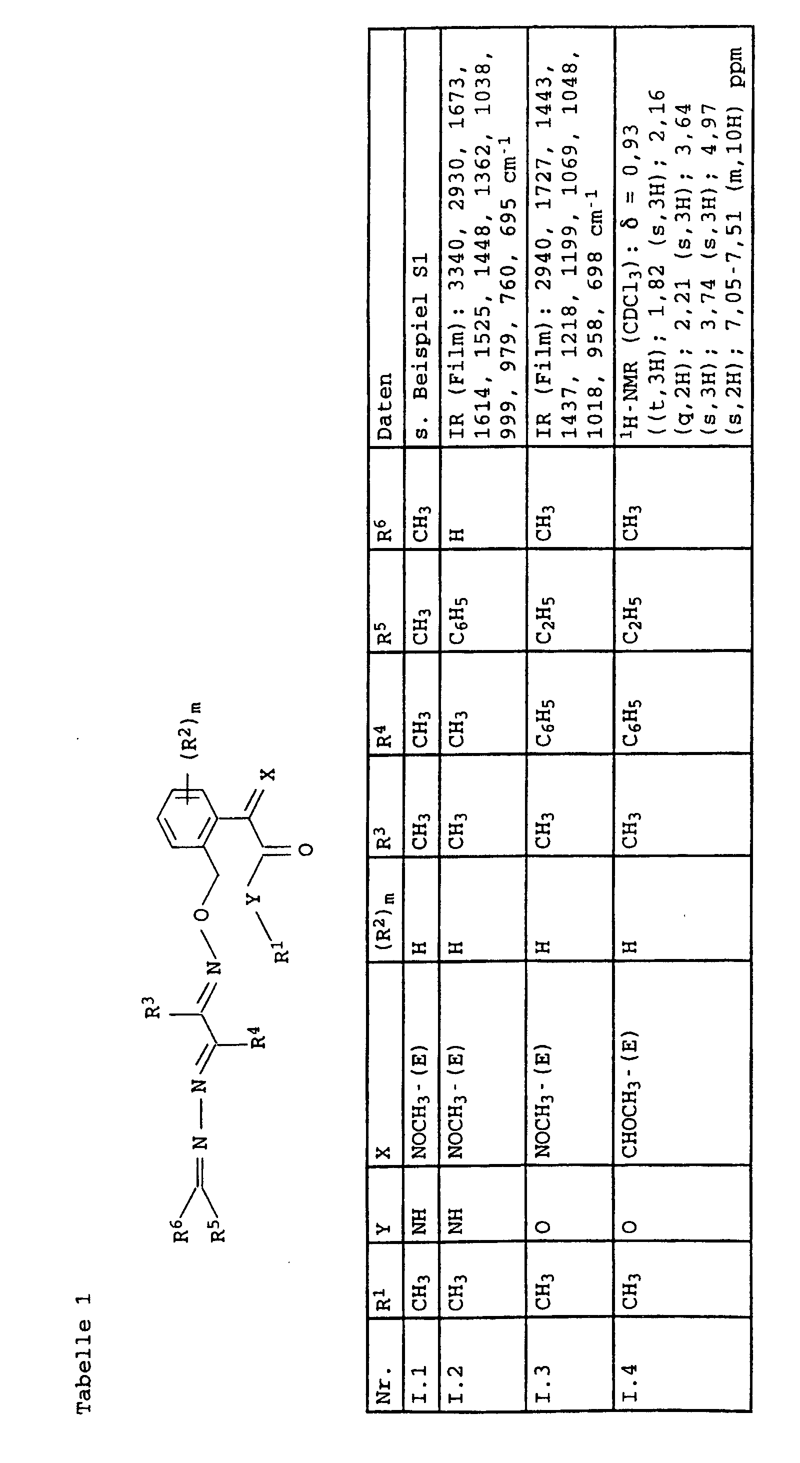

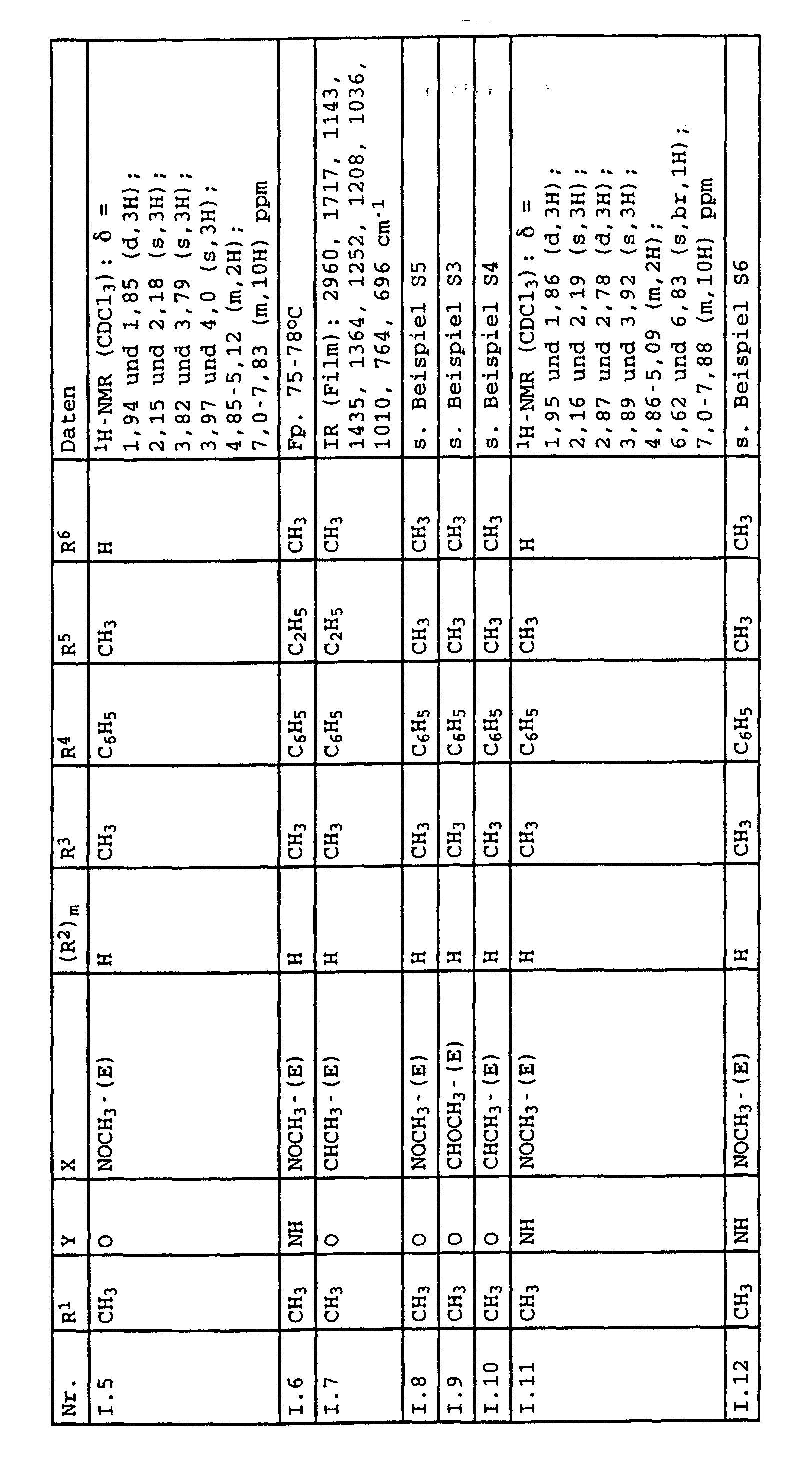

### Anwendungsbeispiele

### 1. Beispiele zur Wirkung gegen Schadpilze: Weizenmehltau

Blätter von in Töpfen gewachsenen Weizenkeimlingen der Sorte "Frühgold" wurden mit wäßriger Spritzbrühe, die mit einer Stammlösung aus 10 % Wirkstoff, 63 % Cyclohexanon und 27 % Emulgiermittel angesetzt wurde, bis zur Tropfnässe besprüht und 24 Stunden nach dem Antrocknen des Spritzbelages mit Sporen des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung visuell in % Befall der gesamten Blattfläche ermittelt.

| Wirkstoff | %-Befall der Blätter nach Applikation von 63 ppm-Wirkstoff enthaltender wäßriger Wirkstoffaufbereitung |
|---|---|
| I.1 | 5 |
| I.2 | 5 |
| I.3 | 15 |
| I.5 | 15 |
| I.7 | 15 |
| I.8 | 15 |
| I.9 | 15 |
| I.10 | 15 |
| Vergleichssubstanz A Verbindung 3.81 aus Tabelle 3 der WO 95/18 789 | 60 |
| Unbehandelt | 75 |

### 2. Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil^{®} LN (Lutensol^{®} AP6, Netzmittel mit Emulgier- und Dispergierwirkung auf der Basis ethoxylierter Alkylphenole) und 10 Gew.-% Emulphor^{®} EL (Emulan^{®} EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. Azinooximether der Formel I 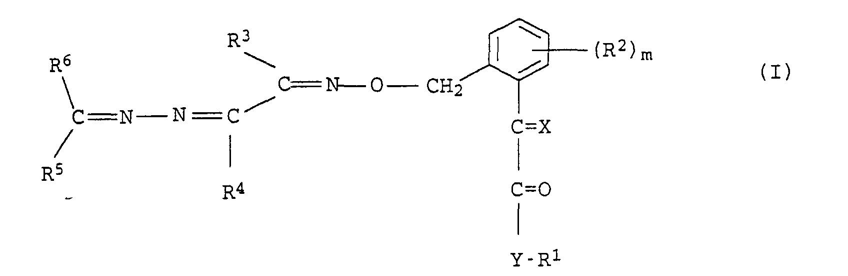 in der die Variablen die folgenden Bedeutungen haben:
X NOCH₃, CHOCH₃ oder CHCH₃;
Y O oder NZ, wobei Z für Wasserstoff oder C₁-C₄-Alkyl steht;
R¹ Wasserstoff oder C₁-C₄-Alkyl;
R² Cyano, Nitro, Halogen, C₁-C₄-Alkyl, Trifluormethyl oder C₁-C₄-Alkoxy;
m 0, 1 oder 2, wobei die Reste R² verschieden sein können, wenn m für 2 steht;
R³ Wasserstoff, Cyano, Alkyl, Halogenalkyl, Alkoxy oder Cycloalkyl;
R⁴, R⁵ R⁶ unabhängig voneinander: Wasserstoff oder gegebenenfalls substituiertes: Alkyl, Alkenyl, Alkinyl, Cycloalkyl, Cycloalkenyl, Heterocyclyl, Aryl oder Heteroaryl,
sowie deren Salze.

2. Verfahren zur Herstellung von Verbindungen der Formel I gemäß Anspruch 1, dadurch gekennzeichnet, daß man ein Benzylderivat der Formel II 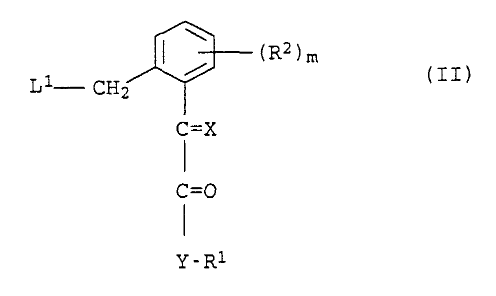 in der L¹ für eine nucleophil austauschbare Abgangsgruppe steht, mit einem Hydroxyimin der Formel III 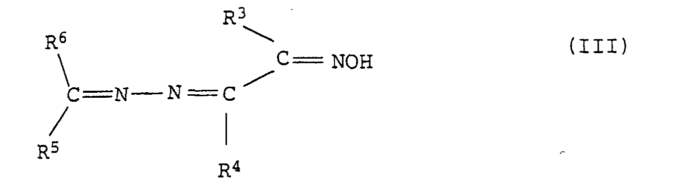 umsetzt.

3. Verfahren zur Herstellung von Verbindungen der Formel I gemäß Anspruch 1, dadurch gekennzeichnet, daß man ein Benzylderivat der Formel II gemäß Anspruch 2 mit einem Hydroxyimin der Formel V 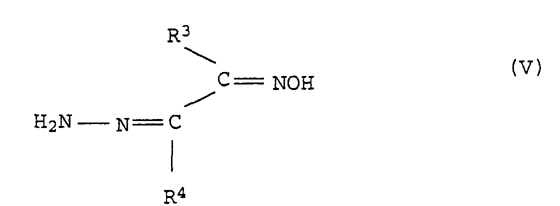 zu einer Verbindung der Formel IX 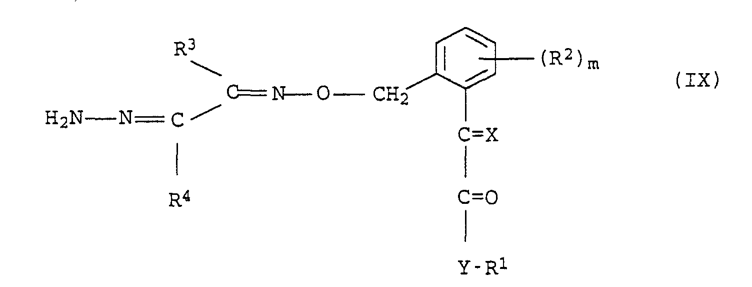 und IX anschließend mit einer Carbonyl-Verbindung der Formel VI 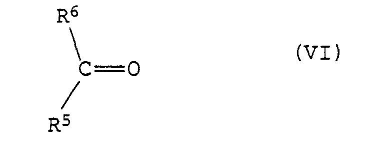 umsetzt.

4. Verfahren zur Herstellung von Verbindungen der Formel I gemäß Anspruch 1, dadurch gekennzeichnet, daß man eine Carbonylverbindung der Formel VIII 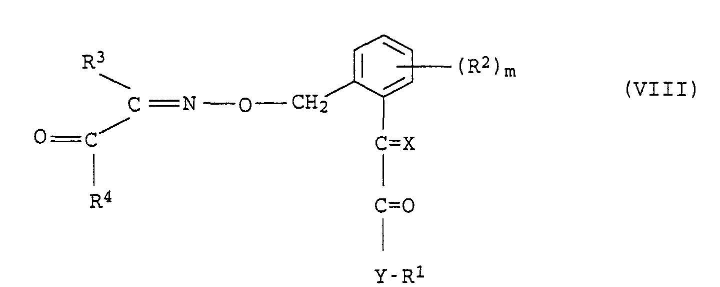 mit Hydrazinhydrat zu einer Verbindung der Formel IX 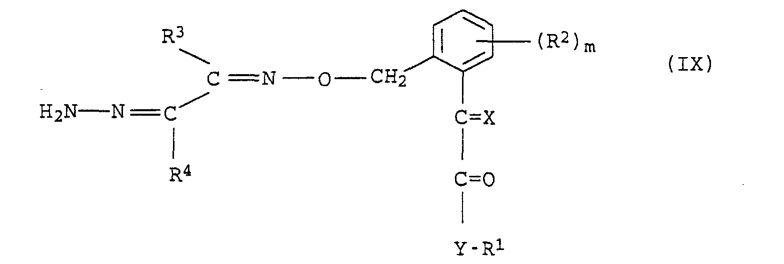 und IX anschließend mit einer Carbonylverbindung der Formel VI 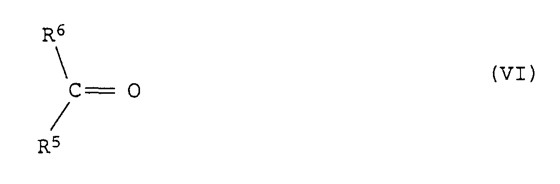 umsetzt.

5. Verfahren zur Herstellung von Verbindungen der Formel I gemäß Anspruch 1, dadurch gekennzeichnet, daß man eine Carbonylverbindung der Formel VIII 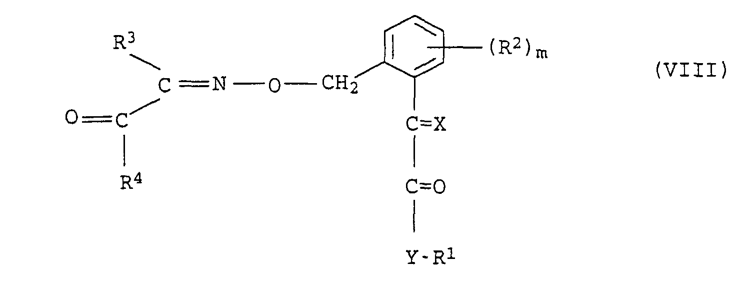 mit einem Hydrazon der Formel VII 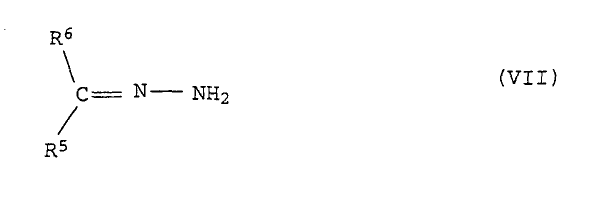 umsetzt.

6. Zur Bekämpfung von tierischen Schädlingen oder von Schadpilzen geeignete Mittel, enthaltend eine Verbindung der Formel I oder eines ihrer Salze gemäß Anspruch 1 und mindestens ein Formulierungshilfsmittel.

7. Mittel nach Anspruch 6 zur Bekämpfung tierischer Schädlinge aus der Klasse der Insekten, Spinntiere oder Nematoden.

8. Verfahren zur Bekämpfung von tierischen Schädlingen oder Schadpilzen, dadurch gekennzeichnet, daß man die Schädlinge oder Schadpilze, deren Lebensraum oder die von ihnen freizuhaltenden Pflanzen, Flächen, Materialien oder Räume mit einer wirksamen Menge einer Verbindung der Formel I oder eines ihrer Salze gemäß Anspruch 1 behandelt.

9. Verwendung der Verbindungen I gemäß Anspruch 1 zur Herstellung von Mitteln gegen tierische Schädlinge oder gegen Schadpilze.

10. Verwendung der Verbindungen I gemäß Anspruch 1 zur Bekämpfung von tierischen Schädlingen oder von Schadpilzen.

11. Verbindungen der Formel IX 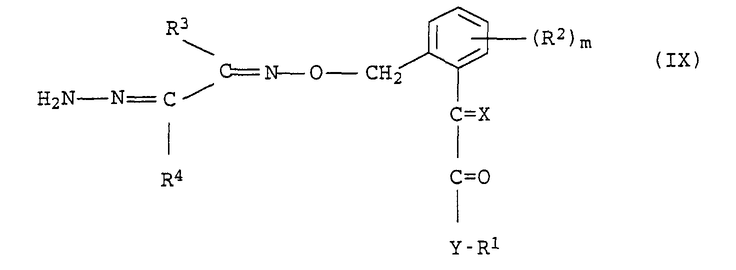 wobei die Variablen die in Anspruch 1 angegebenen Bedeutungen haben.

12. Verbindungen der Formel XI 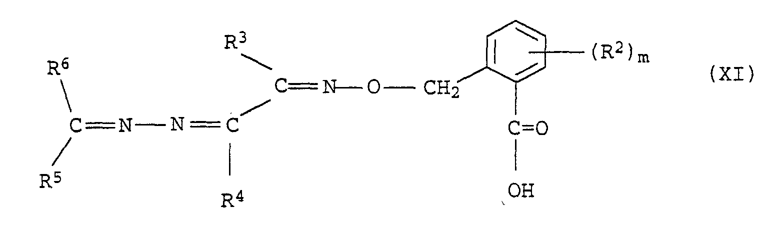 wobei die Variablen die in Anspruch 1 angegebenen Bedeutungen haben.

13. Verbindungen der Formel XII 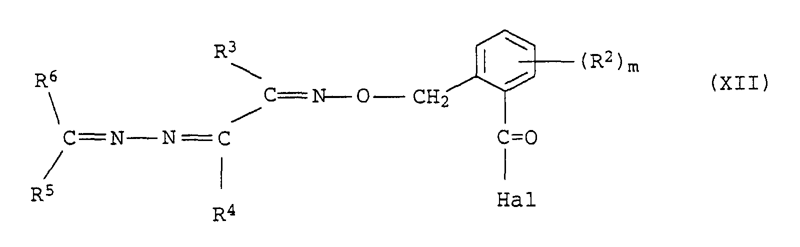 Hal = Halogen, wobei die übrigen Variablen die in Anspruch 1 angegebenen Bedeutungen haben.

14. Verbindungen der Formel XIII 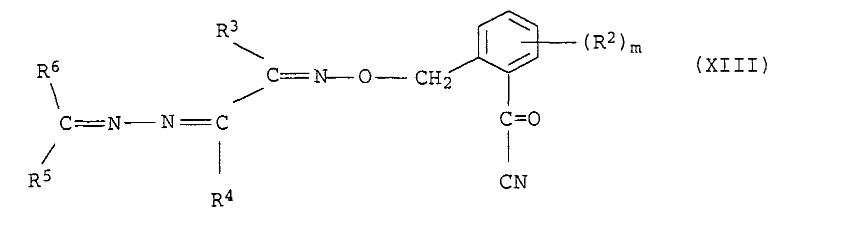 wobei die Variablen die in Anspruch 1 angegebenen Bedeutungen haben.

15. Verbindungen der Formel XIV 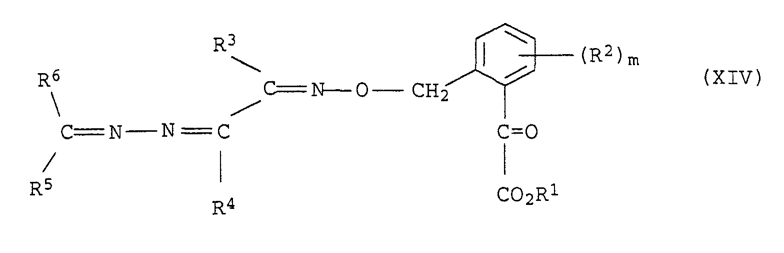 wobei die Variablen die in Anspruch 1 angegebenen Bedeutungen haben.

16. Verbindungen der Formel XV 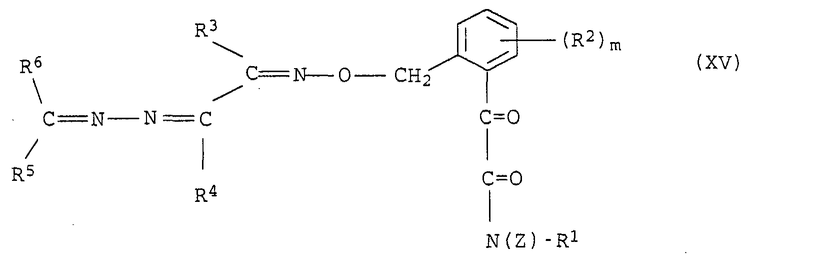 wobei die Variablen die in Anspruch 1 angegebenen Bedeutungen haben und Z für Wasserstoff oder C₁-C₄-Alkyl steht.

17. Verbindungen der Formel XVI 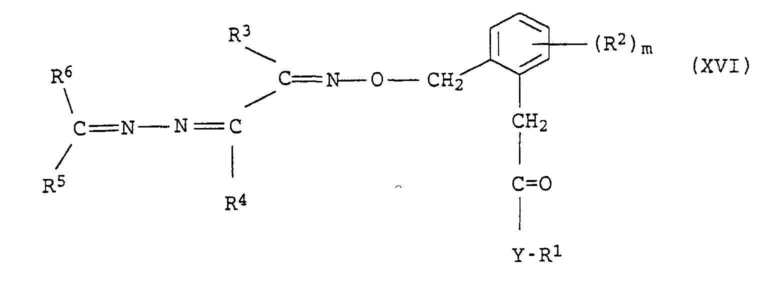 wobei die Variablen die in Anspruch 1 angegebenen Bedeutungen haben.

## Claims

1. An azinooxime ether of the formula I 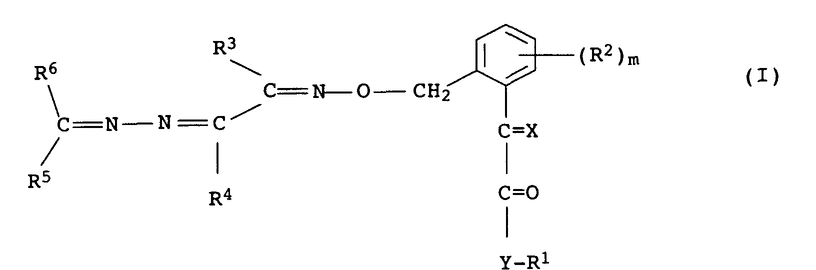 where the variables have the following meanings:
X is NOCH₃, CHOCH₃ or CHCH₃;
Y is O or NZ, Z being hydrogen or C₁-C₄-alkyl;
R¹ is hydrogen or C₁-C₄-alkyl;
R² is cyano, nitro, halogen, C₁-C₄-alkyl, trifluoromethyl or C₁-C₄-alkoxy;
m is 0, 1 or 2, it being possible for the radicals R² to be different if m is 2;
R³ is hydrogen, cyano, alkyl, haloalkyl, alkoxy or cycloalkyl;
R⁴, R⁵, R⁶ independently of one another are: hydrogen or unsubstituted or substituted alkyl, alkenyl, alkynyl, cycloalkyl, cycloalkenyl, heterocyclyl, aryl or heteroaryl,
or its salts.

2. A process for preparing compounds of the formula I as claimed in claim 1, which comprises reacting a benzyl derivative of the formula II 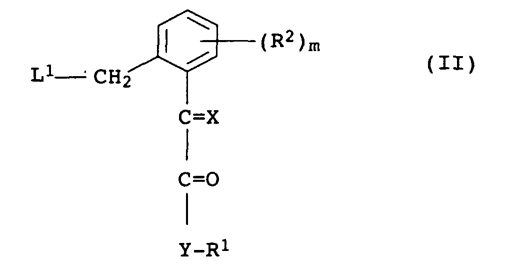 where L¹ is a nucleophilically replaceable leaving group with a hydroxyimine of the formula III

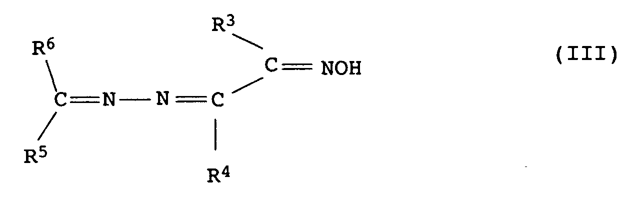

3. A process for preparing compounds of the formula I as claimed in claim 1, which comprises reacting a benzyl derivative of the formula II as set forth in claim 2 with a hydroxyimine of the formula V 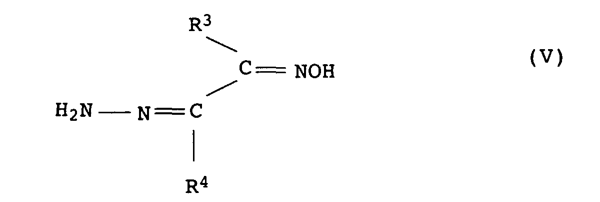 to give a compound of the formula IX 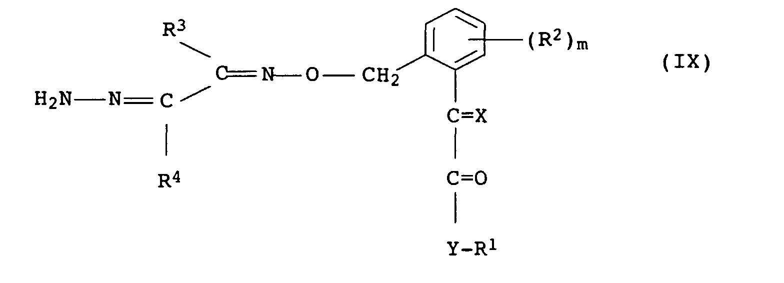 and then reacting IX with a carbonyl compound of the formula VI

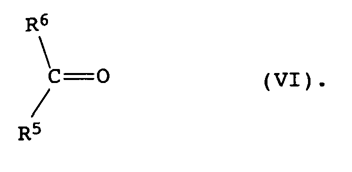

4. A process for preparing compounds of the formula I as claimed in claim 1, which comprises reacting a carbonyl D compound of the formula VIII 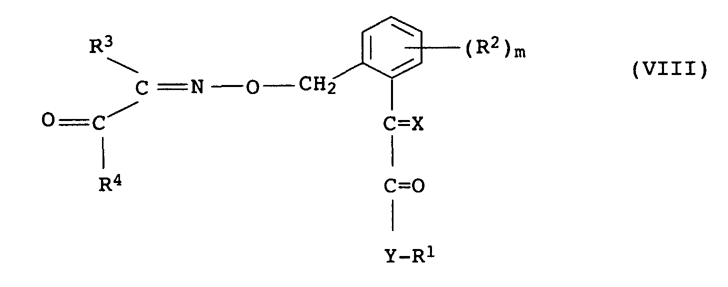 with hydrazine hydrate to give a compound of the formula IX 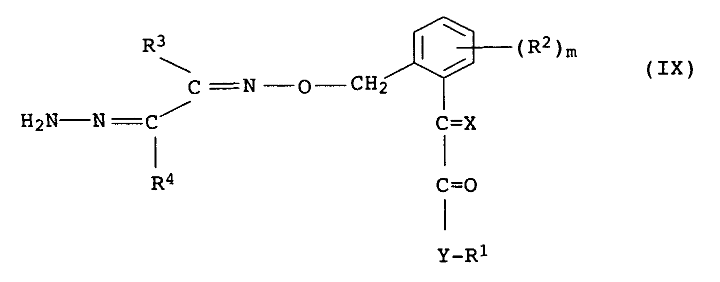 and then reacting IX with a carbonyl compound of the formula VI

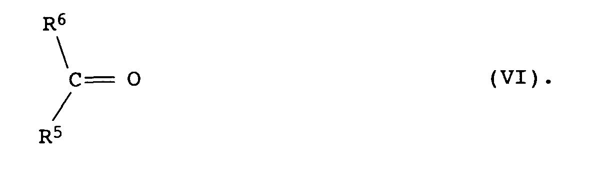

5. A process for preparing compounds of the formula I as claimed in claim 1, which comprises reacting a carbonyl compound of the formula VIII 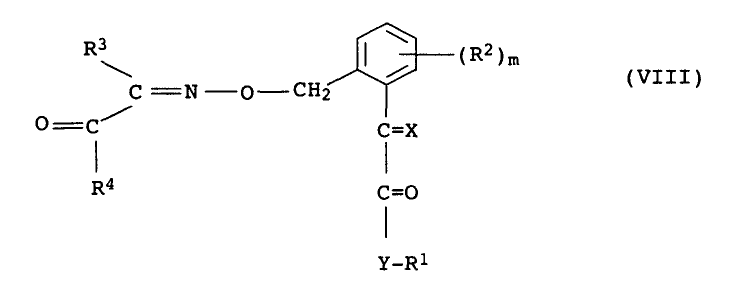 with a hydrazone of the formula VII

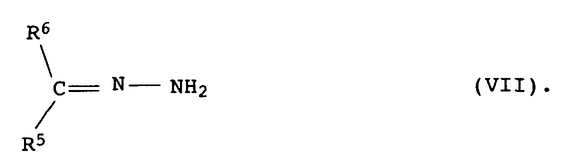

6. A composition suitable for controlling animal pests or harmful fungi, comprising a compound of the formula I or one of its salts as claimed in claim 1 and at least one formulation auxiliary.

7. A composition as claimed in claim 6 for controlling animal pests of the insects, arachnids or nematodes classes.

8. A method of controlling animal pests or harmful fungi, which comprises treating the pests or harmful fungi, their habitat or the plants, surfaces, materials or spaces to be kept free from them with an effective amount of a compound of the formula I or one of its salts as claimed in claim 1.

9. The use of the compounds I as claimed in claim 1 for preparing compositions against animal pests or against harmful fungi.

10. The use of the compounds I as claimed in claim 1 for controlling animal pests or harmful fungi.

11. A compound of the formula IX 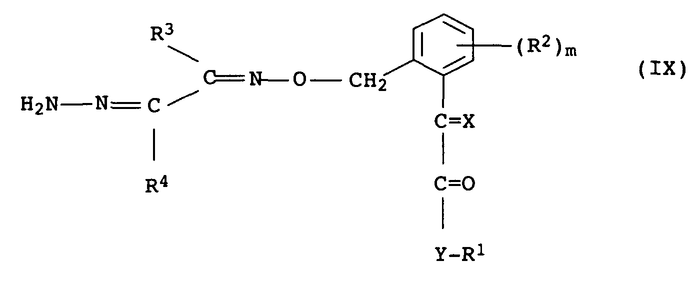 where the variables have the meanings given in claim 1.

12. A compound of the formula XI 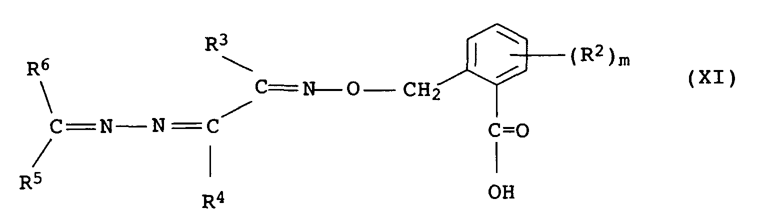 where the variables have the meanings given in claim 1.

13. A compound of the formula XII 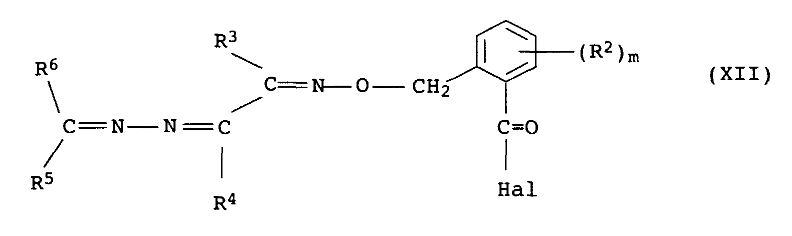 Hal = halogen, where the other variables have the meanings given in claim 1.

14. A compound of the formula XIII 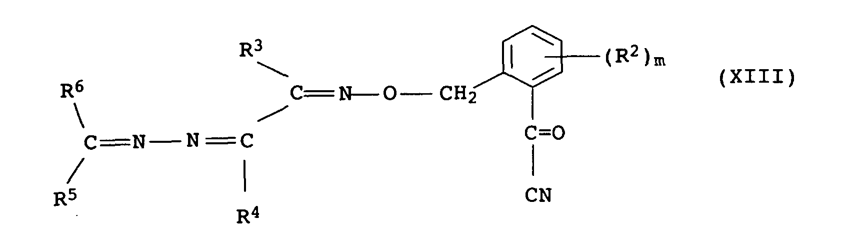 where the variables have the meanings given in claim 1.

15. A compound of the formula XIV 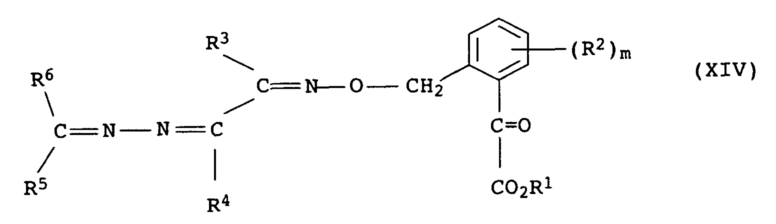 where the variables have the meanings given in claim 1.

16. A compound of the formula XV 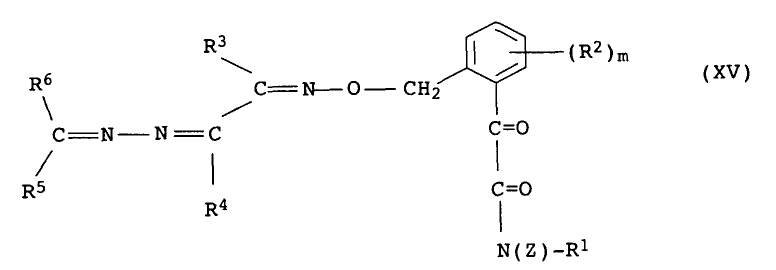 where the variables have the meanings given in claim 1 and Z is hydrogen or C₁-C₄-alkyl.

17. A compound of the formula XVI 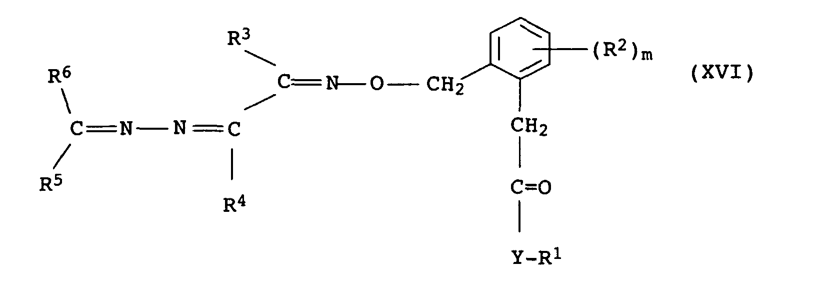 where the variables have the meanings given in claim 1.

## Revendications

1. Ethers d'azinoximes de formule I 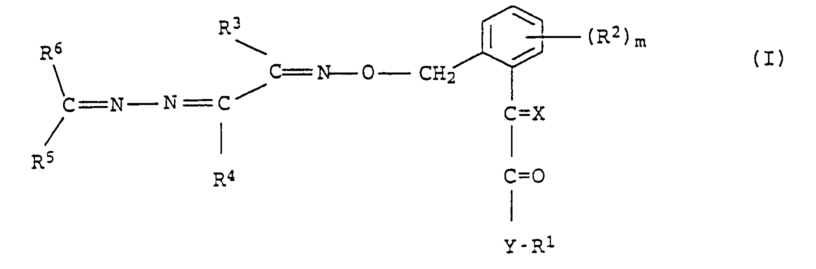 dans laquelle les symboles ont les significations suivantes :
X: NOCH₃, CHOCH₃ ou CHCH₃;
Y : O ou NZ, Z représentent l'hydrogène ou un groupe alkyle en C1-C4 ;
R¹ : l'hydrogène ou un groupe alkyle en C1-C4 ;
R² : un groupe cyano, nitro, un halogène, un groupe alkyle en C1-C4, trifluorométhyle ou alcoxy en C1-C4 ;
m : 0, 1 ou 2, les substituants R² pouvant être différents lorsque m est égal 2 ;
R³ : l'hydrogène, un groupe cyano, alkyle, halogénoalkyle, alcoxy ou cycloalkyle ;
R⁴,R⁵, R⁶, indépendamment les uns des autres : l'hydrogène ou un groupe alkyle, alcényle, alcynyle, cycloalkyle, cycloalcényle, hétarocyclyle, aryle ou hétéroaryle éventuellement substitué,
et leurs sels.

2. Procédé de préparation des composés de formule I de la revendication 1, caractérisé par le fait que l'on fait réagir un dérivé benzylique de formule II 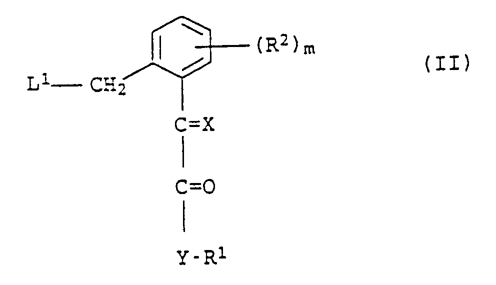 dans laquelle L¹ représente un groupe éliminable par échange nucléophile, avec une hydroxyimine de formule III

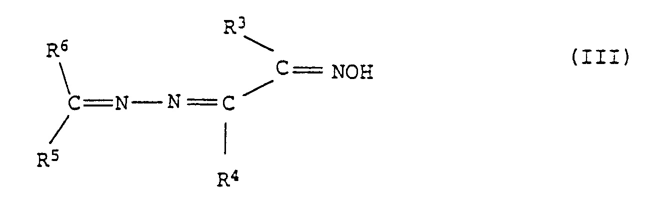

3. Procédé de préparation des composés de formule I de la revendication 1, caractérisé par le fait que l'on fait réagir un dérivé benzylique de formule II de la revendication 2 avec une hydroxyimine de formule V 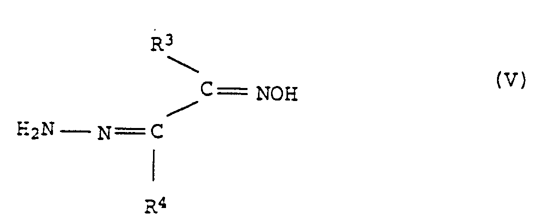 ce qui donne un composé de formule IX 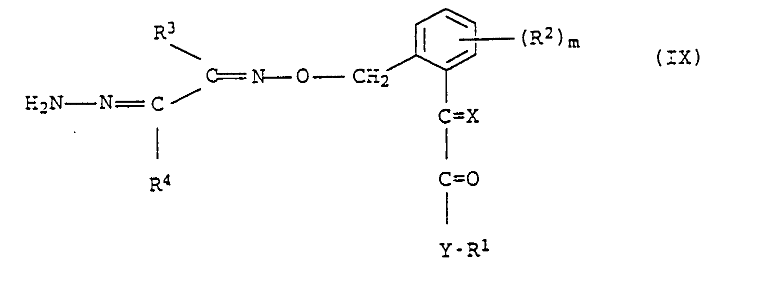 qu'on fait ensuite réagir avec un dérivé carbonylé de formule VI

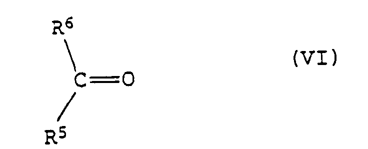

4. Procédé de préparation des composés de formule I de la revendication 1, caractérisé par le fait que l'on fait réagir un dérivé carbonylé de formule VIII 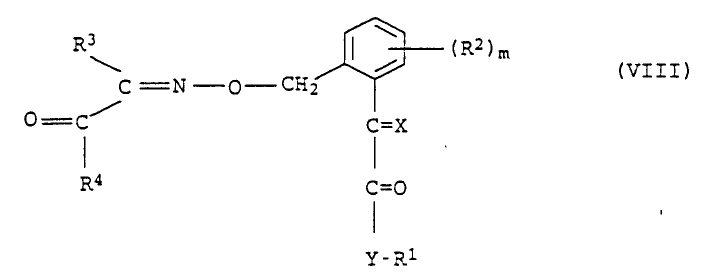 avec l'hydrate d'hydrazine, ce qui donne un composé de formule IX 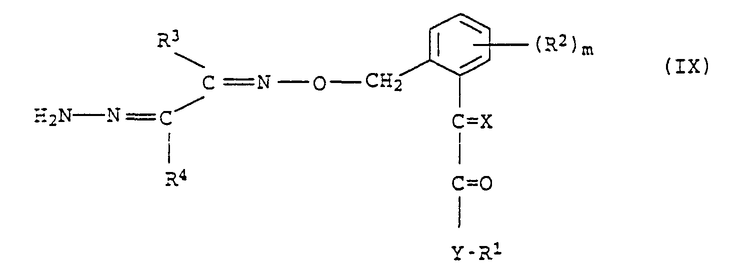 qu'on fait ensuite réagir avec un dérivé carbonylé de formule VI

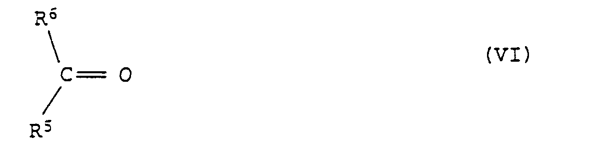

5. Procédé de préparation des composés de formule I de la revendication 1, caractérisé par le fait que l'on fait réagir un dérivé carbonylé de formule VIII 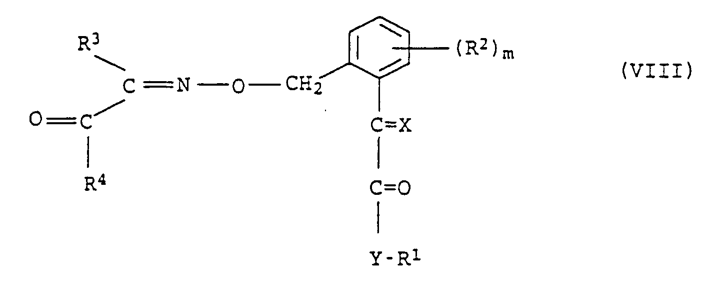 avec une hydrazone de formule VII

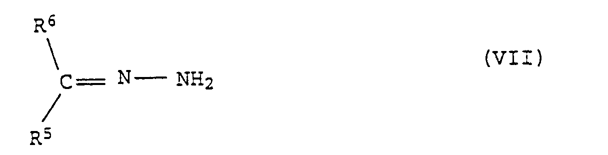

6. Produits appropriés à l'utilisation pour la lutte contre les parasites animaux ou les mycètes nuisibles, contenant un composé de formule I ou l'un de ses sels selon la revendication 1 et au moins un produit auxiliaire de formulation.

7. Produits selon la revendication 6, pour la lutte contre les parasites animaux appartenant aux classes des insectes, des arachnides ou des nématodes.

8. Procédé pour combattre les parasites animaux ou les mycètes nuisibles, caractérisé par le fait que l'on traite les parasites ou les mycètes nuisibles, leur habitat ou les végétaux, aires, matières ou locaux qu'on veut protéger par une quantité efficace d'un composé de formule I ou d'un de ses sels selon la revendication 1.

9. Utilisation des composés I de la revendication 1 pour la préparation de produits prévus pour la lutte contre les parasites animaux ou les mycètes nuisibles.

10. Utilisation des composés I selon la revendication 1 pour la lutte contre les parasites animaux ou les mycètes nuisibles.

11. Composés de formule IX 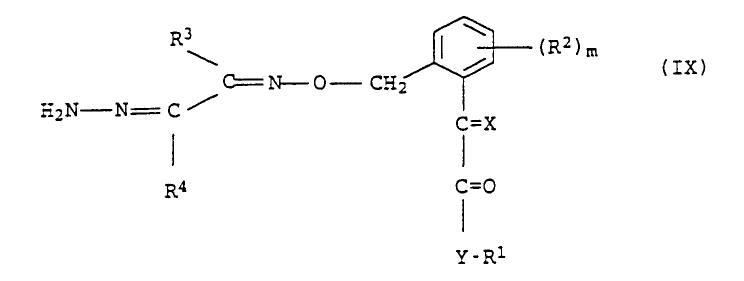 dans laquelle les symboles ont les significations indiquées dans la revendication 1.

12. Composés de formule XI 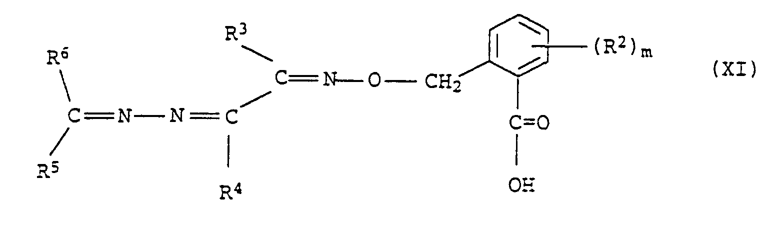 dans laquelle les symboles ont les significations indiquées dans la revendication 1.

13. Composés de formule XII 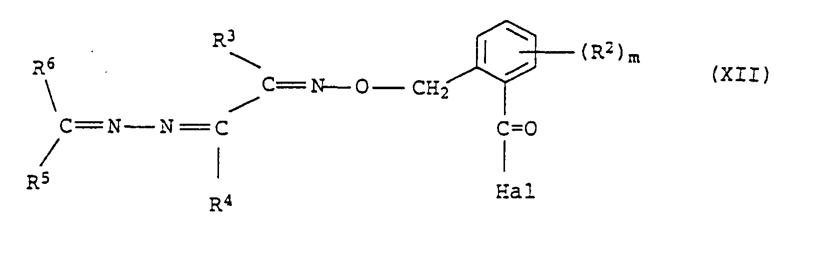 dans laquelle Hal présente un halogène, les autres symboles ayant les significations indiquées dans la revendication 1.

14. Composés de formule XIII 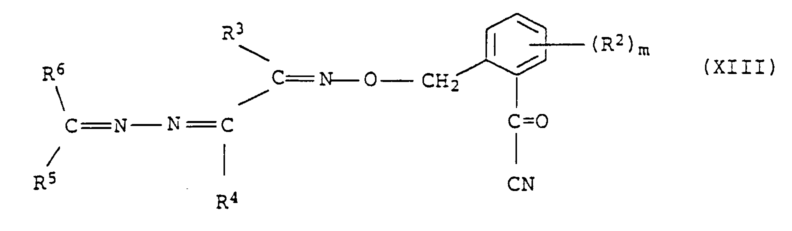 dans laquelle les symboles ont les significations indiquées dans la revendication 1.

15. Composés de formule XIV 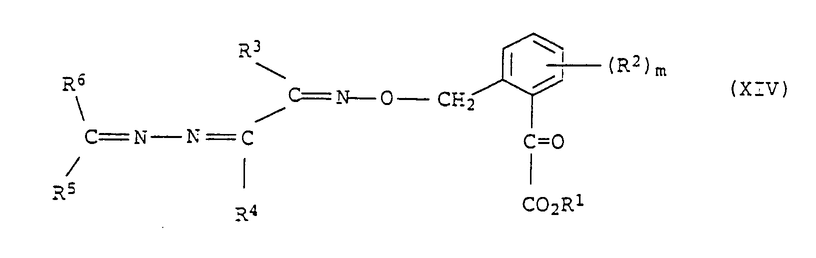 dans laquelle les symboles ont les significations indiquées dans la revendication 1.

16. Composés de formule XV 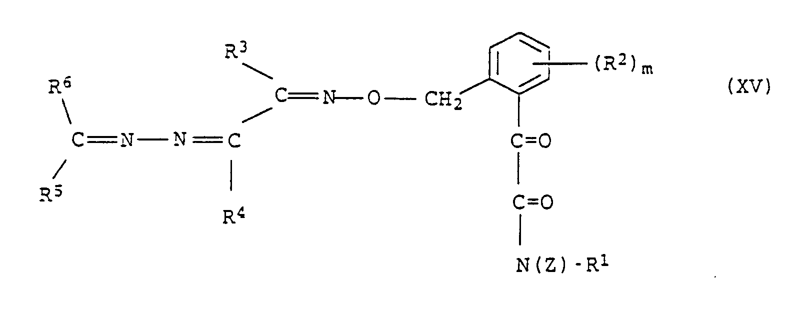 dans laquelle les symboles ont les significations indiquées dans la revendication 1 et Z représente l'hydrogène ou un groupe alkyle en C1-C4.

17. Composés de formule XVI 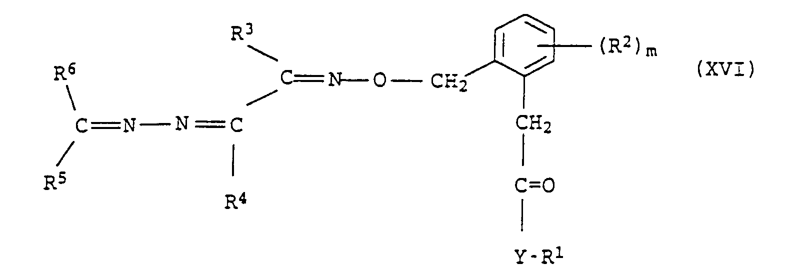 dans laquelle les symboles ont les significations indiquées dans la revendication 1.
